(19) Europäisches Patentamt
European Patent Office
Office européen des brevets

(11) EP 4 281 775 B1

(12) EUROPEAN PATENT SPECIFICATION

(45) Date of publication and mention of the grant of the patent:
**29.04.2026 Bulletin 2026/18**

(21) Application number: **22706944.0**

(22) Date of filing: **21.01.2022**

(51) International Patent Classification (IPC):
***G01N 33/543*** *(2006.01)*

(52) Cooperative Patent Classification (CPC):
**G01N 33/543**

(86) International application number:
**PCT/US2022/013252**

(87) International publication number:
**WO 2022/159663 (28.07.2022 Gazette 2022/30)**

(54) **SYSTEMS AND METHODS FOR BIOMOLECULE PREPARATION**

SYSTEME UND VERFAHREN ZUR BIOMOLEKÜLHERSTELLUNG

SYSTÈMES ET PROCÉDÉS DE PRÉPARATION DE BIOMOLÉCULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.01.2021 US 202163139818 P**

(43) Date of publication of application:
**29.11.2023 Bulletin 2023/48**

(73) Proprietor: **Nautilus Subsidiary, Inc.**
**Seattle WA 98102 (US)**

(72) Inventors:
- **KAPP, Gregory**
**Seattle, Washington 98102 (US)**
- **ROBINSON, Julia**
**Seattle, Washington 98102 (US)**
- **MALLICK, Parag**
**Seattle, Washington 98102 (US)**
- **RINKER, Torri**
**Seattle, Washington 98102 (US)**
- **ANUMALA, Deepthi**
**Seattle, Washington 98102 (US)**

(74) Representative: **HGF**
**HGF Limited**
**4th Floor, 1 City Square**
**Leeds LS1 2ES (GB)**

(56) References cited:
**WO-A1-2017/125442**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 281 775 B1

## Description

This application claims priority to U.S. Provisional Application No. 63/139,818, filed on January 21, 2021.

## BACKGROUND

Polypeptides, including proteins and/or peptides, are commonly found in a broad range of natural, artificial or synthetic environments, including *in vivo* and *ex vivo* environments. Polypeptides may be isolated from these environments for purposes such as research, diagnostics, or commercial usage. Polypeptides may be purified, modified, or otherwise prepared before subsequent usage.

WO 2017/125442 A1 discloses a method for analysing protein variants of a recombinant protein of interest, such as antibodies or Fc-fusion proteins, in a liquid sample of a mammal.

## SUMMARY

The presently claimed invention is as defined in the claims.

The invention provides a method of forming a polypeptide array, comprising:

(a) providing a sample comprising sample polypeptides;

(b) separating the sample polypeptides from the sample in the presence of separation standard polypeptides, wherein the separation standard polypeptides comprise polypeptides derived from a differing source than the sample polypeptides, and wherein each separation standard polypeptide of the separation standard polypeptides comprises a universal and/or unique tag, wherein the universal tag distinguishes the separation standard polypeptides from other polypeptides in the method and wherein the unique tag distinguishes a separation standard polypeptide from all other separation standard polypeptides in the method, wherein the tag is a peptide sequence;

(c) coupling the sample polypeptides to anchoring groups to form sample polypeptide composites, wherein the coupling occurs in the presence of coupling standard polypeptides, wherein the coupling standard polypeptides comprise polypeptides derived from a differing source than the sample polypeptides, and wherein each coupling standard polypeptide of the coupling standard polypeptides comprises a universal and/or unique tag, wherein the universal tag distinguishes the coupling standard polypeptides from other polypeptides in the method and wherein the unique tag distinguishes a coupling standard polypeptide from all other coupling standard polypeptides in the method, wherein the tag is a peptide sequence; and

(d) attaching at least a fraction of the sample polypeptide composites, the separation standard polypeptides and the coupling standard polypeptides to a solid support, whereby each of the sample polypeptide composites, the separation standard polypeptides and the coupling standard polypeptides that is attached to the solid support comprises an address of the polypeptide array.

In one embodiment, the attaching of step (d) occurs in the presence of attachment standard polypeptides, whereby each of the attachment standards that is attached to the solid support comprises an address of the polypeptide array.

In one embodiment, the attachment standard polypeptides comprise attachment standard polypeptide composites, wherein each of the attachment standard polypeptide composites comprises an attachment standard polypeptide coupled to an anchoring group.

In one embodiment, the separation standard polypeptides comprise separation standard polypeptide composites, wherein each of the separation standard polypeptide composites comprises a separation standard polypeptide coupled to an anchoring group.

In one embodiment, the coupling standard polypeptides comprise coupling standard polypeptide composites, wherein each of the coupling standard polypeptide composites comprises a coupling standard polypeptide coupled to an anchoring group.

In one embodiment, the separation standard polypeptides, coupling standard polypeptides or attachment standard polypeptides comprise a synthetic polypeptide, a non-natural amino acid or a synthetically modified amino acid.

In one embodiment, the polypeptide array comprises at least $1 \times 10^4$ different full-length primary protein structures.

In one embodiment, the anchoring groups comprise particles configured to couple to the solid support, optionally wherein the particles comprise a nucleic acid, a polypeptide, a polymer, an inorganic nanoparticle, an organic nanoparticle, a structured nucleic acid particle (SNAP), a nucleic acid origami or a nucleic acid nanoball.

In one embodiment, the method further comprises:

(e) detecting the sample polypeptide composites, the separation standard polypeptides and the coupling standard

polypeptides at addresses of the polypeptide array.

In one embodiment, the method further comprises detecting the attachment standard polypeptides at addresses of the polypeptide array.

In one embodiment, the sample polypeptide composites, the separation standard polypeptides and the coupling standard polypeptides are detected as single polypeptide resolution.

In one embodiment, the method further comprises quantifying the amount of a particular sample polypeptide species in the sample by comparing (i) a number of the addresses attached to a separation standard polypeptide with (ii) the number of the addresses attached to a sample polypeptide composite that comprise the particular sample polypeptide species.

In one embodiment, the method further comprises quantifying the amount of a particular sample polypeptide species in the sample by comparing (i) a number of addresses attached to a coupling standard polypeptide with (ii) a number of addresses attached to a sample polypeptide composite that comprise the particular sample polypeptide species.

In one embodiment, the method further comprises quantifying the amount of a particular sample polypeptide species in the sample by comparing (i) a number of addresses attached to an attachment standard polypeptide with (ii) a number of addresses attached to a sample polypeptide composite that comprise the particular sample polypeptide species.

## BRIEF DESCRIPTION OF THE DRAWINGS

The invention is defined in the claims. Any of the following Figures that do not fall within the scope of the claims do not form part of the claimed invention and are provided for comparative purposes only.

A better understanding of the features and advantages of the present invention will be obtained by reference to the following detailed description that sets forth illustrative embodiments, in which the principles of the invention are utilized, and the accompanying drawings of which:

**FIG. 1A** depicts a sample preparation decision framework, in accordance with some embodiments.

**FIG. 1B** depicts a sample preparation decision framework, in accordance with some embodiments.

**FIG. 1C** depicts a sample preparation decision framework, in accordance with some embodiments.

**FIG. 1D** depicts a sample preparation decision framework, in accordance with some embodiments.

**FIG. 2** depicts a flow chart for methods of separating proteins from a biological sample, in accordance with some embodiments of the present invention.

**FIG. 3** depicts a flow chart for methods of separating proteins from a curated sample, in accordance with some embodiments of the present invention.

**FIG. 4A** illustrates a step of a targeting separation method for isolating a sample polypeptide fraction, in accordance with some embodiments.

**FIG. 4B** illustrates a step of a targeting separation method for isolating a sample polypeptide fraction, in accordance with some embodiments.

**FIG. 4C** illustrates a step of a targeting separation method for isolating a sample polypeptide fraction, in accordance with some embodiments.

**FIG. 4D** illustrates a step of a targeting separation method for isolating a sample polypeptide fraction, in accordance with some embodiments.

**FIG. 4E** illustrates a step of a targeting separation method for isolating a sample polypeptide fraction, in accordance with some embodiments.

**FIG. 4F** illustrates a step of a targeting separation method for isolating a sample polypeptide fraction, in accordance with some embodiments.

**FIG. 4G** illustrates a step of a targeting separation method for isolating a sample polypeptide fraction, in accordance with some embodiments.

**FIG. 5A** shows a step of utilizing a degradable targeting agent for isolating a sample polypeptide fraction, in accordance with some embodiments.

**FIG. 5B** shows a step of utilizing a degradable targeting agent for isolating a sample polypeptide fraction, in accordance with some embodiments.

**FIG. 5C** shows a step of utilizing a degradable targeting agent for isolating a sample polypeptide fraction, in accordance with some embodiments.

**FIG. 5D** shows a step of utilizing a degradable targeting agent for isolating a sample polypeptide fraction, in accordance with some embodiments.

**FIG. 5E** shows a step of utilizing a degradable targeting agent for isolating a sample polypeptide fraction, in accordance with some embodiments.

**FIG. 5F** shows a step of utilizing a degradable targeting agent for isolating a sample polypeptide fraction, in accordance with some embodiments.

**FIG. 5G** shows a step of utilizing a degradable targeting agent for isolating a sample polypeptide fraction, in accordance with some embodiments.

**FIG. 5H** shows a step of utilizing a degradable targeting agent for isolating a sample polypeptide fraction, in accordance with some embodiments.

**FIG. 6A** depicts a step of a method for functionalizing sample polypeptides from a biological source, in accordance with some embodiments.

**FIG. 6B** depicts a step of a method for functionalizing sample polypeptides from a biological source, in accordance with some embodiments.

**FIG. 7A** depicts a step of an alternative method for functionalizing sample polypeptides from a biological source, in accordance with some embodiments.

**FIG. 7B** depicts a step of an alternative method for functionalizing sample polypeptides from a biological source, in accordance with some embodiments.

**FIG. 8A** depicts a step of a method for functionalizing sample polypeptides from a curated source, in accordance with some embodiments.

**FIG. 8B** depicts a step of a method for functionalizing sample polypeptides from a curated source, in accordance with some embodiments.

**FIG. 9A** depicts a method for forming a polypeptide composite coupled to a solid support, in accordance with some embodiments.

**FIG. 9B** depicts a method for forming a polypeptide composite coupled to a solid support, in accordance with some embodiments

**FIG. 10** illustrates the formation of polypeptide composites comprising differing species of anchoring groups, in accordance with some embodiments.

**FIG. 11A** illustrates a method for forming an array of polypeptide composites comprising multiple species of polypeptide composites, in accordance with some embodiments.

**FIG. 11B** illustrates an alternative method for forming an array of polypeptide composites comprising multiple species of polypeptide composites, in accordance with some embodiments

**FIG. 12** shows a flowchart for forming arrays of polypeptide composites coupled to solid supports, in accordance with some embodiments.

**FIG. 13** displays HPLC data for a protein conjugate demonstrating the change in absorption profile between the functionalized protein (upper) and non-functionalized protein (lower).

**FIG. 14A** shows the effect of the presence of surfactants and denaturants on the functionalization of hen-egg white lysozyme.

**FIG. 14B** shows the effect of the presence of surfactants and denaturants on the functionalization of myoglobin.

**FIG. 15A** displays HPLC data for the conjugation of Alexa-Fluor 647-labeled Protein A to a DNA origami anchoring group.

**FIG. 15B** displays negative control HPLC data for the conjugation of Protein A to a DNA origami anchoring group with no reactive handle.

**FIG. 15C** displays HPLC data for the conjugation of Alexa-Fluor 488-labeled maltose-binding protein to a DNA origami anchoring group.

**FIG. 15D** displays HPLC data for the conjugation of TAMRA-labeled ubiquitin to a DNA origami anchoring group.

**FIG. 16** illustrates a schematic view of an anchoring group comprising multiple nucleic acid origami components, in accordance with some embodiments.

**FIGs 17A, 17B,** and **17C** depict top-down views of polypeptide arrays comprising ordered spatial distributions of standard polypeptides, in accordance with some embodiments. **FIG. 17D** depicts a top-down view of a polypeptide array comprising a random spatial distribution of standard polypeptides, in accordance with some embodiments.

## DETAILED DESCRIPTION

The characterization of polypeptide samples derived from natural or engineered systems is often difficult due to the wide or dynamic range of polypeptide abundances found within these systems. For example, a single cell (such as a bacterium or tissue cell) may contain some polypeptides with thousands or even millions of copies and other polypeptides with ten or fewer copies. Consequently, in polypeptide samples with diverse types of polypeptides present, the signal generated by the characterization of the most abundant polypeptides can drown out the signal generated by the least abundant polypeptides. For methods such as mass spectrometry, the analysis of low copy number polypeptides may require collection of enough of the low copy number polypeptide to produce a detectable signal.

Single molecule approaches to polypeptide characterization provide the ability to detect low copy number

polypeptides due to the molecule-by-molecule approach to analysis. Single-molecule approaches to polypeptide characterization, such as affinity binding approaches and Edman degradation sequencing approaches, can provide detectable signal over the full range of polypeptide abundances within a proteome. However, the detection of low copy number polypeptides utilizing a single-molecule approach may be sensitive not only to any detection bias arising from the characterization assay itself, but also detection bias arising due to polypeptide sample preparation methods. For example, if a polypeptide sample preparation method has a known bias against hydrophobic polypeptides, then low copy number hydrophobic polypeptides may be excluded from a sample, thereby limiting or eliminating the characterization of these polypeptides. Moreover, exclusion of polypeptides from a sample can adversely impact the ability to accurately quantify polypeptides in the sample.

The present disclosure describes methods and systems for the preparation of polypeptide samples for any of a variety of purposes, including, for example, polypeptide assays, such as polypeptide characterization assays or polypeptide quantitation assays. The ability to accurately characterize properties of polypeptides or quantify polypeptides from a complex source, such as a biological system, can be compromised due to modification or degradation of sample polypeptides that occur between the time they are removed from the source and the time they are detected. Complex populations of polypeptides, such as those that are processed when performing proteomic scale preparation and detection methods, can be especially compromised by artifacts arising after the polypeptides are removed from biological systems since polypeptides are capable of modifying and degrading each other, often in ways that create chain reactions due to modifications of one polypeptide that increase its ability to modify other polypeptides. The problem of distinguishing native properties of polypeptides from artifacts introduced during preparation or detection of the polypeptides can be alleviated by methods set forth herein that utilize one or more internal standards, such as polypeptide standards, that provide useful information relevant to characterizing the structure or function of one or more sample polypeptides that are processed in a method set forth herein. In some cases, polypeptide standards may provide information about a polypeptide sample preparation processes or about a method used to detect sample polypeptides. The methods and systems described herein may be useful for generating one or more polypeptide fractions with proteome-scale coverage or targeted subfractions of polypeptides from within a sample comprising a polypeptide fraction.

Polypeptide standards can be particularly useful for at least four reasons. First, polypeptide standards can include structural features that represent a range of polypeptide structures relative to a sample, a proteome, or a biological system. Polypeptide standards may comprise determined or known amino acid sequences that give rise to unstructured strands or ordered strands, such as alpha helices, beta-pleated sheets, or even larger tertiary structures or motifs. Polypeptide standards may comprise structural features that are either reactive to a particular modification or inert to the modification. For example, a polypeptide standard can include a recognition sequence that is known or expected to be cleaved by a particular protease. The polypeptide standard can be added to a polypeptide sample and the amount of polypeptide standard that is lost or modified while processing the sample can be used to account for absence of sample polypeptides having the protease recognition sequence and that were expected to be present in the sample. Other polypeptide standards can include recognition sequences for other post-translational modifications that may be made to sample polypeptides while being processed in a method set forth herein, and presence or absence of the modifications in sample polypeptides can be evaluated relative to the extent of modifications made to the standards during processing.

Second, polypeptide standards can include chemical features that encompass a range of possible chemical properties, such as hydrophobicity, hydrophilicity, polarity, surface charge density, and reactivity. For example, polypeptide standards may comprise sequences with consecutive alike amino acids (e.g., RRRRR for a region of high positive-charge density) or consecutive similar amino acids (e.g., IMFIMF for a region of hydrophobicity). A polypeptide standard may provide information on biases in polypeptide preparation processes due to one or more chemical properties (e.g., hydrophobicity, hydrophilicity, charge density, etc.). Polypeptide standards may be composed with polypeptides of varying properties along one or more continua of behavior (e.g., hydrophobic to hydrophilic; polar to nonpolar; positively-charged to neutral to negatively-charged, etc.), thereby providing a measurable standard for biases based upon chemical properties during sample preparation processes.

Third, polypeptide standards can be particularly useful because they can include unique peptide sequences that provide a tag indicating an associated structure or function. For example, a protease sensitive polypeptide can have a protease recognition sequence and a tag sequence. Detecting presence or absence of the tag sequence in a sample having the polypeptide sequence can provide a convenient means to determine whether or not sample polypeptides that were expected to be in the sample were lost due to proteolysis during sample processing. Many standard polypeptides that are used for a particular sample will have tag sequences that are exogenous to the source from which the sample was derived. This allows the standard polypeptides to be readily distinguished from sample polypeptides. However, some sample sources may include one or more endogenous polypeptides that can be usefully exploited as standard polypeptide(s). For example, so called 'housekeeping proteins' or other high abundance polypeptides from a biological source, can be quantified after processing a sample from the source in order to normalize the yield of lower abundance polypeptides from the sample.

Fourth, polypeptide standards can be particularly useful because they can be detected using the same

techniques used to detect sample polypeptides. For example, polypeptide standards can be attached to addresses on an array that includes addresses attached to sample polypeptides, thereby allowing multiplex detection of standard polypeptides and sample polypeptides together. Moreover, polypeptide standards can be provided to arrays in a segregated or directed fashion, or provided a unique or orthogonal detection label, making them readily detectable during polypeptide assays. Similarly, polypeptide standards can be subjected to mass spectrometry detection schemes used for sample polypeptides with minimal modification to the detection scheme that would have been used for sample polypeptides alone. Polypeptide standards can be subjected to mass spectrometry detection after initial analysis by a multiplex detection method.

Described herein are improved workflows for the preparation of polypeptide samples. Given the broad range of sources from which polypeptides may be derived, including biological systems (e.g., an organelle, a cell, a tissue, a microbiome) and curated and/or non-biological systems (e.g., industrial effluent, geological samples), the described workflows offer the skilled person an adaptable framework for selecting a sample preparation method that best suits the polypeptide sample of interest and/or the polypeptide assay to be used.

In an aspect, described herein is a method for forming a polypeptide array comprising providing a polypeptide mixture comprising sample polypeptides and separation standard polypeptides, then separating a plurality of sample polypeptides and a plurality of separation standard polypeptides from the polypeptide mixture to form a separation mixture. The separation mixture may then be combined with coupling standard polypeptides to form a coupling mixture. The coupling mixture may then be coupled to a plurality of anchoring groups, creating polypeptide-anchoring group complexes. Before or after the coupling of the coupling mixture to the anchoring groups, the anchoring groups may be coupled to a solid support at addresses on a surface of the solid support. Consequently, a polypeptide array is formed on the surface of the solid support, comprising sample polypeptides, separation standard polypeptides, and coupling standard polypeptides.

In alternative configurations of the above described method, the coupling standard polypeptides can be combined with the polypeptide sample before the separation mixture is formed. In yet another alternative configuration of the method, the separation standard polypeptides can be omitted such that the polypeptide array does not include the separation standard polypeptides. Similarly, the coupling standard polypeptides can be omitted such that the polypeptide array does not include the coupling standard polypeptides.

In another aspect, described herein is a composition comprising a polypeptide array coupled to a solid support. The polypeptide array comprises a plurality of polypeptides, including sample polypeptides, separation standard polypeptides, and coupling standard polypeptides, with each polypeptide in the plurality of polypeptides coupled to an anchoring group. Each anchoring group is coupled to a surface of the solid support to form the polypeptide array. The plurality of sample polypeptides may be characterized as having been coupled to the array with an overall efficiency relative to the amount of sample polypeptides originally present. The overall efficiency may be calculated as a function of a separation efficiency that is a function of the observed plurality of separation standard polypeptides, and/or a coupling efficiency that is a function of the observed plurality of coupling standard polypeptides. Again, the separation standard polypeptides or coupling standard polypeptides can optionally be omitted from the polypeptide array.

The above described polypeptide array and method for forming a polypeptide array are exemplified with respect to use of separation standard polypeptides and coupling standard polypeptides. The separation standard polypeptides can be useful for characterizing a property of the separation method, characterizing a property of the sample polypeptides and/or quantifying the sample polypeptides. The coupling standard polypeptides can be useful for characterizing a property of the coupling method, characterizing a property of the sample polypeptide-anchoring group complexes and/or quantifying the sample polypeptide-anchoring group complexes. Those skilled in the art will recognize from the teachings of the present disclosure that other types of standard polypeptides can be useful. Standard polypeptides can be introduced at any of a variety of stages of a polypeptide preparation method including, for example, stages set forth herein or known in the art. The standard polypeptides can be useful for characterizing a property of the stage, characterizing a property of sample polypeptides obtained from the stage and/or quantifying sample polypeptides obtained from the stage. For example, a variety of methods set forth herein include a stage of attaching sample polypeptides to a solid support. Attachment standard polypeptides can be combined with a sample, or fraction thereof, prior to or during the attachment stage and the attachment standard polypeptides can be useful for characterizing a property of the attachment stage, characterizing a property of sample polypeptides obtained from the attachment stage and/or quantifying sample polypeptides obtained from the attachment stage.

It will be readily recognized that the compositions, systems, and methods set forth herein may be readily extended from polypeptide arrays to arrays of other single analytes, such as nucleic acids, polysaccharides, cells, and metabolites.

## Definitions

As used herein, the term "sample" refers to a collected substance or material that comprises or is suspected to comprise one or more analytes of interest such as polypeptides. A sample may be modified for purposes such as storage or

stability. A sample may have undergone one or more processes that separate or remove unwanted fractions or impurities from the analyte(s) of interest. For example, a fraction is a type of sample. Alternatively, a sample may not have undergone any processes that separates or removes any unwanted fractions or impurities from the analyte(s) of interest. For example, a fluid, tissue or cell is a type of sample, A sample may include biological and/or non-biological components. As used herein, the terms "biological sample" or "biological source" refer to a sample that is derived from a predominantly biological system or organism, such as one or more viral particles, cells (e.g. individualized cells), organelles (e.g. individualized organelles), tissues, bodily fluids, bone, cartilage, and exoskeleton. A biological sample may comprise a majority of biological material on a mass basis, excluding the weight of fluid within the sample. As used herein, the term "curated sample" or "curated source" refer to a sample that is derived from a predominantly non-biological system, such as processed or manufactured materials, rocks, minerals, metals, ceramics, and polymers. Samples obtained from materials that are substantially modified by processing or manufacturing, including materials produced from biological materials (e.g., cotton fabrics) may be classified as curated materials. Curated samples may also include materials associated with industrial processes (e.g., feeds, reagents, mixtures, products, waste, effluents) where biological materials can exist but are expcted to constitute a minority of available sample material on a mass basis. For example, a purified effluent from a biochemical reactor may be a curated sample that is checked for a presence or absence of biological contamination.

As used herein, the term "fraction" refers to a purified or separated portion of a sample that comprises or is suspected to comprise some, most or all of a particular sample component. For example, a polypeptide fraction may refer to a purified or separated portion of a sample that comprises or is suspected to comprise mostly polypeptides by weight. A fraction may also be characterized with respect to the absence of some, most or all of a particular sample component. For example, a non-polypeptide fraction may refer to a purified or separation portion of a sample that substantially lacks polypeptides or that comprises or is suspected to comprise mostly non-polypeptide substances by weight. Different types of polypeptides can optionally be present in different fractions. For example, a membrane fraction may comprise or be suspected to comprise mostly membrane proteins, and can be substantially devoid of soluble proteins. In another example, a soluble fraction may comprise or be suspected to comprise mostly soluble proteins, and can be substantially devoid of membrane proteins. A sample may undergo one or more processes, such as sample preparation processes set forth herein or known in the art, that cause the sample to be divided into two or more fractions.

As used herein, the term "polypeptide" refers to a molecule comprising two or more amino acids joined by a peptide bond. A polypeptide may refer to a protein or a peptide. A polypeptide may refer to a naturally-occurring molecule, or an artificial or synthetic molecule. A polypeptide may include one or more non-natural, modified amino acids, or non-amino acid linkers. A polypeptide may be modified naturally or synthetically, such as by post-translational modifications.

As used herein, the term "sample polypeptide" refers a polypeptide that is contained within a sample, or is obtained by separation from a sample.

As used herein, the term "separation standard polypeptide" refers to a polypeptide that is used for the purpose of monitoring the performance of a polypeptide separation process. A separation standard polypeptide may be combined with a sample, or fraction thereof, that contains one or more polypeptides that are to be separated from other sample components. A separation standard polypeptide may be derived from the same source as the sample polypeptide (i.e. an endogenous separation standard polypeptide) or from a source that is different from the source of the sample polypeptide (i.e. an exogenous separation standard polypeptide). A separation standard polypeptide may be composed, in part or in whole, of non-natural or abiotic amino acids (D-amino acids), or amino acids joined by a non-amino acid chemical linker.

As used herein, the term "coupling standard polypeptide" refers to a polypeptide that is used for the purpose of monitoring the performance of a polypeptide coupling process. A coupling standard polypeptide may be combined with a sample, or fraction thereof, that contains one or more polypeptides that participate in a coupling process. A coupling standard polypeptide may be derived from the same source as the sample polypeptide (i.e. an endogenous coupling standard polypeptide) or from a source that is different from the source of the sample polypeptide (i.e. an exogenous coupling standard polypeptide).

As used herein, the term "protein" may refer to any single- or multi-chain polypeptide molecule. A protein may have a known or unknown biological function or activity. Proteins can include natural, synthetic, modified, and degraded polypeptides.

As used herein, the term "protein complex" can refer to two or more proteins that are associated or co-localized by covalent or non-covalent bonding between the proteins.

As used herein, the term "peptide" may refer to any short, single polypeptide chain. A peptide may be no more than about 100, 95, 90, 85, 80, 75, 70, 65, 60, 55, 50, 45, 40, 35, 30, 25, 20, 15, 10, 5, or less than about 5 amino acids in length. A peptide may have a known or unknown biological function or activity. Peptides can include natural, synthetic, modified, or degraded proteins or peptides.

As used herein, the term "functionalized" refers to any material or substance that has been modified to include a functional group. A functionalized material or substance may be naturally or synthetically functionalized. For example, a polypeptide can be naturally functionalized with a phosphate, oligosaccharide (*e.g.* glycosyl, glycosylphosphatidylinositol or phosphoglycosyl), nitrosyl, methyl, acetyl, lipid (*e.g.* glycosyl phosphatidylinositol, myristoyl or prenyl), ubiquitin or other

naturally occurring post-translational modification. A functionalized material or substance may be functionalized for any given purpose, including altering chemical properties (e.g., altering hydrophobicity or changing surface charge density) or altering reactivity (e.g. capable of reacting with a moiety or reagent to form a covalent bond to the moiety or reagent).

As used herein, the term "reactive handle" refers to a pendant, reactive functional group (e.g., activated ester, azide) that is attached to a material or substance. A reactive handle may be covalently or non-covalently attached to a material or substance.

As used herein, the term "anchoring group" refers to a molecule or particle that serves as an intermediary attaching a protein or peptide to a surface (e.g., a solid support or a microbead). An anchoring group may be covalently or non-covalently attached to a surface and/or a polypeptide. An anchoring group may be a biomolecule, polymer, particle, nanoparticle, or any other entity that is capable of attaching to a surface or polypeptide. In some cases, an anchoring group may be a structured nucleic acid particle.

As used herein, the term "structured nucleic acid particle" (or "SNAP") refers to a single- or multi-chain polynucleotide molecule having a compacted three-dimensional structure. The compacted three-dimensional structure can optionally have a characteristic tertiary structure. For example, a SNAP can be configured to have an increased number of interactions between regions of a polynucleotide strand, less distance between the regions, increased number of bends in the strand, and/or more acute bends in the strand, as compared to the same nucleic acid molecule in a random coil or other non-structured state. Alternatively or additionally, the compacted three-dimensional structure can optionally have a characteristic quaternary structure. For example, a SNAP can be configured to have an increased number of interactions between polynucleotide strands or less distance between the strands, as compared to the same nucleic acid molecule in a random coil or other non-structured state. In some configurations, the secondary structure (i.e. the helical twist or direction of the polynucleotide strand) of a SNAP can be configured to be more dense than the same nucleic acid molecule in a random coil or other non-structured state. SNAPs may include deoxyribonucleic acid (DNA), ribonucleic acid (RNA), peptide nucleic acid (PNA), and combinations thereof. SNAPs may have naturally-arising or engineered secondary, tertiary, or quaternary structures. Exemplary SNAPs may include nucleic acid nanoballs (e.g. DNA nanoballs), nucleic acid nanotubes (e.g. DNA nanotubes), and nucleic acid origami (e.g. DNA origami). A SNAP may be functionalized to include one or more reactive handles or other moieties.

As used herein, the term "polypeptide composite" refers to a molecule that is formed by the coupling of a polypeptide to one or more anchoring groups. The coupling between molecules in a polypeptide composite may be covalent or non-covalent. For example, a polypeptide composite may be covalently linked by a covalent bond between a reactive handle on a polypeptide with a reactive handle on an anchoring group. In another example, a polypeptide composite may be non-covalently linked by an interaction such as hybridization between complementary oligonucleotides or a receptor-ligand linkage such as a streptavidin-biotin linkage.

As used herein, the term "standard," when used in reference to a plurality of analytes (e.g., polypeptides), refers to a composition of one or more molecules that is utilized for characterizing the quality, quantity or other characteristic of a sample or component thereof. A standard may include one or more polypeptides. A standard can be endogenous to a source from which a sample or sample component, such as a sample polypeptide is derived. For example, the endogenous standard can be a polypeptide standard that is derived from the same source from which the sample or sample polypeptide is derived. Accordingly, an endogenous standard polypeptide will have a peptide sequence that is native to, or otherwise found in, the source from which a particular sample or sample polypeptide is derived. Alternatively, a standard can be exogenous to a source from which a sample or sample component, such as a sample polypeptide, is derived. For example, the exogenous standard can be a polypeptide standard that is derived from a source that is different from the source from which the sample or sample polypeptide is derived. Accordingly, an exogenous standard polypeptide will have a peptide sequence that is not found naturally in the source from which a particular sample or sample polypeptide is derived. A standard may include one or more types of polypeptides in known amounts. A standard may further comprise other non-polypeptide components, including small molecule components, that permit observation and/or quantitation of a sample before or after processing of the sample. As used herein, the term "separation standard" may refer to a standard that is utilized to observe the effects of a separation process on a sample, sample fraction (e.g. a polypeptide fraction) or sample component (e.g. a sample polypeptide). As used herein, the term "coupling standard" may refer to a standard that is utilized to observe the effects of a coupling process on a sample, sample fraction (e.g. a polypeptide fraction) or component of a sample (e.g. a sample polypeptide).

As used herein, the term "species" refers to a molecule with a unique, distinguishable chemical structure. As used herein, the term "polypeptide species" refers to a polypeptide with a unique, distinguishable primary structure. Two polypeptides are of the same species if they possess the same primary structure. Polypeptide variants and isoforms are different species from each other. For example, members of an "anchoring group species" have a unique, distinguishable structure that is common to the members. Anchoring group species may be identified, for example, by common shape and/or conformation, number of coupling sites, or type of coupling sites.

As used herein, the term "polypeptide diversity" refers to the number of different polypeptide species present within a plurality of polypeptides. The different species can differ with respect to a specified characteristic such as peptide

sequence (i.e. polypeptide sequence diversity), length (i.e. polypeptide length diversity), post-translational modification (e.g. polypeptide phosphorylation diversity) or other characteristics set forth herein or known in the art. The number of different polypeptide species present within a plurality of polypeptides can optionally be identified relative to a reference basis, such as the number of polypeptide species in a proteome, within a cell, within a sample, or within a polypeptide fraction. For example, the polypeptide diversity within a polypeptide fraction may refer to the number of species of polypeptides present within the polypeptide fraction relative to the sample from which the polypeptide fraction was derived. The polypeptide diversity may be characterized on a percentage or fractional basis, e.g., 0.1% or 0.001 of all polypeptide species.

As used herein, the term "click reaction" or "bioorthogonal reaction" refers to single-step, thermodynamically-favorable conjugation reaction utilizing biocompatible reagents. A click reaction may utilize no toxic or biologically incompatible reagents (e.g., acids, bases, heavy metals) or generate no toxic or biologically incompatible byproducts. A click reaction may utilize an aqueous solvent or buffer (e.g., phosphate buffer solution, Tris buffer, saline buffer, MOPS, etc.). A click reaction may be thermodynamically favorable if it has a negative Gibbs free energy of reaction, for example a Gibbs free energy of reaction of less than about - 5 kiloJoules/mole (kJ/mol), -10 kJ/mol, -25 kJ/mol, -50 kJ/mol, -100 kJ/mol, - 200 kJ/mol, -300 kJ/mol, -400 kJ/mol, or less than -500 kJ/mol. Exemplary bioorthogonal and click reactions are described in detail in WO 2019/195633A1.

. Exemplary click reactions may include metal-catalyzed azide-alkyne cycloaddition, strain-promoted azide-alkyne cycloaddition, strain-promoted azide-nitrone cycloaddition, strained alkene reactions, thiol-ene reaction, Diels-Alder reaction, inverse electron demand Diels-Alder reaction, [3+2] cycloaddition, [4+1] cycloaddition, nucleophilic substitution, dihydroxylation, thiol-yne reaction, photoclick, nitrone dipole cycloaddition, norbornene cycloaddition, oxanobornadiene cycloaddition, tetrazine ligation, and tetrazole photoclick reactions. Exemplary functional groups or reactive handles utilized to perform click reactions may include alkenes, alkynes, azides, epoxides, amines, thiols, nitrones, isonitriles, isocyanides, aziridines, activated esters, and tetrazines.

As used herein, the term "array" refers to a population of molecules that is attached to one or more solid supports such that the molecules at one address can be distinguished from molecules at other addresses. An array can include different molecules that are each located at different addresses on a solid support. Alternatively, an array can include separate solid supports each functioning as an address that bears a different molecule, wherein the different molecules can be identified according to the locations of the solid supports on a surface to which the solid supports are attached, or according to the locations of the solid supports in a liquid such as a fluid stream. The molecules of the array can be, for example, nucleic acids such as SNAPs, polypeptides, proteins, peptides, oligopeptides, enzymes, ligands, or receptors such as antibodies, functional fragments of antibodies or aptamers. The addresses of an array can optionally be optically observable and, in some configurations, adjacent addresses can be optically distinguishable when detected using a method or apparatus set forth herein.

As used herein, the term "address," when used in reference to an array, means a location in an array where a particular molecule is present. An address can contain only a single molecule, or it can contain a population of several molecules of the same species (i.e. an ensemble of the molecules). Alternatively, an address can include a population of molecules that are different species. Addresses of an array are typically discrete. The discrete addresses can be contiguous, or they can have interstitial spaces between each other. An array useful herein can have, for example, addresses that are separated by less than 100 microns, 50 microns, 10 microns, 5 microns, 1 micron, or 0.5 micron. Alternatively or additionally, an array can have addresses that are separated by at least 0.5 micron, 1 micron, 5 microns, 10 microns, 50 microns or 100 microns. The addresses can each have an area of less than 1 square millimeter, 500 square microns, 100 square microns, 25 square microns, 1 square micron or less.

As used herein, the term "solid support" refers to a rigid substrate that is insoluble in aqueous liquid. The substrate can be non-porous or porous. The substrate can optionally be capable of taking up a liquid (e.g. due to porosity) but will typically be sufficiently rigid that the substrate does not swell substantially when taking up the liquid and does not contract substantially when the liquid is removed by drying. A nonporous solid support is generally impermeable to liquids or gases. Exemplary solid supports include, but are not limited to, glass and modified or functionalized glass, plastics (including acrylics, polystyrene and copolymers of styrene and other materials, polypropylene, polyethylene, polybutylene, polyurethanes, Teflon™, cyclic olefins, polyimides etc.), nylon, ceramics, resins, Zeonor™, silica or silica-based materials including silicon and modified silicon, carbon, metals, inorganic glasses, optical fiber bundles, and polymers.

As used herein, the terms "group" and "moiety" are intended to be synonymous when used in reference to the structure of a molecule. The terms refer to a component or part of the molecule. The terms do not necessarily denote the relative size of the component or part compared to the molecule, unless indicated otherwise. The terms do not necessarily denote the relative size of the component or part compared to any other component or part of the molecule, unless indicated otherwise. A group or moiety can contain one or more atom.

As used herein, the term "sample pretreatment" refers to an *in vitro* process that alters a sample or a fraction of a sample, or alters a fraction of polypeptides within a sample prior to a sample preparation process (e.g., separation, functionalization, conjugation, etc.). Sample pretreatment processes may include processes that improve the chemical

and/or physical properties of a sample or polypeptides within a sample for a subsequent sample preparation process. Sample pretreatment processes may include processes that alter one or more polypeptides within a sample (e.g., drug or drug analog treatments; enzymatic treatments).

As used herein, the term "epitope" refers to a molecule or part of a molecule, which is recognized by or binds specifically to an affinity reagent. Epitopes may include amino acid sequences that are sequentially adjacent in the primary structure of a protein or amino acids that are structurally adjacent in the secondary, tertiary or quaternary structure of a protein. An epitope can optionally be recognized by or bound to an antibody. However, an epitope need not necessarily be recognized by any antibody, for example, instead being recognized by an aptamer, miniprotein or other probe. An epitope can optionally bind an antibody to elicit an immune response. However, an epitope need not necessarily participate in, nor be capable of, eliciting an immune response.

As used herein, the term "affinity reagent" refers to a molecule or other substance that is capable of specifically or reproducibly binding to an analyte (e.g. protein, peptide or unique identifier label) or moiety (e.g. post-translational modification of a protein). An affinity reagent can be larger than, smaller than or the same size as the analyte. An affinity reagent may form a reversible or irreversible bond with an analyte. An affinity reagent may bind with an analyte in a covalent or non-covalent manner. Affinity reagents may include reactive affinity reagents, catalytic reaffinity agents (e.g., kinases, proteases, etc.) or non-reactive affinity reagents (e.g., antibodies or fragments thereof). An affinity reagent can be non-reactive and non-catalytic, thereby not permanently altering the chemical structure of an analyte to which it binds. Affinity reagents that can be particularly useful for binding to proteins include, but are not limited to, antibodies or functional fragments thereof (e.g., Fab' fragments, F(ab')2 fragments, single-chain variable fragments (scFv), di-scFv, tri-scFv, or microantibodies), aptamers, affibodies, affilins, affimers, affitins, alphabodies, anticalins, avimers, miniproteins, DARPins, monobodies, nanoCLAMPs, lectins, or functional fragments thereof. The term "affinity agent" is used synonymously with the term "affinity reagent" herein.

As used herein, the term **"single analyte"** refers to an analyte (*e.g*. protein) that is individually manipulated or distinguished from other analytes. A single analyte can be a single molecule (*e.g.* single protein), a single complex of two or more molecules (*e.g.* a single protein attached to a structured nucleic acid particle or a single protein attached to an affinity agent), a single particle, or the like. A single analyte may be resolved from other analytes based on, for example, spatial or temporal separation from the other analytes. Accordingly, an analyte can be detected at "single-analyte resolution" which is the detection of, or ability to detect, the analyte on an individual basis, for example, as distinguished from its nearest neighbor in an array. Reference herein to a 'single analyte' in the context of a composition, apparatus or method does not necessarily exclude application of the composition, apparatus or method to multiple single analytes that are manipulated or distinguished individually, unless indicated to the contrary.

The term "comprising" is intended herein to be open-ended, including not only the recited elements, but further encompassing any additional elements.

As used herein, the term "each," when used in reference to a collection of items, is intended to identify an individual item in the collection but does not necessarily refer to every item in the collection. Exceptions can occur if explicit disclosure or context clearly dictates otherwise.

### Polypeptide Sample Preparation Workflows

Described herein are methods and system for preparing polypeptide fractions for any of a variety of purposes, including, for example, polypeptide assays such as characterizations assays and sequencing assays. The described polypeptide sample preparation workflows may involve one or more fractionation or targeting processes for the isolation of polypeptide fractions. Fractionation processes may include methods that separate samples into two or more fractions (e.g., a polypeptide fraction and a non-polypeptide fraction; soluble polypeptides and membrane polypeptides). Targeting processes may include methods that selectively separate or isolate desired polypeptides from a sample with a broader composition (e.g., capture or pull-down assays). The selection of a sample preparation workflow may be driven, in whole or in part, by the type of polypeptide fraction to be prepared and the intended use of the polypeptide fraction.

The selection of a polypeptide sample preparation workflow may be influenced by a variety of aligned or competing factors. Factors influencing the choice of sample preparation workflow may include sample type, sample size, sample stability, and mode of use. In some cases, a predominant factor or two or more synergizing factors may influence the selection of a particular workflow. In other cases, two or more competing or offsetting factors may make more than one sample preparation workflow appropriate for generation of a polypeptide fraction.

The selection of a polypeptide sample preparation workflow may also be influenced by the final form in which a polypeptide sample may be produced. For example, polypeptide fractions produced for a mass spectrometry type analysis may be produced in a solution or loosely adhered to a solid support. In contrast, polypeptide fractions produced for an affinity binding assay may be produced in a more ordered array on a solid support. Consequently, in some cases, it may be advantageous to prepare polypeptide fractions in the presence of a solid support or other medium upon which the polypeptide is to be utilized. Certain separation methods, such as targeting, may be configured for *in situ* usage (e.g., on-chip devices), while other methods, such as chromatographic fractionation, may be most easily implemented in bench-scale or larger instruments.

**FIGs. 1A - 1D** depict examples of how various factors may be weighed to determine a possible sample preparation workflow. **FIG. 1A** shows an exemplary workflow decision framework when considering sample types. First, it may be determined if a sample is heterogeneous or homogeneous. A heterogeneous sample may comprise multiple phases (e.g., solids, membranes, aqueous liquids, and/or hydrophobic liquids) or multiple types of components (e.g., cells and free proteins). A homogeneous sample may comprise a uniform solution or mixture (e.g., a soluble fraction from a cellular lysate; a wastewater sample). If a sample is heterogeneous, it may next be desired to retain heterogeneity, or to be homogenized into a homogeneous sample. If the sample remains heterogeneous, it may be subjected to a fractionation or targeting process. If the heterogeneous sample is to be divided into multiple polypeptide fractions, appropriate fractionation or targeting processes may be used. In some cases, a single polypeptide fraction may be isolated by a targeting process. If the sample type is homogeneous, it may also undergo an appropriate fractionation or targeting process. If the homogeneous sample is to be divided into multiple polypeptide fractions, appropriate fractionation or targeting processes may be used. In some cases, a single polypeptide fraction may be isolated by a targeting process.

**FIG. 1B** shows a workflow decision framework when considering the size or mass scale of a sample. If the sample is large or has a bulk application, a fractionation method may be chosen to isolate one or more polypeptide fractions. If the polypeptide fractions are to be utilized in a polypeptide assay, the fractionation method may occur on external instrumentation rather than an assay platform, such as a flow cell, chip, or cartridge. Alternatively, if the sample is small or will be used in part, a targeting method may be chosen to isolate one or more polypeptide fractions. If the polypeptide fractions are to be utilized in a polypeptide assay, the targeting method may occur directly on or within an assay platform, such as a flow cell, chip, or cartridge.

**FIG. 1C** shows a workflow decision framework when considering the time scale or stability of a sample. If a sample is unstable or contains target components that are unstable, a targeting method may be appropriate for rapidly isolating the target components. For example, target polypeptide(s) can be separated from protease enzymes or other enzymes that would post-translationally modify the targeted polypeptide(s). In some cases, a sample may be subjected to a series of two or more sequential targeting or fractionation steps. For example, an unstable polypeptide fraction that has been separated from a sample may be further subjected to additional targeting or fractionation depending upon the utility and stability of a subset of desired polypeptides in the sample. If the polypeptide fractions are to be utilized in a polypeptide assay, the targeting method may occur directly on or within an assay platform, such as a flow cell, chip, or cartridge. If a sample is more stable, it may be prepared in one or more fractions by a slower or more complex fractionation method. If the polypeptide fractions are to be utilized in a polypeptide assay, the fractionation method may occur on external instrumentation rather than an assay platform, such as a flow cell, chip, or cartridge.

**FIG. 1D** shows a workflow decision framework when a polypeptide sample is to be analyzed in a polypeptide assay. The workflow chosen may depend upon whether the intended analysis is qualitative or quantitative. For qualitative analysis, the chosen workflow may further be selected based upon whether the analysis is for the full polypeptide content of the sample, or a partial polypeptide fraction of the sample. For full sample analysis, fractionation methods may be preferred for larger quantities of polypeptides. For partial sample analysis, targeting or fractionation methods may be utilized depending upon what quantity of polypeptides are to be analyzed. For quantitative analysis, the chosen workflow may also be selected based upon whether the analysis is for the full polypeptide content of the sample, or a partial polypeptide fraction of the sample. For full sample analysis, fractionation methods may be preferred for large quantities of polypeptides. In some cases, a sample may undergo multiple types of fractionations or repeated cycles of a fractionation method to provide separation and collection of a majority, or even substantially all polypeptides in the sample. For partial sample analysis, targeting methods may be preferred for smaller polypeptide fractions. In some cases, a sample may undergo multiple types of targeting or repeated cycles of a targeting method to provide separation and collection of a majority, or even substantially all targeted polypeptides in the sample.

A sample may be prepared to form a polypeptide array for any of a variety of purposes, such as a polypeptide assay. In some cases, a method of forming a polypeptide array may comprise one or more of the following steps: 1) providing a sample comprising a plurality of sample polypeptides; 2) separating the plurality of sample polypeptides from the sample in the presence of a plurality of separation standard polypeptides; 3) coupling the plurality of sample polypeptides to a plurality of anchoring groups to form a plurality of sample polypeptide composites, where the coupling occurs in the presence of a plurality of coupling standard polypeptides; and 4) attaching at least a fraction of the sample polypeptide composites of the plurality of sample polypeptide composites to a solid support, whereby each of the sample polypeptide composites that is attached to the solid support comprises an address of a polypeptide array. Optionally, step 4) can further include attaching at least a fraction of the separation standard polypeptides and/or the coupling standard polypeptides to the solid support, whereby each of the separation standard polypeptides and/or the coupling standard polypeptides that is attached to the solid support comprises an address of the polypeptide array. In a further option, step 4) can be carried out in the presence of a plurality of attachment standard polypeptides. The attachment standard polypeptides can be attached to addresses of the polypeptide array.

One or more of the above steps can be omitted from the sample preparation method. For example, a method of forming a polypeptide array may comprise the steps of: 1) providing a sample comprising a plurality of sample polypeptides; 2) coupling the plurality of sample polypeptides to a plurality of anchoring groups to form a plurality of sample polypeptide composites, where the coupling occurs in the presence of a plurality of coupling standard polypeptides; and 3) attaching at least a fraction of the sample polypeptide composites of the plurality of sample polypeptide composites to a solid support, whereby each of the sample polypeptide composites that is attached to the solid support comprises an address of a polypeptide array. Optionally, step 3) can further include attaching at least a fraction of the coupling standard polypeptides to the solid support, whereby each of the coupling standard polypeptides that is attached to the solid support comprises an address of the polypeptide array. In a further option, step 3) can be carried out in the presence of a plurality of attachment standard polypeptides. The attachment standard polypeptides can be attached to addresses of the polypeptide array.

The steps set forth above for a method of forming a polypeptide array may be carried out in the order exemplified above or in an alternative order. For example, a method of forming a polypeptide array may comprise the steps of: 1) providing a sample comprising a plurality of sample polypeptides; 2) coupling the plurality of sample polypeptides to a plurality of anchoring groups to form a plurality of sample polypeptide composites, where the coupling occurs in the presence of a plurality of coupling standard polypeptides; 3) separating the plurality of sample polypeptide composites from the sample; and 4) attaching at least a fraction of the sample polypeptide composites of the plurality of sample polypeptide composites to a solid support, whereby each of the sample polypeptide composites that is attached to the solid support comprises an address of a polypeptide array. Step 3) can optionally be carried out in the presence of a plurality of separation standard polypeptides. As a further option, step 4) can further include attaching at least a fraction of the separation standard polypeptides and/or the coupling standard polypeptides to the solid support, whereby each of the separation standard polypeptides and/or the coupling standard polypeptides that is attached to the solid support comprises an address of the polypeptide array.

Optionally, the plurality of sample polypeptides used in the sample preparation methods set forth above or elsewhere herein may be functionalized with a group that facilitates the coupling of the anchoring group to the sample polypeptide. **FIG. 12** illustrates various configurations of a method for forming a polypeptide array. In one configuration, a sample **1200** comprising a plurality of sample polypeptides is provided for the polypeptide array method. In some cases, the plurality of sample polypeptides is separated from the sample by a sample polypeptide separation process **1210.** Before or after the sample polypeptide separation process **1210,** the plurality of sample polypeptides may be functionalized in a polypeptide functionalization process **1250.** After the sample polypeptide separation process **1210** and/or the polypeptide functionalization process **1250,** the plurality of sample polypeptides may be coupled to a plurality of anchoring groups **1220** and attached to a solid support **1230** to form a polypeptide array **1240** comprising a plurality of sample polypeptides attached to a solid support by a plurality of anchoring groups. In another configuration, the sample **1200** may be coupled to a plurality of anchoring groups **1220.** Before the coupling of the sample **1200** to the plurality of anchoring groups **1220,** the sample **1200** may be functionalized in a polypeptide functionalization process **1250.** After the coupling of the sample **1200** to the plurality of anchoring groups **1220** and/or the polypeptide functionalization process **1250,** the plurality of sample polypeptides may be separated from the sample **1200** in a sample polypeptide separation process **1210.** After the sample polypeptide separation process **1210,** the plurality of polypeptides may be attached to a solid support **1230** to form a polypeptide array **1240** comprising a plurality of sample polypeptides attached to a solid support by a plurality of anchoring groups. In some cases, the coupling of the plurality of sample polypeptides to the anchoring groups **1220** and the binding of the plurality of the sample polypeptides to the solid support **1230** may occur simultaneously, for example by coupling the plurality of sample polypeptides to a plurality of anchoring groups that are already bound to the solid support. A polypeptide array process may include one or more rinsing processes to remove unbound or loosely adhered molecules from the solid support. The attachment of the anchoring groups to the solid support can be covalent or non-covalent in the above method or in other methods set forth herein.

The methods of sample preparation disclosed herein may further include the providing of surfactants to one or more processes of the sample preparation method. In some cases, a first sample preparation process (e.g., sample collection, sample storage, sample polypeptide separation, sample polypeptide functionalization, sample polypeptide coupling, or sample polypeptide binding) may utilize a first surfactant and a second sample preparation process may utilize a second surfactant. In some cases, the first surfactant and the second surfactant may be the same species of surfactant. In other cases, the first surfactant and the second surfactant may be a different species of surfactant. By analogy, the skilled person will readily recognize that the use of surfactants may be extended to additional sample preparation processes. A sample preparation process of a sample preparation method may be repeated. In some cases, the sample preparation process may be repeated under varying conditions, such as the presence of differing surfactants between the first cycle of the process and the second cycle of the process.

## Samples and Sample Collection

Samples of the present disclosure may initially contain any of a variety of materials derived from an environment of interest. In some cases, a sample may be reasonably expected to contain polypeptides, such as samples derived from living or deceased organisms. In other cases, a sample may be reasonably expected to contain no or minimal polypeptides, such as surface swabs from a clean room or pulverized extraterrestrial rock material. In yet other cases, the presence of polypeptides in a sample may be unknown. A sample of the present disclosure may be collected for any of a variety of purposes, including, but not limited to research, medical diagnostics, forensics, and commercial quality assurance.

A sample may be derived from an organism or an organism-derived substance or material. A sample may comprise any type of organism, including animals, non-human animals, humans, plants, fungi, bacteria, protozoa, archaea, viruses, or combinations thereof. The organism may be a domesticated, modified, or engineered organism, such as poultry, livestock, genetically-modified crops, non-modified crops, transgenic animals, transgenic plants, or production strains of microorganisms (e.g., *E. coli, S. cerevisiae*). A sample may comprise a substance derived from an organism, such as an extracellular secretion or debris from a deceased cell. A sample may be collected from a 2D cell culture line, a 3D cell culture line, a plant tissue sample, an animal tissue sample, a non-human animal tissue sample, a fungal tissue sample, a cultured tissue sample, a human patient-derived tissue sample, a veterinary patient-derived tissue sample, a skin or tissue swab, a tissue biopsy sample, a bodily fluid sample (e.g., blood plasma, blood serum, whole blood, urine, cerebrospinal fluid, saliva, semen, vaginal secretions, tears, mucus), a fecal sample, a cellular lysate, a fixed tissue sample (e.g., FFPE), a single-cell organism, a tissue-derived single cell, a secreted sample, an environmental sample, a microbial sample, a microbiome sample, a biofilm sample, or a curated sample.

A sample may be prepared to form one or more fractions comprising polypeptides. A polypeptide fraction may comprise sample polypeptides derived from a biological source such as an organism set forth above. In some cases, a polypeptide fraction may comprise sample polypeptides from a plurality of differing organisms, such as a microbiome-derived sample or a blood sample, or from a plurality of the same organism such as blood samples from a number of humans pooled into a single sample. A polypeptide fraction may comprise sample polypeptides derived from a curated source. A polypeptide fraction may comprise sample polypeptides derived from a curated source such as a forensic sample, an industrial sample, a consumer product, a geological sample, an archeological sample, a paleontological sample, and an extraterrestrial sample.

A standard polypeptide may have a peptide sequence that is endogenous to one or more of the organisms or other sources set forth above or elsewhere herein. Alternatively, a standard polypeptide may have a peptide sequence that is exogenous to one, more than one, or all of the organisms or other sources set forth above or elsewhere herein. In some configurations, a standard polypeptide may have a peptide sequence that is not found or not known to be found in any organism.

A polypeptide fraction formed from a sample may have full proteomic polypeptide coverage, or may contain some subset of a full proteome. For example, a polypeptide sample may provide sufficient amounts of polypeptides to represent the full proteome of a biological source from which the tissue is derived. In another example, a blood plasma sample may only represent the portion of a proteome that is secreted into, or otherwise present in, the bloodstream of an organism from which the sample is derived. A full proteomic sample may be considered a sample that displays the full sequence diversity or content of polypeptides that may be present within a cell, tissue, biological fluid or organism. A partial proteomic sample may be considered a sample that displays a portion of the full sequence diversity or content of polypeptides that may be present within a cell, tissue, biological fluid or organism. A proteomic sample may comprise about 0.000001%, 0.000005%, 0.00001%, 0.00005%, 0.0001%, 0.0005%, 0.001%, 0.005%, 0.01%, 0.05%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, or about 100% of the polypeptide sequence diversity or content of a cell, tissue, or organism. A proteomic sample may comprise at least about 0.000001%, 0.000005%, 0.00001%, 0.00005%, 0.0001%, 0.0005%, 0.001%, 0.005%, 0.01%, 0.05%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, or more than 95% of the polypeptide sequence diversity or content of a cell, tissue, or organism. Alternatively or additionally, a proteomic sample may comprise no more than about 95%, 90%, 85%, 80%, 75%, 70%, 65%, 60%, 55%, 50%, 45%, 40%, 35%, 30%, 25%, 20%, 15%, 10%, 5%, 1%, 0.5%, 0.1%, 0.05%, 0.01%, 0.005%, 0.001%, 0.0005%, 0.0001%, 0.00005%, 0.00001%, 0.000005%, 0.000001%, or less than about 0.000001% of the polypeptide sequence diversity or content of a cell, tissue, or organism.

Samples of the present disclosure may be derived from any of a variety of environments. Samples may be collected in natural, artificial, altered, engineered, or anthropogenic environments. Samples may comprise a gas phase, liquid phase, solid phase, or a combination thereof. Samples may be collected from the gas phase, liquid phase, solid phase, or a combination thereof. Exemplary samples collected from the gas phase may include directly captured particulates or aerosols (e.g., dust, viral particles or aerosols), particulates captured by air filtration, or swabs of surface-sedimented airborne particulates. Exemplary samples collected from the liquid phase may include water

samples, blood or other bodily fluid samples, or liquid rinses of solid surfaces. Exemplary samples collected from the solid phase may include soil samples, rock samples, biopsy or tissue samples, textile samples, or virtually any other solid material or substance.

A sample may be collected in any of a variety of environments. A sample may be collected by a human collector. A sample may be collected by an automated or robotic system. A sample may be collected with the aid of a collection device. A collection device may be a passive device (e.g., a filter or trap) or an active device (e.g., an intravenous or finger prick blood draw). A sample may be collected for any of a variety of purposes, including biological, ecological, paleontological, archeological, medical, diagnostic, clinical, or forensic purposes. A sample may be collected from a pre-existing sample, collected previously for a similar purpose (polypeptide analysis) or for a different purpose (nucleic acid analysis, phenotypic analysis, or an unanalyzed archival sample). A sample may be collected from any of a variety of locations, including air, water, soil, sand, rock, vegetation, wildlife, extraterrestrial, and anthropogenic objects. A sample may be collected in any of a variety of ecosystems within any known biome, including an oceanic, polar ice, arctic tundra, taiga, mountain, temperate deciduous forest, temperate evergreen forest, warm evergreen forest, tropical forest, tropical evergreen forest, chaparral, desert, savanna, semiarid, or temperate grassland ecosystem. A medical, clinical, or diagnostic sample may be collected in a medical, clinical, or diagnostic facility (e.g., hospital, medical lab, diagnostic facility), or in a non-medical environment (e.g., a farm or home). A medical, clinical, or diagnostic sample may be collected by a trained collector (e.g., a phlebotomist) or may be self-collected by a test subject (e.g., a urine or stool sample).

Standard polypeptides can be utilized during one or more of the sample collection processes set forth above or elsewhere herein. The standard polypeptides can be useful for characterizing the quality or quantity of the sample collected, characterizing the quality or quantity of sample polypeptides derived from the sample, characterizing a property of sample or the polypeptides obtained from the sample, or the like. For example, modification or degradation that is observed for sample polypeptides but not for standard polypeptides following collection and analysis can be attributed to the sample as opposed to the collection or analysis processes. Desired standard polypeptides can be introduced to a sample or fraction thereof prior to or during one or more of the sample collection processes set forth above or during another a process or step set forth above or elsewhere herein.

A sample may be collected into a sample vessel. A sample vessel may comprise any container capable of holding a sample. A sample vessel may comprise a vial, tube, jar, flask, cylinder, one or more wells of a multiwell plate, flow cell, capillary tube, vesicle, droplet, dipstick or bag. A sample vessel may be waterproof, corrosion-proof, leakproof, optically opaque or transparent, inert. A sample vessel may comprise a degradable material, such as a photolytic or thermally-decomposable polymer. A sample vessel may be sealable or unsealable. A sample vessel may be sterilized and/or sealed prior to the collection of a sample. A sample may be placed in a sample vessel in a sterile or non-sterile environment. A sample may be placed in a sample vessel by a method that prevents or minimizes contamination, e.g., glove bag, fume hood, positive pressure, gas flushing. A sample vessel may be used for transportation and/or storage of a sample to a location for a characterization assay. A sample vessel can contain a standard polypeptide before or after a sample is added to the vessel.

A sample may have a known or unknown age. A sample may have a known or unknown history of environmental insults. A sample may comprise a forensic, paleontological or archeological specimen. A sample may be collected immediately before a characterization assay. A sample may be collected from a living, senescent, deceased, or non-biological subject. In some cases, the age or history of a sample may be determined by a characterization assay. A sample may be fully or partially degraded, or fully intact. A standard polypeptide can be added to the sample to provide a basis for distinguishing degradation or modification that has occurred due to age or history of the sample from degradation or modification that occurs while preparing or analyzing the sample. More specifically, degradation or modification that occurs for a standard polypeptide(s) can be attributed to a technical artifact of the sample preparation or analysis process, whereas degradation or modification of sample polypeptides that are not also observed for the standard polypeptide(s) can be attributed to age or history of the sample. Comparison between sample polypeptides from samples having a different age or history can also be helpful in this regard.

A sample may be homogeneous or heterogeneous. A sample may be processed before a characterization assay to render the sample homogeneous. A sample may be homogenized by any appropriate method, including crushing, pulverization, grinding, mixing, and blending. A heterogeneous sample may be homogenized to produce a homogeneous sample. A sample may be subdivided or fractionated. A sample may be subdivided or fractionated to isolate, separate, refine, or concentrate constituent components of the sample. For example, a homogeneous tissue sample may be lysed to isolate membrane, cytoplasmic, and/or nuclear fractions. Whether or not a sample has been lysed, the contents of the sample can be fractionated by methods known in the art of biochemistry or analytical chemistry such as centrifugation, density-gradient centrifugation, chromatography (e.g. size exclusion chromatography, ionic exchange chromatography, reverse phase chromatography, affinity chromatography, thin layer chromatography etc.), liquid-phase extraction, solid-phase extraction, gel electrophoresis, filtration, distillation or the like. A homogeneous sample may be subdivided or fractionated to produce two or more heterogeneous fractions. A homogeneous sample may be subdivided or fractionated to produce at least one homogenous fraction, or to produce two or more homogeneous fractions, such as for performing

replicate or repeat characterization assays.

Standard polypeptides can be utilized during one or more of the homogenization or fractionation processes set forth above or elsewhere herein. The standard polypeptides can be useful for characterizing a property of the process, characterizing a property of sample polypeptides obtained from the process and/or quantifying sample polypeptides obtained from the process. For example, standard polypeptide(s) having expected separation properties when subjected to one or more of the separation techniques set forth above can be used to identify properties of sample polypeptides that co-fractionate with the standard polypeptide(s). Exemplary properties include, but are not limited to, polypeptide length, polypeptide isoelectric point, polypeptide hydrophobicity, polypeptide hydrophilicity, polypeptide hydrodynamic radius, polypeptide charge, polypeptide mass, polypeptide charge to mass ratio, polypeptide pKa, presence or absence of post-translational modifications on polypeptides, polypeptide structure, stability of polypeptides to proteases, or the like. Desired standard polypeptides can be introduced to a sample or fraction thereof prior to or during one or more of the homogenization or fractionation processes set forth above or elsewhere herein.

A sample may have a known or unknown stability. Stability may refer to the tendency of the sample, or particular components within the sample, to undergo chemical changes that alter the composition of the sample relative to the composition at the moment of its collection. In some cases, stability may specifically refer to the tendency of particular components of the sample, such as a polypeptide fraction or sample polypeptide, to degrade after sample collection. A sample may be stable, conditionally stable, partially unstable, or unstable. A stable sample may experience little or no observable chemical degradation or alteration. A conditionally stable sample may experience little or no observable chemical degradation or alteration within a specified set of environmental conditions (e.g., within 24 hours when stored at 20 °C or less). For example, less than 10%, 5%, 1% or even less of a stable or partially stable sample may experience observable chemical degradation or alteration. A partially unstable sample may experience chemical degradation or alteration of particular components while other components remain unchanged. For example, less than 50%, 25%, 10%, 5%, 1% or even fewer of the polypeptide species in the sample may experience observable chemical degradation or alteration. An unstable sample may experience chemical degradation or alteration regardless of environmental conditions after sample collection. Global or component-specific instability or degradation may be described by a kinetic rate law, such as a first- or second-order reaction rate.

A sample may be treated before, during or after collection to prevent or inhibit degradation or alteration of a polypeptide fraction. The sample may be in any of a variety of states including, for example, an intact biological sample, crude lysate, fraction or other state set forth herein or known in the art. A sample may be treated to prevent or inhibit physical, chemical or biological processes that degrade or alter a polypeptide fraction or sample polypeptide. Polypeptides in a sample may be subject to chemical and/or biological processes such as oxidation, reduction, cleavage (hydrolytic or enzymatic), complexation, agglomeration, or post-translational modification. A sample containing one or more sample polypeptides may be treated to prevent or inhibit polypeptide degradation or alteration processes to obtain an instantaneous or near-instantaneous polypeptide or polypeptide panel at the time of sample collection. A sample may be treated to prevent or inhibit one or more specific chemical or biological processes that degrade or alter the sample polypeptide(s). For example, a protease inhibitor may be added to a sample to reduce or eliminate enzymatic polypeptide cleavage. A sample may be treated to cease all polypeptide activity, thereby preventing any biological or enzymatic polypeptide degradation or alteration processes. For example, a sample may be treated with a denaturant (e.g., guanidinium chloride or heat) to denature all polypeptides, thereby inhibiting or preventing polypeptide-mediated or polypeptide-related biological processes. A sample may be treated with one or more chemical agents to control the sample chemistry. A sample may be treated with one or more chemical agents, including acids, bases, buffers, salts, oxidizers, reductants, chelating agents, oxygen scavengers, surfactants, detergents, crosslinking reagents, aqueous solvents, and non-aqueous solvents.

Standard polypeptides can be useful for evaluating the stability of sample polypeptides during a treatment or process set forth above or elsewhere herein. For example, standard polypeptide(s) having known or expected susceptibility to various types of proteases or other reagents can be used during the treatment or process. The quantity or characteristic(s) of degradation that occurs for sample polypeptides can be determined by normalization with respect to the quantity or characteristic(s) of degradation that occurs for the standard polypeptides. Exemplary characteristics include, but are not limited to, presence or absence of protease recognition sites in a sample polypeptide, location of protease recognition sites in a sample polypeptide, and presence or absence of post-translational modifications that alter the extent of proteolysis. Desired standard polypeptides can be introduced to a sample or fraction thereof prior to or during one or more of the treatments or processes set forth above or elsewhere herein.

A sample may undergo one or more treatments that stop biological activities that affect polypeptide expression, composition, and/or abundance. The effect of such treatments may be to capture a near instantaneous profile of a proteome within a biological system. The one or more treatments may include stopping or inhibiting transcription and/or translation, degrading or removing nucleic acids, stopping or inhibiting post-translational modification of polypeptides, and stopping or inhibiting polypeptide degradation. A sample may undergo a treatment method such as heat treatment, cold treatment, freezing, enzymatic treatment (e.g., endonucleases, exonucleases, proteases), chemical treatment, or

combinations thereof. Chemical treatments to stop or inhibit biological processes in samples may include addition of protease inhibitors, denaturants, acids, bases, salts, crosslinking reagents or surfactants. In some cases, chemical treatments may be combined with temperature treatments (e.g., heating, cooling) to increase the effectiveness of the treatment. A sample may undergo a treatment to stop or inhibit biological activity in the presence of a detergent. Without wishing to be bound by theory, the presence of a detergent during a sample treatment may separate some unwanted impurities from the polypeptide fraction of the sample, thereby enhancing or improving the separation and/or isolation of a polypeptide fraction from the sample. A treatment to stop biological activities may occur immediately after sample collection, or within a reasonable timeframe, for example no more than about 30 seconds (s), 1 minute (min), 2 mins, 3 mins, 4 mins, 5 mins, 10 mins, 15 mins, 20 mins, 30 mins, 45 mins, 1 hour (hr), 3 hrs, 6 hrs, 12 hrs, or no more than about 24 hrs. A treatment to stop biological activities may commence upon cell lysis, for example, by lysing the cell(s) in the presence of the treatment. Alternatively, a treatment to stop biological activities can be initiated after cell lysis, for example, within the time periods exemplified above or during a collection or fractionation process set forth herein. Polypeptide standards can be used to distinguish effects of the treatments on one or more sample polypeptides. For example, modification or degradation that is observed for sample polypeptides that are subjected to a treatment set forth above or elsewhere herein, but not observed for standard polypeptides in an untreated sample, can be attributed to the treatment.

A sample or sample polypeptide may be stored after collection. A sample or sample polypeptide may be stored after collection for a specified amount of time. A sample or sample polypeptide may be stored after collection at a specified temperature and/or pressure. A sample or sample polypeptide may be stored after collection at a specific humidity or under a specific inert atmosphere (e.g. in a vacuum or under inert gas). A sample or sample polypeptide may be stored after collection for a sufficient amount of time to permit the continuance or completion of a biological process including, for example, a biological process that was initiated prior to the collection process. A sample or sample polypeptide may be stored after collection for a sufficient amount of time to permit the completion of a post-collection treatment process such as a biological, chemical or physical process. The post-collection treatment process can involve only components that were present in the sample, or with the sample polypeptide, prior to initiating the collection process or alternatively the post-collection treatment process can involve one or more exogenous reagent that was added to the sample or sample polypeptide during or after initiation of the collection process. In some cases, a sample or sample polypeptide may be stored while awaiting or undergoing transport to a different location (e.g., from a collection site to an analysis facility).

A sample or sample polypeptide may be stored at a specified temperature. A sample or sample polypeptide may be stored at a temperature sufficient to freeze liquid in the sample. A sample or sample polypeptide may be stored at a temperature sufficient to prevent freezing of liquid in the sample. A sample or sample polypeptide may be stored at a temperature of about - 80 °C, - 70 °C, - 60 °C, - 50 °C, - 40 °C, - 30 °C, - 20 °C, - 10 °C, - 5 °C, 0 °C, 4 °C, 10°C, 20 °C, 30°C, 37 °C, 40 °C, 50 °C, 60 °C, 70 °C, 80 °C, 90 °C, or about 95 °C. A sample or sample polypeptide may be stored at a temperature of at least about - 80 °C, - 70 °C, - 60 °C, - 50 °C, - 40 °C, - 30 °C, - 20 °C, - 10 °C, - 5 °C, 0 °C, 4 °C, 10 °C, 20°C, 30°C, 37 °C, 40 °C, 50 °C, 60°C, 70 °C, 80 °C, 90 °C, or at least about 95 °C. Alternatively or additionally, a sample or sample polypeptide may be stored at a temperature of no more than about 95 °C, 90 °C, 80 °C, 70 °C, 60 °C, 50 °C, 40 °C, 37 °C, 30 °C, 20 °C, 10 °C, 4 °C, 0 °C, - 5 °C, - 10 °C, - 20 °C, - 30 °C, - 40 °C, - 50 °C, -60 °C, - 70 °C, or about - 80 °C.

A sample or sample polypeptide may be stored for a specific amount of time. A sample or sample polypeptide may be stored at a specific temperature for a specific amount of time. A sample or sample polypeptide may be stored for no more than a specific amount of time to prevent alteration, degradation or aging of the sample or sample polypeptide. A sample or sample polypeptide may be stored for about 1 min, 30 mins, 1 hr, 3 hrs, 6 hrs, 12 hrs, 1 day, 2 days, 3 days, 4 days, 5 days, 6 days, 7 days, 10 days, 2 weeks (wks), 3 wks, 4 wks, 1 month (mth), 2 mths, 3 mths, 6 mths, 9 mths, 1 year (yr), 2 yrs, 3 yrs, 4 yrs, 5 yrs, 10 yrs, 20 yrs, 25 yrs, 50 yrs, or about 100 yrs. A sample or sample polypeptide may be stored for at least about 1 min, 30 mins, 1 hr, 3 hrs, 6 hrs, 12 hrs, 1 day, 2 days, 3 days, 4 days, 5 days, 6 days, 7 days, 10 days, 2 wks, 3 wks, 4 wks, 1 mth, 2 mths, 3 mths, 6 mths, 9 mths, 1 yr, 2 yrs, 3 yrs, 4 yrs, 5 yrs, 10 yrs, 20 yrs, 25 yrs, 50 yrs, or at least about 100 yrs. Alternatively or additionally, a sample or sample polypeptide may be stored for no more than about 100 yrs, 50 yrs, 25 yrs, 20 yrs, 10 yrs, 5 yrs, 4 yrs, 3 yrs, 2 yrs, 1 yr, 9 mths, 6 mths, 3 mths, 2 mths, 1 mth, 4 wks, 3 wks, 2 wks, 10 days, 7 days, 6 days, 5 days, 4 days, 3 days, 2 days, 1 day, 12 hrs, 6 hrs, 3 hrs, 1 hr, 30 mins, 1 min, or less than about 1 min.

A sample or sample polypeptide may be transported from a collection site to another site for subsequent processing or analysis. The sites may be within a single facility or campus. Alternatively, the sites may be located at different facilities or campuses, for example, in different cities, counties, states, countries, territories, time zones, or other geographically or politically delineated locations. During transport, a sample or sample polypeptide may be stored in a controlled environment. A controlled environment may prevent exposure of a sample to variable or detrimental environmental conditions. A controlled environment may prevent exposure of a sample to undesirable levels of heat, cold, humidity, radiation, vibration, pressure, or contaminants. A controlled environment may prevent a sample from undergoing biological, physical or chemical processes that affect the chemical composition or function of the sample. A controlled environment may prevent degradation or alteration of a sample polypeptide or polypeptide fraction of a sample. A sample or sample polypeptide may be stored in a controlled environment based upon instructions provided by an individual or entity providing the sample or sample polypeptide, or by an individual or entity receiving the sample or sample polypeptide.

A controlled environment may include various forms of environmental regulation, including heaters, ovens, humidifiers, dehumidifiers, inert gas, vacuum, insulation, padding, shielding, lighting, and filtration (liquid or gas). A sample or sample polypeptide may be collected according to a prescribed or pre-defined protocol. A prescribed or pre-defined protocol may be specified by an individual or entity collecting the sample or sample polypeptide, an individual or entity that will receive the sample or sample polypeptide, or by an accepted standard protocol, such as an academic standard protocol, a commercial standard protocol, or an industrial standard protocol. A sample or sample polypeptide may be collected in a duplicate or replicate fashion. A sample may be divided into multiple sub-samples for the purposes of quality control or data verification. A sample may be divided into multiple sub-samples, with each sub-sample intended for a different type of disposal, storage, modification, manipulation or analysis. For example, a tissue sample may be divided into three separate sub-samples, with each sub-sample intended for either 1) a polypeptide assay, 2) nucleic acid characterization assay, or 3) a small-molecule metabolite characterization assay.

Standard polypeptides can be useful for evaluating the stability of sample polypeptides during a storage or transport process set forth above or elsewhere herein. For example, standard polypeptide(s) having known or expected susceptibility to various physical, chemical or biological processes that are known os suspected to occur during storage or transport can be used. The quantity or characteristic(s) of degradation or other change that occurs for sample polypeptides can be determined by normalization with respect to the quantity or characteristic(s) of degradation or other change that occurs for the standard polypeptides. Desired standard polypeptides can be introduced to a sample or fraction thereof prior to or during one or more of the storage or transport processes set forth above or elsewhere herein.

A sample or fraction thereof may be collected utilizing a collection kit. A collection kit may be specific to a particular assay to be performed on the sample. For example, a collection kit for a polypeptide assay may include polypeptide-specific reagents to protect and/or preserve polypeptides within a sample. A collection kit may include one or more sample vessels, one or more reagents, instructions for use of the sample collection kit and optionally intermediate sample vessels, a sealant for the vessel(s), a label for the vessel(s) such as a barcode or radio frequency identification device (RFID), or packaging for transport and/or storage of the sample vessel(s). A sample collection kit may include one or more reagents for any of a variety of purposes, including sample preservation, sample stability, sample quality control, processing and/or purification, and sample storage. A sample collection kit may include reagents such as buffers, acids, bases, solvents, denaturants, surfactants, detergents, reactants, labels (e.g., fluorophores, radiolabels), indicator dyes, enzymes, enzyme inhibitors, oxygen scavengers, water scavengers, humectants, affinity reagents (e.g., antibodies), or other capture agents (e.g., biotinylated particles). A sample collection kit may include one or more reagents in liquid or solid form. A sample collection kit may include one or more separate reagents and/or polypeptide standards that are added to the sample collection vessel before or after sample collection. A sample collection kit may include one or more reagents and/or polypeptide standards that are provided within the sample collection vessel. For example, reagents and/or polypeptide standards may be provided in a crystallized or coated form on a surface of the collection vessel, or may be in a liquid solution within the collection vessel. A sample collection kit may have a pre-determined shelf life due to the provided reagents or other materials.

A sample collection kit for a polypeptide assay may vary depending upon the type of characterization to be performed. For example, a collection kit may be specific to a sample component (e.g., blood plasma from whole blood) or may be non-specific to a sample (e.g., full proteome from a tissue or blood sample). A sample collection kit may be sample-specific (e.g., blood, urine, tissue, fecal, cell swab, etc.) or may be sample-agnostic. A sample collection kit may include one or more polypeptide standards that are useful for characterizing a process carried out with the kit or product produced by the process. A sample-agnostic collection kit may be capable of preserving and/or preparing a protein fraction for analysis regardless of the nature or form of the sample.

A sample collection kit for a polypeptide assay may be utilized according to a provided set of instructions. The instructions may be directed to use of polypeptide standards in accordance with teachings set forth herein. A sample collection kit may be utilized by a technician or self-collecting subject. A technician utilizing a sample collection kit may be specifically trained in the proper utilization of the sample collection kit. A sample collection kit protocol may employ one or more intermediate steps before collection of the sample is complete. Intermediate steps during sample collection may be performed in the sample collection vessel or in a separate medium (provided with the kit or provided by the collector). For example, a blood sample may be fractionated by a phlebotomist, with only the red blood cell or plasma fraction saved for collection. A sample collection kit may include indicator dyes, litmus strips, or other methods of confirming successful sample collection and/or preparation. A sample collection kit may include a sealant (e.g., an adhesive or sticker) to ensure that a sample has not been tampered with or damaged during storage or transport. A sample collection kit may include a label for sample tracking by the collector or the analysis facility. A label for a sample collection vessel may include a serial number, RFID, bar code or QR code. A label for a sample collection vessel may be pre-printed or pre-applied to a sample collection vessel, or may be placed by a collector.

A sample kit may comprise one or more reagents that are intended to perform one or more steps of a sample preparation process. For example, a sample kit may comprise one or more reagents that lyse cells, stop biological processes (e.g., protein synthesis or degradation), stop degradative processes (e.g., oxidation), functionalize sample

polypeptides to add reactive handles to the polypeptides, crosslink polypeptides, or couple sample polypeptides to anchoring groups to form polypeptide composites. In some cases, a sample kit may comprise two or more reagents that are added in a step-wise fashion according to a set of instructions. In other cases, a sample kit may comprise one or more reagents that are added simultaneously to perform one or more sample preparation processes set forth herein. A sample kit can include one or more standard polypeptides that are added in a stepwise fashion or simultaneously to perform one or more sample preparation processes set forth herein. In some configurations, the use of a sample kit may comprise one or more of the steps of: 1) adding a sample to a handling or storage vessel; 2) adding one or more reagents or standard polypeptide to the handling or storage vessel; 3) combining the sample, the one or more reagents and/or the standard polypeptide(s) in the handling or storage vessel to form a sample mixture; 4) forming a plurality of sample polypeptide composites and/or standard polypeptide composites in the handling or storage vessel in the sample mixture; 5) applying the sample mixture to a solid support; and 6) removing a waste fraction from the solid support to form a polypeptide array.

A sample may undergo one or more sample pretreatment processes during or after separation from a natural source. A sample pretreatment process may occur before the sample undergoes a sample preparation process for a polypeptide assay. In particular configurations, a sample pretreatment process may occur before, during or after a step of a sample preparation process set forth herein. A sample pretreatment process may comprise one or more *in vitro* processes that alter a sample or a fraction of polypeptides within the sample. A sample pretreatment process may alter a sample or a fraction of polypeptides within the sample in a predictable fashion (e.g., treatment with an enzyme with a known and/or characterized selectivity or reactivity). A sample pretreatment process may alter a sample or a fraction of polypeptides within the sample in an unknown, unpredictable, and/or uncharacterized fashion. For example, a sample may be treated with a drug or drug analog that has the possibility of physically or chemically altering one or more polypeptides within the sample. Exemplary sample pretreatment processes may include, without limitation, oxidation, reduction, denaturation, lysis, homogenization, digestion, labeling (e.g., fluorophores, barcodes, radiolabels, isotopes), dilution, concentration, disruption of polypeptide complexes, formation of polypeptide complexes, crosslinking of polypeptides to other biomolecules, addition of post-translational modifications, and removal of post-translational modifications. In some cases, a sample pretreatment process may comprise the cross-linking of a polypeptide to one or more neighboring or adjacent biomolecules (e.g., polypeptides, nucleic acids, polysaccharides, lipids, etc.). Cross-linking of polypeptides to neighboring biomolecules may capture short-term or long-term spatial associations between related biological components that may be disrupted by subsequent sample preparation processes.

A sample pretreatment process may include contacting a sample, or fraction thereof, with an enzyme that causes post-translational modification of a polypeptide (e.g. sample polypeptide or standard polypeptide). An enzyme that causes any of a variety of post translational modifications can be used including, but not limited to, enzymes that add or remove a moiety set forth herein. For example, a sample, or fraction thereof, can be treated with a kinase to add a phosphate moiety to a polypeptide or with a phosphatase that removes a phosphate moiety from a polypeptide. The phosphate moiety can be present on a serine, threonine, tyrosine, aspartate or glutamate residue of the polypeptide. A sample, or fraction thereof, can be treated with an N-terminal acetyltransferase to add an acetyl moiety to the N-terminus of a polypeptide or with an acetyltransferase that adds an acetyl moiety to a lysine of the polypeptide. Acetyl moieties can optionally be removed by a deacetylase enzyme. A glycosyltransferase or galactosyltransferase can be used to add a carbohydrate moiety to a polypeptide. For example, a serine or threonine residue can have an O-linked glycosyl moiety, or an asparagine residue can have an N-linked glycosyl moiety. All or part of a carbohydrate moiety can be removed from a polypeptide using an appropriate enzyme such as $\alpha$2-3,6,8,9-neuraminidase; $\beta$1,4-galactosidase; $\beta$-N-acetylglucosaminidase; endo-$\alpha$-N-acetylgalactosaminidase or PNGase F. A proline, lysine, asparagine, aspartate or histidine amino acid can be hydroxylated by a hydroxylase. A polypeptide can be methylated by an arginine methyltransferase or lysine methyltransferase. A polypeptide can be ubiquitinated using ubiquitin-activating enzyme, ubiquitin-conjugating enzyme, and ubiquitin ligase. Ubiquitin moieties can be removed form polypeptides using a deubiquitinating enzyme or protease.

A sample pretreatment process may include contacting a sample, or fraction thereof, with chemical reagent that produces a chemically modified polypeptide (such as a chemically modified sample polypeptide or a chemically modified standard polypeptide). Cysteine can be reacted with 5,5-dithio-bis-(2 nitrobenzoic acid) (DTNB) to form TNB modified cysteine. Optionally, the TNB modified cysteine can be reacted with $C{\equiv}N^-$, $SO_3^{2-}$, or $RS^-$ nucleophiles to form cysteine-S-CN, cysteine-S-$SO_3$ or cysteine-S-S-R adducts, respectively, wherein R is an organic moiety such as a hydrocarbon. Cysteine can also be modified with an alkylating reagent (e.g. amide, ketone, carboxylic acid, ester or heterocyclic strained ring) or alkene (e.g. 4-vinylpyridine or maleimide) to form a cysteine thioether. Amino moieties in polypeptides can be acylated, for example, via reaction with succinic anhydride or maleic anhydride; guanidinated, for example, via reaction with O-methylisourea; amidinated, for example, by reaction with methylacetimidate or methylpicolinimidate; acetylated, for example, via reaction with acetic anhydride; carbamylated, for example, via reaction with cyanate; arylated, for example, via reaction with 1-fluoro-2,4-dinitro-benzene or trinitrobenzenesulfonic acid; deaminated, for example, via reaction with nitrous acid; acylated, for example, via reaction with succinic anhydride; or reductively alkylated, for example, via reaction with formaldehyde and $NaBH_4$. Carbonyl moieties in polypeptides can be converted to esters or amides using reagents known in the art. Particularly useful reagents for modifying carbonyl moieties are carbodiimides such as 1-ethyl-3-(3-dimethylaminopropyl)carbodiimide or dicyclohexylcarbodiimide. Histidine can be reacted with ethoxy formic anhydride or subjected to Bamberger cleavage. Photoreactive reagents can be useful for modifying a variety of moieties in a polypeptide. Exemplary reagents and techniques for chemical modification of polypeptides are set forth, for example, in Means and Feeney Bioconjugate Chem. 1, 2-12 (1990); Matthews et al. Meth. Enz. 208:468-496 (1991); and Glazer, The Proteins 3rd Ed. Vol. 2 (1976).

A sample pretreatment process may include crosslinking a polypeptide to another reagent or entity, such as a polypeptide. Exemplary crosslinkers include heterobifunctional or homobifunctional crosslinkers, both of which have a spacer moiety flanked by reactive moieties. The spacers can either be cleavable, for example, using conditions that are relatively mild to preserving the integrity of the crosslinked species, or the spacers can be effectively non-cleavable under the conditions of use. Zerolength crosslinkers, having no spacer moiety, can also be useful. Any of a variety of reactive moieties including those exemplified above or others known in the art can be used. Exemplary crosslinking reagents are set forth in Wong, Chemistry of Protein Conjugation and Cross-Linking, CRC Press, Boca Raton, FL (1991) or available commercially from Sigma Aldrich (St. Louis, MO) or Thermo Fisher (Waltham, MA).

A standard molecule (e.g. standard polypeptide) can be utilized during one or more of the sample pretreatment processes set forth above or elsewhere herein. The standard can be useful for characterizing the reactivity of reagents used for modifying polypeptides. For example, a standard polypeptide having known reactivity toward a chemical reagent or enzyme can be used to measure the extent to which the reactions occurred. Another useful type of standard is a standard polypeptide or other molecule that is modified when an unwanted side reaction occurs, such that the modification can be observed to determine if unwanted reactions occurred for one or more polypeptide in a sample. Pretreatment standard polypeptides (or other pretreatment standard molecules) can be introduced to a sample or fraction thereof prior to or during a pretreatment process or step set forth above or elsewhere herein.

A sample may comprise a plurality of sample polypeptides. In some cases, a sample preparation method may be configured to capture or collect all or a maximal quantity of sample polypeptides from a sample. In other cases, a sample preparation may be configured to capture or collect a specific subset of sample polypeptides from a sample. A sample may comprise sample polypeptides and non-target polypeptides, where the non-target polypeptides are intended to be excluded after collection or capture of the sample polypeptides. Non-target polypeptides may include damaged polypeptides, degraded polypeptides, truncated polypeptides, small polypeptides (e.g., less than about 100, 75, 50, 40, 30, 25, 20, 15, 10, or less than 10 amino acids) or large polypeptides (e.g., greater than about 5000, 10000, 15000, 20000, 25000, or more than 25000 amino acids). A sample polypeptide content before a sample preparation method may be measured, for example by UV absorption, Biuret methods, colorimetric dye methods, or fluorescent dye methods. A sample polypeptide content may be measured on a mass or molar basis. The same measurements can be used for standard polypeptides. A sample preparation method may produce a polypeptide fraction or polypeptide array comprising a plurality of sample polypeptides. The plurality of sample polypeptides may comprise at least about 0.000001%, 0.000005%, 0.00001%, 0.00005%, 0.0001%, 0.0005%, 0.001%, 0.005%, 0.01%, 0.05%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, 99.5%, 99.9%, 99.99%, 99.999%, 99.9999%, 99.99999% or more than 99.99999% of the sample polypeptide content of a sample on a mass or molar basis. Alternatively or additionally, the plurality of sample polypeptides may comprise no more than about 99.99999%, 99.9999%, 99.999%, 99.99%, 99.9%, 99.5%, 99%, 95%, 90%, 85%, 80%, 75%, 70%, 65%, 60%, 55%, 50%, 45%, 40%, 35%, 30%, 25%, 20%, 15%, 10%, 5%, 1%, 0.5%, 0.1%, 0.05%, 0.01%, 0.005%, 0.001%, 0.0005%, 0.0001%, 0.00005%, 0.00001%, 0.000005%, 0.000001%, or less than 0.000001% of the sample polypeptide content of a sample on a mass or molar basis.

A plurality of polypeptides can be characterized on the basis of polypeptide sequence content. The polypeptide sequence content for a fraction of polypeptides obtained from a particular sample can be measured as the percent of sequences in the sample that are present in the fraction. A basis for polypeptide sequence content may include genetic analysis (e.g., known or predicted translated amino acid sequences of identified genes) and/or measurements of polypeptide sequence content in other polypeptide samples. The measure is typically grounded in a sequence string length (also referred to as a "word' length) of a specified length, for example, a string length of 3 amino acids, 5 amino acids, 8 amino acids, 10 amino acids, 12 amino acids, 15 amino acids, 20 amino acids, 25 amino acids, 50 amino acids, 100 amino acids or more. Accordingly, the number of sequences having a particular string length in a sample is compared to the number of sequences having that string length in a fraction obtained from the sample. The sample can be a biological source or sample derived from a biological source. The fraction can be a plurality of polypeptides in a liquid sample, in an array or in another composition set forth herein. The fraction of sample polypeptides may comprise a polypeptide sequence content that includes at least about 0.000001%, 0.000005%, 0.00001%, 0.00005%, 0.0001%, 0.0005%, 0.001%, 0.005%, 0.01%, 0.05%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, 99.5%, 99.9%, 99.99%, 99.999%, 99.9999%, 99.99999% or more than 99.99999% of the polypeptide sequence content of a sample, when comparing sequences having a selected sequence string length. Alternatively or additionally, the fraction of sample polypeptides may comprise a polypeptide sequence content that includes no more than about 99.99999%, 99.9999%, 99.999%, 99.99%, 99.9%, 99.5%, 99%, 95%, 90%,

85%, 80%, 75%, 70%, 65%, 60%, 55%, 50%, 45%, 40%, 35%, 30%, 25%, 20%, 15%, 10%, 5%, 1%, 0.5%, 0.1%, 0.05%, 0.01%, 0.005%, 0.001%, 0.0005%, 0.0001%, 0.00005%, 0.00001%, 0.000005%, 0.000001%, or less than 0.000001% of the polypeptide sequence content of a sample, when comparing sequences having a selected sequence string length.

An internal standard may be combined with a sample before, during, or after sample collection. The internal standard may comprise polypeptides or other components that permit quality control of any subsequent sample processing. Internal standards may be used to assess the quality and/or viability of a sample after handling, storage, or sample preparation processing. Internal standards may be used to determine the success or efficiency of sample preparation processing. Internal standards may permit normalization within a sample or across a group of samples. For example, an internal standard may be utilized to determine the differential separation, functionalization, or conjugation of particular polypeptides within a sample on the basis of a particular property (e.g., size, hydrophobicity, surface charge density, etc.). In another example, an internal standard may be utilized across a group of samples to assess the variability of a sample preparation process based upon observed differences in presence of the internal standards in each prepared sample.

An internal standard comprising a plurality of standard polypeptides may be combined with a plurality of sample polypeptides during a sample preparation method. The total quantity of a standard polypeptides combined with a plurality of sample polypeptides may be adjusted depending upon the intended use of the standard polypeptides for quantitative or qualitative characterization of a sample preparation method. A mass fraction of sample polypeptides in a polypeptide mixture may be calculated as a ratio or percentage of a plurality of sample polypeptides to the total quantity of the plurality of sample polypeptides and the total quantity of a plurality of standard polypeptides (e.g., sample collection standard polypeptides, sample handling standard polypeptides, separation standard polypeptides, functionalization standard polypeptides, coupling standard polypeptides, and attachment standard polypeptides) provided to a sample preparation method. For example, a mixture of a plurality of sample polypeptides with a mass of 9 nanograms and a plurality of separation standard polypeptides with a mass of 1 nanograms would have a sample polypeptide mass fraction of 90% or 0.9. A sample polypeptide mixture comprising a plurality of standard polypeptides may have a sample polypeptide mass or molar fraction of at least about 0.000001%, 0.000005%, 0.00001%, 0.00005%, 0.0001%, 0.0005%, 0.001%, 0.005%, 0.01%, 0.05%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, 99.5%, 99.9%, 99.99%, 99.999%, 99.9999%, 99.99999% or more than 99.99999%. Alternatively or additionally, a sample polypeptide mixture comprising a plurality of standard polypeptides may have a sample polypeptide mass or molar fraction of no more than about 99.99999%, 99.9999%, 99.999%, 99.99%, 99.9%, 99.5%, 99%, 95%, 90%, 85%, 80%, 75%, 70%, 65%, 60%, 55%, 50%, 45%, 40%, 35%, 30%, 25%, 20%, 15%, 10%, 5%, 1%, 0.5%, 0.1%, 0.05%, 0.01%, 0.005%, 0.001%, 0.0005%, 0.0001%, 0.00005%, 0.00001%, 0.000005%, 0.000001%, or less than 0.000001%.

A sample polypeptide mixture may comprise a plurality of standard polypeptides, in which the plurality of standard polypeptides comprises one or more types of standard polypeptides (e.g., coupling standards, separation standards, attachment standards, etc.), such as, for example, at least about 1, 2, 3, 4, 5, 6, 7, 8, 9, 10, 11, 12, 13, 14, 15 ,16, 17, 18, 19, 20, or more than 20 types of standard polypeptides. Alternatively or additionally, a sample polypeptide mixture may comprise a plurality of standard polypeptides, in which the plurality of standard polypeptides comprises one or more types of standard polypeptides, such as, for example, no more than about 20, 19, 18, 17, 16, 15, 14, 13, 12, 11, 10, 9, 8, 7, 6, 5, 4, 3, 2 , or less than 2 types of standard polypeptides. In some cases, differing types of standard polypeptides may be present in a sample polypeptide mixture in identical or similar amounts (e.g., within about 20%, 15%, 10%, 5%, 1%, 0.5%, 0.1%, or less than 0.1% of an average amount or concentration). Differing types of standard polypeptides may be present in differing amounts in a sample polypeptide mixture. For example, a plurality of standard polypeptides may contain about 20% coupling standard polypeptides and 10% separation standards on a molar basis. Differences in amount of a type of standard may arise due to sample preparation conditions. For example, standards may be introduced in similar or identical quantities at each step of a sample prepration process, in which differences in amounts of each type of standard may be correlated to a relative efficiency of a step of the process. Alternatively, standards may be introduced in differing amoutns throughout a sample preparation process for other reasons, such as the relative detection sensititivity of a type of standard, or a relative expense of a particular type of standard. A plurality of standard polypeptides may comprise at least about 0.000000001%, 0.00000001%, 0.0000001%, 0.000001%, 0.00001%, 0.0001%, 0.001%, 0.01%, 0.1%, 1%, 5%, 10%, 15%, 20%, 25%, 30%, 35%, 40%, 45%, 50%, 55%, 60%, 65%, 70%, 75%, 80%, 85%, 90%, 95%, 99%, 99.9%, or more than 99.% of a type of standard polypeptide on a mass or molar basis. Alternatively or additionally, a plurality of standard polypeptides may comprise no more than about 99.9%, 99%, 95%, 90%, 85%, 80%, 75%, 70%, 65%, 60%, 55%, 50%, 45%, 40%, 35%, 30%, 25%, 20%, 15%, 10%, 5%, 1%, 0.1%, 0.01%, 0.001%, 0.0001%, 0.00001%, 0.000001%, 0.0000001%, 0.00000001%, 0.000000001%, or less than 0.000000001% of a type of standard polypeptide on a mass or molar basis.

### Internal Standards

A polypeptide fraction may be combined with one or more internal standards to form a polypeptide mixture.

Internal standards may include standard polypeptides (also referred to herein as "internal standard polypeptides"), or internal standard non-polypeptides. Any of a variety of the standard polypeptides set forth herein can be used as internal standard polypeptides. Several configurations of the methods, compositions and systems of the present disclosure are exemplified with reference to standard polypeptides. It will be understood that internal standard non-polypeptides can be used as alternatives or supplements in the methods, compositions and systems of the present disclosure.

An internal standard may be any composition, such as a standard polypeptide, that is combined with a polypeptide fraction in order to: 1) provide a history of the polypeptide fraction any time during or after sample collection, 2) provide qualitative or quantitative measures of efficiency in various processing steps, 3) provide positive or negative controls for reagents or steps used in a polypeptide assay, or 4) a combination thereof. An internal standard may provide a history of a polypeptide fraction by displaying chemical properties that are linked to aspects of sample collection, handling, and storage. For example, an internal standard may provide information on sample age (e.g., by conversion of a solid support standard by a slow chemical reaction), sample temperature (e.g., polypeptides that aggregate at high or low temperatures), sample pH (e.g., indicator dyes), sample oxidation, or sample degradation.

An internal standard may be provided to a polypeptide fraction as a composition comprising one or more components. Internal standards may include internal standard polypeptides, and internal standard non-polypeptides. Internal standards may include small molecule components such as dyes, indicators, and enzymatic substrates. Internal standards may include biomolecule monomers (e.g., nucleotides, amino acids) with detectable labels (e.g., radiolabels, deuterium). Incorporation of labeled biomolecule monomers may provide evidence of intended or unintended on-going biological processes in a sample. In some cases, polypeptides containing incorporated labeled amino acids may be excluded from a polypeptide assay due to their likely formation or modification after sample collection. In other cases, polypeptides containing incorporated labeled amino acids may be included in a polypeptide assay due to their likely formation or modification after sample collection. In some cases, an internal standard may comprise a paired solid support and enzyme that provides an estimate of sample age or storage history. For example, an enzyme/substrate system may be included with a frozen sample to show improper storage if the presence of a converted substrate is detected. In another example, an enzyme/substrate system that produces a detectable product on a timescale comparable to the period of time that a sample is stored, transported or collected may be used to qualitatively or quantitatively characterize a sample after collection, transport or storage. Enzyme/substrate systems may be chosen based upon the expected length of collection, handling, and/or storage.

Internal standards may comprise a plurality of internal standard polypeptides. Internal standard polypeptides may be chosen based upon the information for which they are utilized to provide, the nature of the polypeptide fraction with which they will be combined, and the nature of the polypeptide assay within which they will be utilized. An internal standard, such as sample collection standard polypeptides, sample handling standard polypeptides, separation standard polypeptides, functionalization standard polypeptides, coupling standard polypeptides, and attachment standard polypeptides, comprising a plurality of polypeptides may comprise a single species of polypeptide. An internal standard, such as sample collection standard polypeptides, sample handling standard polypeptides, separation standard polypeptides, functionalization standard polypeptides, coupling standard polypeptides, and attachment standard polypeptides, comprising a plurality of polypeptides may comprise one or more species of polypeptide. An internal standard, such as sample collection standard polypeptides, sample handling standard polypeptides, separation standard polypeptides, functionalization standard polypeptides, coupling standard polypeptides, and attachment standard polypeptides, comprising a plurality of polypeptides may comprise two or more different species of polypeptide. An internal standard comprising a plurality of polypeptides may comprise a species of polypeptide that is a native species of a sample with which the internal standard polypeptides will be combined (e.g., an *E. coli* polypeptide as an internal standard for an *E. coli* sample). An internal standard comprising a plurality of polypeptides may not comprise any species of polypeptide that is a native species of a sample with which the internal standard polypeptides will be combined (e.g., no *E. coli* polypeptides contained in an internal standard for an *E. coli* sample). An internal standard may comprise polypeptides derived from the same source as a sample polypeptide. For example, an internal standard for a human sample may comprise polypeptides derived from the same human or a different human. Alternatively, an internal standard may comprise polypeptides derived from a differing source from a sample polypeptide. Internal standard polypeptides, such as sample collection standard polypeptides, sample handling standard polypeptides, separation standard polypeptides, functionalization standard polypeptides, coupling standard polypeptides, and attachment standard polypeptides, may be derived from a biological source, such as an animal, plant, bacteria, fungi, protist, archaea, or virus. An internal standard may comprise polypeptides derived from a biological source that is orthogonal to a sample with which the internal standard polypeptides will be combined. For example, a human sample may be combined with internal standard polypeptides from a species of bacteria or plant. An internal standard, such as sample collection standard polypeptides, sample handling standard polypeptides, separation standard polypeptides, functionalization standard polypeptides, coupling standard polypeptides, and attachment standard polypeptides, may comprise polypeptides derived from a curated source. An internal standard may comprise polypeptides that are synthetic or artificial polypeptides. An internal standard may comprise a polypeptide with a modified or non-natural amino acid (e.g., a fluorescent amino acid, a biotinylated amino acid). An internal standard may

comprise an engineered polypeptide with a known or unknown biological function. An internal standard may comprise an engineered polypeptide with no known biological function. Engineered polypeptides may include polypeptides with amino acid sequences that are not known to exist in nature but are readily recognizable by a detection method (e.g., NAVTILVS, SAMPLEPREP, KEITHWILLIAMGNESHINPHDPATENTAGENT). An internal standard may comprise an isotopically labeled polypeptide where some atoms are replaced by isotopes to alter the molecular mass without altering other polypeptide properties. An internal standard may comprise a tag that is configured to make the internal standard readily identifiable or detectable during an assay. A tag may include a polypeptide tag (e.g., a peptide tag, a covalent peptide tag, a protein tag, etc.) or an oligonucleotide tag. A polypeptide array may comprise two or more differing types of internal standards (e.g., a functionalization standard and a purification standard), in which each type of standard is labeled with a differing tag that readily identifies the type of standard on the polypeptide array.

In some configurations, a sample may comprise one or more polypeptides or non-polypeptide compounds that may be utilized as internal standards. Depending upon sample types, certain polypeptide or non-polypeptide species may be present in a sample in a predictable fashion and may be present as impurities, inerts, or passengers during a polypeptide sample preparation process or a substituent step thereof. The presence or absence of the polypeptide or non-polypeptide species may be qualified or quantified after a polypeptide sample preparation process or a substituent process thereof to determine the outcome of the overall process or the substituent step.

A polypeptide standard may have a particular sequence length. A polypeptide standard may be at least about 10, 20, 30, 40, 50, 60, 70, 80, 90, 100, 125, 150, 200, 250, 300, 350, 400, 450, 500, 1000, 2000, 3000, 4000, 5000, 6000, 7000, 8000, 9000, 10000, 15000, 20000, 30000 or more than 30000 amino acid residues in length. Alternatively or additionally, a polypeptide standard may be no more than about 30000, 20000, 15000, 10000, 9000, 8000, 7000, 6000, 5000, 4000, 3000, 2000, 1000, 500, 450, 400, 350, 300, 250, 200, 150, 125, 100, 90, 80, 70, 60, 50, 40, 30, 20, 10, or less than 10 amino acids in length. A polypeptide standard may have a molecular weight of at least about 500 Daltons (Da), 1 kiloDalton (kDa), 5 kDa, 10 kDa, 20 kDa, 30 kDa, 40 kDa, 50 kDa, 60 kDa, 70 kDa, 80 kDa, 90 kDa, 100 kDa, 200 kDa, 300 kDa, 400 kDa, 500 kDa, 600 kDa, 700 kDa, 800 kDa, 900 kDa, 1 megaDalton (MDa), 2 MDa, 3 MDa or more than 3 MDa. Alternatively or additionally, a polypeptide standard may have a molecular weight of no more than about 3 MDa, 2 MDa, 1 MDa, 900 kDa, 800 kDa, 700 kDa, 600 kDa, 500 kDa, 400 kDa, 300 kDa, 200 kDa, 100 kDa, 90 kDa, 80 kDa, 70 kDa, 60 kDa, 50 kDa, 40 kDa, 30 kDa, 20 kDa, 10 kDa, 5 kDa, 1 kDa, 500 Da, or less than 500 Da. An internal standard may comprise a plurality of polypeptides with a known length or weight distribution. For example, an internal standard may comprise a monomodal distribution of proteins from a weight of about 1 kDa to 20 MDa, with a peak near 500 kDa. Alternatively, an internal standard may comprise a polymodal distribution with groupings of polypeptides between 1 kDa to 50 kDa, 50 kDa to 250 kDa, 250 kDa to 1 MDa, and 1 MDa to 20 mDa.

Alternatively or additionally, internal standards may be composed with polypeptide distributions reflecting other polypeptide properties or characteristics. Internal standards may contain mixtures of polypeptides distinguished by properties such as hydrophobicity, hydrophilicity, isoelectric point, folded/misfolded, modification state (e.g., phosphorylated/unphosphorylated; inquinated/non-inquinated), pKa, mass, charge to mass ratio, presence or absence of post-translational modifications, solubility in aqueous solvents, membrane solubility, etc. Internal standards may be composed to approximate or imitate an expected property distribution for a polypeptide fraction or sample polypeptide derived from a sample. Internal standards may be composed to approximate or imitate an expected property distribution for a polypeptide fraction or sample polypeptide derived from a process set forth herein. Internal standards may be composed to deviate from an expected property distribution for a polypeptide fraction derived from a sample or process set forth herein. For a given polypeptide property or characteristic (e.g., size, weight, isoelectric point, etc.), a polypeptide distribution in an internal standard may follow any type of distribution, such as a Poisson distribution, binomial distribution, beta-binomial distribution, hypergeometric distribution, or bimodal distribution.

An internal standard may further comprise non-polypeptide components, such as small molecule compounds or non-polypeptide macromolecules. Non-polypeptide internal standard components may be selected as analogs or proxies for polypeptides or may be selected as representative of chemical and/or physical properties of polypeptides. For example, non-polypeptide components (e.g., polymeric nanoparticles) may be prepared to represent an expected range of one or more polypeptide properties, such as size, hydrodynamic radius, hydrophobicity, hydrophilicity, surface charge density, etc. Non-polypeptide internal standards may include chemical indicators (e.g., pH indicators, oxygen or radical scavengers) that provide measurable or detectable information on sample conditions. A non-polypeptide internal standard component may comprise an inert, undetectable, or orthogonal species that is not detected in a sample unless experiencing deleterious or detrimental environmental conditions (i.e., a "canary-in-the-coal-mine" compound). For example, an internal standard may comprise a branched or dendrimeric compound comprising cleavable fragments under certain conditions (e.g., high or low pH). The cleavable fragments may only be detected (e.g., by mass spectrometry, IR, UV, fluorescence, etc.) if released by cleavage of the compound. In another example, an internal standard may comprise a compound that is initially inert or undetectable but, under certain conditions, reacts to form a detectable compound (e.g., forming a fluorophore). The newly formed detectable compound may form a detectable species that is orthogonal to other detectable species utilized on a polypeptide assay platform (e.g., forming a fluorophore with an

emission wavelength that is unique from other utilized fluorophores). A non-polypeptide internal standard may comprise a designed or engineered compound that provides multiple reporters for different conditions. An internal standard may comprise a branched or dendrimeric compound comprising polypeptides and non-polypeptide standards, thereby limiting the amount of required polypeptides or other compounds to be supplied in a polypeptide internal standard.

An internal standard may be added to a sample or a polypeptide fraction derived from a sample at any point prior to or during the preparation of a polypeptide fraction, including, for example, prior to or during sample collection, sample handling, sample storage, polypeptide separation, polypeptide purification, polypeptide composite formation, attachment of a polypeptide to a solid support and polypeptide composite deposition. A polypeptide fraction may comprise a plurality of internal standards, where each internal standard of the plurality of internal standards is added before or during a particular processing step, or is added for use of characterizing a particular processing step. As such, some internal standards may be referred to herein with reference to the processing step for which they are utilized. For example, a polypeptide assay may utilize sample collection internals standards, sample storage internal standards, sample separation internal standards, sample chromatography internal standards, sample extraction internal standards, sample functionalization internal standards, sample, coupling internal standards, and binding internal standards. In some cases, a polypeptide fraction may comprise a single internal standard that is added at a particular process during the preparation of a polypeptide fraction.

Prior to use as an internal standard, the behavior of an internal standard may be characterized for each known or anticipated processing step in a sample preparation method. The behavior characterization may include characterizations of component loss, component degradation, and process efficiency (e.g., percentage of polypeptides functionalized during a functionalization reaction). For internal standards comprising multiple types or species of polypeptides, the behavior characterizations may be evaluated as a function of the types or species of polypeptides. For example, percentage of internal standard polypeptides functionalized during a functionalization reaction may be provided as a function of polypeptide property (e.g., size, weight, isoelectric point, etc.). Process efficiencies for each known step of a polypeptide preparation process (e.g., separation, functionalization, coupling to anchoring groups, coupling to a solid support) may be characterized as a bulk property of an internal standard, or as a function of polypeptide distribution within an internal standard.

The behavior of an internal standard during a sample preparation method may be correlated to the behavior of a sample during the sample preparation method. In some cases, the behavior of an internal standard may be directly proportional to the behavior of a sample during a sample preparation method. For example, on a weight basis, the percentage of sample polypeptides recovered from a separation process may be proportional to the percentage of internal standard polypeptides recovered from the separation process. In another example, a known property of an internal standard can be useful in attributing a similar property to sample polypeptides that co-fractionate with the internal standard. In other cases, the behavior of an internal standard, such as sample collection internals standards, sample storage internal standards, sample separation internal standards, sample functionalization internal standards, sample, coupling internal standards, and binding internal standards, may be non-proportional to the behavior of a sample during a sample preparation method. For example, on a mass basis, the percentage of sample polypeptides recovered from a separation process may increase or decrease relative to the percentage of internal standard polypeptides recovered from the separation process.

In some cases, an overall efficiency of preparation for a polypeptide fraction derived from a sample preparation method may be calculated based upon efficiencies of individual steps as determined by the measurement of internal standards. An overall efficiency of preparation for a polypeptide for a polypeptide fraction during a sample preparation method may be calculated, for example, as a product of efficiencies for the N processing steps of the method. The overall efficiency may be expressed as a product of efficiencies for steps 1 to N, expressed as:

$$E_O = \prod_1^N (E_n)^\alpha \qquad (1)$$

where $E_O$ is the overall efficiency, $E_n$ is the efficiency of the nth step of the sample preparation method (e.g., collection, storage, separation of polypeptides, coupling of anchoring groups, coupling to solid support, etc.), and $\alpha$ is a proportionality exponent. When the proportionality exponent, $\alpha$, has a value of 1, this indicates that the behavior of the sample in processing step n is proportional to the behavior of the internal standard during the processing step. When the proportionality exponent, $\alpha$, has a value greater than 1, this indicates that processing step is less efficient for the sample than the internal standard. When the proportionality exponent, $\alpha$, has a value less than 1, this indicates that processing step is more efficient for the sample than the internal standard. Equation 1 may be utilized to estimate changes in overall efficiency for sample preparation when processing methods are altered (e.g., adding steps, removing steps, changing processes, etc.).

An internal standard polypeptide, such as a separation standard polypeptide or a coupling standard polypeptide may comprise a detectable label. A detectable label may be a pendant group that is coupled or conjugated to an internal

standard polypeptide (e.g., a fluorophore) or may be directly incorporated within the polypeptide (e.g., a radiolabel or atomic isotope). A detectable label coupled, conjugated, or incorporated with an internal standard polypeptide may comprise a fluorescent group, a luminescent group, a phosphorescent group, an enzyme, a radiolabel, a moiety comprising a non-standard isotope (e.g., heavy or light isotope), or a nucleic acid barcode. In some configurations, a detectable label may comprise a functional group comprising one or more non-standard isotopes that are readily detectable (e.g., by NMR or mass spectrometry). In some configurations, a detectable label may comprise an orthogonal functional group that would provide specific identification or analysis of an internal standard polypeptide. In some configurations, an internal standard polypeptide may comprise a fluorescent labeling group selected from the group consisting of FITC, Alexa Fluor® 350, Alexa Fluor® 405, Alexa Fluor® 488, Alexa Fluor® 532, Alexa Fluor® 546, Alexa Fluor® 555, Alexa Fluor® 568, Alexa Fluor® 594, Alexa Fluor® 647, Alexa Fluor® 680, Alexa Fluor® 750, Pacific Blue, Coumarin, BODIPY FL, Pacific Green, Oregon Green, Cy3, Cy5, Pacific Orange, TRITC, Texas Red, R-Phycoerythrin, and Allophycocyanin (APC). In some cases, the label may be an Atto dye, for example Atto 390, Atto 425, Atto 430, Atto 465, Atto 488, Atto 490, Atto 495, Atto 514, Atto 520, Atto 532, Atto 540, Atto 550, Atto 565, Atto 580, Atto 590, Atto 594, Atto 610, Atto 611, Atto 612, Atto 620, Atto 633, Atto 635, Atto 647, Atto 655, Atto 680, Atto 700, Atto 725, Atto 740, Atto MB2, Atto Oxa12, Atto Rho101, Atto Rho12, Atto Rho13, Atto Rho14, Atto Rho3B, Atto Rho6G, or Atto Thio12.

In some cases, the resulting composition of a sample preparation method may be a polypeptide array comprising a plurality of sample polypeptides and internal standard polypeptides coupled to a solid support with an overall efficiency of sample polypeptide capture that can be calculated based upon the internal standards. In some configurations, the composition may comprise a plurality of polypeptides, where the plurality of polypeptides comprises a plurality of sample polypeptides, a plurality of separation standard polypeptides, and a plurality of coupling standard polypeptides. The composition may further comprise a plurality of anchoring groups, where each polypeptide of the plurality of polypeptides may be coupled to an anchoring group of the plurality of anchoring groups to form a plurality of polypeptide composites, each of the polypeptide composites including a polypeptide from the plurality of polypeptides and an anchoring group from the plurality of anchoring groups. Further, the composition may comprise a solid support, where each anchoring group of the plurality of anchoring groups may be coupled to the solid support. When composed, the plurality of sample polypeptides may be coupled to the solid support with an overall sample preparation efficiency that may be calculated based upon a separation efficiency and a coupling efficiency, where the separation efficiency may be calculated as a function of the plurality of separation standard polypeptides, and wherein the coupling efficiency is calculated as a function of the plurality of coupling standard polypeptides. In some configurations, the overall sample preparation efficiency is linearly proportional to the separation efficiency, the coupling efficiency, or a combination of both (e.g., $\alpha$ is 1 in Equation 1). In some configurations, the overall sample preparation efficiency is non-linearly proportional to the separation efficiency, the coupling efficiency, or a combination of both (e.g., $\alpha$ is greater or less than 1 in Equation 1).

A polypeptide array may comprise an assay internal standard, in which the assay intenal standard is configured to provide information on the outcome of the assay or a step thereof. For example, a polypeptide assay utilizing a denaturation step may include a denaturation internal standard comprising a ladder of polypeptides containing a same epitope with differing degrees of epitope accessibility (i.e., ranging from fully buried within a tertiary structure to exposed at the surface), in which success of the denaturation step is determined by relatively uniform detection of the same epitope for each polypeptide of the ladder of polypeptides. In another example, an internal standard may comprise a protein containing one or more disulfide bridges, in which presence or absence of a disulfide bridge (e.g., due to an oxidation or reduction step of a polypeptide assay) is determined by detection or lack thereof of a known epitope buried or exposed by the presence or absence of the disulfide bridge. In another example, a fluorosequencing assay may comprise an internal standard peptide for each sequenced amino acid that is configured to provide information on the rate of false positive or false negative detections of the sequenced amino acid.

An internal standard may comprise a plurality of polypeptides, in which the plurality of polypeptides is characterized by a range of chemical, physical, and/or biological diversity, such as epitope diversity (e.g., all or a subset of all 8000 possible trimer epitopes), net electrical charge diversity (e.g., ranging from positive to negative net charge), mass diversity (e.g., ranging from peptide to ultra-high molecular weight), hydrodynamic radius diversity, species diversity (e.g., different variants of a same polypeptide from different host organisms), proteoform diversity (e.g., all known proteoforms of a polypeptide), genetic diversity (e.g., all or a subset of all polypeptides derived from all single-nucleotide polymorphisms of a gene), or any other conceivable measure of chemical, physical, or biological diversity.

An internal standard may comprise a plurality of polypeptides, in which the plurality of polypeptides comprises an affinity agent characterization standard. An affinity agent characterization standard may comprise a plurality of polypeptides that are utilized to measure and/or characterize a binding property of an affinity agent, such as a binding specificity, binding affinity, and/or binding promiscuity. An affinity agent characterization standard may comprise a plurality of polypeptides that is utilized across a plurality of assays to provide increased characterization of a known affinity agent or initial characterization of a new or unknown affinity agent.

An internal standard may comprise a quantitation standard. A quantitation standard may be configured to provide a measure of dynamic range for a polypeptide assay. For example, a quantitation standard may be used to determine a

dynamic range of a single-molecule polypeptide assay (e.g., polypeptide identification, polypeptide sequencing, etc.). A quantitation standard may comprise a standard polypeptide with a known concentration, in which the standard polypeptide has an expected quantity on a polypeptide array when deposited. For example, a quantitation may comprise a standard polypeptide whose expected abundance on a polypeptide array is expected to match a particular dynamic range of polypeptides from a sample (e.g., single molecule, tens of molecules, hundreds of molecules, etc.). In some cases, a quantitation standard may comprise a concentration ladder of polypeptides, in which each type of polypeptides in the ladder of polypeptides is used to confirm the detection sensitivity of a particular portion of a dynamic range for detection. In some cases, a quantitation standard may comprise a high-concentration polypeptide that provides an alternative form of quantitation. For example, a polypeptide array may comprise a high-concentration quantitation standard that can be quantitated by an alternative assay such as a Western Blot. In some cases, a quantitation standard may comprise a fluorescent protein (e.g.,, green fluorescent protein, red fluorescent protein, yellow fluorescent protein, etc.). A fluorescent protein may be conjugated or coupled to a quantitation standard polypeptide to provide a detectable label that makes the quantitation standard polypeptide readily identifiable on a polypeptide array.

A polypeptide array may comprise a fraction of internal standard moieties, as measured by a fraction of array addresses containing an internal standard moiety, or as measured by a fraction of internal standard moieties relative to all moieties coupled to a polypeptide array. A polypeptide array may have an internal standard fraction of at least about 0.000000001, 0.000000005, 0.00000001, 0.00000005, 0.0000001, 0.0000005, 0.000001, 0.000005, 0.00001, 0.00005, 0.0001, 0.0005, 0.001, 0.005, 0.01, 0.05, 0.1, 0.5, 0.9, or greater than 0.9. Alternatively or additionally, a polypeptide array may have an internal standard fraction of no more than about 0.9, 0.5, 0.1, 0.05, 0.01, 0.005, 0.001, 0.0005, 0.0001, 0.00005, 0.00001, 0.000005, 0.000001, 0.0000005, 0.0000001, 0.00000005, 0.00000001, 0.000000005, 0.000000001, or less than 0.000000001.

**Polypeptide Isolation**

A sample may be processed or prepared to isolate a polypeptide fraction from a sample. Methods and compositions may be exemplified below with reference to proteins; however, it will be understood that the examples can be extended to peptides or other polypeptides. A sample may be processed to isolate some or all available polypeptides within a sample. A sample may be processed to isolate a subset of all available polypeptides within a sample. A sample with unknown characteristics may be processed or prepared according to a polypeptide isolation method even if no polypeptides are found to exist in the sample after characterization. For example, a sterilized instrument may be swabbed for residual proteins, with the swabbings to be prepared and analyzed for possible protein contamination.

A sample preparation process may isolate or capture polypeptides from a sample with a known efficiency. A sample preparation process may isolate about 0.000001%, 0.00001%, 0.0001%, 0.001%, 0.01%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, 99.9%, 99.99%, 99.999%, or more than 99.999% of the available polypeptides within a sample on a molar or mass basis. A sample preparation process may isolate at least about 0.000001%, 0.00001%, 0.0001%, 0.001%, 0.01%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, 99.9%, 99.99%, 99.999%, or more than about 99.999% of the available polypeptides within a sample on a molar or mass basis. Alternatively or additionally, a sample preparation process may isolate no more than about 99.999%, 99.99%, 99.9%, 99%, 95%, 90%, 85%, 80%, 75%, 70%, 65%, 60%, 55%, 50%, 45%, 40%, 35%, 30%, 25%, 20%, 15%, 10%, 5%, 1%, 0.5%, 0.1%, 0.01%, 0.001%, 0.0001%, 0.00001%, 0.000001% or less than about 0.000001% of the available polypeptides within a sample on a molar or mass basis.

A sample preparation process may isolate or capture a subset of polypeptides from a sample with a known efficiency. A subset of polypeptides may comprise a specific or targeted group of polypeptides, e.g., membrane proteins, soluble proteins, nuclear proteins, cytoplasmic proteins, extracellular proteins, or blood plasma proteins. A sample preparation process may isolate about 5%, 10%, 15%, 20%, 25%, 30%, 35%, 40%, 45%, 50%, 55%, 60%, 65%, 70%, 75%, 80%, 85%, 90%, 95%, 99%, 99.9%, 99.99%, 99.999%, or more than 99.999% of a subset of available polypeptides within a sample on a sequence composition, molar or mass basis. A sample preparation process may isolate at least about 5%, 10%, 15%, 20%, 25%, 30%, 35%, 40%, 45%, 50%, 55%, 60%, 65%, 70%, 75%, 80%, 85%, 90%, 95%, 99%, 99.9%, 99.99%, 99.999%, or more than about 99.999% of a subset of available polypeptides within a sample on a sequence composition, molar or mass basis. Alternatively or additionally, a sample preparation process may isolate no more than about 99.999%, 99.99%, 99.9%, 99%, 95%, 90%, 85%, 80%, 75%, 70%, 65%, 60%, 55%, 50%, 45%, 40%, 35%, 30%, 25%, 20%, 15%, 10%, 5%, or less than about 5% of a subset of available polypeptides within a sample on a sequence composition, molar or mass basis.

A sample preparation process may isolate or capture a sufficient quantity of polypeptides for a polypeptide assay. An isolated polypeptide fraction may consist of at least about 0.1 picograms (pg), 0.5 pg, 1 pg, 5 pg, 10 pg, 50 pg, 100 pg, 500 pg, 1 nanogram (ng), 5 ng, 10 ng, 15 ng, 20 ng, 25 ng, 30 ng, 40 ng, 50 ng, 75 ng, 100 ng, 200 ng, 250 ng, 500 ng, 750 ng, 1 microgram ($\mu$g), 5 $\mu$g, 10 $\mu$g, 20 $\mu$g, 50 $\mu$g, 100 $\mu$g, or more than about 100 $\mu$g. Alternatively or additionally, an isolated

polypeptide fraction may consist of no more than about 100 μg, 50 μg, 20 μg, 10 μg, 5 μg, 1 μg, 750 ng, 500 ng, 250 ng, 200 ng, 100 ng, 75 ng, 50 ng, 40 ng, 30 ng, 20 ng, 15 ng, 10 ng, 5 ng, 1 ng, 500 pg, 100 pg, 50 pg, 10 pg, 5 pg, 1 pg, 0.5 pg, 0.1 pg, or less than 0.1 pg.

An isolated polypeptide fraction may have a characterizable polypeptide diversity. The polypeptide diversity of a polypeptide fraction may be characterized as being homogeneous or heterogeneous. A polypeptide fraction with homogeneous polypeptide diversity may be comprised almost exclusively of a single protein species. A polypeptide fraction may be considered to have heterogeneous polypeptide diversity if it consists of more than one polypeptide species. A polypeptide fraction may be considered to have homogeneous polypeptide diversity if it contains a single polypeptide species above a threshold value on a mass or molar basis. A polypeptide fraction may be considered to have heterogeneous polypeptide diversity if it contains no single polypeptide species above a threshold value on a mass or molar basis. A polypeptide fraction with homogeneous polypeptide diversity may contain a single polypeptide species above a threshold value of about 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, 99.99%, 99.999%, 99.9999%, or about 99.99999% on a mass or molar basis. A polypeptide fraction with homogeneous polypeptide diversity may contain a single polypeptide species above a threshold value of at least about 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, 99.99%, 99.999%, 99.9999%, or greater than about 99.99999% on a mass or molar basis. Alternatively or additionally, a polypeptide fraction with homogeneous polypeptide diversity may contain a single polypeptide species above a threshold value of no more than about 99.99999%, 99.9999%, 99.999%, 99.99%, 99.9%, 99.8%, 99.7%, 99.6%, 99.5%, 99.4%, 99.3%, 99.2%, 99.1%, 99%, 98%, 97%, 96%, 95%, 94%, 93%, 92%, 91%, 90%, or less than 90% on a mass or molar basis.

A sample may undergo one or more purification processes to remove one or more waste fractions comprising impurities (polypeptide or non-polypeptide) or sample fractions that are not of interest, thereby yielding an isolated or purified polypeptide fraction. An isolated polypeptide fraction may be substantially free of impurities. For example, an isolated polypeptide fraction may be substantially free of one, some or all species of impurities set forth herein or known in the art. In some cases an isolated polypeptide fraction may have a substantially reduced amount of one, some or all species of impurities set forth herein or known in the art. The scope of what constitutes an impurity may depend upon the nature of the polypeptide assay to be performed. Impurities may include small molecule components (e.g., amino acids, nucleotides, vitamins, coenzymes, glucose, etc.), other non-polypeptide biomolecules (e.g., lipids, nucleic acids, polysaccharides, etc.), salts, heavy metals, solvents, and non-biological chemicals (e.g., an inorganic impurity, an organic impurity, an acid, a base, a mineral, a polymer, a metal, a semiconductor, a ceramic, etc.). In some cases, an isolated polypeptide fraction may be free of a subset of polypeptides that are not relevant to the characterization to be performed. An isolated polypeptide fraction may be purified to remove unwanted polypeptides, such as degraded proteins, agglomerated proteins, damaged proteins, truncated proteins, low-molecular weight proteins or peptides, high-molecular weight proteins, organelle-specific proteins (e.g., membrane proteins, nuclear proteins, etc.), or specific species of polypeptides that are to be excluded from an analysis. Certain contaminants may be removed by an enzymatic, chemical or physiological method. In some cases, a sample may be prepared for a polypeptide assay with one or more impurities remaining. For example, in certain detection assays, the relative abundance of impurities may be unimportant provided a target polypeptide is still detectable within an impure polypeptide fraction.

An isolated polypeptide fraction may be analyzed for relative or absolute purity prior to or during a polypeptide assay set forth herein. Relative purity may be considered as the amount of one or more impurities within an isolated polypeptide fraction (e.g., nucleic acid content, metal content). Relative purity may be presented as an absolute amount (e.g., 10 parts per billion (ppb) iron) or an amount relative to a pre-purification level (e.g., iron at 0.001% of pre-purification amount). The pre-purification level can be determined as the amount of the one or more impurities in the original source for the isolated polypeptide fraction (e.g. biological fluid, cell, tissue etc.) or in an initial sample (e.g. blood sample) or extract (e.g. crude cell lysate) of the original source. The pre-purification level can be determined empirically or based on a known or suspected level. A polypeptide fraction may be considered free of an impurity if the impurity is below a detectable threshold for a standard method of measurement. An absolute impurity may be considered as the total amount of impurity in a polypeptide fraction on a dry mass basis (e.g., 1000 parts per million (ppm) or 0.1 wt % impurity) or on a molar basis. Purity of one or more polypeptides in an isolated polypeptide fraction can be determined relative to the amount of one or more standard polypeptide set forth herein.

An isolated polypeptide fraction may be purified until the abundances of one or more impurities falls below a threshold level. The threshold level may be an impurity level below which the impurity will not substantially interfere with the results of a polypeptide assay. The threshold level of an impurity may be about 0.1 ppb, 1 ppb, 10 ppb, 50 ppb, 100 ppb, 200 ppb, 500 ppb, 1 ppm, 5 ppm, 10 ppm, 50 ppm, 100 ppm, 500 ppm, 1000 ppm, or more than about 1000 ppm. The threshold level of an impurity may be at least about 0.1 ppb, 1 ppb, 10 ppb, 50 ppb, 100 ppb, 200 ppb, 500 ppb, 1 ppm, 5 ppm, 10 ppm, 50 ppm, 100 ppm, 500 ppm, 1000 ppm, or more than about 1000 ppm. Alternatively or additionally, the threshold level of a component may be about 1000 ppm, 500 ppm, 100 ppm, 50 ppm, 10 ppm, 5 ppm, 1 ppm, 500 ppb, 100 ppb, 50 ppb, 10 ppb, 5 ppb, 1 ppb, 0.5 ppb, 0.1 ppb or less than about 0.1 ppb.

An isolated polypeptide fraction may be purified until the abundance of one, some or all impurities falls below a threshold level. The threshold level may be an impurity level (e.g. a total impurity level) below which the impurities will not substantially interfere with the results of a polypeptide assay. The threshold level of one, some or all impurities may be about 10 weight percent (wt %), 9 wt%, 8 wt%, 7 wt%, 6 wt%, 5 wt%, 4 wt%, 3 wt%, 2 wt%, 1 wt%, 0.5 wt%, 0.1 wt%, 0.05 wt%, 0.01 wt%, 0.005 wt%, 0.001 wt%, 0.0005 wt%, 0.0001 wt%, 0.00005 wt%, 0.00001 wt%, or less. Alternatively or additionally, the threshold level of all impurities may be no more than about 10 weight percent (wt %), 9 wt%, 8 wt%, 7 wt%, 6 wt%, 5 wt%, 4 wt%, 3 wt%, 2 wt%, 1 wt%, 0.5 wt%, 0.1 wt%, 0.05 wt%, 0.01 wt%, 0.005 wt%, 0.001 wt%, 0.0005 wt%, 0.0001 wt%, 0.00005 wt%, 0.00001 wt%, or less than 0.00001 wt%.

A polypeptide fraction produced by a sample preparation process may comprise polypeptides of natural or synthetic origin. Polypeptides may contain post-translational modifications or post-synthesis modifications. In some cases, polypeptides may be treated to add or remove post-translational modifications or post-synthesis modifications. Such modifications can be made as part of a sample pretreatment method, such as those set forth elsewhere herein. Post-translational or post-synthesis modifications may include myristoylation, palmitoylation, isoprenylation, prenylation, farnesylation, geranylgeranylation, lipoylation, flavin moiety attachment, Heme C attachment, phosphopantetheinylation, retinylidene Schiff base formation, dipthamide formation, ethanolamine phosphoglycerol attachment, hypusine, beta-Lysine addition, acylation, acetylation, deacetylation, formylation, alkylation, methylation, C-terminus amidation, arginylation, polyglutamylation, polyglyclyation, butyrylation, gamma-carboxylation, glycosylation, glycation, polysialylation, malonylation, hydroxylation, iodination, nucleotide addition, phosphoate ester formation, phosphoramidate formation, phosphorylation, adenylylation, uridylylation, propionylation, pyrolglutamate formation, S-glutathionylation, S-nitrosylation, S-sulfenylation, S-sulfinylation, S-sulfonylation, succinylation, sulfation, glycation, carbamylation, carbonylation, isopeptide bond formation, biotinylation, carbamylation, oxidation, reduction, pegylation, ISGylation, SUMOylation, ubiquitination, neddylation, pupylation, citrullination, deamidation, elminylation, disulfide bridge formation, proteolytic cleavage, isoaspartate formation, racemization, and protein splicing.

A sample preparation process that is used to isolate a polypeptide fraction from a sample may vary depending upon the type of sample and the specific assay to be performed. In some cases, a sample may be prepared for a proteomic-scale assay, thus employing high-efficiency capture of the full proteome, or a substantial portion thereof, within the sample. In other cases, a sample may be prepared for a specific fraction of polypeptides, thus utilizing high-efficiency capture of that fraction of the polypeptides. Likewise, in some cases, a sample may be prepared for single-molecule quantitation, benefitting from high-purity polypeptide fractions. In other cases, a sample may be prepared to only determine the presence of one or more biomarkers, where the presence of impurities is of lesser concern. As such, sample preparation procedures may be varied with regard to the method of isolating polypeptides and the method of removing impurities.

Sample preparation to isolate or separate protein fractions may involve several steps such as those set forth herein. Certain steps that are set forth herein may be omitted, varied or reordered depending upon the nature of the sample to be prepared. For example, steps used to obtain a protein sample from a fabric for forensic purposes may be different than the steps used to obtain a full proteomic protein sample from a pellet of bacteria. There may be different methods of protein isolation or separation used for biological samples (e.g., cells, blood) as opposed to curated samples (e.g., textiles, rocks). There may also be different methods of protein isolation or separation for protein fractions derived from solid media (e.g., tissue, single cells, fabrics, filters) as opposed to liquid media (bodily fluids, water samples). A standard polypeptide can be added prior to or during any of the steps set forth herein or known in the art. The added standard polypeptide can be used to evaluate a characteristic or quantity of one or more polypeptides obtained using the step.

A procedure for protein isolation or separation from a biological sample may be configured to suit a particular sample, for example, based on characteristics such as whether polypeptides are possibly localized on or within a biological compartment (e.g., an organism, tissue, cell or organelle) or are free in solution (e.g., proteins in blood plasma or other biological fluid). For example, a blood sample may be prepared differently if all polypeptides (including those in red or white blood cells) are to be captured rather than only cell free polypeptides from blood plasma. **FIG. 2** depicts a flowchart for a general method of isolating polypeptide fractions from biological samples. The starting point in the process may depend upon whether polypeptides are to be derived from a sample containing polypeptides within a solid medium **200** or a sample in which free-solution polypeptides are to be retained **201.** For a sample in which polypeptides are to be derived from within a solid medium **200,** the sample **200** may be treated to break up or disrupt a cell wall if one is present. A cell wall may be disrupted **210** mechanically, enzymatically, or chemically. A cell wall may be disrupted **210** in the presence of a detergent. A cell wall may be disrupted **210** in the presence of a standard polypeptide, such as a cell wall disruption standard polypeptide. Also, for a sample in which polypeptides are to be derived from within a solid medium **200,** the sample **200** may be treated to lyse or disrupt a cell membrane if one is present. A cell membrane may be disrupted **220** mechanically (e.g., shearing, french press or sonication) or chemically (e.g., exposure of the cell to hypotonic or hyperionic solution, pH extremes, lytic enzymes or the like). A cell membrane may be disrupted **220** in the presence of a detergent. A cell membrane may be disrupted **220** in the presence of a standard polypeptide, such as a membrane disruption standard polypeptide. A cell wall or cell membrane can be disrupted using methods known in the art such as those set forth in Scopes, Protein Purification: Principles and Practice, 3rd Ed., Springer Advanced Texts in Chemistry (1993); Sambrook et

al., Molecular Cloning: A Laboratory Manual, 3rd edition, Cold Spring Harbor Laboratory, New York (2001) or in Ausubel et al., Current Protocols in Molecular Biology, John Wiley and Sons, Baltimore, MD (1998).

After a sample **200** has been sufficiently disrupted, the resulting material may include fragments of cell walls, cell membranes, nuclear membranes, organelles, capsids, nucleic acids, and other biological materials, as well as polypeptides that are suspended in solution. It may be necessary to extract additional polypeptides **230** from fragments of the disrupted sample **200** (e.g., membrane proteins, organelle proteins). The additional polypeptides may be extracted **230** in the presence of a detergent. The additional polypeptides may be extracted **230** in the presence of a standard polypeptide, such as an extraction standard polypeptide. After the extraction of additional polypeptides **230,** a polypeptide fraction may be separated, isolated, or precipitated **240** from the non-polypeptide fragments of the sample **230.** In some cases, the non-polypeptide fragments may be retained for one or more additional rounds of polypeptide separation and extraction. Any suitable method may be utilized for polypeptide separation, isolation or precipitation, including, for example, precipitation such as high salt or organic solvent based separations; centrifugation to separate membranes and insoluble matter from soluble matter; filtration to separate matter in solution from insoluble matter; liquid-phase extraction to separate matter based on solvation properties; solid-phase extraction to separate matter based on affinity for a solid support having predefined chemical properties; chromatography, such as affinity chromatography to separate matter based on affinity for a particular analyte, ionic exchange chromatography to separate matter based on ionic charge, size exclusion chromatography to separate matter based on size; hydrophobic exchange chromatography; reverse phase chromatography and other methods known in the art such as those set forth in Scopes Protein Purification: Principles and Practice, 3rd Ed., Springer Advanced Texts in Chemistry (1993). Separation, isolation, or precipitation **240** of a polypeptide fraction may occur in the presence of a detergent. Separation, isolation, or precipitation **240** of a polypeptide fraction may occur in the presence of a standard polypeptide, such as a separation standard polypeptide, isolation standard polypeptide, or precipitation standard polypeptide.

A particularly useful method for separating polypeptides of interest from other sample components is selective adsorption on insoluble supports such as resins, beads, particles or the like. For example, particles can be used to adsorb polypeptides based on attraction between the polypeptides and the surface of the nanoparticles. Polypeptides in a biological fluid, such as plasma, can form a polypeptide layer that coats the particles on the particle surface, forming a protein corona. The composition and quantity of corona polypeptides is a function of the physicochemical properties of the particles. Particles can be sleeted or engineered with surface properties that reproducibly produces variation in the corona in terms of identity and/or quantity of polypeptides. Particles can be contacted with a sample such that polypeptide coronas form on the particles. The polypeptide coated particles can be separated from the sample (e.g. a magnet can be used to attract paramagnetic or magnetic particles, gravity can be used to attract dense particles etc.) and then the polypeptides can be released for further processing. An exemplary super- paramagnetic iron oxide nanoparticle (SPION) can be coated with a thin layer of silica by a modified Stöber process using tetraethyl orthosilicate (TEOS). Other exemplary SPIONs can be coated with poly(dimethylaminopropyl methacrylamide) or poly(ethylene glycol). These and other particles, methods for making the particles and methods for their use are set forth in Blume et al. Nat. Commun. 11, 3662 (2020); US Pat. Nos. 10,866,242, 10,272,050, or 10,022,334; or US Pat. App. Pub. Nos. 2019/0350870 A1, 2020/0138728 A1 or 2020/0206145 . Another useful capture substrate is pyridinyl carboxaldehyde derivative attached to a resin or other particle. See, for example, Howard et al. ACS Chem. Biol. 15, 1401-1407 (2020).

The particles can be attached to a standard polypeptide and the standard polypeptide can be used to evaluate a characteristic of polypeptides that are known or expected to form a corona and/or the standard polypeptide can be used to evaluate a characteristic of polypeptides that are known or expected to be released from the corona. In some configurations, particles having different surfaces can be attached to unique standard polypeptides that indicate the type of surface the particle has, the type of polypeptide that is expected to form a corona on the particle, or the type of polypeptide that is expected to be released from the corona. The particles can be attached to standard polypeptides using any of a variety of attachment mechanisms known in the art or set forth herein, including for example, covalent bonding, non-covalent bonding, adsorption, or the like. A standard polypeptide can be attached to a particle before or after the particle being contacted with a sample. In some cases, a standard polypeptide can be present in a sample that is contacted with the particle, such that the standard polypeptide attaches to the particle, for example by adsorbing with sample polypeptides to form a polypeptide corona.

Following separation, isolation, or precipitation **240** of a polypeptide fraction, the polypeptide fraction may be further purified **250** to remove residual impurities, yielding a final purified polypeptide fraction **260.** Impurities removed **250** may include metals, salts, or remaining non-polypeptide biological fragments. Exemplary purification techniques may include, for example, those set forth above or in Scopes Protein Purification: Principles and Practice, 3rd Ed., Springer Advanced Texts in Chemistry (1993).

A purified polypeptide fraction **260** may be subjected to one or more additional rounds of purification to arrive at a substantially purified polypeptide fraction **260.** The skilled person will recognize that certain steps of the process shown in **FIG. 2** can be rearranged or run in parallel. For example, free-solution polypeptides may be separated **240** from non-polypeptide biological materials before polypeptides have been extracted from the non-polypeptide biological materials.

Separation of polypeptides from the non-polypeptide biological materials may occur in parallel after separation **240,** with extracted polypeptides combined with free-solution polypeptides before or after final purification **250.**

**FIG. 2** also depicts an exemplary separation process for samples in which free-solution polypeptides are to be selectively retained **201.** The sample **201** may be processed **235** to separate unwanted components from the free-solution polypeptides. Methods such as centrifugation, size-exclusion chromatography, liquid chromatography, other techniques set forth herein, or a combination thereof, may be utilized to separate unwanted components (e.g., cells) from the free-solution polypeptides. This separation may be utilized to remove components that greatly differ in size from the free-solution polypeptides. The processing **235** may occur in the presence of a detergent. The processing **235** may occur in the presence of a standard polypeptide, such as a separation standard polypeptide. After processing **235** the sample **201,** the purified fraction may be subjected to separation, isolation, or precipitation **240** of a polypeptide fraction. Any suitable method may be utilized for polypeptide separation, isolation or precipitation, including methods set forth above or elsewhere herein. Separation, isolation, or precipitation **240** of a polypeptide fraction may occur in the presence of a detergent. Separation, isolation, or precipitation **240** of a polypeptide fraction may occur in the presence of a standard polypeptide, such as a separation standard polypeptide, isolation standard polypeptide, or precipitation standard polypeptide. Following separation, isolation, or precipitation **240** of a polypeptide fraction, the polypeptide fraction may be further purified **250** to remove residual impurities, yielding a final purified polypeptide fraction **260.** Impurities removed **250** may include metals, salts, or remaining non-polypeptide biological fragments. Exemplary purification techniques may include methods set forth above or elsewhere herein. A purified polypeptide fraction **260** may be subjected to one or more additional rounds of purification to arrive at a substantially purified polypeptide fraction **260.**

**FIG. 3** depicts a process for isolating or separating a polypeptide fraction from a curated sample. The procedure for polypeptide isolation or separation from a curated or environmental sample may be configured based upon whether the sample **300** is in a solid or liquid medium. If the sample **300** comprises a liquid medium, the sample may be moved directly to a polypeptide extraction process **320.** If the sample **300** comprises a solid medium, the processing of the sample may be configured based upon whether the sample **300** may be altered. For example, a sample may be an artifact, forensic specimen or paleontological specimen. In another example, a polypeptide sample may be isolated from a solid surface such as an air filter or liquid filter without damaging the filter. If the sample can be altered, it may undergo a preliminary processing **305** that may improve the extraction of polypeptide from the solid sample. The preliminary processing **305** may include crushing, grinding, pulverization, heating, cooling, steam treatment, or chemical dissolution. After an optional preliminary processing step **305,** the sample **300** may be transferred into a solvent extraction process **310.** The solvent extraction process **310** may involve the use of one or more solvents to loosen or solvate polypeptides bound to the solid medium. The solvent extraction process **310** may involve additional extraction enhancers such as heat, denaturants, or mixing. A solvent mixture for a solvent extraction process **310** may include a detergent. A solvent mixture for a solvent extraction process **310** may include a standard polypeptide, such as an extraction standard polypeptide. The solvent extraction process **310** may result in a liquid medium containing free-solution polypeptides. A liquid medium containing free-solution polypeptides may be subjected to a polypeptide precipitation process **320.** The free-solution polypeptides in the liquid medium may be separated, isolated, or precipitated **320** from the liquid medium of the sample. In some cases, the liquid medium may be retained for one or more additional rounds of polypeptide separation and extraction. Any suitable method may be utilized for polypeptide separation, isolation or precipitation, including the methods listed above. Separation, isolation, or precipitation **320** of a polypeptide fraction may occur in the presence of a detergent. Separation, isolation, or precipitation **320** of a polypeptide fraction may occur in the presence of a standard polypeptide, such as a separation standard polypeptide, isolation standard polypeptide, or precipitation standard polypeptide. Following separation, isolation, or precipitation **320** of a polypeptide fraction, the polypeptide fraction may be further purified **330** to remove residual impurities, yielding a final purified polypeptide fraction **340.** Impurities removed **330** may include metals, salts, or remaining non-polypeptide biological fragments. Exemplary purification techniques may include the methods listed above. A purified polypeptide fraction **340** may be subjected to one or more additional rounds of purification to arrive at a final purified polypeptide fraction **340.**

Polypeptide fractions may be prepared to remove certain polypeptides. Polypeptides may be separated or excluded from a polypeptide fraction if they are larger than a particular size. Polypeptides may be separated or excluded from a polypeptide fraction if they are smaller than a particular size. A polypeptide fraction may be treated to reduce the size of large polypeptides, for example by protease treatment or hydrolysis. Polypeptides may be separated or excluded from a polypeptide fraction based on size by methods such as density-gradient centrifugation, size-exclusion chromatography, filtration, dialysis, gel electrophoresis or other methods set forth herein for polypeptide separation. A polypeptide fraction may be treated to generate a peptide fraction, wherein the average length of the peptides in the peptide fraction is substantially smaller than the average length of the polypeptides in the polypeptide fraction. Peptides generated from a polypeptide fraction may have an average size that is at least 50%, 25%, 10%, 5%, or less of the average size of the polypeptides in the polypeptide fraction. A polypeptide fraction may undergo one or more processes to alter the size distribution of a polypeptide fraction, for example protease treatment followed by a separation process such as size exclusion chromatography.

A sample comprising a plurality of polypeptides may be processed or prepared to produce one or more polypeptide fractions. A sample comprising a plurality of polypeptides may be processed or prepared to produce a plurality of polypeptide fractions. A plurality of polypeptide fractions may be produced such that each polypeptide fraction has a different physical attribute. For example, polypeptide fractions may be distinguished by comprising polypeptides of differing types (e.g., extracellular, membrane, cytoplasmic, etc.), differing size ranges, differing chemical properties (e.g., hydrophobicity), differing isoelectric points, polarity, pKa etc. A plurality of polypeptide fractions may be produced by separating fractions on the basis of a polypeptide property, such as polypeptide size, polypeptide isoelectric point, polarity, pKa, polypeptide hydrophobicity, polypeptide hydrodynamic radius, polypeptide state, or the presence or absence of post-translational modifications. A sample may be separated into about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100 or more than 100 polypeptide fractions. A sample may be separated into at least about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100 or more than 100 polypeptide fractions. Alternatively or additionally, a sample may be separated into no more than about 100, 95, 90, 85, 80, 75, 70, 65, 60, 55, 50, 45, 40, 35, 30, 25, 20, 15, 10, 9, 8, 7, 6, 5, 4, 3, 2 or less than 2 polypeptide fractions.

The preparation of polypeptide fractions from a sample may involve one or more separation processes that isolate or purify polypeptides from non-polypeptide sample components. The preparation of polypeptide fractions from a sample may involve one or more separation processes that separate a first plurality of polypeptides from a second plurality of polypeptides. Polypeptide fractions may be produced by any suitable method. A polypeptide fraction separation process may comprise a fractionation method, a targeting method, or a combination thereof. Fractionation methods may include bulk methods that separate polypeptides based upon polypeptide properties. Exemplary fractionation methods may include chromatographic methods (e.g., size exclusion chromatography, reverse-phase liquid chromatography, ion exchange chromatography, hydrophobic exchange chromatography, hydrophobic interaction chromatography, affinity chromatography, hydrophilic interaction liquid chromatography, mixed-mode chromatography, 2-dimensional liquid chromatography, etc.), centrifugation methods (e.g., ultracentrifugation, density gradient centrifugation, etc.), electrophoretic methods (e.g., 1-D or 2-D SDS-page gel electrophoresis, isoelectric focusing, free-flow electrophoresis, pulsed-field electrophoresis, field flow fractionation, etc.), solid phase adsorption, solid phase absorption, and precipitation methods (e.g., acetone precipitation, ethanol precipitation, methanol precipitation, salting-in, salting-out, hydrophilic polymer precipitation, polyelectrolyte flocculation, polyvalent cation precipitation, etc.). Targeting methods may include any separation method that separates a polypeptide species or group of polypeptide species from a plurality of polypeptide species with a desired specificity. Targeting methods may include the use of capture reagents or pull-down reagents to specifically target and isolate target polypeptides within a plurality of polypeptides. In some cases, a targeting method may utilize a capture reagent or a pull-down reagent that specifically couples with or binds to a subset of polypeptides or polypeptide species within a plurality of polypeptides or polypeptide species (e.g., antibodies, aptamers, etc.). In some cases, a targeting method may utilize a capture reagent or a pull-down reagent that is specifically coupled with or bound to a subset of polypeptides or polypeptide species within a plurality of polypeptides or polypeptide species (e.g., binding ligands, binding substrates).

In certain polypeptide assays, a polypeptide fraction may be displayed as an array of polypeptides on a solid support. A separation method for producing polypeptides as an array on a solid support may be configured to occur in the presence of the solid support. In some configurations, a solid support may be contained within a fluidic device such as a flow cell, microfluidic device, cartridge, or chip that is configured to facilitate a separation process for polypeptide sample preparation, such as a polypeptide fractionation method or a polypeptide targeting method. In some configurations, a solid support may be provided with a plurality of capture agents that are coupled to a surface of the solid support and are configured to couple to or bind with a polypeptide or polypeptide species. In other configurations, a plurality of polypeptides may be contacted with a pull-down reagent that is configured to couple to a solid support and is further configured to couple to or bind with a polypeptide or polypeptide species.

A polypeptide fraction may be produced by a targeting separation method. A targeting separation method may comprise the steps of: 1) contacting or combining a sample with a targeting agent that is configured to bind a sample polypeptide of a plurality of sample polypeptides; 2) coupling a sample polypeptide from the plurality of sample polypeptides to the targeting agent; 3) separating a coupled sample polypeptide and targeting agent from the separation mixture; and optionally 4) binding the sample polypeptide to a solid support. The targeting agent may comprise a capture reagent or a pull down reagent. In some configurations, the separation mixture may comprise a plurality of sample polypeptides and a plurality of separation standard polypeptides. In some configurations, the separation mixture may comprise a plurality of sample polypeptides, where the plurality of sample polypeptides comprises target sample polypeptides and non-target sample polypeptides. The targeting agent may be configured to couple to or bind with target sample polypeptides of the plurality of sample polypeptides. In some configurations, the targeting agent may comprise an anchoring group that is configured to couple with a solid support, or may be configured to couple with an anchoring group that is configured to couple with a solid support.

A targeting agent may be directly contacted with a sample to couple a polypeptide from the sample. For example,

a targeting agent may be contacted with a non-disrupted cell sample or a non-disrupted tissue sample (i.e., not homogenized or lysed) to directly remove a polypeptide from the cell sample or the tissue sample without further disruption of the cell or tissue. In some cases, a targeting separation method may comprise the steps of: 1) contacting or combining a sample with a targeting agent that is configured to bind a polypeptide; 2) coupling a polypeptide from the sample to the targeting agent; 3) separating a coupled polypeptide and targeting agent from the sample; and optionally 4) binding the polypeptide to a solid support. The targeting agent may comprise a capture reagent or a pull down reagent. In some configurations, the sample may comprise a plurality of sample polypeptides and a plurality of separation standard polypeptides. In some configurations, the sample may comprise a plurality of sample polypeptides, where the plurality of sample polypeptides comprises target sample polypeptides and non-target sample polypeptides. The targeting agent may be configured to couple to or bind with target sample polypeptides of the plurality of sample polypeptides. In some configurations, the targeting agent may be configured to couple with a solid support, or may be configured to couple with an anchoring group that is configured to couple with a solid support.

**FIGs. 4A - 4F** depict various methods for utilizing targeting to isolate a polypeptide fraction from a sample. **FIGs. 4A - 4B** depict a pull down method for targeting polypeptides in a plurality of polypeptides derived from a sample. The plurality of polypeptides may comprise target polypeptides **420** that contain a target moiety **425** (e.g., an epitope, an amino acid sidechain, a functional group, reactive handle, oligonucleotide, etc.) that is configured to couple to a capture reagent **440** comprising a coupling group **445** (e.g., affinity reagent, complementary functional group, complementary reactive handle, complementary oligonucleotide, etc.) that is configured to couple to the target moiety **425.** The plurality of polypeptides may also comprise non-target polypeptides **430.** The capture reagent **440** may be configured to couple to a solid support **410.** The plurality of polypeptides may be contacted with a plurality of capture reagents **440,** thereby allowing the coupling groups **445** of the capture reagents **440** to couple the target moiety **425** of the target polypeptides **420.** The capture reagents **440** that are coupled to the target polypeptides **420** may be coupled to the solid support **410,** thereby separating the target polypeptides **420** from the non-target polypeptides **430.** The non-target polypeptides **430** may be rinsed or washed from the solid support **410,** leaving behind a plurality of target polypeptides **420** coupled to the solid support **410.**

**FIGs. 4C - 4D** depict a pull down method for targeting polypeptides in a plurality of polypeptides derived from a sample. The plurality of polypeptides may comprise target polypeptides **420** that contain a target moiety **425** (e.g., an epitope, an amino acid sidechain, a functional group, reactive handle, oligonucleotide, etc.) that is configured to couple to a capture reagent **440** comprising a coupling group **445** (e.g., affinity reagent, complementary functional group, complementary reactive handle, complementary oligonucleotide, etc.) that is configured to couple to the target moiety **425.** The plurality of polypeptides may also comprise non-target polypeptides **430.** The capture reagent **440** may be coupled to a solid support **410.** The plurality of polypeptides may be contacted with a plurality of capture reagents **440** coupled to the solid support, thereby allowing the coupling groups **445** of the capture reagents **440** to couple the target moiety **425** of the target polypeptides **420,** thereby separating the target polypeptides **420** from the non-target polypeptides **430.** The non-target polypeptides **430** may be rinsed or washed from the solid support **410,** leaving behind a plurality of target polypeptides **420** coupled to the solid support **410.**

**FIGs. 4E - 4G** depict an indirect pull down method for targeting polypeptides in a plurality of peptides derived from a sample. The plurality of polypeptides may comprise target polypeptides **420** that contain a target moiety **425** (e.g., an epitope, an amino acid sidechain, a functional group, reactive handle, oligonucleotide, etc.) that is configured to couple to a capture reagent **441** comprising a coupling group **445** (e.g., affinity reagent, complementary functional group, complementary reactive handle, complementary oligonucleotide, etc.) that is configured to couple to the target moiety **425.** The plurality of polypeptides may also comprise non-target polypeptides **430.** The capture reagent **441** may be coupled to a solid support **410.** The plurality of polypeptides may be contacted with a plurality of transfer pull down reagents **440.** In some configurations, the transfer pull down reagents **440** may comprise a binding target **448** (e.g., a small molecule substrate, a nucleic acid, a polypeptide, etc.) that binds the target polypeptide **420.** The binding target **448** may bind a target polypeptide **420** then be transported to a surface of the solid support **410** where the target polypeptides may contact the capture reagents **441.** The coupling group **445** of the capture reagent **440** may couple to the target moiety **425** of the target polypeptide **420,** thereby separating the target polypeptide **420** from the non-target polypeptides **430.** The non-target polypeptides **430** may be rinsed or washed from the solid support **410,** leaving behind a plurality of target polypeptides **420** coupled to the solid support **410.**

Targeting assays that utilize a capture agent or pull down reagent may comprise any suitable components for the selective removal of target polypeptides from a plurality of sample polypeptides. The capture agent or pull down agent may comprise a retaining component and one or more coupling components. The retaining component may provide a structure or scaffold for providing a necessary utility to a capture agent or a pull down reagent. A retaining component may comprise a body that can be free in a liquid medium, such as a particle or nanoparticle. In some cases, a retaining component may comprise a particle such as an organic nanoparticle (e.g., polymers, biopolymers, dendrimers, hydrogels, micelles, liposomes, colloids, carbon nanoparticles, etc.), an inorganic nanoparticle (e.g., $SiO_2$ nanoparticles, $Fe_2O_3$ nanoparticles, quantum dots, etc.), or a biomolecule (e.g., a cellulose nanoparticle, a structured nucleic acid particle, a polypeptide, etc.).

In some cases, a polymer retaining component may comprise an anionic polymer or a cationic polymer. In some cases, a biopolymer retaining component may comprise a polysaccharide, a polypeptide (e.g., a native or engineered protein), or an oligonucleotide. In some cases, a retaining component may comprise a structured nucleic acid particle (SNAP), such as a nucleic acid origami or a nucleic acid nanoball. A retaining component may further comprise a layer, shell, or coating that alters the properties of the retaining component. For example, a retaining component particle may comprise a metal, polymer or biopolymer coating, or a surface layer of functional groups that are configured to permit coupling or conjugation of other moieties to the particle surface. A retaining component, such as a polymer or biopolymer, may be structured to have an increased surface electrical charge. For example, a protein may be modified to have a larger surface electrical charge than a native protein. A retaining component may be configured to couple to a solid support by a covalent or non-covalent interaction. In some configurations, a capture reagent or pull down reagent may comprise an anchoring group that is configured to couple a polypeptide to a solid support. A retaining component may comprise a fixed support or phase, such as a resin. A fixed retaining component may be configured as, for example, an affinity chromatography medium.

A capture agent or pull down reagent may further comprise coupling groups that are configured to couple a polypeptide to the capture agent or pull down reagent. Coupling groups may be configured to form a covalent or non-covalent interaction with a polypeptide. A coupling group may comprise a functional group or reactive handle that is configured to form a covalent bond with a functional group or reactive handle on a polypeptide. A reactive handle on a capture reagent or pull down reagent may comprise a functional group that is configured to undergo a click reaction. A coupling group may include a group that is configured to form a non-covalent interaction with a polypeptide or a group coupled to a polypeptide. Exemplary non-covalent interactions may include nucleic acid oligonucleotide hybridization and receptor-ligand or binder-small molecule pairs, such as streptavidin-biotin, glutathione-glutathione-S-transferase, maltose-maltose binding protein, and chitin-chitin binding protein. In some configuration, a coupling group may comprise a binding ligand that selectively scavenges targeted polypeptides from a sample. For example, polypeptides with specific binding functions, such as G-proteins, transcription factors, promoter polypeptides, repressor polypeptides, and histones, may be targeted by capture reagents or pull down reagents displaying nucleic acid sequences that are specific to the binding polypeptide. A targeting agent, such as a capture reagent or pull down reagent, may be configured to couple to one or more internal standard polypeptides of a plurality of internal standard polypeptides, such as sample collection standard polypeptides, sample handling standard polypeptides, separation standard polypeptides, functionalization standard polypeptides, coupling standard polypeptides, and attachment standard polypeptides.

A targeting agent, such as a capture reagent or pull down reagent, may be configured to couple with a sample polypeptide by a chemical linkage, such as a chemical bond, a catalyzed bond, or an enzymatically-catalyzed linkage. A capture reagent or pull down reagent may comprise one or more chemically-modifying groups, such as post-translational modification groups (e.g., SUMO, ubiquitin, acetyl, formyl, phosphate, glycans, isoprenoids, etc.) that are coupled or conjugated to the capture reagent or pull down reagent. The capture reagent or pull down reagent comprising the chemically-modifying group may be combined with a sample polypeptide in the presence of an enzyme (e.g., ubiquitin-E3 ligase, acetylase, isoprenoid transferase, etc.) that is configured to catalyze attachment of the sample polypeptide to the chemically-modifying group at any available attachment site. A sample polypeptide that is available for a chemical modification may become linked to the capture reagent or pull down reagent, thereby permitting it to be isolated from a sample.

A capture reagent or pull down reagent for a targeting separation method may be configured to couple to one or more polypeptides. A capture reagent or pull down reagent may be configured to couple to a plurality of polypeptides. A capture reagent or pull down reagent for a targeting separation method may be configured to couple to about 1, 2, 3, 4, 5, 6, 7, 8, 9, 10, 15, 20, 25, 30, 35, 40, 45, 50, 60, 70, 80, 90, 100, 125, 150, 175, 200, 250, 300, 400, 500, 600, 700, 800, 900, 1000, or more than 1000 polypeptides. A capture reagent or pull down reagent for a targeting separation method may be configured to couple to at least about 1, 2, 3, 4, 5, 6, 7, 8, 9, 10, 15, 20, 25, 30, 35, 40, 45, 50, 60, 70, 80, 90, 100, 125, 150, 175, 200, 250, 300, 400, 500, 600, 700, 800, 900, 1000, or more than 1000 polypeptides. Alternatively or additionally, a capture reagent or pull down reagent for a targeting separation method may be configured to couple to no more than about 1000, 900, 800, 700, 600, 500, 400, 300, 250, 200, 175, 150, 125, 100, 90, 80, 70, 60, 50, 40, 30, 25, 20, 15, 10, 9, 8, 7, 6, 5, 4, 3, 2 or less than 2 polypeptides.

In some cases, a capture reagent or a pull down reagent may be configured to act as a transfer agent or intermediary for targeting polypeptides during a separation process. A transfer agent or intermediary may couple to a polypeptide, then transfer the polypeptide to an anchoring group that is coupled to a solid support, or configured to be coupled to a solid support. **FIGs. 4E - 4G** depict an example of a transfer agent. A transfer agent or intermediary may release a polypeptide to the anchoring group, or may be configured to bind to the anchoring group, thereby coupling the polypeptide to the anchoring group.

A capture reagent or a pull down reagent utilized for a targeting separation method may degrade during usage. For example, a capture agent utilizing a nucleic acid capture agent may be degraded by the presence of particular enzymes when contacted with some cellular or lysate samples (e.g., restriction enzymes). A capture reagent or a pull down reagent may be designed to degrade during usage. Degradation can optionally be initiated by application of a degrading

reagent (e.g. oxidant, reducing agent, enzyme) or stimulus (e.g. light or heat). For example, a capture agent comprising photocleavable linkers may degrade into subcomponents upon application of radiation of a particular wavelength. In another example, a nucleic acid-based capture agent can be degraded by an enzyme such as a nuclease or by a chemical agent such as piperidine. The degradable capture reagent or pull down reagent may be degraded before, during, or after contact with a plurality of polypeptides. The degradable capture reagent or pull down reagent may be degraded before, during, or after contact with a solid support or a plurality of anchoring groups.

**FIGs. 5A - 5H** depict various configurations of capture reagents with degradable properties. **FIG. 5A** depicts a capture reagent comprising a plurality of subunits **540** (e.g., a structured nucleic acid particle, a cluster of linked nanoparticles). Each subunit **540** of the plurality of subunits comprises a coupling group **545** that is configured to couple with a polypeptide and a coupling group **548** that is configured to be coupled with a solid support or an anchoring group. The capture reagent comprising the plurality of subunits **540** is contacted with a plurality of polypeptides **520** comprising coupling groups **525** that are configured to be coupled by the polypeptide coupling groups **545** of the capture reagent. **FIG. 5B** shows the capture reagent subunits **540** with coupled polypeptides **520** after the capture reagent has undergone a degradation process (e.g., enzymatic degradation, photolytic degradation, chemical degradation, etc.). The polypeptide-coupled subunits **540** may be contacted with a solid support **510** comprising a plurality of anchoring groups **530** with coupling groups **535** that are configured to couple with the coupling group **548** of the capture reagent subunit **540.** As shown in **FIG. 5C,** after contacting, the subunits **540** may be coupled to the anchoring groups **530,** thereby coupling the polypeptides **520** to the solid support **510.**

**FIGs. 5D - 5F** show an example of a capture reagent or pull down reagent for a targeting separation method with a designed degradable structure. As shown in **FIG. 5D,** the capture reagent comprises a structured nucleic acid particle **550** (e.g., a DNA nanoball) comprising surface-displayed coupling groups **558** that are configured to couple with polypeptides and surface-displayed coupling groups **545** that are configured to be coupled with a solid support or an anchoring group. **FIG. 5E** shows an exploded view of the particle when the scaffold of the capture reagent or pull down reagent is unfolded. The structured nucleic acid particle **550** comprises a plurality of concatemerized subunits **551,** with each subunit **551** comprising a polypeptide coupling group **558** and a coupling group **545** that is configured to be coupled with a solid support or an anchoring group. The concatemerized subunits **551** are linked by a degradation site **552** (e.g., a restriction site, a photocleavable linker, etc.) that is configured to be cleaved under suitable conditions. **FIG. 5F** depicts a capture reagent **550** contacted with a plurality of polypeptides **520,** thereby coupling the polypeptides to the subunits **551.** The subunits each comprise a free coupling group **555** that may facilitate attachment of the subunits to a solid support or an anchoring group.

**FIGs. 5G - 5H** show an additional example of a capture reagent or pull down reagent for a targeting separation method with a designed degradable structure. **FIG. 5G** depicts a capture reagent or pull down reagent comprising a plurality of nanoparticle subunits **560** (e.g., organic or inorganic nanoparticles) that are linked by degradable linkers **552** (e.g., nucleic acid restriction sites, photocleavable linkers, etc.). Each nanoparticle subunit **560** comprises a coupling group **558** that is configured to couple with a polypeptide and a coupling group **555** that is configured to be coupled with a solid support or an anchoring group. The capture reagent may be contacted with a plurality of polypeptides **520,** where each polypeptide comprises a coupling group **525** that is configured to be coupled with a capture reagent. **FIG. 5H** depicts the capture reagent or pull down reagent after a degradation process. Each subunit **560** of the capture reagent may be detached from other subunits **560.** Each subunit may be further coupled to a polypeptide **520** and comprise a coupling group **555** that is configured to couple with a solid support or an anchoring group.

A targeting agent, such as a capture reagent or a pull down reagent may be combined with a sample or a composition comprising polypeptides in an amount that constitutes a deficit, at parity, or an excess relative to the total amount of polypeptides present, or the amount of targeted polypeptides present. The amount of a targeting agent contacted or combined with a sample or composition comprising polypeptides may be determined by a level of confidence for the amount of polypeptide present in a sample or polypeptide fraction. For example, the total amount of a polypeptide fraction may be known on a weight basis, but the total number of individual polypeptides within the polypeptide fraction may not be known. Likewise, the total number of individual polypeptides within a polypeptide fraction may be known, but the total number of individual target polypeptides may not be known. A targeting agent may be provided to a targeting separation method in excess to ensure that a sufficient quantity of polypeptide coupling groups are present to capture all targeted polypeptides. When a degradable targeting agent or a capture reagent comprising an anchoring group is utilized in a targeting separation method, an excess of the capture reagent may be utilized to control the display of polypeptides on the surface of a solid support. For example, a degradable capture reagent may degrade into a mixture of polypeptide-coupled subunits and uncoupled subunits. When the mixture of subunits deposits on a solid support, the amount of uncoupled subunits present may control the average spacing between polypeptide-containing sites on the solid support.

The quantity of a targeting reagent utilized in a targeting separation method may be calculated based upon the total available number of polypeptide-coupling groups on the utilized targeting reagent. A targeting reagent may be provided to a targeting separation method at a particular ratio of polypeptide-coupling groups to available or possibly available polypeptides. A targeting reagent may be provided to a targeting separation method at a particular ratio of

polypeptide-coupling groups to available or possibly available polypeptides of about 1:1000, 1:500, 1:100, 1:50, 1:10, 1:5, 1:2, 1:1, 2:1, 5:1, 10:1, 50:1, 100:1, 500:1, 1000:1, or more than 1000:1. A targeting reagent may be provided to a targeting separation method at a particular ratio of polypeptide-coupling groups to available or possibly available polypeptides of at least about 1:1000, 1:500, 1:100, 1:50, 1:10, 1:5, 1:2, 1:1, 2:1, 5:1, 10:1, 50:1, 100:1, 500:1, 1000:1, or more than 1000:1. Alternatively or additionally, a targeting reagent may be provided to a targeting separation method at a particular ratio of polypeptide-coupling groups to available or possibly available polypeptides of no more than about 1000:1, 500:1, 100:1, 50:1, 10:1, 5:1, 2:1, 1:1, 1:2, 1:5, 1:10, 1:50, 1:100, 1:500, 1:1000, or less than 1:1000.

A polypeptide fraction prepared by a polypeptide separation method comprising a plurality of sample polypeptides may include polypeptides of a particular size. A polypeptide within a polypeptide fraction may be at least about 0.1 Daltons (Da), 0.5 Da, 1 Da, 5 Da, 10 Da, 50 Da, 100 Da, 200 Da, 300 Da, 400 Da, 500 Da, 600 Da, 700 Da, 800 Da, 900 Da, 1 kiloDalton (kDa), 1.5 kDa, 2 kDa, 2.5 kDa, 3 kDa, 3.5 kDa, 4 kDa, 4.5 kDa, 5 kDa, 6 kDa, 7 kDa, 8 kDa, 9 kDa, 10 kDa, 15 kDa, 20 kDa, 25 kDa, 30 kDa, 40 kDa, 50 kDa, 60 kDa, 70 kDa, 80 kDa, 90 kDa, 100 kDa, 200 kDa, 300 kDa, 400 kDa, 500 kDa, 600 kDa, 700 kDa, 800 kDa, 900 kDa, 1000 kDa, 1200 kDa, 1400 kDa, 1600 kDa, 1800 kDa, 2000 kDa, 2500 kDa, 3000 kDa, 3500 kDa, 4000 kDa, or more than 4000 kDa. Alternatively or additionally, a polypeptide within a polypeptide fraction may be no more than about 4000 kDa, 3500 kDa, 3000 kDa, 2500 kDa, 2000 kDa, 1800 kDa, 1600 kDa, 1400 kDa, 1200 kDa, 1000 kDa, 900 kDa, 800 kDa, 700 kDa, 600 kDa, 500 kDa, 400 kDa, 300 kDa, 200 kDa, 100 kDa, 90 kDa, 80 kDa, 70 kDa, 60 kDa, 50 kDa, 40 kDa, 30 kDa, 25 kDa, 20 kDa, 15 kDa, 10 kDa, 9 kDa, 8 kDa, 7 kDa, 6 kDa, 5 kDa, 4.5 kDa, 4 kDa, 3.5 kDa, 3 kDa, 2.5 kDa, 2 kDa, 1.5 kDa, 1 kDa, 900 Da, 800 Da, 700 Da, 600 Da, 500 Da, 400 Da, 300 Da, 200 Da, 100 Da, 50 Da, 10 Da, 5 Da, 1 Da, 0.5 Da, or less than 0.5 Da.

A polypeptide within a polypeptide fraction prepared by a polypeptide separation method comprising a plurality of sample polypeptides may contain a minimum or maximum number of amino acid residues. A polypeptide may contain at least about 2, 3, 4, 5, 6, 7, 8, 9, 10, 15, 20, 25, 30, 35, 40, 45, 50, 60, 70, 80, 90, 100, 125, 150, 200, 250, 300, 400, 500, 600, 700, 800, 900, 1000, 1500, 2000, 3000, 4000, 5000, 6000, 7000, 8000, 9000, 10000, 15000, 20000, 30000, 40000 or more than 40000 amino acid residues. Alternatively or additionally, a polypeptide may contain no more than about 40000, 30000, 20000, 15000, 10000, 9000, 8000, 7000, 6000, 5000, 4000, 3000, 2000, 1500, 1000, 900, 800, 700, 600, 500, 400, 300, 200, 250, 200, 150, 125, 100, 90, 80, 70, 60, 50, 45, 40, 35, 30, 25, 20, 15, 10, 9, 8, 7, 6, 5, 4, 3, or less than 3 amino acid residues.

A polypeptide fraction prepared by a polypeptide separation method comprising a plurality of sample polypeptides may comprise a characterized percentage of a total quantity of sample polypeptides in a sample. A percentage of a total quantity of sample polypeptides may be calculated with respect to an appropriate basis, such as compared to an original sample from which the sample polypeptides are derived, as compared to a crude extract prepared from a sample, or as compared to a prior fraction or a prior sample. A polypeptide fraction comprising a plurality of sample polypeptides may contain at least about 0.000001%, 0.000005%, 0.00001%, 0.00005%, 0.0001%, 0.0005%, 0.001%, 0.005%, 0.01%, 0.05%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, 99.9%, 99.99%, 99.999%, 99.9999%, 99.99999%, or more than 99.99999% of the total quantity of sample polypeptides on a mass or molar basis. Alternatively or additionally, a polypeptide fraction comprising a plurality of sample polypeptides may contain no more than about 99.99999%, 99.9999%, 99.999%, 99.99%, 99.9%, 99.5%, 99%, 95%, 90%, 85%, 80%, 75%, 70%, 65%, 60%, 55%, 50%, 45%, 40%, 35%, 30%, 25%, 20%, 15%, 10%, 5%, 1%, 0.5%, 0.1%, 0.05%, 0.01%, 0.005%, 0.001%, 0.0005%, 0.0001%, 0.00005%, 0.00001%, 0.000005%, 0.000001%, or less than 0.000001% of the total quantity of sample polypeptides on a mass or molar basis.

A polypeptide fraction prepared by a polypeptide separation method comprising a plurality of sample polypeptides may have a characterized or characterizable polypeptide diversity after a separation process. A polypeptide diversity of sample polypeptides may be calculated with respect to an appropriate basis, such as compared to an original sample from which the sample polypeptides are derived, as compared to a crude extract prepared from a sample, or as compared to a prior fraction or a prior sample. A polypeptide fraction comprising a plurality of sample polypeptides may have a polypeptide diversity of at least about 0.000001%, 0.000005%, 0.00001%, 0.00005%, 0.0001%, 0.0005%, 0.001%, 0.005%, 0.01%, 0.05%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, 99.9%, 99.99%, 99.999%, 99.9999%, 99.99999%, or more than 99.99999% of the total quantity of polypeptide species of the sample from which the plurality of sample polypeptides was derived on a mass or molar basis. Alternatively or additionally, a polypeptide fraction comprising a plurality of sample polypeptides may have a polypeptide diversity of no more than about 99.99999%, 99.9999%, 99.999%, 99.99%, 99.9%, 99.5%, 99%, 95%, 90%, 85%, 80%, 75%, 70%, 65%, 60%, 55%, 50%, 45%, 40%, 35%, 30%, 25%, 20%, 15%, 10%, 5%, 1%, 0.5%, 0.1%, 0.05%, 0.01%, 0.005%, 0.001%, 0.0005%, 0.0001%, 0.00005%, 0.00001%, 0.000005%, 0.000001%, or less than 0.000001% of the total quantity of polypeptide species of the sample from which the plurality of sample polypeptides was derived on a mass or molar basis.

A polypeptide fraction may have a characterized or characterizable polypeptide diversity with respect to a known proteome or sub-proteome of an organism from which the sample was derived. A polypeptide diversity of sample polypeptides of a proteome or sub-proteome may be calculated with respect to an appropriate basis, such as compared to

an original sample from which the sample polypeptides are derived, as compared to a crude extract prepared from a sample, or as compared to a prior fraction or a prior sample. A polypeptide fraction may have a characterized or characterizable polypeptide diversity of at least about 0.000001%, 0.000005%, 0.00001%, 0.00005%, 0.0001%, 0.0005%, 0.001%, 0.005%, 0.01%, 0.05%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 100%, 110%, 120%, 150%, 200%, 300%, 400%, 500%, 1000%, or more than 1000% relative to a proteome or sub-proteome on a mass or molar basis. Alternatively or additionally, a polypeptide fraction may have a characterized or characterizable polypeptide diversity of no more than about 1000%, 500%, 400%, 300%, 200%, 150%, 120%, 110%, 100%, 95%, 90%, 85%, 80%, 75%, 70%, 65%, 60%, 55%, 50%, 45%, 40%, 35%, 30%, 25%, 20%, 15%, 10%, 5%, 1%, 0.5%, 0.1%, 0.05%, 0.01%, 0.005%, 0.001%, 0.0005%, 0.0001%, 0.00005%, 0.00001%, 0.000005%, 0.000001%, or less than 0.000001% relative to a proteome or sub-proteome on a mass or molar basis.

The sample polypeptide content of a polypeptide mixture may be characterized before a polypeptide separation process. The sample polypeptide content of a polypeptide mixture may be characterized as a mass fraction of sample polypeptides relative to total polypeptide content of a polypeptide mixture. For example, a polypeptide mixture containing a mixture of 90% sample polypeptides by weight and 10% internal standard polypeptide by weight may have a characterized sample polypeptide mass fraction of 90% or 0.9. A polypeptide mixture may have a sample polypeptide mass fraction of about 0.01, 0.05, 0.1, 0.15, 0.2, 0.25, 0.3, 0.35, 0.4, 0.45, 0.5, 0.55, 0.6, 0.65, 0.7, 0.75, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 0.991, 0.992, 0.993, 0.994, 0.995, 0.996, 0.997, 0.998, 0.999, or more than 0.999. A polypeptide mixture may have a sample polypeptide mass fraction of at least about 0.01, 0.05, 0.1, 0.15, 0.2, 0.25, 0.3, 0.35, 0.4, 0.45, 0.5, 0.55, 0.6, 0.65, 0.7, 0.75, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 0.991, 0.992, 0.993, 0.994, 0.995, 0.996, 0.997, 0.998, 0.999, or more than 0.999. Alternatively or additionally, a polypeptide mixture may have a sample polypeptide mass fraction of no more than about 0.999, 0.998, 0.997, 0.996, 0.995, 0.994, 0.993, 0.992, 0.991, 0.99, 0.98, 0.97, 0.96, 0.95, 0.94, 0.93, 0.92, 0.91, 0.9, 0.89, 0.88, 0.87, 0.86, 0.85, 0.84, 0.83, 0.82, 0.81, 0.8, 0.75, 0.7, 0.65, 0.55, 0.5, 0.45, 0.4, 0.35, 0.3, 0.25, 0.2, 0.15, 0.1, 0.05, 0.01 or less than 0.01.

A polypeptide separation process may occur in a medium that facilitates the separation process. A polypeptide mixture solvent composition may comprise a fluid medium, such as an aqueous solvent. In some cases, a polypeptide separation process may occur in a polypeptide mixture separation composition that is in contact with the solid support. A polypeptide mixture solvent composition for storage of a sample or partially purified sample may be utilized for a polypeptide separation process. A polypeptide mixture solvent composition may comprise one or more polypeptide composites in solution or suspension. A polypeptide mixture solvent composition may be formulated to be a homogeneous liquid medium. A polypeptide mixture solvent composition may be formulated to be a single-phase liquid medium. A polypeptide mixture solvent composition may be formulated to be a multi-phase liquid medium, such as an oil-in-water emulsion or a water-in-oil emulsion. For a polypeptide mixture solvent composition formulated as an emulsion, anchoring groups or polypeptide composites may be solvated or suspended within the dissolved phase.

### Polypeptide Functionalization

Polypeptides (e.g. sample polypeptides or standard polypeptides) or a polypeptide fraction may be treated with one or more modifying agents to add functional groups to some or all polypeptides within a sample or polypeptide fraction. The methods and compositions set forth below may generally be exemplified with reference to a modifying agent, functional group, polypeptide and/or a protein; however, it will be understood that the examples can be extended to populations of modifying agents, functional groups, polypeptides, and/or proteins. For example, a population having the same species of protein can be used with a population of the same species of modifying agent and/or functional group, a population having different species of protein can be used with a population of the same species of modifying agent and/or functional group, a population having the same species of protein can be used with a population of different species of modifying agent and/or functional group, or a population having different species of protein can be used with a population of different species of modifying agent and/or functional group. Moreover, methods and compositions may be exemplified below with reference to proteins; however, it will be understood that the examples can be extended to other polypeptides such as sample polypeptides or standard polypeptides.

A functional group may be added to a polypeptide from a polypeptide fraction for any of a variety of purposes, including altering chemical properties (e.g., altering electrical charge, altering hydrophobicity, altering reactivity), adding groups that improve detectability of polypeptides (e.g., fluorophores, luminescent particles, radiolabels), modulating activity (e.g. modification of amino acids at or near the active site of an enzyme, or binding to an inhibitor or activator of the enzyme) or adding groups that alter the separability of polypeptides (e.g., magnetic particles, hydrophobic particles, charged particles, linkers for attachment to solid-phase materials etc.).

A polypeptide fraction may be treated with one or more modifying agents to add a reactive functional group to some or all polypeptides within a polypeptide fraction. In some cases, a polypeptide fraction may be treated with one or

more modifying agents to add a functional group to some or all polypeptides that are configured to interact with a second functional group. A polypeptide with a functional group may interact with a second molecule with a second functional group to form a polypeptide composite. A polypeptide composite may be formed for any of a variety of purposes, including altering the polypeptide's chemical properties (e.g., altering electrical charge, altering hydrophobicity, altering reactivity), altering the polypeptide's activity (e.g. inhibiting or activating enzymatic activity), adding groups that improve detectability of the polypeptide (e.g., fluorophores, luminescent particles, radiolabels), or adding groups that alter the separability of the polypeptide (e.g., magnetic particles, hydrophobic particles, charged particles).

A polypeptide from a sample or a polypeptide fraction may be functionalized with one or more functional groups. A functional group may be added to a polypeptide at a terminal amino acid residue, for example at the terminal carboxyl moiety or side chain moiety of the C-terminal amino acid or at the terminal amino moiety or side chain moiety of the N-terminal amino acid. A functional group may be added to a polypeptide at the side chain of any given non-terminal amino acid within the polypeptide. A functional group may be added to a polypeptide at any amino acid containing an amine or thiol side chain. A functional group may be added to a cysteine, asparagine, glutamine, lysine, histidine, tryptophan, or arginine amino acid residue. A polypeptide may be functionalized at more than one amino acid (e.g., all or nearly all amine side chains may be functionalized for a polypeptide). A polypeptide may have a functional group added to one or more type of side chain. For example, some or all side chains having amines may be functionalized, some or all side chains having carbonyls may be functionalized, some or all side chains having phosphates may be functionalized, some or all side chains having hydroxyls may be functionalized, or some or all side chains having thiols may be functionalized. Functionalization can be carried out to add the same functional group to multiple side chains of the polypeptide and the multiple side chains can optionally be the same or different type of side chain. A polypeptide may be functionalized to add more than one type of functional group to the polypeptide (e.g., adding azides to all amines and NHS esters to all thiols) and different types of functional groups can be added to the same type of side chain. Alternatively, different types of functional groups can be added to different types of side chains respectively. A polypeptide may be functionalized with more than one type of functional group with all functional groups added for the same purpose (e.g., increasing reactivity). A polypeptide may be functionalized with more than one type of functional group with differing functional groups added for differing purposes (e.g., increasing reactivity and altering hydrophobicity).

A polypeptide may be functionalized at any stage of a sample preparation process set forth herein or known in the art. A polypeptide may be functionalized before it has been separated, isolated or extracted from a sample. A polypeptide may be functionalized while it is still contained within a sample. A polypeptide may be functionalized during the processing of a sample but before any polypeptides have been separated from the sample (e.g., after cell lysis). A polypeptide may be functionalized during or after separation, isolation, or extraction from a sample. A functionalization process may be repeated one or more times to ensure complete or near-complete functionalization of all polypeptides derived from a sample.

**FIG. 6A** depicts a scheme for preparing polypeptides that are modified with a functional group in an intact biological sample. In this scheme, the polypeptides are modified with a functional group before they are extracted from the sample to yield a functionalized polypeptide fraction. A biological material **600** (e.g., a cell) may be contacted with a reagent **660** that is configured to functionalize a polypeptide. The reagent **660** may be provided with one or more additional reagents that are necessary to induce a reaction between the reagent **660** and any available polypeptides. The reagent **660** may be contacted with the biological material in the presence of a detergent. The reagent **660** may be contacted with the biological material in the presence of a standard polypeptide such as a reagent standard polypeptide. The biological material **600** may include any biological component, including but not limited to cell walls, cell membranes **670,** nuclear membranes **672,** organelles **674,** genetic materials **650** (e.g., chromosomes, plasmids), fibrils or other structural components, capsids, cartilage, bone, exoskeleton, exocellular proteins, cytoplasmic proteins **610,** nuclear proteins **620,** organelle proteins **630,** and membrane-bound or membrane-linked proteins **640.** The reagent **660** may react with any available polypeptides in sample to form functionalized polypeptides, such as functionalized cytoplasmic proteins **611,** functionalized nuclear proteins **621,** functionalized organelle proteins **631,** and functionalized membrane proteins **641.** The reagent **660** may incidentally functionalize other non-polypeptide components of the sample. After the sample has been functionalized with the reagent **660,** the sample may be processed as described above to separate, isolate, or extract the polypeptides from the sample, producing a polypeptide fraction including the functionalized polypeptides, and a waste fraction composing other components such as the genetic materials **650** and fragmented cellular debris **680** (e.g., membrane fragments, organelle fragments). The polypeptide fraction may be separated, isolated, or extracted from the non-polypeptide fraction in the presence of a detergent. The polypeptide fraction may be separated, isolated, or extracted from the non-polypeptide fraction in the presence of a standard polypeptide, such as a separation standard polypeptide, isolation standard polypeptide, or extraction standard polypeptide.

**FIG. 6B** depicts a scheme for preparing polypeptides that are modified with a functional group, the polypeptides being from an intact biological sample. In this scheme, the polypeptides are modified with a functional group after they are extracted from the sample to yield a functionalized polypeptide fraction. A biological material **600** (e.g., a cell) may be processed as described herein to release polypeptides contained within the biological material **600.** The biological

material **600** may include any biological component, including but not limited to cell walls, cell membranes **670,** nuclear membranes **672,** organelles **674,** genetic materials **650** (e.g., chromosomes, plasmids), fibrils or other structural components, capsids, cartilage, bone, exoskeleton, exocellular proteins, cytoplasmic proteins **610,** nuclear proteins **620,** organelle proteins **630,** and membrane-bound or membrane-linked proteins **640.** After the biological material **600** has been processed to release polypeptides, the polypeptides may be contacted with a reagent **660** that is configured to functionalize a polypeptide. The reagent **660** may be provided with one or more additional reagents that are necessary to induce a reaction between the reagent **660** and any available polypeptides. The reagent **660** may functionalize the available polypeptides to produce functionalized polypeptides, such as functionalized cytoplasmic proteins **611,** functionalized nuclear proteins **621,** functionalized organelle proteins **631,** and functionalized membrane proteins **641.** Functionalized polypeptides may be extracted, isolated, or separated from the non-polypeptide components (e.g., genetic material **650,** cellular debris **680**) to yield a polypeptide fraction. The reagent **660** may be introduced before or after the polypeptides have been separated from the residual non-polypeptide components.

**FIG. 7A** depicts a scheme for preparing polypeptides that are modified with a functional group, the sample polypeptides being from a sample and in solution phase (e.g., blood plasma, water samples). The sample may comprise polypeptides **710** suspended or solvated within a liquid medium, as well as other components **700** (e.g., cells, viral particles, non-biological particles) that are not of interest in a polypeptide assay. The polypeptides **710** may be separated from the other components before the polypeptides **710** are functionalized. Methods such as precipitation, centrifugation, filtration, liquid-phase extraction, solid-phase extraction, size-exclusion chromatography, and liquid chromatography, or a combination thereof, may be utilized to separate, isolate, or extract the other components **700** from the solution phase polypeptides **710.** After the solution phase polypeptides **710** have been collected in a polypeptide fraction, the polypeptides may be contacted with a functionalizing reagent **720** that is configured to add a functional group to the polypeptide. The functionalizing reagent **720** may be provided with one or more additional reagents that are necessary to induce a reaction between the reagent **720** and any available polypeptides. The functionalizing reagent **720** may functionalize the available polypeptides to produce a functionalized polypeptide fraction **740.** The polypeptide fraction may undergo additional purification and/or preparation processes before or after the functionalization step.

**FIG. 7B** depicts an alternative scheme for preparing a polypeptide fraction comprising polypeptides that are modified with a functional group from a sample where the polypeptides of interest are in solution phase (e.g., blood plasma). The sample may comprise polypeptides **710** suspended or solvated within a liquid medium, as well as other components **700** (e.g., cells, viral particles) that are not of interest in a polypeptide assay. The sample may be contacted with a functionalizing reagent **720** before the polypeptides **710** of interest have been separated from any other components **700.** The functionalizing reagent **720** may be provided with one or more additional reagents that are necessary to induce a reaction between the reagent **720** and any available polypeptides. The functionalizing reagent **720** may functionalize solution phase polypeptides **710** as well as other components **700** to produce functionalized polypeptides **711** mixed with functionalized biological materials **701.** After the functionalization step, the functionalized polypeptides **711** may be separated, isolated, or extracted from the other functionalized biological materials **701,** thereby yielding a functionalized polypeptide fraction **740.**

**FIG. 8A** depicts a scheme for preparing a polypeptide fraction comprising polypeptides that are modified with a functional group when the sample is to be extracted from a curated solid medium **800** (e.g., rock, a filter). The solid medium **800** may include adhering polypeptides **810** or adhering non-polypeptide components **820** (e.g., mineral particles). The solid medium **800** may be subjected to one or more washing or extraction processes that separate the polypeptides **810** from the solid medium **800** and any non-polypeptide components **820.** The separated polypeptides **810** may be contacted with a functionalizing reagent **830** to produce a functionalized polypeptide fraction **811.** The functionalizing reagent **830** may be provided with one or more additional reagents that are necessary to induce a reaction between the reagent **830** and any available polypeptides.

**FIG. 8B** depicts an alternative scheme for preparing a polypeptide fraction comprising polypeptides that are modified with a functional group when the sample is to be extracted from a curated solid medium **800** (e.g., rock, a filter). The solid medium **800** may include adhering polypeptides **810** or adhering non-polypeptide components **820** (e.g., mineral particles). The entire sample may be contacted with a functionalizing reagent **830.** The functionalizing reagent **830** may be provided with one or more additional reagents that are necessary to induce a reaction between the reagent **830** and any available adhering polypeptides **810** to produce a functionalized polypeptide fraction **811.** The solid medium **800** may be subjected to one or more washing or extraction processes that separate the functionalized polypeptide fraction **811** from the solid medium **800** and any non-polypeptide components **820.**

Standard polypeptides can be particularly useful in methods for preparing polypeptides modified with functional groups, such as those exemplified above in reference to **FIGs 6 - 8.** One or more different standard polypeptides, such as species of standard polypeptide set forth elsewhere herein, can be present during at least one of the steps in each scheme. One or more different standard polypeptides can be added before, during or after any of the steps, for example, to provide a means to characterize the step or subsequent step(s). Standard polypeptides present during a particular step can optionally be used to characterize a product of the step. Exemplary characterizations include, but are not limited to

quantifying sample polypeptides or other product(s) of a method or step exemplified herein, identifying a property of a sample polypeptide or other product of a method or step exemplified herein, or determining the quality of a sample polypeptide or other product of a method or step exemplified herein. The standard polypeptides can optionally be functionalized as exemplified in the methods above, for example, the standard polypeptides being treated simultaneously with, and in the same sample as, sample polypeptides. A standard polypeptide can have a known or predicted reactivity toward one or more reagent that is used in a functionalization reaction. In some cases a plurality of different standard polypeptides can be present in a functionalization reaction, wherein each of the different standard polypeptides has a different reactivity toward one or more reagents used in the functionalization reaction. As such, one or more standard polypeptides can be used as functionalization standards.

Polypeptides may be conjugated with a functional group for any of a variety of purposes. A functional group may be added to a polypeptide to increase or alter the reactivity of the polypeptide. In some cases, a polypeptide may be functionalized with a functional group that is configured to react with another chemical species by a "click" reaction (see, for example, U.S. Pat. Nos. 6,737,236 and 7,427,678 ) such as azide alkyne Huisgen cycloaddition reactions, which use a copper catalyst (see, for example, U.S. Pat. Nos. 7,375,234 and 7,763,736 ); or Copper-free Huisgen reactions ("metal-free click") using strained alkynes or triazine-hydrazine moieties which can link to aldehyde moieties (see, for example, U.S. Pat. No. 7,259,258

). A polypeptide may be functionalized with a functional group to facilitate coupling or conjugation of the polypeptide to other molecules or materials. For example, a polypeptide may be coupled or conjugated to a complementary species such as a biomolecule, fluorescent label, or a surface. The polypeptide may be coupled or conjugated to the complementary species by a covalent or non-covalent interaction of the functional group on the polypeptide with a complementary functional group, chemical species or active site on the complementary species. Functional groups for coupling or conjugation of a polypeptide to a complementary species may comprise a single-stranded nucleic acid, a double-stranded nucleic acid, a nucleophile, an electrophile, a coordination compound (e.g., a silane), or a moiety capable of forming hydrogen bonds (e.g., alcohols, carboxylic acids, ketones).

The extent of functionalization of a functionalized polypeptide fraction may be characterized or quantified. The extent of functionalization may include one or both of two aspects: 1) the quantity of polypeptides within a polypeptide fraction that receive at least one functionalization; and 2) the average number of functionalizations per polypeptide within a polypeptide fraction. A polypeptide fraction may be functionalized to ensure that all polypeptides contain at least one added functional group. A polypeptide fraction may be functionalized until there is an average of at least one functional group per polypeptide. In some cases, an individual polypeptide from a polypeptide fraction may have no added functional groups. In some cases, an individual polypeptide from a polypeptide fraction may have more than one added functional group. In some cases, a polypeptide may have a functional group on every available target for functionalization (e.g., all side chain amines are functionalized with an azide). The extent of functionalization for individual molecules within a polypeptide fraction may follow some quantifiable distribution, such as a Poisson distribution, binomial distribution, beta-binomial distribution, hypergeometric distribution, or bimodal distribution. The extent of functionalization may be specific to a single type of functional group if more than one functional group is added to a polypeptide fraction. The extent of functionalization may be a global measure of functionalization if more than one functional group is added to a polypeptide fraction. The extent of functionalization for a functionalized polypeptide fraction may be measured by any suitable method. For example, quantity of a polypeptide fraction with at least one functionalization may be assessed by conjugating a macromolecule (e.g., PEG) to functionalized polypeptides, then separating by a method such as size-exclusion chromatography to quantify the relative amounts of functionalized and non-functionalized polypeptide in a polypeptide sample. Likewise, functionalization of a polypeptide may cause a change in property (e.g., hydrophobicity, polarity) that may permit chromatographic separation on the basis of the property (e.g., hydrophobic exchange chromatography, reverse-phase liquid chromatography). In another example, the average number of functionalizations per molecule may be determined by conjugation of labeled dyes to the added functional groups, thereby permitting quantitation of total dye conjugated by a colorimetric or fluorescent assay. Functionalization of polypeptides may be directly measured by an analytical technique such as mass spectrometry (MS), or spectroscopy (e.g., infrared, UV-vis, Raman, etc.).

A plurality of functionalized polypeptide may be coupled to a plurality of molecules to determine an extent of functionalization for the plurality of polypeptides. The plurality of functionalized polypeptides may be coupled to macromolecules such as biopolymers ( e.g., polypeptides, nucleic acids, polysaccharides). Macromolecules may be coupled or conjugated to polypeptides by a cleavable or reversible linker (e.g., a photocleavable linker). Macromolecules may be larger than a polypeptide (e.g., by weight or radius) or chemically dissimilar relative to a functionalized polypeptide (e.g., by hydrophobicity polarity, etc.) to permit fractionation of polypeptides by number of macromolecules incorporated. For example, size-exclusion chromatography may be utilized to produce fractions of polypeptides coupled to 1 macromolecule, 2 macromolecules, 3 macromolecules, etc. Relative quantities of recovered fractions may be correlated to the relative numbers of functionalizations per polypeptide molecule. In some cases, the estimated number of functionalizations per polypeptide may comprise a size proxy (e.g., larger polypeptides may contain more amino acid residues that are functionalized). In some cases, each size-based polypeptide fraction may be individually coupled to differing anchoring

groups to provide identifiable fractions based upon polypeptide size, approximate number of functionalized amino acids, or approximate solvent accessible area of each polypeptide fraction.

A suitable functionalized polypeptide fraction for a polypeptide assay may have a quantified extent of functionalization that meets a threshold value. The threshold value (e.g., a maximum or minimum threshold value) may represent the extent of functionalization needed to obtain an accurate characterization of polypeptide fraction. The threshold value for extent of functionalization may depend upon the polypeptide assay to be performed. For example, a qualitative assay (e.g., presence or absence of a biomarker) may require a lower threshold for extent of functionalization to ensure detection of a polypeptide. In another example, a quantitative assay (e.g., polypeptide sizing) may have an upper threshold for extent of functionalization if an excess of functionalization may exceed a detection limit of a measurement assay. In some cases, the extent of functionalization may be quantified as the average number of functionalizations per polypeptide molecule or per mass of polypeptide. In some cases, the extent of functionalization may be quantified as the percentage of polypeptides to receive at least one functional group. The threshold average number of functionalizations per polypeptide molecule may be at least about 0.1, 0.2, 0.3, 0.4, 0.5, 0.6, 0.7, 0.8, 0.9, 1, 1.5, 2, 3, 4, 5, 6, 7, 8, 9, 10, 15, 20, 25, 50 or greater than 50 functionalizations per molecule. Alternatively or additionally, the average number of functionalizations per polypeptide molecule may be no more than about 50, 25, 20, 15, 10, 9, 8, 7, 6, 5, 4, 3, 2, 1, 0.9, 0.8, 0.7, 0.6, 0.5, 0.4, 0.3, 0.2, 0.1, or less than 0.1 functionalizations per molecule. The threshold percentage of functionalized polypeptide in a polypeptide fraction may be at least about 1%, 5%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, 99.9%, or greater than 99.9% of the total polypeptide in the fraction measured by mass or molarity. Alternatively or additionally, the threshold percentage of functionalized polypeptide in a polypeptide fraction may be no more than about 99.9%, 99%, 98%, 97%, 96%, 95%, 94%, 93%, 92%, 91%, 90%, 85%, 80%, 75%, 70%, 65%, 60%, 55%, 50%, 45%, 40%, 35%, 30%, 25%, 20%, 15%, 10%, 5%, 1%, or less than 1% of the total polypeptide in the fraction measured by mass or molarity.

The extent of functionalization for one or more sample polypeptides may be quantified relative to the extent of functionalization for one or more standard polypeptides, such as standard polypeptide(s) having a known or predicted reactivity with one or more reagents used in the functionalization reaction. Accordingly, a threshold value for functionalization of one or more sample polypeptide may be quantified relative to functionalization of one or more standard polypeptide. Moreover, properties of functionalized sample polypeptides may be characterized relative to known or expected properties of functionalized standard polypeptides.

In some cases, a functionalization standard may include one or more polypeptides that are configured to provide a quantitative measure of polypeptide functionalization. For example, a functionalization standard may comprise a polypeptide that contains an amino acid abundance or overabundance (e.g., relative to a natural proteomic amino acid abundance) of an amino acid residue that is functionalized (e.g., a 200 residue polypeptide comprising 100 lysine residues). Such a polypeptide may provide a more sensitive measure for the extent of functionalization. In another example, a functionalization standard may comprise a polypeptide comprising an amino acid abundance or overabundance (e.g., relative to a natural proteomic amino acid abundance) of an amino acid residue that is off target for a functionalization chemistry (e.g., a 200 residue polypeptide comprising 100 arginine residues, for a lysine-targeting functionalization chemistry). In another example, a functionalization standard may comprise a polypeptides comprising functionalization target residues (e.g., lysine, cysteine, etc.) in differing sequence contexts. For example, a functionalization standard may comprise a polypeptide comprising lysine residues that are buried in a tertiary structure to assess the extent of functionalization of lysines based upon accessibility. In some cases, a functionalization standard may comprise a plurality of polypeptides that are configured to determine an effect of functionalization of a polypeptide on affinity agent binding to a polypeptide epitope. For example, a functionalization polypeptide standard may comprise a plurality of polypeptides, in which each polypeptide of the plurality of polypeptides comprises a same epitope, and in which each polypeptide of the plurality of polypeptides comprises a functionalized amino acid residues (e.g., a functionalized lysine, etc.) with a known position relative to the epitope (e.g., within at least 1, 2, 3, 4, 5, or more than 5 amino acid residues of the epitope). Such a functionalization standard may provide a quantitative or qualitative measure of the effect of the functionalized amino acid on affinity agent binding to the epitope.

Functionalization reactions may be repeated for a particular polypeptide fraction. A functionalization reaction may be repeated for a particular polypeptide fraction to obtain a larger extent of functionalization. A functionalization reaction may be repeated under differing conditions to functionalize polypeptides that are resistant to functionalization under the initial reaction conditions. Alternatively, a functionalization reaction may be performed only once or for a limited amount of time to prevent excessive functionalization of a plurality of polypeptides. For example, functionalization can be carried out under differing denaturation conditions to allow increased access to side chain moieties that are differentially accessible under those conditions. For example, functionalization can be carried out at different temperatures (e.g. temperatures higher than the physiological range for the sample from which the polypeptides are derived), different pH (e.g. pH higher or lower than the physiological range for the sample from which the polypeptides are derived), different ionic strengths (e.g. ionic strength lower or higher than the physiological range for the sample from which the polypeptides are derived), different polarity (e.g. polarity that is higher or lower than the physiological range for the sample from which the

polypeptides are derived), different species of chemical denaturant and/or different concentrations of chemical denaturant. A functionalization reaction may be repeated using a differing reagent that more effectively targets any residues not functionalized during the first functionalization reaction. In some cases, additional reagent may be supplied to a functionalization reaction during the course of the reaction to replenish depleted reagents, shift a chemical equilibrium, or alter a reaction rate. In some cases, a plurality of sample polypeptides may be functionalized then separated to isolate functionalized polypeptides from unfunctionalized polypeptides. In such cases, the unfunctionalized polypeptide fraction may be functionalized a second time to ensure near-complete or complete functionalization of all sample polypeptides.

In some cases, functionalization may be carried out with co-solvents or alternative solvents. A functionalization solvent may be aqueous or non-aqueous. A solvent or co-solvent may be an organic solvent or an anhydrous solvent. Solvent choice may be influenced by the target for functionalization and the specificity of the reaction between the polypeptide and the functionalization compound. For example, altering a solvent from DMSO to aqueous PBS buffer may shift the selectivity of a dinitroimidazole reaction with a thiol-containing sidechain from lysine to cysteine. A functionalization solvent may also comprise a catalyst, such as a dissolved catalyst or a solid catalyst, that promotes an increased rate or specificity of polypeptide functionalization. For example, the reaction of an amine-containing polypeptide with a proton-acceptor catalyst in an anhydrous solvent (e.g., diisopropylethylamine (DiPEA), triethylamine (TEA), N-methyl morpholine (NMM)) may favor reaction with the amine over hydrolysis. A functionalization reaction may occur in the presence of an enzyme that is configured to modify an amino acid side chain or add a functional group to an amino acid side chain or terminal residue.

A functionalization reaction may be chosen for a polypeptide fraction based upon the speed of the reaction. Selection of functionalization reactions may be based upon various factors, including the stability of the sample, the time available to prepare the sample, the known or expected chemical composition of the sample, the available functionalizing reagents, and the type of characterization assay to be performed. For example, a time-sensitive sample may be functionalized with an enzymatic or catalytic approach (e.g., sterically-hindered bases for amine side chains), or in the presence of a high concentration of the functionalization reagent. In another example, a sample may be shelf stable for up to a day at room temperature. This sample may be functionalized with an anhydrous functionalization chemistry such as carbene reactions (slower due to the steps of rendering a polypeptide sample anhydrous). A functionalization reaction may occur in the presence of a surfactant and/or a detergent. A functionalization reaction may occur in the presence of a co-solvent that is configured to improve the rate, conversion or selectivity of a functionalization reaction. For example, an aqueous functionalization medium may be modified to include a miscible co-solvent (e.g., ethanol, methanol, acetone) or an immiscible co-solvent (e.g., dimethyl ether, carbon tetrachloride) during a functionalization reaction.

Functional groups capable of rapidly forming covalent bonds with other chemical species may be of particular interest for the functionalization of polypeptides. In general, functional groups of interest will include most common species for bioconjugation. Such functional groups may include "click" reagents that are capable of forming highly specific products with complementary functional groups in a rapid and irreversible fashion.

A functionalization reaction of a polypeptide may be performed in a fashion that ensures that some or all reactive sites in a polypeptide become functionalized. For example, an amine-functionalizing chemistry (e.g., NHS-azide conjugation) may be performed with an excess of the azide-containing compound to ensure complete reaction of all amine side chains. A functionalization reaction may be performed with a functional group:polypeptide molar ratio of about 1:1000, 1:500, 1:250, 1:100, 1:50, 1:25, 1:10, 1:5, 1:4, 1:3, 1:2, 1:1, 2:1, 3:1, 4:1, 5:1, 6:1, 7:1, 8:1, 9:1, 10:1, 20:1, 25:1, 30:1, 50:1, 100:1, 250:1, 500:1, 1000:1, or more. A functionalization reaction may be performed with a functional group:polypeptide molar ratio of at least about 1:1000, 1:500, 1:250, 1:100, 1:50, 1:25, 1:10, 1:5, 1:4, 1:3, 1:2, 1:1, 2:1, 3:1, 4:1, 5:1, 6:1, 7:1, 8:1, 9:1, 10:1, 20:1, 25:1, 30:1, 50:1, 100:1, 250:1, 500:1, 1000:1, or more. Alternatively or additionally, a functionalization reaction may be performed with a functional group:polypeptide molar ratio of no more than about 1000:1, 500:1, 250:1, 100:1, 50:1, 30:1, 25:1, 20:1, 10:1, 9:1, 8:1, 7:1, 6:1, 5:1, 4:1, 3:1, 2:1, 1:1, 1:2, 1:3, 1:4, 1:5, 1:10, 1:25, 1:50, 1:100, 1:250, 1:500, 1:1000, or more.

A functionalization reaction may occur at a fixed temperature. In some cases, a functionalization reaction may occur at a first temperature for a fixed amount of time, then a second temperature for a fixed amount of time. A functionalization reaction between a polypeptide and a reactive group may occur at a temperature of about - 80 °C, - 70 °C, - 60 °C, - 50 °C, - 40 °C, - 30 °C, - 20 °C, - 10 °C, - 5° C, 0 °C, 4 °C, 10 °C, 20°C, 30°C, 37 °C, 40 °C, 50 °C, 60 °C, 70 °C, 80 °C, 90 °C, or about 95 °C. A functionalization reaction between a polypeptide and a reactive group may occur at a temperature of at least about - 80 °C, - 70 °C, - 60 °C, - 50 °C, - 40 °C, - 30 °C, - 20 °C, - 10 °C, - 5 °C, 0 °C, 4 °C, 10 °C, 20 °C, 30°C, 37 °C, 40 °C, 50 °C, 60 °C, 70 °C, 80 °C, 90 °C, or at least about 95 °C. Alternatively or additionally, a functionalization reaction between a polypeptide and a reactive group may occur at a temperature of no more than about 95 °C, 90 °C, 80 °C, 70 °C, 60 °C, 50 °C, 40 °C, 37 °C, 30 °C, 20 °C, 10°C, 4°C, 0 °C, - 5 °C, - 10°C, - 20 °C, - 30 °C, - 40 °C, - 50 °C, -60°C, - 70 °C, or about - 80 °C.

A functionalization reaction between a polypeptide and a reactive group may occur for a fixed amount of time. A functionalization reaction between a polypeptide and a reactive group may occur for about 1 min, 30 mins, 1 hr, 2 hrs, 3 hrs, 4 hrs, 5 hrs, 6 hrs, 7 hrs, 8 hrs, 9 hrs, 10 hrs, 11 hrs, 12 hrs, 13 hrs, 14 hrs, 15 hrs, 16 hrs, 17 hrs, 18 hrs, 19 hrs, 20 hrs, 21 hrs,

22 hrs, 23 hrs, 1 day, 2 days, 3 days, 4 days, 5 days, 6 days, 7 days, 10 days, 2 wks, 3 wks, 4 wks, 1 mth, or more than 1 mth. A functionalization reaction between a polypeptide and a reactive group may occur for at least about 1 min, 30 mins, 1 hr, 2 hrs, 3 hrs, 4 hrs, 5 hrs, 6 hrs, 7 hrs, 8 hrs, 9 hrs, 10 hrs, 11 hrs, 12 hrs, 13 hrs, 14 hrs, 15 hrs, 16 hrs, 17 hrs, 18 hrs, 19 hrs, 20 hrs, 21 hrs, 22 hrs, 23 hrs, 1 day, 2 days, 3 days, 4 days, 5 days, 6 days, 7 days, 10 days, 2 wks, 3 wks, 4 wks, 1 mth, or more than 1 mth. Alternatively or additionally, a functionalization reaction between a polypeptide and a reactive group may occur for no more than about 1 mth, 4 wks, 3 wks, 2 wks, 10 days, 7 days, 6 days, 5 days, 4 days, 3 days, 2 days, 1 day, 23 hrs, 22 hrs, 21 hrs, 20 hrs, 19 hrs, 18 hrs, 17 hrs, 16 hrs, 15 hrs, 14 hrs, 13 hrs, 12 hrs, 11 hrs, 10 hrs, 9 hrs, 8 hrs, 7 hrs, 6 hrs, 5 hrs, 4 hrs, 3 hrs, 2 hrs, 1 hr, 30 mins, 1 min, or less than 1 min.

In some cases, chemical conjugation techniques may be applied for creating biomaterial-biomolecule conjugates. Functional groups used for bioconjugation may be native to the biomolecule or may be incorporated synthetically. In the illustrations below, R and R' may be a biomolecule (for example, but not limited to: SNAPs, polypeptides, nucleic acids, carbohydrates, lipids, metabolites, small molecules, monomers, oligomers, polymers) and/or a solid support.

A polypeptide or particle can be modified to incorporate a reactive moiety that will in turn participate in a subsequent attachment reaction, such as a reaction for attaching a particle to a polypeptide, a reaction for attaching a particle to a solid support or a reaction for attaching a polypeptide to a solid support. Functionalizing one or both of such reaction partners may improve the efficiency or speed of subsequent attachment between the partners. For example, a sulfhydryl group (-SH) or amine ($-NH_2$) of a polypeptide or particle may be functionalized to allow for greater reactivity or efficiency of an attachment reaction. Exemplary functionalization chemistries are set forth below. For example, a reaction set forth below can be used to attach a reactive moiety to a particle, and R and R' may represent the particle or the reactive moiety. In other cases, a reaction set forth below can be used to attach a reactive moiety to a polypeptide, and R and R' may represent the polypeptide or the reactive moiety. Similarly, a reaction set forth below can be used to attach a reactive moiety to a solid support and, R and R' may represent the solid support or the reactive moiety. It will be understood that the reactions set forth below can be used to attach a particle to a polypeptide and R and R' may represent the polypeptide or the particle.

Amine moieties can be functionalized to attach a reactive moiety or other moiety. In some cases, an isothiocyanate moiety may react with nucleophiles such as amines, sulfhydryls, the phenolate ion of tyrosine side chains or other molecules to form an isothiourea linkage.

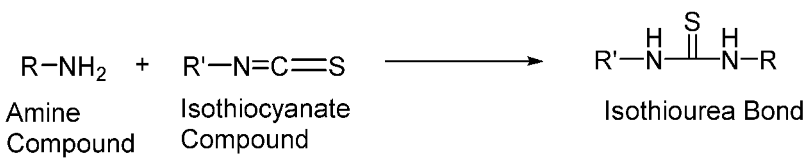


An isocyanate moiety can react with an amine to form a stable isourea linkage.

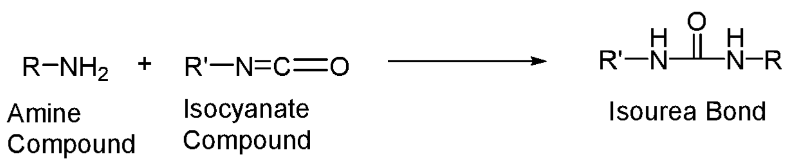


An acyl azide can react with a primary amine to form an amide linkage.

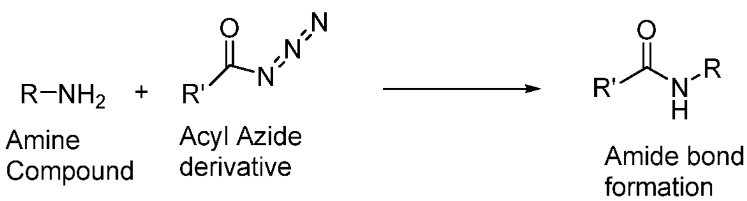


An N-hydroxysuccinimide (NHS) ester can react with an amine to form an amide linkage.

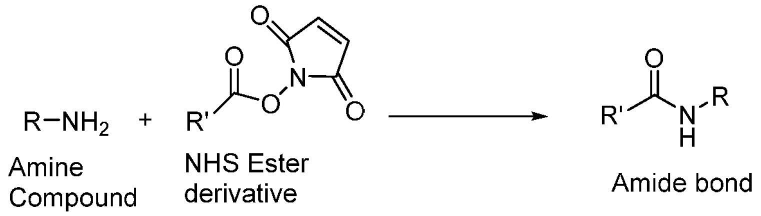


A sulfonyl chloride can react with a primary amine to form a sulfonamide linkage.

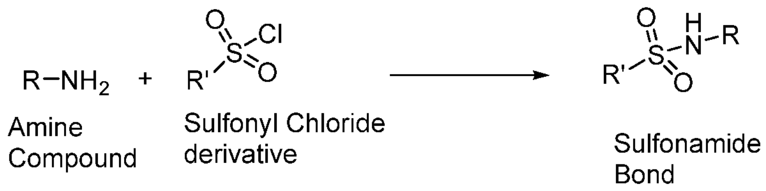


Carbonyl moieties such as aldehydes, ketones, and glyoxals can react with amines to form Schiff base intermediates and the addition of sodium borohydride or sodium cyanoborohydride can reduce the Schiff base intermediate to form a secondary amine linkage.

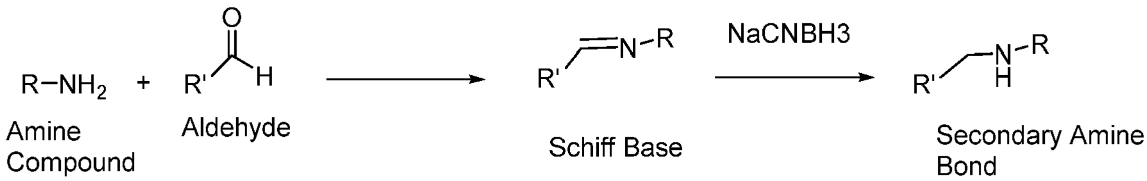


An epoxide or oxirane can react with a primary amine, sulfhydryl, or hydroxyl to create a secondary amine, thioether, or ether linkage, respectively.

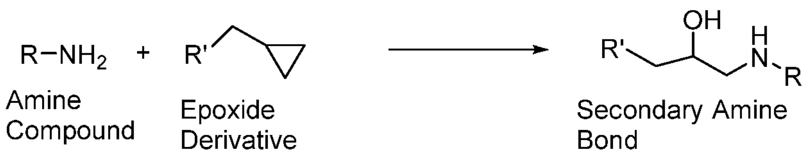


A carbonate can react with nucleophiles such as amines to form a carbamate linkage.

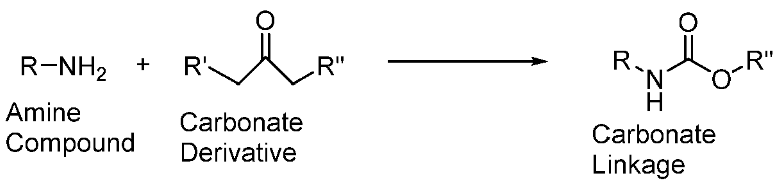


An aryl halide, such as fluorobenzene derivative, can react with an amine to form an arylamine linkage. Other nucleophiles such as thiol, imidazolyl, and phenolate groups can also react with an aryl halide to form a stable linkage.

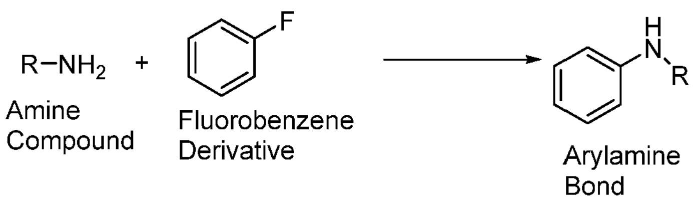


An amine can react with an imidoester to form an amidine linkage.

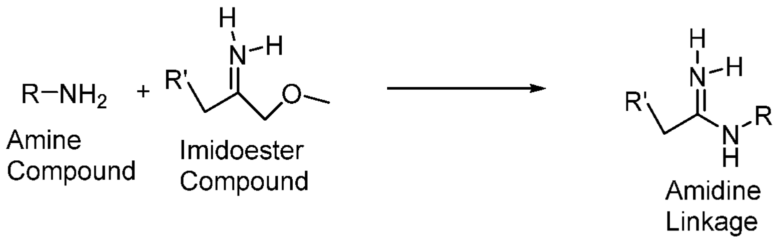


A carbodiimide can be used as a zero-length crosslinking agents to mediate formation of an amide or phosphoramidate linkage between a carboxylate group and an amine, or between a phosphate and an amine, respectively. Carbodiimides are zero-length reagents because in forming these bonds no additional chemical structure is introduced between the conjugating molecules. A carbodiimide can be used to activate a phosphate to an intermediate phosphate ester that in turn reacts with an amine to form a phosphoramidate linkage.

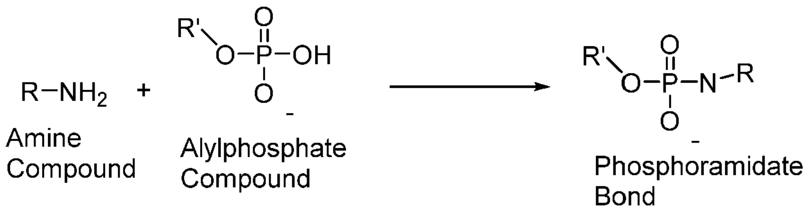


Squarates and cyanomethyl acyl sulfonamides, such as those set forth in Abbasov et al., Nature Chemistry 13: 1081-1092 (2021), can be used to modify amino moieties on lysines.

In some cases, a thiol may be functionalized or modified. For example, the thiol group of cysteine is the most nucleophilic functional group found among the 20 polypeptideogenic amino acids. Through careful control of pH, selective modification over other nucleophilic amino acid residues such as lysine can be readily achieved. Moreover, thiol modification of oligonucleotides may be used to enable derivatization. In some cases, the unique nucleophilicity of thiols can be exploited for selective reaction with a number of alternative electrophiles, which allow efficient and selective attachment to be achieved. For example, one such group includes α-halocarbonyls, with iodoacetamide based reagents finding particular utility. Higher thiol selectivity may be achieved using less electrophilic bromo- and even chloro-derivatives, though reactivity is also reduced. Methylsulfonyl heteroaromatic derivatives can also be used for thiol-specific conjugation. In other cases, alternative thiol-functional groups, such as disulfide-bridging pyridazinediones, carbonylacrylic reagents, and cyclopropenyl ketones may be utilized for bioconjugation.

Three forms of activated halogen derivatives that can be used for functionalization of sulfhydryls include haloacetyl, benzyl halides, and alkyl halides. In each of these compounds, the halogen group may be easily displaced by an attacking nucleophilic substance to form an alkylated derivative with loss of HX (where X is the halogen and the hydrogen comes from the nucleophile).

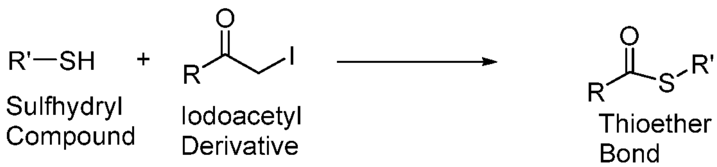


The double bond of a maleimide can undergo an alkylation reaction with a sulfhydryl to form a thioether linkage.

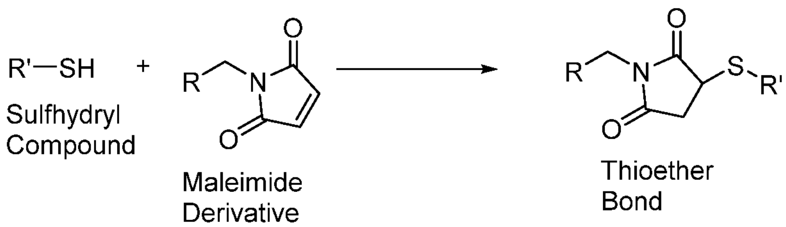


A sulfhydryl can react with an aziridine to form a thioether bond.

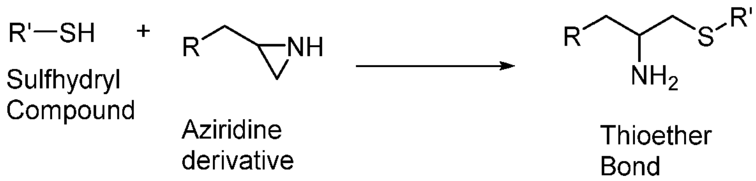


An acryloyl can be reacted with a sulfhydryl to create a thioether linkage.

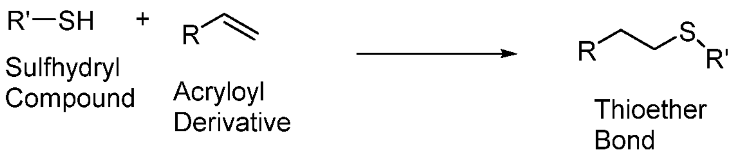


Although aryl halides are commonly used to modify amine-containing molecules to form aryl amine derivatives, they also may react quite readily with sulfhydryl groups. For example, a fluorobenzene can react with a sufhydryl to form an aryl thioether linkage. Conjugates formed with sulfhydryl groups are reversible by cleaving with an excess of thiol (such as DTT).

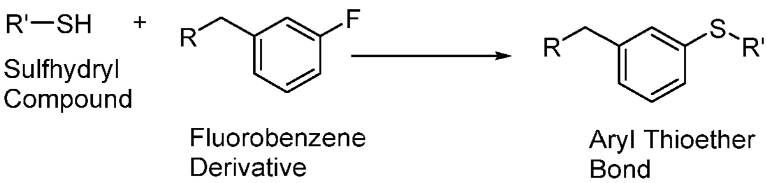


A vinyl sulfone can be reacted with a sulfhydryl to form a beta-thiosulfonyl linkage.

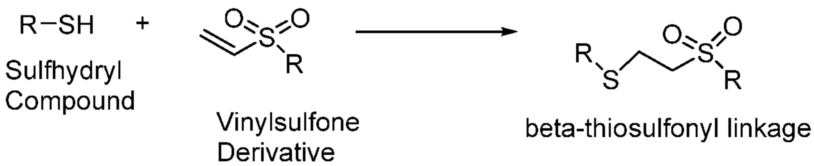


Compounds containing a disulfide group can participate in a disulfide exchange reaction with another thiol.

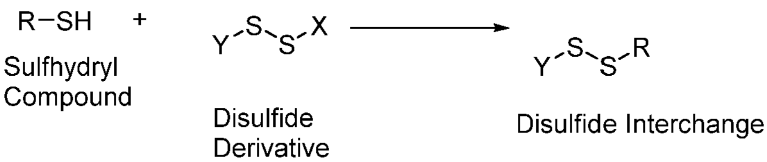


For example, a pyridyl dithiol can undergo an interchange reaction with a free sulfhydryl to yield a single mixed disulfide product.

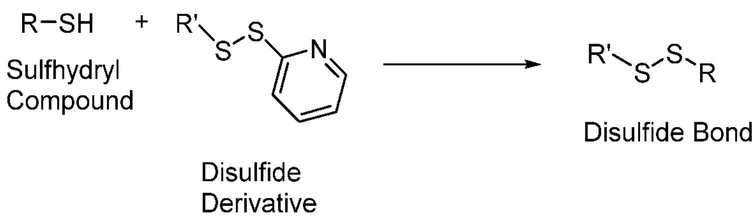


In another example, sulfhydryl groups activated with the leaving group 5-thio-2-nitrobenzoic acid can be used to couple free thiols by disulfide interchange. The disulfide of Ellman's reagent can undergo disulfide exchange with a free sulfhydryl to form a mixed disulfide with concomitant release of one molecule of the chromogenic substance 5-sulfido-2-nitroben-zoate, also called 5-thio-2-nitrobenzoic acid (TNB). The TNB-thiol group can again undergo interchange with a

sulfhydryl to yield a disulfide crosslink. Upon coupling with a sulfhydryl compound, the TNB group is released.

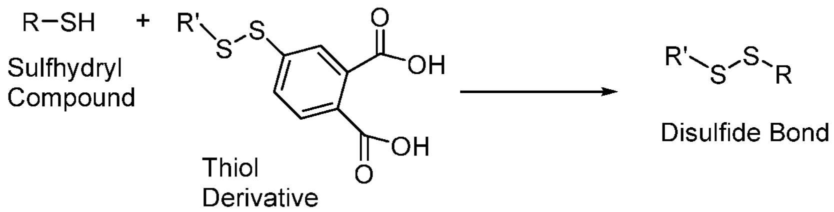


In some cases, disulfide reduction may be performed using thiol-containing compounds such as TCEP, DTT, 2-mercaptoethanol, or 2-mercaptoethylamine. Dinitroimidazoles, such as 1,4-dinitorimidazoles can react with cysteine under acidic to neutral conditions to form a (4-nitroimidazole)-thiol product. See, for example, Luo et al., Nat. Comm. 10: 142 (2019).

Optionally, a carboxylate may be utilized for functionalization or modification of a polypeptide, particle or other substance. For example, *N,N'*-Carbonyl diimidazole (CDI) can react with carboxylic acids under nonaqueous conditions to form N-acylimidazoles of high reactivity. An active carboxylate can then react with amines to form amide bonds or with hydroxyl groups to form ester linkages.

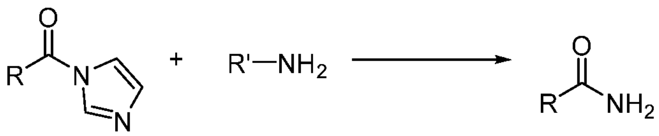

In some cases, carbodiimides function as zero-length crosslinking agents capable of activating a carboxylate group for coupling with an amine-containing compound for attachment. Carbodiimides can be used to mediate the formation of amide or phosphoramidate linkages between a carboxylate and an amine or a phosphate and an amine. *N,N'-Disuccinimidyl* carbonate (DSC) is highly reactive toward nucleophiles. In aqueous solutions, DSC can be hydrolyzed to form two molecules of *N*-hydroxysuccinimide (NHS) with release of one molecule of $CO_2$. In nonaqueous environments, the reagent can be used to activate a hydroxyl group to a succinimidyl carbonate derivative. DSC-activated hydroxylic compounds can be used to conjugate with amine-containing molecules to form stable crosslinked products.

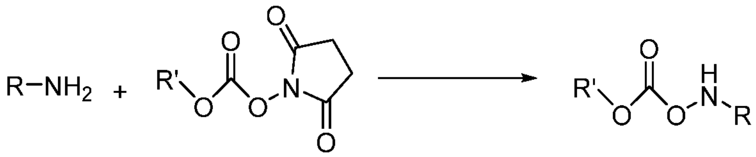

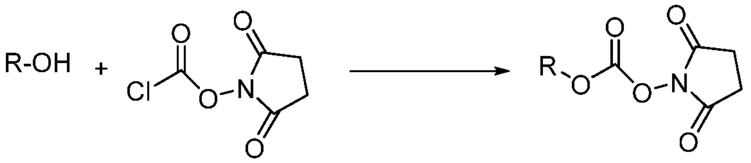

In some cases, sodium periodate can be used to oxidize hydroxyl groups on adjacent carbon atoms, forming reactive aldehyde moieties suitable for coupling with amine- or hydrazide-containing molecules for conjugation. Optionally, reactive alkyl halogen compounds can be used to specifically modify hydroxyl groups for attachment.

In some cases, modification reagents can add a receptor or ligand moiety to a polypeptide, particle or other substance set forth herein. For example, amines, carboxylates, sulfhydryls, carbohydrate groups and other reactive sites can be functionalized with a biotin or (strept)avidin moiety. In some cases, photoreactive biotinylation reagents are used to add a biotin group to a particle or polypeptide, for example, when not containing convenient functional groups for modification. Alternatively, a linkage set forth above can be used. For example, carboxylate-containing biotin compounds can be coupled to amines *via* a carbodiimide-mediated reaction using EDC. In some cases, NHS-iminobiotin can be used to label amine-containing molecules with an iminobiotin moiety. In some cases, Sulfo-NHS-SS-biotin (also known as NHS-SS-biotin) can be used to modify amine-containing polypeptides, paricles or other substances. In some cases, 1-biotinamido-4-[4'-(maleimidomethyl) cyclohexane-carboxamido]butane reacts with sulfhydryls to form stable thioether linkages. In some cases, N-[6-(biotinamido)hexyl]-3'-(2'-pyridyldithio)propionamide, where the reagent contains a 1,6-diaminohexane spacer group which is attached to biotin's valeric acid side chain, may be modified at the terminal amino group of the spacer *via* an amide linkage with the acid precursor of SPDP to create a terminal, sulfhydryl-reactive group. The pyridyl disulfide end of biotin-HPDP may react with free thiol groups to form a disulfide bond with loss of pyridine-2-thione.

Polypeptide modifications set forth herein can be performed in complex samples, including for example, samples having some or all of a proteome. Conditions can be deployed to achieve selective modification of a given type of amino acids. For example, IA-alkyne or EBX2-alkyne can be used to selectively modify cysteines; 2,3 STP-alkyne, ArSq-alkyne or EBA-alkyne can be used to selectively modify lysines; SuTEx2-alkyne or PTAD-alkyne can be used to selectively modify tyrosines; MeTet-alkyne, HC-alkyne or Az-alkyne can be used to selectively modify aspartates and glutamates; CP-alkyne, HMN-alkyne, or MMP-alkyne can be used to selectively modify tryptophans; CP-alkyne can be used to selectively modify histidines, and PhGO-alkyne can be used to selectively modify arginines within a proteome sample, for example, as set forth in Zanon et al., 10.33774/chemrxiv-2021-w7rss-v2 (2021).

A polypeptide may be attached to a particle by a covalent bond or a non-covalent bond. The attachment can occur between a reactive moiety on the polypeptide and a reactive moiety on the particle. The reactive moiety on the polypeptide can be endogenous to the polypeptide, for example, being a reactive moiety of an amino acid side chain group. Alternatively, the reactive moiety can be exogenous to the polypeptide, for example, being produced by functionalization of the polypeptide. Similarly, the reactive moiety on the particle can be endogenous to the structure of the particle or an exogenous moiety that is a modification or addition to the composition of the particle. Reactive moieties on a polypeptide or particle can participate in forming a covalent or non-covalent bond between a particle and polypeptide. Exemplary reactive moieties and attachment configurations include, but are not limited to those set forth above in the context of functionalizing polypeptides or those set forth below. Also, set forth below are methods and compositions for modifying polypeptides or particles to include reactive moieties for use in various attachment configurations.

Any of a variety of covalent or non-covalent chemistries can be used to attach a polypeptide to a particle. Attachment of a polypeptide to a particle can employ chemical conjugation, bioconjugation, enzymatic conjugation, photo-conjugation, thermal-conjugation, or a combination thereof. (Spicer et. al., Chemical Reviews, 118:7702-7743 (2018), or Hermanson, "Bioconjugate Techniques", Academic Press; 3rd Edition, 2013

). Chemistries and methods set forth herein in the context of attaching polypeptides to particles can also be used to functionalize polypeptides to incorporate reactive moieties, functionalize particles to incorporate reactive moieties, or attach particles to other substances such as surfaces, solid supports, sites of an array, or other particles. Optionally, chemistries used in one step of a method set forth herein are orthogonal to chemistries used for other steps. For example, chemistry used to attach polypeptides to particles can be orthogonal to chemistries used to functionalize the polypeptides, functionalize the particles and/or attach the particles to a solid support.

Bioorthogonal chemistries can facilitate selective modification of polypeptides or selective attachment of polypeptides to particles in complex biological milieus, for example, to prevent non-polypeptide molecules from attaching to particles or quenching desired reactions. However, bioorthogonal chemistries need not be deployed in a method set forth herein, for example, when polypeptides are separated from their native milieu or isolated from other biological components. Accordingly, a wide range of other chemistries can be used. In some cases, polypeptides can be modified or attached to particles in non-aqueous solvents, for example, in situations where the polypeptides need not be in a native state.

A polypeptide can be attached to a particle using a bioorthogonal reaction or click chemistry (see, for example, U.S. Pat. Nos. 6,737,236 and 7,427,678

); azide alkyne Huisgen cycloaddition reactions, which use a copper catalyst (see, for example, U.S. Pat. Nos. 7,375,234 and 7,763,736

); Copper-free Huisgen reactions ("metal-free click") using strained alkynes or triazine-hydrazine moieties which can link to aldehyde moieties (see, for example, U.S. Pat. No. 7,259,258 ); triazine chloride moieties which can link to amine moieties; carboxylic acid moieties which can link to amine moieties using a coupling reagent, such as EDC; thiol moieties which can link to thiol moieties; alkene moieties which can link to dialkene moieties that are coupled through Diels-Alder reactions; and acetyl bromide moieties which can link to thiophosphate moieties (see, for example, WO 2005/065814 ). A functional group may be configured to react via a click reaction (e.g., metal-catalyzed azide-alkyne cycloaddition, strain-promoted azide-alkyne cycloaddition, strain-promoted azide-nitrone cycloaddition, strained alkene reactions, thiol-ene reaction, Diels-Alder reaction, inverse electron demand Diels-Alder reaction, [3+2] cycloaddition, [4+1] cycloaddition, nucleophilic substitution, dihydroxylation, thiol-yne reaction, photoclick, nitrone dipole cycloaddition, norbornene cycloaddition, oxanobornadiene cycloaddition, tetrazine ligation, tetrazole photoclick reactions). Exemplary silane-derivative click reactants may include alkenes, alkynes, azides, epoxides, amines, thiols, nitrones, isonitriles, isocyanides, aziridines, activated esters, and tetrazines (e.g., dibenzocyclooctyne - azide, methyltetrazine - transcyclooctylene, epoxide - thiol, etc.). A click reaction can provide an advantageous method of rapidly forming a bond under biologically conducive conditions (e.g., room temperature, aqueous solvents).

Copper-Catalyzed Azide-Alkyne Cycloadditions (CuAAC) can be utilized for attachment of two substances, such as attachment of a particle to a polypeptide. In some cases, the (3 + 2) cycloaddition between an azide and alkyne can yield a mixture of two triazole isomers. To achieve conjugation via CuAAC, a copper(I) catalyst can either be added directly, or generated *in situ* by reduction of an initial copper(II) complex, for example, using ascorbic acid.

Strain-Promoted Azide-Alkyne Cycloadditions (SPAAC) may be utilized for attachment of two substances, such as attachment of a particle to a polypeptide. Highly strained cyclooctynes can react with azides to form triazoles. In some cases, supramolecular host-guest interactions can also be used to promote azide-alkyne cycloaddition.

Inverse-electron demand Diels-Alder reactions (IEDDA) may be utilized for attachment of two substances, such as attachment of a particle to a polypeptide. For example, an IEDDA reaction between 1,2,4,5-tetrazines and strained alkenes or alkynes may be employed. Useful reactive moieties include, for example, strained *trans*-cyclooctenes, functionalized norbornene derivatives, triazines, or spirohexene. In some cases, hetero-Diels-Alder cycloaddition of maleimides and furans may be utilized for attachment. A particularly useful reaction occurs between methyltetrazine (mTz) and transcyclooctene to yield a dihydropyridazine linkage, which may isomerize to a corresponding 1,4-dihydro-isomers or be oxidized to give a pyridazine product. In some cases, oxime and hydrazone may be utilized for attachment of two substances, such as attachment of a particle to a polypeptide. For example, attachment via hydrazone formation can be achieved via difunctional crosslinking.

In some cases, a Diels-Alder reaction can involve covalent coupling of a diene with an alkene to form a six-membered ring.

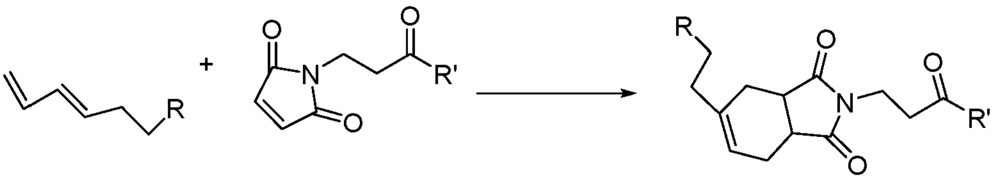

In some cases, transition metal complexes may be utilized for attachment of two substances, such as attachment of a particle to a polypeptide. The nature of late transition metals may make a transition metal complex well suited to the manipulation of unsaturated and polarizable functional groups (olefins, alkynes, aryl iodides, arylboronic acids, etc.). For example, a Pd(0)-catalyst can be used to mediate allyl carbamate deprotections or Suzuki-Miyaura cross-coupling. In other examples, a ruthenium catalyst may be used. For example, with ruthenium complexes, S-allylcysteine can be introduced into polypeptides by a variety of methods, including conjugate addition of allyl thiol to dehydroalanine, direct allylation of cysteine, desulfurization of allyl disulfide, or metabolic incorporation as a methionine surrogate in methionine auxotrophic *E. coli.*

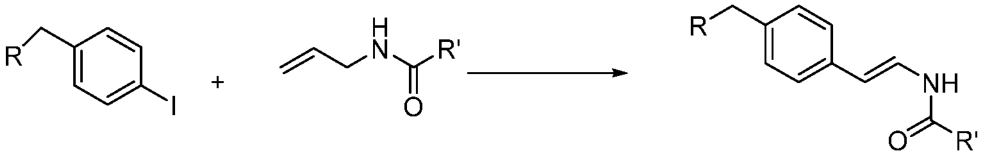

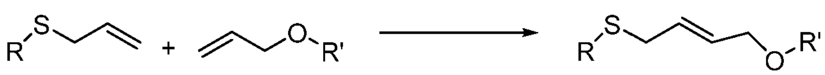

In some cases, complex formation with boronic acid derivatives may be used for attachment of substances, such as attachment of a particle to a polypeptide. For example, boronic acid derivatives are able to form ring structures with other molecules having neighboring functional groups consisting of 1,2- or 1,3-diols, 1,2- or 1,3-hydroxy acids, 1,2- or 1,3-hydroxylamines, 1-2- or 1,3-hydroxyamides, 1,2- or 1,3-hydroxyoximes, as well as various sugars or biomolecules containing these species.

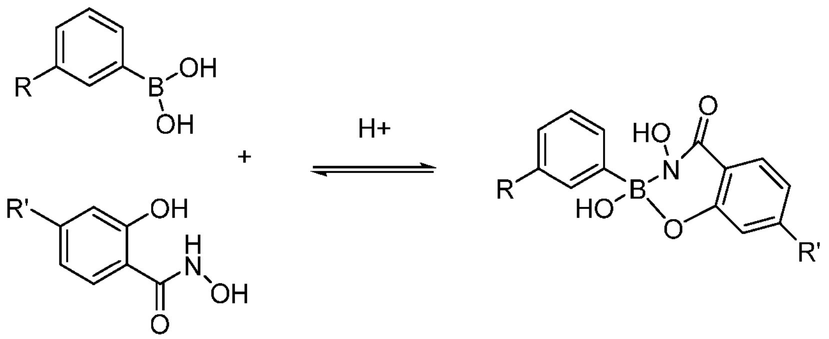

In some cases, enzyme-mediated conjugation may be utilized to attach substances, such as attachment of a particle to a polypeptide. Enzyme-mediated conjugation may proceed via transglutaminases, peroxidases, sortase, SpyTag-SpyCatcher, or a combination thereof. Photo conjugation and activation may proceed via photoacrylate cross-linking reaction, photo thiol-ene reaction, photo thiol-yne reaction, or a combination thereof. In some cases, attachment or conjugation may proceed via noncovalent interactions, these may be through self-assembling peptides, binding sequences, host-guest chemistry, complementary hybridization of nucleic acids, or a combination thereof.

A polypeptide can be attached to a particle by a receptor-ligand binding interaction. For example, binding of (strept)avidin to the small molecule biotin may be used. (Strept)avidin may be attached to a first substance, such as a particle, and biotin may be attached to a second substance, such as a polypeptide, thereby allowing the substances to become attached via binding of the (strept)avidin to the biotin. Other receptor ligand pairs that can be used instead of (strept)avidin-biotin include, but are not limited to, antibodies and their epitopes, aptamers and their epitopes, complementary nucleic acid molecules, lectins and carbohydrates, or nucleic acids and nucleic acid binding polypeptides. Further examples of useful receptor-ligand pairs include probes set forth herein and the targets to which they bind. A polypeptide, particle or other substance set forth herein can be functionalized to include a receptor moiety or ligand moiety, for example, using functionalization chemistries set forth herein.

A reactive handle comprising a functional group may be coupled or conjugated to a polypeptide by a molecule comprising a linking group. A linking group may include molecules or macromolecules that increase the length or size of the functionalization groups. A linking group may include homobifunctional linkers, hetereobifunctional linkers, or other polyfunctional linkers. A linking group may be coupled or conjugated to a polypeptide before a second molecule is coupled or conjugated to the linking groups. A linking group may comprise a flexible region (e.g., a PEG linker) or may be a rigid linker (e.g., a polyunsaturated alkyl group). A rigid linker may facilitate controlling the orientation of a polypeptide when coupled to an anchoring group. A linking group may incorporate a detectable label that facilitates measurement of functional group incorporation or extent of functionalization. For example, an incorporated group in a linking group may comprise a molecule with a measurable and/or quantifiable absorbance (e.g., chromophores) or emission signal (e.g., fluorophores, radiolabels, isotopes, etc.)

A linking group may incorporate a reporting molecule. A reporting molecule may comprise a molecule that is formed or released upon functionalization of a polypeptide. For example, a polypeptide may be functionalized with a functionalizing compound comprising a fluorescent leaving group (e.g., NHS ester coupled to a fluorophore). In some cases, released reporting molecules may be measured to quantify an extent of functionalization.

A functionalized polypeptide fraction may be prepared and/or stored in a functionalized polypeptide solvent composition. A functionalized polypeptide solvent composition may comprise one or more polypeptide composites in solution or suspension. A functionalized polypeptide solvent composition may be formulated to be a homogeneous liquid medium. A functionalized polypeptide solvent composition may be formulated to be a single-phase liquid medium. A functionalized polypeptide solvent composition may be formulated to be a multi-phase liquid medium, such as an oil-in-water emulsion or a water-in-oil emulsion. For a functionalized polypeptide solvent composition formulated as an emulsion, anchoring groups or polypeptide composites may be solvated or suspended within the dissolved phase. In some cases, a functionalized polypeptide composition may be stored in a non-fluid phase, such as polypeptide immobilized on a solid, encapsulated polypeptides (e.g., alginates), or dried or lyophilized polypeptides.

A functionalized polypeptide fraction may comprise a functionalization internal standard. The functionalization internal standard may comprise a plurality of functionalization standard polypeptides. Functionalization standard polypeptides may include polypeptides that undergo functionalization in the same reaction as sample polypeptides. Functionalization standard polypeptides may include polypeptides that were functionalized in a different setting than the sample polypeptides by the same reaction as the sample polypeptides. Functionalization standard polypeptides may include functionalized polypeptides that have had functional groups added by a different method than the method utilized to functionalize the sample polypeptides. A functionalization internal standard may also include non-polypeptide components that may be used to determine the conditions of a polypeptide functionalization method, such as pH indicator dyes or

competitor reagents.

**Anchoring Groups and Polypeptide Coupling**

Polypeptides may be coupled or conjugated to other molecules or materials. The polypeptide(s) can be sample polypeptides, standard polypeptides or other polypeptides, such as those set forth herein. The sample polypeptides can be from a source, sample or fraction including, for example, those set forth herein. In some cases, a polypeptide may be coupled or conjugated to other molecules or materials during or after a polypeptide functionalization reaction. In other cases, a polypeptide may be coupled or conjugated to other molecules or materials without a polypeptide functionalization reaction. For example, a polypeptide within a cell may be directly coupled to a molecule or material. A polypeptide may be coupled or conjugated to another molecule or material before or after the polypeptide has been separated from a sample comprising the polypeptide. The methods and compositions set forth below may generally be exemplified with reference to a functionalized polypeptide; however, it will be understood that the examples can be extended to a population having the same species of functionalized polypeptide, a population having the same species of unfunctionalized polypeptide, a population having different species of functionalized polypeptide, or a population having different species of unfunctionalized polypeptide.

Multiple functionalized or unfunctionalized polypeptides, whether being the same or different species, can be attached to the same molecule or material, for example, the functionalized polypeptides can be attached to a surface to form an array of addresses where each address has a different coupled or conjugated molecule attached or the functionalized polypeptides can be attached to a molecule such as a polymer having multiple attachment sites. Alternatively, multiple functionalized or unfunctionalized polypeptides, whether being the same or different species, can be attached to a population of molecules, beads or particles such that each molecule, bead or particle is attached to a different functionalized or unfunctionalized polypeptide. Moreover, methods and compositions may be exemplified below with reference to proteins; however, it will be understood that the examples can be extended to other polypeptides.

The addition of one or more functional groups on a polypeptide molecule may facilitate the coupling or conjugation of the polypeptide molecule to another molecule or material. In some cases, a polypeptide may be coupled or conjugated to one or more additional molecules to form a polypeptide composite. A polypeptide composite may be formed with a small molecule such as a fluorophore, or a large molecule such as another biomolecule (e.g., polypeptide or nucleic acid). The polypeptide composite may alter the solution properties of a polypeptide from which it is formed. A polypeptide composite may increase or decrease the solubility of a polypeptide, or may render a polypeptide amphiphilic by drawing it toward a liquid/gas or liquid/liquid interface. A polypeptide composite may be formed for any necessary purpose, including localizing or depositing a polypeptide at a surface or adding a detection label to a polypeptide (e.g., fluorophore, radiolabel, nucleic acid tag, streptavidin tag). In some cases, a polypeptide composite may permit characterization of a polypeptide by adding a new functionality or creating a new possible interaction with the polypeptide. For example, a horseradish peroxidase enzyme may be coupled or conjugated to each added functional group on a polypeptide, thereby permitting polypeptide size to be estimated by the activity level of the enzyme.

In some cases, a polypeptide or a plurality of polypeptides may be directly coupled or conjugated onto a solid substrate surface such as a surface at an address of an array, or a surface of a flow cell, microwell or microbead. For example, a polypeptide that has been functionalized with an NHS group may be contacted with a silicon surface covered in an aminated silane monolayer, thereby forming a covalent bond between the polypeptide and the silane. In other cases, a polypeptide may be functionalized with a magnetic nanoparticle that is configured to couple or conjugate with a complementary magnetic material (e.g., a surface with an array of embedded or tethered magnetic nanoparticles).

Polypeptides from a functionalized or unfunctionalized polypeptide fraction may be coupled or conjugated to one or more anchoring groups. An anchoring group may comprise a particle that mediates or facilitates the binding of the polypeptide to a substrate or surface. An anchoring group may comprise a particle that couples a sample polypeptide to a solid support. An anchoring group may comprise a particle such as a nucleic acid particle, a polypeptide, a polymer, an inorganic nanoparticle, an organic nanoparticle, or a combination thereof. An anchoring group may interact with a surface by an interaction such as electrostatic adhesion, magnetic adhesion, covalent bonding, ionic bonding, hydrogen bonding, or coordinate bonding. An anchoring group may interact with a surface in a reversible fashion or an irreversible fashion.

A polypeptide (e.g. a sample polypeptide or standard polypeptide) from a plurality of sample polypeptides may be coupled or conjugated to an anchoring group by a reversible or irreversible interaction. A polypeptide of a plurality of polypeptides may be coupled to an anchoring group of a plurality of anchoring groups by a covalent bond. In some configurations, a polypeptide of a plurality of polypeptides may be coupled to an anchoring group of a plurality of anchoring groups by a click reaction or other covalent coupling chemistry exemplified elsewhere herein. A polypeptide of a plurality of polypeptides may be coupled to an anchoring group of a plurality of anchoring groups by a non-covalent interaction. In some configurations, the non-covalent interaction may be an electrostatic interaction, magnetic interaction, a hydrogen bond, or a binding interaction. In some configurations, the non-covalent hydrogen bond interaction may comprise nucleic acid hybridization. In other configurations, the non-covalent binding interaction may comprise a receptor-ligand interaction

or a receptor-small molecule interaction, such as streptavidin-biotin, FITC-anti-FITC antibody, or digoxigenin-anti-digoxigenin antibody or other non-covalent interaction exemplified elsewhere herein.

An anchoring group may comprise a macromolecule or particle that possesses a positive or negative overall surface charge density. An anchoring group may comprise a macromolecule or particle that possesses a positive or negative region of surface charge density. The surface charge density of an anchoring group may be the opposite charge of a surface that a polypeptide conjugate is to be deposited upon. The surface charge density of an anchoring group may be neutral. The surface charge density of an anchoring group may be uniform over the available surface area of the anchoring group. A uniform surface charge density may increase the speed and/or likelihood of the anchoring group depositing upon a surface or material. Regions of positive or negative surface charge density of an anchoring group may be localized to one or more regions of the anchoring group structure. Localized surface charge density on an anchoring group may cause a polypeptide conjugate containing the anchoring group to deposit on a surface or material with a uniform or controlled orientation. Surface charge density of an anchoring group or a polypeptide conjugate containing an anchoring group may be measured by a suitable method such as electrophoretic measurement of zeta potential. A surface charge density of an anchoring group or a polypeptide conjugate containing an anchoring group may be determined by experimental measurement, computational modeling, or a combination thereof.

Anchoring groups may comprise one or more macromolecules. A suitable macromolecule for an anchoring group may include a macromolecule with a uniform or localized region of positive or negative surface charge density. A macromolecule in an anchoring group may possess a controlled or engineered structure, including a feature such as a polypeptide coupling or conjugation site, or a surface bonding site. A polypeptide coupling or conjugation site on an anchoring group may comprise a functional group configured to react with a functional group of a functionalized or unfunctionalized polypeptide, thereby forming a covalent bond between the anchoring group and the polypeptide. Suitable macromolecules may include nucleic acids, proteins, or polymers. A nucleic acid anchoring group may comprise a structured nucleic acid particle (SNAP) such as a DNA nanoball, DNA nanotube, or DNA origami. A polypeptide-based anchoring group may include an engineered or non-engineered polypeptide that has a tendency to deposit on a surface or material. A polypeptide for a polypeptide-based anchoring group may be prepared for conjugation to a polypeptide from a polypeptide fraction by methods similar to those described above. A polymer-based anchoring group may include ionic or nonionic polymers.

In other configurations, an anchoring group may comprise a particle, such as a nanoparticle, that provides a plurality of attachment sites for two or more binding components, and optionally one or more label components. In some configurations, a particle may comprise a surface that is functionalized, can be functionalized, or is otherwise modifiable to provide attachment sites for polypeptide coupling. In some configurations, a particle may provide a template for a shell, surface coating, or surface layer (e.g., a surface coating comprising a polymer or hydrogel coating, a surface layer of functional groups) that contains or can be modified to contain attachment sites for detectable probe components. A surface coating may comprise a polymer, biopolymer, metal, or metal oxide. In some configurations, an anchoring group may effectively function as a label component (e.g., a fluosphere or quantum dot). A particle for an anchoring group may comprise a surface coating or surface layer that comprises a surface electrical charge. The surface electrical charge may comprise a net positive charge or a net negative charge. An anchoring group may be formulated or modified to comprise a plurality of functional groups that are configured to couple to a solid support by a covalent or non-covalent interaction. In some configurations, the plurality of functional groups may comprise a functional group selected from the group consisting of an alkyl, alkenyl, alkynyl, phenyl, halide, hydroxyl, carbonyl, aldehyde, acyl halide, ester, carboxylate, carboxyl, carboalkoxy, methoxy, hydroperoxy, ether, hemiacetal, hemiketal, acetal, ketal, orthoester, epoxide, carboxylic anhydride, carboxamide, amine, ketimine, aldimine, imide, azide, azo, cyanate, isocyanate, nitrate, nitrile, isonitrile, nitrosoxy, nitro, nitroso, oxime, pyridyl, carbamate, sulfhydryl, sulfide, disulfide, sulfinyl, sulfonyl, sulfinom, sulfo, thiocyanate, isothiocyanate, carbonothioyl, thioester, thionoester, phosphino, phosphono, phosphonate, phosphate, borono, boronate, and a borinate. In some configurations, an anchoring group may be modified to comprise a functional group that is configured to undergo a click reaction. In other configurations, an anchoring group may be modified to comprise a functional group that is configured to undergo a chemical cross-linking or a photo-initiated cross-linking reaction.

An anchoring group may comprise a detectable label that permits detection of the anchoring group. A detectable label may comprise a fluorescent label, a luminescent label, a radiolabel, an enzymatic tag, or a nucleic acid label or barcode. An anchoring group may be conjugated with a detectable label. The conjugated detectable label may be conjugated by a covalent bond (e.g., a reactive dye) or a non-covalent interaction (e.g., hybridization of a nucleic acid tag, an intercalation dye). A detectable label may be used to quantify anchoring groups in solution or detect anchoring groups at individual locations on a substrate.

An anchoring group or a linkage between an anchoring group and a polypeptide may further comprise a linker. A linker may comprise a bifunctional, trifunctional, or polyfunctional linker. A bifunctional linker may comprise a homobifunctional linker or a heterobifunctional linker. A linker may include a reporting molecule that is released upon successful coupling of an anchoring group to a polypeptide. For example, a click-to-release strategy may be utilized to covalently couple a polypeptide comprising a click handle with an anchoring group comprising a second click handle (e.g., inverse-electron demand Diels Alder click-to-release).

An anchoring group may be configured to be coupled or conjugated to a functionalized or unfunctionalized polypeptide (e.g. sample polypeptide or standard polypeptide). Functional groups capable of rapidly forming covalent bonds with functionalized or unfunctionalized polypeptides may be of particular interest for the functionalization of anchoring groups. In general, functional groups of interest will include most common species for bioconjugation. Such functional groups may include "click" reagents that are capable of forming highly specific products with complementary functional groups in a rapid and irreversible fashion. Exemplary functionalization chemistries are described above. An anchoring group may comprise one or more sites for polypeptide coupling or conjugation. An anchoring group with more than one attachment site may be capable of coupling or conjugating more than one polypeptide. An anchoring group may comprise a fixed number of polypeptide attachment sites, such as about 1, 2, 3, 4, 5, 6, 7, 8, 9, 10, 20, 30, 40, 50, 75, 100, 200, 300, 400, 500, 1000, 5000, 10000, 50000, 100000, 500000, 1000000, or more than 1000000 attachment sites. An anchoring group may comprise a fixed number of polypeptide attachment sites, such as at least about 1, 2, 3, 4, 5, 6, 7, 8, 9, 10, 20, 30, 40, 50, 75, 100, 200, 300, 400, 500, 1000, 5000, 10000, 50000, 100000, 500000, 1000000, or more than 1000000 attachment sites. Alternatively or additionally, an anchoring group may comprise a fixed number of polypeptide attachment sites, such as no more than about 1000000, 500000, 100000, 50000, 10000, 5000, 1000, 500, 400, 300, 200, 100, 75, 50, 40, 30, 20, 10, 9, 8, 7, 6, 5, 4, 3, 2, or less than 2 attachment sites.

A coupling or conjugation reaction between a functionalized polypeptide and an anchoring group may be performed in a fashion that yields substantially complete coupling or conjugation of all polypeptides, all anchoring groups, or a combination of both. For example, polypeptide may be supplied in excess such that nearly all anchoring groups become coupled or conjugated to a polypeptide by completion of the reaction. Alternatively, an anchoring group may be supplied in excess such that all polypeptides in a polypeptide fraction become coupled or conjugated to an anchoring group by completion of the reaction. A coupling or conjugation reaction may be performed with a polypeptide: anchoring group ratio of about 1:1000, 1:500, 1:250, 1:100, 1:50, 1:25, 1:10, 1:5, 1:4, 1:3, 1:2, 1:1, 2:1, 3:1, 4:1, 5:1, 6:1, 7:1, 8:1, 9:1, 10:1, 20:1, 25:1, 30:1, 50:1, 100:1, 250:1, 500:1, 1000:1, or more. A conjugation reaction may be performed with a polypeptide: anchoring group ratio of at least about 1:1000, 1:500, 1:250, 1:100, 1:50, 1:25, 1:10, 1:5, 1:4, 1:3, 1:2, 1:1, 2:1, 3:1, 4:1, 5:1, 6:1, 7:1, 8:1, 9:1, 10:1, 20:1, 25:1, 30:1, 50:1, 100:1, 250:1, 500:1, 1000:1, or more. Alternatively or additionally, a conjugation reaction may be performed with a polypeptide:anchoring group ratio of no more than about 1000:1, 500:1, 250:1, 100:1, 50:1, 30:1, 25:1, 20:1, 10:1, 9:1, 8:1, 7:1, 6:1, 5:1, 4:1, 3:1, 2:1, 1:1, 1:2, 1:3, 1:4, 1:5, 1:10, 1:25, 1:50, 1:100, 1:250, 1:500, 1:1000, or more.

A conjugation reaction between a functionalized or unfunctionalized polypeptide (e.g., sample polypeptide or standard polypeptide) and an anchoring group may occur at a fixed temperature. In some cases, a coupling or conjugation reaction may occur at a first temperature for a fixed amount of time, then a second temperature for a fixed amount of time. A coupling or conjugation reaction between a functionalized or unfunctionalized polypeptide and an anchoring group may occur at a temperature of about - 80 °C, - 70 °C, - 60 °C, - 50 °C, - 40 °C, - 30 °C, - 20 °C, - 10 °C, - 5 °C, 0 °C, 4 °C, 10 °C, 20 °C, 30 °C, 37 °C, 40 °C, 50 °C, 60 °C, 70 °C, 80 °C, 90 °C, or about 95 °C. A coupling or conjugation reaction between a functionalized polypeptide and an anchoring group may occur at a temperature of at least about - 80 °C, - 70 °C, - 60 °C, - 50 °C, - 40 °C, -30 °C, -20 °C, -10 °C, -5 °C, 0 °C, 4 °C, 10 °C, 20°C, 30°C, 37 °C, 40 °C, 50 °C, 60°C, 70 °C, 80 °C, 90 °C, or about 95 °C. Alternatively or additionally, a conjugation reaction between a functionalized or unfunctionalized polypeptide and an anchoring group may occur at a temperature of no more than about 95 °C, 90 °C, 80 °C, 70 °C, 60 °C, 50 °C, 40 °C, 37 °C, 30 °C, 20 °C, 10 °C, 4 °C, 0 °C, - 5 °C, - 10 °C, - 20 °C, - 30 °C, - 40 °C, - 50 °C, -60 °C, - 70 °C, or about - 80 °C.

A conjugation reaction between a functionalized or unfunctionalized polypeptide (e.g., sample polypeptide or standard polypeptide) and an anchoring group may occur for a fixed amount of time. A conjugation reaction between a functionalized or unfunctionalized polypeptide and an anchoring group may occur for about 1 min, 30 mins, 1 hr, 2 hrs, 3 hrs, 4 hrs, 5 hrs, 6 hrs, 7 hrs, 8 hrs, 9 hrs, 10 hrs, 11 hrs, 12 hrs, 13 hrs, 14 hrs, 15 hrs, 16 hrs, 17 hrs, 18 hrs, 19 hrs, 20 hrs, 21 hrs, 22 hrs, 23 hrs, 1 day, 2 days, 3 days, 4 days, 5 days, 6 days, 7 days, 10 days, 2 wks, 3 wks, 4 wks, 1 mth, or more than 1 mth. A conjugation reaction between a functionalized or unfunctionalized polypeptide and an anchoring group may occur for at least about 1 min, 30 mins, 1 hr, 2 hrs, 3 hrs, 4 hrs, 5 hrs, 6 hrs, 7 hrs, 8 hrs, 9 hrs, 10 hrs, 11 hrs, 12 hrs, 13 hrs, 14 hrs, 15 hrs, 16 hrs, 17 hrs, 18 hrs, 19 hrs, 20 hrs, 21 hrs, 22 hrs, 23 hrs, 1 day, 2 days, 3 days, 4 days, 5 days, 6 days, 7 days, 10 days, 2 wks, 3 wks, 4 wks, 1 mth, or more than 1 mth. Alternatively or additionally, a conjugation reaction between a functionalized or unfunctionalized polypeptide and an anchoring group may occur for no more than about 1 month, 4 wks, 3 wks, 2 wks, 10 days, 7 days, 6 days, 5 days, 4 days, 3 days, 2 days, 1 day, 23 hrs, 22 hrs, 21 hrs, 20 hrs, 19 hrs, 18 hrs, 17 hrs, 16 hrs, 15 hrs, 14 hrs, 13 hrs, 12 hrs, 11 hrs, 10 hrs, 9 hrs, 8 hrs, 7 hrs, 6 hrs, 5 hrs, 4 hrs, 3 hrs, 2 hrs, 1 hr, 30 mins, 1 min, or less than 1 min.

An anchoring group may be coupled or conjugated to a functionalized or unfunctionalized polypeptide (e.g., sample polypeptide or standard polypeptide). An anchoring group may be coupled or conjugated to a functionalized polypeptide before or after the anchoring group has been deposited on a surface. **FIG. 9A** depicts a broad scheme for forming a polypeptide composite then depositing the polypeptide composite on a surface or material. A polypeptide **910** comprising a first functional group **920** may be contacted with an anchoring group **940** containing a second functional

group **930.** The first functional group **920** may be configured to form a covalent bond with the second functional group **930.** The reaction between the first functional group **920** and the second functional group **930** forms a polypeptide composite **950.** The reaction between the polypeptide **910** and the anchoring group **940** may occur in the presence of a detergent. The reaction between the polypeptide **910** and the anchoring group **940** may occur in the presence of a standard polypeptide, such as a coupling standard polypeptide. After a polypeptide composite **950** has been formed, the polypeptide composite **950** may be contacted with a surface or material **960** to deposit the polypeptide composite **950** on the surface or material **960.** The polypeptide composite **950** may become attached to the surface or material **960** by a covalent or non-covalent interaction. The deposition of the polypeptide composite **950** on the surface or material **960** may occur in the presence of a detergent. The deposition of the polypeptide composite **950** on the surface or material **960** may occur in the presence of a standard polypeptide, such as a deposition standard polypeptide.

**FIG. 9B** depicts an alternate scheme for forming a polypeptide composite. An anchoring group **940** comprising a second functional group **930** is brought in contact with a surface or material **960.** The anchoring group **940** may become coupled to the surface or material **960** by a covalent or non-covalent interaction. The deposition of the anchoring group **950** on the surface or material **960** may occur in the presence of a detergent. The deposition of the anchoring group **950** on the surface or material **960** may occur in the presence of a standard polypeptide, such as a deposition standard polypeptide. The deposited anchoring group **940** on the surface or material **960** may be subsequently contacted with a polypeptide **910** containing a first functional group **920.** The first functional group **920** may be configured to form a covalent bond with the second functional group **930.** The reaction between the first functional group **920** and the second functional group **930** forms a polypeptide composite **950** that is deposited on a surface or material **960.** The reaction between the polypeptide **910** and the anchoring group **940** may occur in the presence of a detergent. The reaction between the polypeptide **910** and the anchoring group **940** may occur in the presence of a standard polypeptide, such as a coupling standard polypeptide.

A polypeptide composite (e.g., sample polypeptide composite or standard polypeptide composite) may be prepared and/or stored in a polypeptide composite solvent composition. A polypeptide composite solvent composition may comprise one or more polypeptide composites in solution or suspension. A polypeptide composite solvent composition may be formulated to be a homogeneous liquid medium. A polypeptide composite solvent composition may be formulated to be a single-phase liquid medium. A polypeptide composite solvent composition may be formulated to be a multi-phase liquid medium, such as an oil-in-water emulsion or a water-in-oil emulsion. For a polypeptide composite solvent composition formulated as an emulsion, anchoring groups or polypeptide composites may be solvated or suspended within the dissolved phase.

A polypeptide composite solvent composition may comprise a stable solution or suspension of polypeptide composites (e.g., sample polypeptide composites or standard polypeptide composites). A stable solution or suspension of polypeptide composites may exhibit no detectable sedimentation or agglomeration of polypeptide composites over a fixed time period such as about 1 min, 5 mins, 10 mins, 30 mins, 1 hr, 2 hrs, 3 hrs, 6 hrs, 12 hrs, 1 day, 2 days, 3 days, 4 days, 5 days, 6 days, 1 week, 2 wks, 3 wks, 4 wks, 1 mth, 3 mths, 6 mths, 1 yr, or more than 1 yr.

A polypeptide conjugate solvent composition may be utilized for the storage of prepared polypeptide conjugates(e.g., sample polypeptide composites or standard polypeptide composites). A polypeptide conjugate solvent composition may be utilized for the deposition of polypeptide conjugates on a substrate. In some cases, polypeptide conjugates may be deposited on a substrate in a polypeptide conjugate solvent composition without further modification of the solvent composition. In other cases, polypeptide conjugates may be deposited on a substrate in a polypeptide conjugate solvent composition that is modified at the time of deposition (e.g., adding a component, adjusting a component concentration, changing the composition pH). In other cases, a polypeptide conjugate solvent composition may be exchanged (e.g., by dialysis, filtration, or extraction) for a deposition solvent composition before polypeptide conjugates are deposited on a substrate.

A coupled or conjugated polypeptide fraction may comprise a coupling internal standard. The coupling internal standard may comprise a one or a plurality of coupling standard polypeptides. Coupling standard polypeptides may include polypeptides that undergo coupling by the same coupling method as sample polypeptides. Coupling standard polypeptides may include polypeptides that were coupled in a different setting than the sample polypeptides by the same method as the sample polypeptides. Coupling standard polypeptides may include coupled polypeptides that have had anchoring groups added by a different method than the method utilized to couple the sample polypeptides. A coupling internal standard may also include non-polypeptide components that may be used to determine the conditions of a polypeptide coupling method, such as pH indicator dyes or competitor reagents. A coupling standard may comprise a polypeptide that characterizes the functionalization and/or deposition efficiency of low- or no-residue polypeptides for a particular functionalization chemistry. For example, a coupling standard for a lysine-targeting functionalization chemistry may comprise polypeptides with few lysines (e.g., no more than about 5, 4, 3, 2, 1, or 0 lysine residues), in which the polypeptides with few lysines are less likely to conjugate to an anchoring group. A coupling standard for a low- or no-residue polypeptide may be measured in a downstream fraction (e.g., after coupled polypeptide conjugates are separated from unused reagents) or by deposition of the standard on the chip (e.g., presence of the coupling standard when no standard is expected).

A plurality of polypeptides may be separated into a plurality of polypeptide fractions where each polypeptide fraction is distinguished by a unique species of coupled or conjugated anchoring group. For example, a sample mixture comprising sample polypeptides and separation standard polypeptides may be formed by individually coupling sample polypeptides to a first species of anchoring group and separation standard polypeptides to a second species of anchoring group, then combining the two polypeptide fractions into a sample mixture. Species of anchoring groups may be distinguished by shape; configuration (e.g., presence or absence of modifying groups, presence or absence of coupling groups, etc.); presence, absence, or type of detectable label (e.g., a fluorophore); or coupling specificity. Two or more species of anchoring groups may be configured to self-assemble into an array. In some configurations, two or more species of anchoring groups may self-assemble due to complementary coupling groups (e.g., nucleic acids) on each species of anchoring group.

Differing species of anchoring groups may be formed for the purpose of distinguishing different types of polypeptides (e.g. different types of sample polypeptides or different types of standard polypeptides). In some configurations, a polypeptide sample may be divided into separate fractions (e.g., by size, by location in cell, by hydrophobicity, etc.), with each separate fraction being placed on a different species of anchoring group. Each of the fractions can optionally include a different type of standard polypeptide, wherein the differences can be detected to indicate a characteristic common to the sample polypeptides in the respective fraction. In other configurations, sample polypeptides may be coupled to one species of anchoring group and a standard or control polypeptide may be coupled to a different species of anchoring group. **FIG. 10** illustrates a method of forming differing species of polypeptide composites by selectively capturing polypeptides from a polypeptide sample onto differing structured nucleic acid particles (SNAPs). A square species of SNAP comprising an amine reactive group **1020** and a triangular species of SNAP comprising a DBCO reactive group **1030** are contacted with a polypeptide sample comprising differentially functionalized polypeptides, including carboxylated polypeptides **1010,** activated ester-labeled polypeptides **1011,** azide-labeled polypeptides **1012,** and hydroxyl-labeled polypeptides **1013.** Due to the relative reactivities of the SNAP-based reactive groups and the polypeptide-based reactive groups, the square species of SNAP **1020** may covalently conjugate to the activated ester-labeled polypeptide **1011** to form a polypeptide-coupled SNAP. Likewise, the triangular species of SNAP **1030** may covalently conjugate to the activated ester-labeled polypeptide 1012 to form a polypeptide-coupled SNAP.

A sample polypeptide composite fraction prepared by a sample polypeptide coupling method comprising a plurality of sample polypeptides may comprise a characterized percentage of the total quantity of sample polypeptides in a sample. A sample polypeptide composite fraction comprising a plurality of sample polypeptides may contain at least about 0.000001%, 0.000005%, 0.00001%, 0.00005%, 0.0001%, 0.0005%, 0.001%, 0.005%, 0.01%, 0.05%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, 99.9%, 99.99%, 99.999%, 99.9999%, 99.99999%, or more than 99.99999% of the total quantity of sample polypeptides on a mass basis. Alternatively or additionally, a sample polypeptide composite fraction comprising a plurality of sample polypeptides may contain no more than about 99.99999%, 99.9999%, 99.999%, 99.99%, 99.9%, 99.5%, 99%, 95%, 90%, 85%, 80%, 75%, 70%, 65%, 60%, 55%, 50%, 45%, 40%, 35%, 30%, 25%, 20%, 15%, 10%, 5%, 1%, 0.5%, 0.1%, 0.05%, 0.01%, 0.005%, 0.001%, 0.0005%, 0.0001%, 0.00005%, 0.00001%, 0.000005%, 0.000001%, or less than 0.000001% of the total quantity of sample polypeptides on a mass basis.

## Preparation of Polypeptide Arrays

A polypeptide array may be formed by coupling a plurality of polypeptides from a sample to a solid support. Some or all of the polypeptides can optionally be conjugated to anchoring groups to form polypeptide composites. Optionally, the anchoring group can mediate attachment of the polypeptide to the solid support. In an exemplary method, a polypeptide array comprising a plurality of polypeptide composites may be formed by one or more of the steps of: 1) providing a sample comprising a plurality of sample polypeptides; 2) separating the plurality of sample polypeptides from the sample in the presence of a plurality of separation standard polypeptides; 3) coupling the plurality of sample polypeptides to a plurality of anchoring groups to form a plurality of sample polypeptide composites, wherein the coupling occurs in the presence of a plurality of coupling standard polypeptides; 4) attaching each sample polypeptide composite of the plurality of sample polypeptide composites to a solid support at an address of a plurality of addresses, thereby forming a polypeptide array comprising the plurality of sample polypeptide composites attached to the solid support at the plurality of addresses; and optionally 5) repeating any one of steps 1) - 4) at least once. In some cases, one or more of steps 1) - 3) may occur in the presence of the solid support. In some cases, all of steps 1) - 4), and optionally step 5), may occur in the presence of the solid support. For example, certain above-described targeting separation methods may be facilitated by coupling a targeting agent to the solid support, then coupling a sample polypeptide from the plurality of sample polypeptides to the targeting agent, thereby coupling the sample polypeptide to the solid support. In other cases, one or more, or all of steps 1) - 5) may occur in the absence of the solid support. For example, certain above-described fractionation methods may be performed on an offline instrument, followed by coupling of sample polypeptides to anchoring groups occurring in the presence of the solid support. The attaching of step 4) may be carried out in the presence of a plurality of attachment

standard polypeptides, and as a further option the attachment standard polypeptides may be attached to the solid support at individual addresses, respectively. In yet a further option, the attaching of step 4) may further include attaching each separation standard polypeptide of the plurality of separation standard polypeptides to the solid support at an address of the plurality of addresses, and/or attaching each coupling standard polypeptide of the plurality of coupling standard polypeptides to the solid support at an address of the plurality of addresses.

An anchoring group or a polypeptide composite containing an anchoring group may be deposited on a substrate surface or material. The methods and compositions set forth below may generally be exemplified with reference to an anchoring group or polypeptide composite; however, it will be understood that the examples can be extended to a population having the same species of anchoring groups, a population having different species of anchoring groups, a population having the same species of polypeptide composite, or a population having different species of polypeptide composite. Moreover, methods and compositions may be exemplified below with reference to proteins; however, it will be understood that the examples can be extended to other polypeptides.

A polypeptide may be conjugated to an anchoring group before or after deposition of the anchoring group on a solid support. The deposition of an anchoring group on a solid support or material may be driven by a physical phenomenon such as electrostatic interactions, magnetic interactions, hydrophobic interactions, hydrophilic interactions, covalent bonding, or non-covalent bonding. In some cases, the deposition of an anchoring group may be due to the electrostatic interaction between a negatively-charged anchoring group and a positively-charged solid support (or other material), or vice versa.

Polypeptides need not necessarily be conjugated to anchoring groups in a method of forming an array of polypeptides. For example, a polypeptide array comprising a plurality of polypeptides may be formed by one or more of the steps of: 1) providing a sample comprising a plurality of sample polypeptides; 2) separating the plurality of sample polypeptides from the sample in the presence of a plurality of separation standard polypeptides; 3) attaching each sample polypeptide of the plurality of sample polypeptides to a solid support at an address or a plurality of addresses, thereby forming a polypeptide array comprising the plurality of sample polypeptides attached to the solid support at the plurality of addresses; and optionally 4) repeating any one of steps 1) - 3) at least once. In some cases, step 1) or 2) may occur in the presence of the solid support. In some cases, all of steps 1) - 3), and optionally step 4), may occur in the presence of the solid support. For example, certain above-described targeting separation methods may be facilitated by coupling a targeting agent to the solid support, then coupling a sample polypeptide from the plurality of sample polypeptides to the targeting agent, thereby coupling the sample polypeptide to the solid support. In other cases, steps 1) or 2) may occur in the absence of the solid support. For example, certain above-described fractionation methods may be performed on an offline instrument. The attaching of step 3) may be carried out in the presence of a plurality of attachment standard polypeptides, and as a further option the attachment standard polypeptides may be attached to the solid support at individual addresses, respectively. In yet a further option, the attaching of step 3) may further include attaching each separation standard polypeptide of the plurality of separation standard polypeptides to the solid support at an address of the plurality of addresses.

A polypeptide array may be formed such that a single polypeptide of a plurality of polypeptides is coupled to a unique address on a solid support (i.e. no more than one of the polypeptides is coupled to the unique address). A polypeptide array may be formed such that two or more polypeptides of a plurality of polypeptides are coupled to a unique address on a solid support. A polypeptide array may be formed such that a single sample polypeptide of a plurality of sample polypeptides is coupled to a unique address on a solid support (i.e. no more than one of the sample polypeptides is coupled to the unique address). A polypeptide array may be formed such that two or more sample polypeptides of a plurality of sample polypeptides are coupled to a unique address on a solid support. A polypeptide array may be formed such that a single standard polypeptide of a plurality of standard polypeptides is coupled to a unique address on a solid support (i.e. no more than one of the standard polypeptides is coupled to the unique address). A polypeptide array may be formed such that two or more sample polypeptides of a plurality of sample polypeptides are coupled to a unique address on a solid support. A polypeptide array may be formed such that a single anchoring group of a plurality of anchoring groups is coupled to a unique address on a solid support (i.e. no more than one of the anchoring groups is coupled to the unique address). A polypeptide array may be formed such that two or more anchoring groups of a plurality of anchoring groups are coupled to a unique address on a solid support. A polypeptide array may be formed such that a single polypeptide composite of a plurality of polypeptide composites is coupled to a unique address on a solid support (i.e. no more than one of the polypeptide composites is coupled to the unique address). A polypeptide array may be formed such that two or more polypeptide composites of a plurality of polypeptide composites are coupled to a unique address on a solid support. A polypeptide array may be formed such that a single sample polypeptide composite of a plurality of sample polypeptide composites is coupled to a unique address on a solid support (i.e. no more than one of the sample polypeptide composites is coupled to the unique address). A polypeptide array may be formed such that two or more sample polypeptide composites of a plurality of sample polypeptide composites are coupled to a unique address on a solid support. A polypeptide array may be formed such that a single standard polypeptide composite of a plurality of standard polypeptide composites is coupled to a unique address on a solid support (i.e. no more than one of the standard polypeptide

composites is coupled to the unique address). A polypeptide array may be formed such that two or more standard polypeptide composites of a plurality of standard polypeptide composites are coupled to a unique address on a solid support.

The quantity of polypeptides (e.g. sample polypeptides and/or standard polypeptides), anchoring groups, and/or polypeptide composites (e.g. sample polypeptide composites and/or standard polypeptide composites) coupled to a solid support at a unique address on the solid support may be controlled by the size of the binding sites on the solid support relative to the size of the polypeptides, anchoring groups and/or polypeptide composites. For example, steric exclusion can be exploited, whereby individual binding sites are sized to accommodate only a single polypeptide, anchoring group and/or polypeptide composite. The quantity of polypeptides, anchoring groups, and/or polypeptide composites coupled to a solid support at a unique address on the solid support may be controlled by the use of surface modification groups (e.g., steric groups) to obstruct deposition, or alteration of concentrations of deposited components, such as anchoring groups or polypeptide composites.

Polypeptides or polypeptide composites may be deposited on a solid support surface or material to form a patterned, ordered, or unordered array of polypeptide composites. In some cases, the solid support surface or material may be structured, engineered, or fabricated to control where the deposition of polypeptide composites may occur. The solid support surface or material may contain localized or uniform regions of positive or negative surface charge density that promote electrostatic interactions with an anchoring group of a polypeptide composite. A solid support surface or material may be deposited with a coating, layer, or functional group that alters the surface charge density of the surface or material to promote electrostatic interactions with an anchoring group of a polypeptide composite. A solid support surface or material may be functionalized with a chemical species that permits direct covalent attachment of an anchoring group to the surface or material. In some configurations, an anchoring group of a plurality of anchoring groups may be coupled to the solid support by a covalent bond. The covalent bond may be formed between a first reactive handle on the anchoring group of the plurality of anchoring groups and a second reactive handle on the solid support.

Before, during, or after deposition of polypeptides on a polypeptide array, a detectable quencher may be contacted with a plurality of polypeptide conjugates. A detectable quencher may be configured to couple to an anchoring group that does not comprise a polypeptide. A detectable quencher may comprise a complementary coupling moiety that is configured to couple or conjugate to a coupling moiety of an anchoring group. For example, an detectable quencher comprising a methyltetrazine (mTz) reactive group may be configured to conjugate to an anchoring group comprising a transcyclooctene (TCO) coupling moiety. In another example, a detectable quencher may comprise an oligonucleotide that is complementary to a coupling oligonucleotide of an anchoring group. In some cases, a detectable quencher may comprise a fluorescent or luminescent moiety. In a particular case, a detectable quencher may comprise a fluorescent moiety that is configured to comprise a fiducial element when an anchoring group coupled to the fluorescent quencher is deposited on a polypeptide array.

A solid support may be formulated or modified to comprise a plurality of functional groups that are configured to couple to a polypeptide (e.g. sample polypeptide or standard polypeptide) or an anchoring group by a covalent or non-covalent interaction. In some configurations, the plurality of functional groups may comprise a functional group selected from those set forth herein in the context of functional groups for anchoring groups.

A solid support may be configured to form a non-covalent interaction with a polypeptide (e.g. sample polypeptide or standard polypeptide) or an anchoring group. In some cases, the non-covalent interaction may comprise an electrostatic interaction, a magnetic interaction, a hydrogen bond, or a binding interaction. In some configurations, the non-covalent hydrogen bond interaction may comprise nucleic acid hybridization. In other configurations, the non-covalent binding interaction may comprise a receptor-ligand interaction or a receptor-small molecule interaction, such as streptavidin-biotin, FITC-anti-FITC antibody, or digoxigenin-anti-digoxigenin antibody.

A solid support may comprise a material with desired characteristics such as hydrophobicity or hydrophilicity, amphipathicity, low adhesion of particular chemical or biological species, and particular chemical, optical, electrical, or mechanical properties. In some cases, a solid support material may be chosen for its compatibility with a detection technique or method (e.g., confocal fluorescent microscopy). For example, a material may be selected due to its low autofluorescence characteristic if a fluorescent detection method is to be utilized. A solid support may be a solid surface to which molecules can be covalently or non-covalently attached. Non-limiting examples of solid supports include slides, coverslips, surfaces of elements of devices, membranes, flow cells, wells, chambers, and macrofluidic chambers. Solid supports used herein may be flat or curved, or can have other shapes, and can be smooth or textured. In some cases, solid support surfaces may contain wells (e.g. microwells or nanowells). In some cases, solid support surfaces may contain one or more microwells in combination with one or more nanowells. A solid support may comprise polymers, glasses, semiconductors (e.g., silicon, germanium), ceramics, metals, minerals, a combination thereof, or other materials. In some instances, a solid support may comprise components made of a glass such as silicon dioxide, borosilicate glass, fused silica, or quartz. In other instances, a solid support may comprise an optical glass or a photochromatic glass. In some cases, a glass with a high sodium or potassium content may be selected as a material for a fluidic device component. A solid support may be fabricated from polymers or plastics such as polycarbonate, polyethylene, polypropylene, polyethylene terephthalate, polyvinyl chloride, polymethyl methacrylate, polydimethylsiloxane, polystyrene acrylics, latex and others. A solid support may comprise metals, metal oxides, and metal alloys such as stainless steel, brass, bronze, aluminum, gold, chromium, titanium, titanium oxide, tin oxide, zirconium oxide, or silicon dioxide. A solid support may comprise carbohydrates such as dextrans or cellulose. In some cases, a solid support may comprise two or more components with different (e.g. plastic vs. glass) or differing (e.g. borosilicate vs. quartz glass) material types. The solid support may have properties that are modified by the presence of polypeptide or polypeptide composites (e.g. intrinsic fluorescence that is blocked or shifted due to binding of polypeptide). A solid support may be patterned to create addresses having one or more of the materials or structural features exemplified above. The addresses may optionally be separated from each other by interstitial regions that lack the materials or have a different material from the addresses. The interstitial regions can optionally be selected from the materials or structural features exemplified above.

A solid support may be contained within a fluidic device. The fluidic device may comprise a flow cell, a microfluidic device, a cartridge, a tube such as a capillary tube, a channel, or a chip. In some configuration, a fluidic device may comprise a plurality of solid supports. A solid support of the plurality of solid supports may be fluidically isolated or fluidically connected to one or more additional solid supports.

A solid support or an address on a solid support may be characterized by a thickness or depth. The thickness of a solid support may be uniform or may vary over the body of the solid support. The thickness of the solid support may be altered by a fabrication, forming or machining process. In some cases, a solid support or address may have a thickness of about 1 micrometer (μm), 10 μm, 50 μm, 100 μm, 250 μm, 500 μm, 750 μm, 1 millimeter (mm), 5 mm, 1 centimeter (cm), 10 cm or more. In some cases, a solid support may have a thickness of at least about 1 micrometer (μm), 10 μm, 50 μm, 100 μm, 250 μm, 500 μm, 750 μm, 1 millimeter (mm), 5 mm, 1 centimeter (cm), 10 cm or more than 10 cm. Alternatively or additionally, a solid support or address may have a thickness of no more than about 10 cm, 1 cm, 5 mm, 1 mm, 750 μm, 500 μm, 250 μm, 100 μm, 50 μm, 10 μm, 1 μm or less.

A solid support or address may comprise one or more surface coatings. A surface coating may be organic or inorganic. In some cases, a surface coating may be deposited by a suitable deposition process, e.g., atomic layer deposition, chemical vapor deposition, self-assembling monolayers. In some cases, a surface coating may be patterned by a suitable patterning process, e.g., dry etch, wet etch, lift-off, deep UV lithography or combination thereof. A deposited surface coating may have a uniform thickness or a variable thickness over a surface of a solid support. In some cases, a surface coating may comprise an atomic or molecular monolayer. In some cases, a surface coating may comprise a self-assembled monolayer. In some cases, a surface coating may comprise a metal or metal oxide layer. In some cases, a surface coating may comprise a silane layer (e.g., ethoxy-, methoxy- or chloro- silane), a phosphonate layer, or a phosphate layer. In some cases, a surface coating may comprise a polymer, a gel such as a hydrogel, a mineral, a ceramic, or an ink. A surface coating may provide a surface electrical charge density, such as a net positive charge or a net negative charge. A solid support may be patterned to create addresses having one or more of the surface coatings exemplified above. The addresses may optionally be separated from each other by interstitial regions that lack the surface coatings or have a different surface coating from the addresses. The interstitial regions can optionally be selected from the surface coatings exemplified above.

A surface coating on a solid support or address may be characterized by a particular thickness. A surface coating may be at least about 1 Angstrom (Å), 5 Å, 1 nanometer (nm), 5 nm, 10 nm, 20 nm, 30 nm, 40 nm, 50 nm, 100 nm, 250 nm, 500 nm, 1 micrometer (μm), 5 μm, 10 μm, 50 μm, 100 μm or more. Alternatively or additionally, a surface coating may be no more than about 100 μm, 50 μm, 10 μm, 5 μm, 1 μm, 500 nm, 250 nm, 100 nm, 50 nm, 40 nm, 30 nm, 20 nm, 10 nm, 5 nm, 1 nm, 5 Å, 1 Å or less.

A solid support or address may comprise one or more surfaces that are coated with a layer of metal or metal oxide. A metal or metal oxide layer may comprise a particular species depending upon the preferable chemistry. Candidate metals or metal oxides may include zirconium oxide ($ZrO_2$), hafnium (Hf), gold (Au), titanium dioxide ($TiO_2$), aluminum (Al), aluminum oxide ($Al_2O_3$) or a combination thereof. A solid support may be patterned to create addresses having one or more of the metals or metal oxides exemplified above. The addresses may optionally be separated from each other by interstitial regions lack a particular metal or metal oxide. The interstitial regions can optionally be selected from the metals or metal oxides exemplified above.

In some cases, a solid support or address may be optically opaque. In some cases, a solid support or address may be optically clear at one or more wavelengths. In some cases, the solid support may be partially optically clear, or may be optically clear in some regions. For example, a solid support may be optically opaque in regions that are not functionalized (e.g. interstitial regions), and optically clear in regions that are functionalized (e.g. addresses).

The deposition of polypeptide composites on a solid support surface or material may be controlled for sufficient separation between neighboring polypeptides or polypeptide composites. For a polypeptide assay, the polypeptides or polypeptide composites may be deposited with sufficient separation to locate each polypeptide composite at an address or location on a solid support surface or material. In some cases, each polypeptide of a plurality of polypeptides may be located at a unique address or location on a solid support surface or material. In other cases, more than one polypeptide may be located at an optically-observable address or location on a solid support surface or material. Separation between

neighboring deposited polypeptide composites may be controlled by the solid support surface or material, the polypeptide composites, or by a combination thereof. A solid support surface or material may be modified to mediate the deposition of polypeptide composites at binding sites. A solid support surface may be modified to form a patterned or ordered array of polypeptide coupling sites, for example by lithographic techniques. Suitable lithographic techniques may include photolithography, Dip-Pen nanolithography, nanoimprint lithography, nanosphere lithography, nanoball lithography, nanopillar arrays, nanowire lithography, scanning probe lithography, thermochemical lithography, thermal scanning probe lithography, local oxidation nanolithography, molecular self-assembly, stencil lithography, and electron-beam lithography. Areas of the solid support surface or material between binding sites may be modified to discourage or prevent deposition of polypeptide composites. A solid support may comprise an unpatterned or non-patterned solid support, such as a surface comprising a uniform coating of functional groups. Deposition of polypeptide composites may be prevented by surface groups or materials that sterically obstruct a polypeptide composite from depositing on the surface, such as tethered polyethylene glycol (PEG) macromolecules or nucleic acid material such as sheared salmon sperm DNA. Deposition of polypeptide composites may be prevented by surface groups that electrostatically or magnetically repel polypeptide composites. For example, a negatively charged anchoring group may be repelled from areas of a solid support surface that have been functionalized with negatively charged groups such as a carboxylic acids, organophosphates, organosulfates, or combinations thereof.

A polypeptide array may be formed such that each address or location of the array is a particular average distance from its nearest neighbor or adjacent address. An address may be separated from its nearest neighbor or adjacent address by at least about 5 nm, 10 nm, 20 nm, 25 nm, 50 nm, 75 nm, 100 nm, 110 nm, 120 nm, 130 nm, 140 nm, 150 nm, 160 nm, 170 nm, 180 nm, 190 nm, 200 nm, 210 nm, 220 nm, 230 nm, 240 nm, 250 nm, 260 nm, 270 nm, 280 nm, 290 nm, 300 nm, 310 nm, 320 nm, 330 nm, 340 nm, 350 nm, 360 nm, 370 nm, 380 nm, 390 nm, 400 nm, 410 nm, 420 nm, 430 nm, 440 nm, 450 nm, 460 nm, 470 nm, 480 nm, 490 nm, 500 nm, 600 nm, 700 nm, 800 nm, 900 nm, 1 μm, or more than 1 μm. Alternatively or additionally, an optically-observable address may be separated from its nearest neighbor or adjacent address by no more than about 1 μm, 900 nm, 800 nm, 700 nm, 600 nm, 500 nm, 490 nm, 480 nm, 470 nm, 460 nm, 450 nm, 440 nm, 430 nm, 420 nm, 410 nm, 400 nm, 390 nm, 380 nm, 370 nm, 360 nm, 350 nm, 340 nm, 330 nm, 320 nm, 310 nm, 300 nm, 290 nm, 280 nm, 270 nm, 260 nm, 250 nm, 240 nm, 230 nm, 220 nm, 210 nm, 200 nm, 190 nm, 180 nm, 170 nm, 160 nm, 150 nm, 140 nm, 130 nm, 120 nm, 110 nm, 100 nm, 75 nm, 50 nm, 25 nm, 20 nm, 10 nm, 5 nm, or less than 5 nm.

Covalent bonds may be formed between an anchoring group and a solid support. A covalent bond may be formed directly between an anchoring group and a solid support. A covalent bond may be formed between a functional group on an anchoring group and a solid support. For example, an anchoring group functionalized with an organosilane group may be bonded to a silicon solid support by a coordination bond. A covalent bond may be formed between a functional group on an anchoring group and a functional group on a solid support. For example, an anchoring group containing an activated ester functional group may be bonded to a solid support containing an aminated functional group (e.g., 3 amino-propyl triethoxysilane on a silicon surface). In some cases, an anchoring group may be coupled to a solid support by a click reaction between a reactive handle coupled to the anchoring group and a reactive handle coupled to the solid support.

A plurality of anchoring groups or polypeptide composites may be deposited on a solid support with a known or characterized efficiency. In certain cases where the available number of binding sites on a solid support exceeds the size of the plurality of anchoring groups or polypeptide composites, the efficiency of deposition may be measured based upon the fraction of the plurality of anchoring groups or polypeptide composites that are deposited on the solid support. In certain cases where the plurality of anchoring groups or polypeptide composites exceeds the available number of binding sites on a solid support, the efficiency of deposition may be measured based upon the fraction of available binding sites on the solid support that are occupied after deposition.

The binding efficiency of a plurality of polypeptides, anchoring groups or polypeptide composites to a solid support may be quantified based upon a percentage or fraction of the plurality of polypeptides, anchoring groups or polypeptide composites that are deposited on the solid support. The binding efficiency of a plurality of polypeptides, anchoring groups or polypeptide composites may be at least about 1%, 5%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, 99.5%, 99.9%, 99.99%, 99.999%, 99.9999%, 99.99999%, 99.999999%, or more than 99.999999% based upon the available number of polypeptides, anchoring groups or polypeptide composites in the plurality. Alternatively or additionally, the binding efficiency of a plurality of polypeptides, anchoring groups or polypeptide composites may be no more than about 99.999999%, 99.99999%, 99.9999%, 99.999%, 99.99%, 99.9%, 99.5%, 99%, 98%, 97%, 96%, 95%, 94%, 93%, 92%, 91%, 90%, 85%, 80%, 75%, 70%, 65%, 60%, 55%, 50%, 45%, 40%, 35%, 30%, 25%, 20%, 15%, 10%, 5%, 1%, or less than about 1% based upon the available number of polypeptides, anchoring groups or polypeptide composites in the plurality.

A sample polypeptide fraction, such as a sample polypeptide composite fraction, bound to a solid support comprising a plurality of sample polypeptides may comprise a characterized percentage of the total quantity of sample polypeptides in a sample. The fraction bound to the solid support may contain at least about 0.000001%, 0.000005%, 0.00001%, 0.00005%, 0.0001%, 0.0005%, 0.001%, 0.005%, 0.01%, 0.05%, 0.1%, 0.5%, 1%, 5%, 10%, 15%, 20%, 25%,

30%, 35%, 40%, 45%, 50%, 55%, 60%, 65%, 70%, 75%, 80%, 85%, 90%, 95%, 99%, 99.9%, 99.99%, 99.999%, 99.9999%, 99.99999%, or more than 99.99999% of the total quantity of sample polypeptides on a mass basis. Alternatively or additionally, the fraction bound to the solid support may contain no more than about 99.99999%, 99.9999%, 99.999%, 99.99%, 99.9%, 99.5%, 99%, 95%, 90%, 85%, 80%, 75%, 70%, 65%, 60%, 55%, 50%, 45%, 40%, 35%, 30%, 25%, 20%, 15%, 10%, 5%, 1%, 0.5%, 0.1%, 0.05%, 0.01%, 0.005%, 0.001%, 0.0005%, 0.0001%, 0.00005%, 0.00001%, 0.000005%, 0.000001%, or less than 0.000001% of the total quantity of sample polypeptides on a mass basis.

The binding efficiency of a plurality of polypeptides, anchoring groups or polypeptide composites to a solid support may be quantified based upon a percentage or fraction of the available binding sites on the solid support that become occupied with a polypeptide, anchoring group or polypeptide composite. The occupancy rate of solid support binding sites may be at least about 1%, 5%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, 99.5%, 99.9%, 99.99%, 99.999%, 99.9999%, 99.99999%, 99.999999%, or more than 99.999999% based upon the total number of available binding sites. Alternatively or additionally, the occupancy rate of solid support binding sites may be no more than about 99.999999%, 99.99999%, 99.9999%, 99.999%, 99.99%, 99.9%, 99.5%, 99%, 98%, 97%, 96%, 95%, 94%, 93%, 92%, 91%, 90%, 85%, 80%, 75%, 70%, 65%, 60%, 55%, 50%, 45%, 40%, 35%, 30%, 25%, 20%, 15%, 10%, 5%, 1%, or less than about 1% based upon the total number of available binding sites.

More than one polypeptide, anchoring group or polypeptide composite may deposit on a solid support at a unique location, address, or binding site on the solid support. In some cases, the number of binding sites with more than one polypeptide, anchoring group or polypeptide composite may be minimized to single molecule detection during a polypeptide assay. In other cases, more than one polypeptides anchoring group or polypeptide composite may be deposited at a plurality, at a majority, or at all available binding sites, such as during a bulk polypeptide assay. A solid support comprising a plurality of deposited polypeptides, anchoring groups or polypeptide composites may be characterized or quantified to determine the number of binding sites with more than one polypeptide, anchoring group or polypeptide composite. A solid support binding site may contain more than one polypeptide, anchoring group or polypeptide composite, such as, for example, about 2, 3, 4, 5, 6, 7, 8, 9, 10, or more polypeptides, anchoring groups or polypeptide composites. Binding sites with more than one deposited polypeptide, anchoring group or polypeptide composite may exist according to some quantifiable distribution, such as a Poisson distribution, binomial distribution, beta-binomial distribution, hypergeometric distribution, or bimodal distribution.

The percentage of binding sites on a solid support with more than one polypeptide, anchoring group or polypeptide composite may be quantified based upon the observed number of molecules detected at each unique location on the solid support. The number of excess molecules at a unique location on a solid support may be quantified by detection of excess fluorescence, luminescence, scintillation, or size (e.g., as characterized by atomic force microscopy). The percentage of binding sites on a solid support with more than one polypeptide, anchoring group or polypeptide composite may be no more than about 99%, 95%, 90%, 85%, 80%, 75%, 70%, 65%, 60%, 55%, 50%, 45%, 40%, 35%, 30%, 25%, 20%, 15%, 10%, 9%, 8%, 7%, 6%, 5%, 4%, 3%, 2%, 1%, 0.5%, 0.1%, 0.05%, 0.01%, 0.005%, 0.001%, 0.0001%, 0.00001%, 0.000001%, 0.0000001%, or less than about 0.0000001% of all available binding sites. Alternatively or additionally, the percentage of binding sites on a solid support with more than one polypeptide, anchoring group or polypeptide composite may be at least about 0.0000001%, 0.000001%, 0.00001%, 0.0001%, 0.001%, 0.005%, 0.01%, 0.05%, 0.1%, 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 99% or more than about 99% of all available binding sites. In some cases, there may be no observed binding sites on a solid support with more than one deposited polypeptide, anchoring group or polypeptide composite.

A polypeptide, anchoring group or polypeptide composite may be deposited on a solid support under conditions that encourage the deposition of the polypeptide, anchoring group or polypeptide composite at a binding site on the solid support surface. The deposition conditions may depend upon the chemical composition of the polypeptide, anchoring group or polypeptide composite; the chemical composition of the solid support; the type of interaction between the solid support and the polypeptide, anchoring group or polypeptide composite; the number or diversity of polypeptide species to be deposited, or a combination thereof. Deposition may occur under externally applied physical conditions, such as electric fields, magnetic fields, heating, cooling, or combinations thereof. In some cases, a polypeptide, anchoring group or polypeptide composite may be deposited on a solid support under a solvent condition that promotes deposition of the polypeptide, anchoring group or polypeptide composite. A solvent for deposition may be varied by chemical composition, ionic strength, pH, electrical conductivity, magnetic permeability, heat capacity, thermal conductivity, reactivity, density, viscosity, polarity, aqueous miscibility, and combinations thereof. The chemical composition of a solvent for deposition of polypeptides, anchoring groups or polypeptide composites may be varied by solvent types and amounts, salt types and amounts, metal types and amounts, surfactant types and amounts, constituent pH, constituent pKa, and constituent reactivity. In some cases, a solvent for the deposition of polypeptides, anchoring groups or polypeptide composites may be composed to enhance the interactions between polypeptides, anchoring groups or polypeptide composites and a solid

support, for example the electrostatic bonding of an anchoring group to a solid support. Without wishing to be bound by theory, a deposition solvent for polypeptides, anchoring groups or polypeptide composites may increase the thermodynamic potential for deposition of a polypeptide, anchoring group or polypeptide composite. A deposition solvent may comprise a dispersing agent, such as a surfactant or detergent, that reduces or prevents aggregation of polypeptides, anchoring groups or polypeptide composites before deposition. A deposition solvent may comprise a charged species, such as a cation or anion that mediates charge interactions between polypeptides, anchoring groups or polypeptide composites and a solid support. In some cases, a polypeptide composite solvent composition may be utilized as a deposition solvent.

The deposition of a plurality of polypeptides or polypeptide composites on a solid support by an electrostatic interaction may be driven by the presence of a surfactant. A plurality of polypeptides or polypeptide composites may be contacted with a solid support in the presence of one or more surfactants that facilitate an electrostatic interaction between the polypeptides or polypeptide composites and the solid support. A deposition solvent composition may include a surfactant species, such as a cationic surfactant, an anionic surfactant, a zwitterionic surfactant, or an amphoteric surfactant. The polypeptides or polypeptide composites may be contacted with a solid support in a deposition solvent composition that comprises a surfactant at a weight percentage of about 0.0001%, 0.0005%, 0.001%, 0.002%, 0.003%, 0.004%, 0.005%, 0.006%, 0.007%, 0.008%, 0.009%, 0.01%,%, 0.02%, 0.03%, 0.04%, 0.05%, 0.06%, 0.07%, 0.08%, 0.09%,%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, or more than 10%. The polypeptides or polypeptide composites may be contacted with a solid support in a deposition solvent composition that comprises a surfactant at a weight percentage of at least about 0.0001%, 0.0005%, 0.001%, 0.002%, 0.003%, 0.004%, 0.005%, 0.006%, 0.007%, 0.008%, 0.009%, 0.01%,%, 0.02%, 0.03%, 0.04%, 0.05%, 0.06%, 0.07%, 0.08%, 0.09%,%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, or more than 10%. Alternatively or additionally, the polypeptides or polypeptide composites may be contacted with a solid support in a deposition solvent composition that comprises a surfactant at a weight percentage of no more than about 10%, 9%, 8%, 7%, 6%, 5%, 4%, 3%, 2%, 1%, 0.9%, 0.8%, 0.7%, 0.6%, 0.5%, 0.4%, 0.3%, 0.2%, 0.1%,0.09%, 0.08%, 0.07%, 0.06%, 0.05%, 0.04%, 0.03%, 0.02%, 0.01%,0.009%, 0.008%, 0.007%, 0.006%, 0.005%, 0.004%, 0.003%, 0.002%, 0.001%, 0.0005%, 0.0001%, or less than 0.0001% .

The deposition of a plurality of polypeptides or polypeptide composites on a solid support by an electrostatic interaction may be driven by the presence of a salt. Exemplary salts may include salts comprising an alkali metal, alkaline earth metal, or a transition metal. In some cases, the salt may comprise a sodium salt, a calcium salt, a magnesium salt, or a potassium salt (e.g., NaCl, $CaCl_2$, $MgCl_2$, or KCl). A plurality of polypeptides or polypeptide composites may be contacted with a solid support in the presence of one or more salts that facilitate an electrostatic interaction between the polypeptides or polypeptide composites and the solid support. The polypeptides or polypeptide composites may be contacted with a solid support in a deposition solvent composition that comprises a salt at a concentration of about 0.001 moles/liter (M), 0.002 M, 0.003M, 0.004M, 0.005M, 0.006M, 0.007M, 0.008M, 0.009M, 0.01M, 0.02 M, 0.03M, 0.04M, 0.05M, 0.06M, 0.07M, 0.08M, 0.09M, 0.1M, 0.2 M, 0.3M, 0.4M, 0.5M, 0.6M, 0.7M, 0.8M, 0.9M, 1M, 1.1M, 1.2M, 1.3M, 1.4M, 1.5M, 1.6M, 1.7M, 1.8M, 1.9M, 2M, 2.1M, 2.2M, 2.3M, 2.4M, 2.5M, 2.6M, 2.7M, 2.8M, 2.9M, 3M, 3.1M, 3.2M, 3.3M, 3.4M, 3.5M, 3.6M, 3.7M, 3.8M, 3.9M, 4M, 4.1M, 4.2M, 4.3M, 4.4M, 4.5M, 4.6M, 4.7M, 4.8M, 4.9M, 5M, or more than 5M. The polypeptides or polypeptide composites may be contacted with a solid support in a deposition solvent composition that comprises a salt at a concentration of at least about 0.001M, 0.002 M, 0.003M, 0.004M, 0.005M, 0.006M, 0.007M, 0.008M, 0.009M, 0.01M, 0.02 M, 0.03M, 0.04M, 0.05M, 0.06M, 0.07M, 0.08M, 0.09M, 0.1M, 0.2 M, 0.3M, 0.4M, 0.5M, 0.6M, 0.7M, 0.8M, 0.9M, 1M, 1.1M, 1.2M, 1.3M, 1.4M, 1.5M, 1.6M, 1.7M, 1.8M, 1.9M, 2M, 2.1M, 2.2M, 2.3M, 2.4M, 2.5M, 2.6M, 2.7M, 2.8M, 2.9M, 3M, 3.1M, 3.2M, 3.3M, 3.4M, 3.5M, 3.6M, 3.7M, 3.8M, 3.9M, 4M, 4.1M, 4.2M, 4.3M, 4.4M, 4.5M, 4.6M, 4.7M, 4.8M, 4.9M, 5M, or more than 5M. Alternatively or additionally, the polypeptide or polypeptide composites may be contacted with a solid support in a deposition solvent composition that comprises a salt at a concentration of about 5M, 4.9M, 4.8M, 4.7M, 4.6M, 4.5M, 4.4M, 4.3M, 4.2M, 4.1M, 4M, 3.9M, 3.8M, 3.7M, 3.6M, 3.5M, 3.4M, 3.3M, 3.2M, 3.1M, 3M, 2.9M, 2.8M, 2.7M, 2.6M, 2.5M, 2.4M, 2.3M, 2.2M, 2.1M, 2M, 1.9M, 1.8M, 1.7M, 1.6M, 1.5M, 1.4M, 1.3M, 1.2M, 1.1M, 1M, 0.9M, 0.9M, 0.8M, 0.7M, 0.6M, 0.5M, 0.4M, 0.3M, 0.2M, 0.1M, 0.09M, 0.08M, 0.07M, 0.06M, 0.05M, 0.04M, 0.03M, 0.02M, 0.01M, 0.009M, 0.008M, 0.007M, 0.006M, 0.005M, 0.004M, 0.003M, 0.002M, 0.001M, or less than 0.001M.

The deposition of a plurality of polypeptides or polypeptide composites on a solid support by an electrostatic interaction may be driven by a solvent pH. A plurality of polypeptides or polypeptide composites may be contacted with a solid support in the presence of a deposition solvent composition with a particular pH. In some cases, a plurality of polypeptides or polypeptide composites in contact with a solid support may be deposited on the solid support by a change in the deposition solvent pH. The pH change can be about 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, or more than 7 pH units. The pH change can be at least about 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, or more than 7 pH units. Alternatively

or additionally, the pH change can be no more than about 7, 6.9, 6.8, 6.7, 6.6, 6.5, 6.4, 6.3, 6.2, 6.1, 6, 5.9, 5.8, 5.7, 5.6, 5.5, 5.4, 5.3, 5.2, 5.1, 5, 4.9, 4.8, 4.7, 4.6, 4.5, 4.4, 4.3, 4.2, 4.1, 4, 3.9, 3.8, 3.7, 3.6, 3.5, 3.4, 3.3, 3.2, 3.1, 3, 2.9, 2.8, 2.7, 2.6, 2.5, 2.4, 2.3, 2.2, 2.1, 2, 1.9, 1.8, 1.7, 1.6, 1.5, 1.4, 1.3, 1.2, 1.1, 1, 0.9, 0.8, 0.7, 0.6, 0.5, 0.4, 0.3, 0.2, 0.1, or less than 0.1 pH units.

A solid support may be contacted with a solvent or medium before polypeptides or polypeptide composites are deposited. The contacting of a solvent or medium before polypeptide or polypeptide composite deposition may enhance or facilitate the deposition of polypeptide or polypeptide composites on a solid support surface. The solvent or medium contacted with the solid support before deposition may have the same composition as the solvent or medium used during deposition. For example, if a polypeptide composite comprises a polypeptide and a structured nucleic acid particle (SNAP) and the composite is to be deposited using a storage solvent, then the solid support may be incubated with storage solvent that has no polypeptide composites in it. The contacting of the solid support with the solvent or medium may occur for a sufficient amount of time to prepare the solid support for polypeptide or polypeptide composite deposition. A solid support may be contacted with a solvent or medium for about 1 min, 10 mins, 15 mins, 30 mins, 1 hr, 2 hrs, 3 hrs, 6 hrs, 12 hrs, 1 day, or more than 1 day before deposition. A solid support may be contacted with a solvent or medium for at least about 1 min, 10 mins, 15 mins, 30 mins, 1 hr, 2 hrs, 3 hrs, 6 hrs, 12 hrs, 1 day, or more than 1 day before deposition. Alternatively or additionally, a solid support may be contacted with a solvent or medium for no more than about 1 day, 12 hrs, 6 hrs, 3 hrs, 2 hrs, 1 hr, 30 mins, 15 mins, 10 mins, 1 min, or less than 1 min.

A polypeptide, anchoring group or polypeptide composite may be deposited on a solid support in a stable or conditionally stable configuration. A stable configuration may comprise a configuration of deposited anchoring groups on a solid support surface is not interrupted over a fixed period of time. For example, a deposited anchoring group may be considered to be in a stable configuration if it does not dissociate from a solid support, even in the presence of a denaturing or dissociating compound. A conditionally stable configuration of deposited anchoring groups on a solid support surface may comprise a stable configuration that can be destabilized, rearranged, or dissociated under known or characterized conditions. For example, a deposited anchoring group on a solid support may dissociate from a solid support when the temperature exceeds a threshold value.

The stability of deposited polypeptides, anchoring groups or polypeptide composites on a solid support surface or material may be characterized or quantified. Several compositions and methods for characterizing or quantifying this stability are set forth below. In some configurations, the stability of deposited anchoring groups can be characterized or quantified relative to an internal standard. For example, the stability of one or more sample polypeptide composites can be evaluated relative to the stability of one or more standard polypeptide composites. Optionally, a plurality of different standard polypeptide composites on a solid support can have different stabilities when subjected to one or more condition. The different stabilities can be known or predicted, such that a loss of one, more than one or all of the standard polypeptide composites indicates that one, more than one or all of the sample polypeptide composites may have been lost. As such loss of sample polypeptides due to technical conditions used during array processing or detection can be distinguished from loss (or absence) of particular sample polypeptides due to a characteristic of interest to an observer.

The stability of deposited polypeptides, anchoring groups or polypeptide composites on a solid support surface or material may be characterized or quantified based upon the rate of loss from the surface or material or the rate of rearrangement on the surface or material. In some cases, the stability of deposited polypeptides, anchoring groups or polypeptide composites may be characterized or quantified by characterizing their presence or absence at each address on the solid support. The characterizing may be repeated one or more times. The repeating the characterization of the presence or absence of polypeptides, anchoring groups or polypeptide composites at each address may occur consecutively, after an elapsed time period, or with successive processes (e.g., rinsing processes to remove unbound or loosely bound molecules). The presence or absence of polypeptides, anchoring groups or polypeptide composites at each address may be characterized by, for example, fluorescence microscopy, surface plasmon resonance, atomic force microscopy, or any other suitable method. A configuration of deposited polypeptides, anchoring groups or polypeptide composites may be considered stable or conditionally stable if there is no observed loss or rearrangement over a fixed period of time. A configuration of deposited polypeptides, anchoring groups or polypeptide composites may be considered stable or conditionally stable if their loss or rearrangement is beneath a threshold level (e.g. the threshold can be the level at which the polypeptides, anchoring groups or polypeptide composites are detectable using a method set forth herein or known in the art) over a fixed period of time. Loss of polypeptides, anchoring groups or polypeptide composites may be quantified directly, for example by imaging a solid support at an initial time and a later time and counting unique, spatial addresses that have lost fluorescent polypeptides, anchoring groups or polypeptide composites at the later time. In some cases, a configuration of deposited polypeptides, anchoring groups or polypeptide composites may be considered stable or conditionally stable if there is gain, loss or rearrangement of the polypeptides, anchoring groups or polypeptide composites beneath a threshold level (e.g. the detectability threshold) over a fixed period of time that corresponds to one or more cycles of a polypeptide assay. A cycle may refer to a sequence of processes that occur during a polypeptide assay (e.g., washes, rinses, binding of affinity reagents, imaging, etc.). Loss or gain of polypeptides, anchoring groups or polypeptide composites may be quantified indirectly, for example by measuring changes in total fluorescence, total

luminescence, total scintillation, or combinations thereof between an initial and final time point.

The stability of polypeptides, anchoring groups or polypeptide composites may be quantified by a magnitude of gain or loss, or by a rate of gain or loss on a solid support. The magnitude or rate can be determined over a fixed amount of time or over a fixed number of cycles of a polypeptide assay. A polypeptide assay may be performed if the magnitude or rate of gain or loss from a solid support is less than a maximum value. The magnitude or rate of gain or loss from a solid support may be measured over a fixed period of time, such as at least about 1 s, 30 s, 1 min, 5 mins, 10 mins, 15 mins, 20 mins, 30 mins, 1 hr, 3 hrs, 6 hrs, 12 hrs, 24 hrs, or more than 24 hours. The magnitude or rate of gain or loss on a solid support may be measured over a fixed number of assay cycles, such as at least about 1 cycle, 2, 3, 4, 5, 6, 7, 8, 9, 10, 15, 20, 25, 30, 35, 40, 45, 50, 60, 70, 75, 80, 90, 100, 125, 150, 175, 200, 250, 300, 350, 400, 450, 500 or more than 500 cycles.

The magnitude of gain or loss, or the rate of gain or loss, of polypeptides, anchoring groups or polypeptide composites from a solid support may be quantified. The quantity may be measured over a full polypeptide assay or a period of time within the course of a polypeptide assay. A magnitude of gain or loss may be given as a fraction or percentage of polypeptides, anchoring groups or polypeptide composites observed to be absent at the end of a period of time (e.g., 10% loss is equivalent to a fraction of 0.10). A rate of gain or loss may be given as a fraction or percentage of polypeptides, anchoring groups or polypeptide composites observed to have been lost over a period of time (e.g., 10% loss per cycle). A polypeptide assay may have a maximum quantity or rate of gain or loss of polypeptides, anchoring groups or polypeptide composites from a solid support over a period of time. If the quantity or rate of gain or loss from a solid support exceeds a maximum magnitude or rate, the predictive capability of the polypeptide assay may be diminished.

A quantity of gain or loss of polypeptides, anchoring groups or polypeptide composites from a solid support over a period of time may be no more than about 10%, 9%, 8%, 7%, 6%, 5%, 4%, 3%, 2%, 1%, 0.5%, 0.1%, 0.05%, 0.01%, 0.005%, 0.001%, 0.0001%, 0.00001%, 0.000001%, 0.00000001%, 0.000000001%, 0.0000000001%, or less than 0.0000000001% over a fixed time period (e.g., second, minute, hour, day). Alternatively or additionally, a quantity of gain or loss of polypeptides, anchoring groups or polypeptide composites from a solid support over a period of time may be at least about 0.0000000001%, 0.000000001%, 0.00000001%, 0.0000001%, 0.000001%, 0.00001%, 0.0001%, 0.001%, 0.005%, 0.01%, 0.05%, 0.1%, 0.5%, 1%, 2%, 3%, 4%, 5%, 6%, 7%, 8%, 9%, 10%, or more than 10% over a fixed time period. Alternatively or additionally, a quantity of gain or loss of polypeptides, anchoring groups or polypeptide composites from a solid support over a period of time may be no more than about 10%, 9%, 8%, 7%, 6%, 5%, 4%, 3%, 2%, 1%, 0.5%, 0.1%, 0.05%, 0.01%, 0.005%, 0.001%, 0.0001%, 0.00001%, 0.000001%, 0.00000001%, 0.000000001%, 0.0000000001%, or less than 0.0000000001% per cycle of a polypeptide assay. Alternatively or additionally, a quantity of gain or loss of polypeptides, anchoring groups or polypeptide composites from a solid support over a period of time may be at least about 0.0000000001%, 0.000000001%, 0.00000001%, 0.0000001%, 0.000001%, 0.00001%, 0.0001%, 0.001%, 0.005%, 0.01%, 0.05%, 0.1%, 0.5%, 1%, 2%, 3%, 4%, 5%, 6%, 7%, 8%, 9%, 10%, or more than 10% per cycle of a polypeptide assay.

A rate of gain or loss of polypeptides, anchoring groups or polypeptide composites from a solid support may be quantified. The rate of gain or loss may increase or decrease over the course of a polypeptide assay, or during a particular step of a polypeptide assay. A rate of gain or loss of polypeptides, anchoring groups or polypeptide composites from a solid support may be no more than about 10%, 9%, 8%, 7%, 6%, 5%, 4%, 3%, 2%, 1%, 0.5%, 0.1%, 0.05%, 0.01%, 0.005%, 0.001%, 0.0001%, 0.00001%, 0.000001%, 0.00000001%, 0.000000001%, 0.0000000001%, or less than 0.0000000001% per unit of time (e.g., second, minute, hour, day). Alternatively or additionally, a rate of gain or loss from a solid support may be at least about 0.0000000001%, 0.000000001%, 0.00000001%, 0.0000001%, 0.000001%, 0.00001%, 0.0001%, 0.001%, 0.005%, 0.01%, 0.05%, 0.1%, 0.5%, 1%, 2%, 3%, 4%, 5%, 6%, 7%, 8%, 9%, 10%, or more than 10% per unit of time. Alternatively or additionally, a quantity of gain or loss from a solid support may be no more than about 10%, 9%, 8%, 7%, 6%, 5%, 4%, 3%, 2%, 1%, 0.5%, 0.1%, 0.05%, 0.01%, 0.005%, 0.001%, 0.0001%, 0.00001%, 0.000001%, 0.00000001%, 0.000000001%, 0.0000000001%, or less than 0.0000000001% per cycle of a polypeptide assay. Alternatively or additionally, a quantity of gain or loss from a solid support may be at least about 0.0000000001%, 0.000000001%, 0.00000001%, 0.0000001%, 0.000001%, 0.00001%, 0.0001%, 0.001%, 0.005%, 0.01%, 0.05%, 0.1%, 0.5%, 1%, 2%, 3%, 4%, 5%, 6%, 7%, 8%, 9%, 10%, or more than 10% per cycle of a polypeptide assay.

One or more polypeptides, anchoring groups, or polypeptide composites may be removed from a solid support. A polypeptide, anchoring group, or polypeptide composite may be removed from a solid support by a passive mechanism, such as dissociation. A polypeptide, anchoring group, or polypeptide composite may be removed from a solid support by an active mechanism, such as applying a removing fluid, enzymatically degrading the polypeptide, anchoring group, or polypeptide composite, photolytically degrading the polypeptide, anchoring group, or polypeptide composite, or chemically degrading the polypeptide, anchoring group, or polypeptide composite. A polypeptide, anchoring group, or polypeptide composite may be removed by a removing fluid comprising an acid, a base, a surfactant, a denaturant, a chaotrope, a salt, or a combination thereof. An anchoring group may be configured to bind a sample polypeptide by a photocleavable linker that may be cleaved under irradiation. An anchoring group may be configured to comprise one or more enzymatic targets (e.g., restriction enzyme nucleic acid sequences) or chemical targets (e.g., photocleavable linkers) that permit the anchoring group to be enzymatically, chemically, or photolytically degraded. The enzymatic,

chemical, or photolytic degradation may release the anchoring group from the solid support or may partially or completely degrade the anchoring group. An anchoring group may comprise an enzymatically, chemically, or photolytically degradable detectable label, such as a standard polypeptide or nucleic acid barcode, that can be released after a particular process (e.g., binding to a solid support).

One or more polypeptides, anchoring groups, or polypeptide composites may be controllably removed from a solid support. Polypeptides, anchoring groups, or polypeptide composites may be removed from a solid support on an individual basis, in discrete batches or groups, or in bulk. Polypeptides, anchoring groups, or polypeptides comprising photocleavable linkers may be removed by focused irradiation at a single address or group of addresses, thereby releasing one or a group of molecules from the solid support. Solid supports comprising arrays of multiple species of polypeptides, anchoring groups, or polypeptide composites may be enzymatically or chemically treated with reagents that target particular species for degradation or separation from the solid support. For example, a particular species of anchoring group may comprise one or more of the same restriction site such that treatment with the corresponding restriction enzyme degrades that particular anchoring group, thereby releasing it from the solid support.

A polypeptide array may be formed with more than one species of anchoring groups. Differing species of anchoring groups may be configured to form a self-patterning or self-assembling array. In some configurations, an anchoring group may comprise one or more coupling groups that are configured to form interactions with anchoring groups of the same species, thereby grouping polypeptides coupled to anchoring groups of the same species. In other configurations, an anchoring group may comprise one or more coupling groups that are configured to form interactions with anchoring groups of a differing species, thereby creating adjacent polypeptides coupled to differing species of anchoring groups. **FIGs. 11A - 11B** depict methods of self-arrangement by the coupling of anchoring groups to each other. **FIG. 11A** illustrates a mixture of anchoring groups comprising a first species of anchoring group **1110** and a second species of anchoring group **1120.** The first species of anchoring group is configured with coupling groups that are configured to selectively interact with coupling groups of the first species. The second species of anchoring group is configured with coupling groups that are configured to selectively interact with coupling groups of the second species. When combined the, the anchoring groups may couple to complementary anchoring groups to form clusters of assembled anchoring groups of the first species **1115** or clusters of assembled anchoring groups of the second species **1125. FIG. 11B** illustrates a mixture of anchoring groups comprising a first species of anchoring group **1112** and a second species of anchoring group **1122.** The first species of anchoring group is configured with coupling groups that are configured to selectively interact with coupling groups of second species. The second species of anchoring group is configured with coupling groups that are configured to selectively interact with coupling groups of the second species. When combined the, the anchoring groups may couple to complementary anchoring groups to form clusters of assembled anchoring groups of alternating species **1138.**

Pluralities of differing species of anchoring groups or polypeptide composites may be assembled into arrays. A first species of anchoring group or polypeptide composite may be configured to bind, assemble, or associate with a second species of anchoring group or polypeptide composite. A first species of anchoring group or polypeptide composite may be configured to bind, assemble or associate with two or more differing species of anchoring group or polypeptide composite. A species of anchoring group or polypeptide composite may be configured to bind, assemble, or associate with one or more anchoring groups or polypeptide composites of the same species. A first species of anchoring group or polypeptide composite may be configured to bind, assemble, or associate with one or more anchoring groups or polypeptide composites of the same species and one or more anchoring groups or polypeptide composites of a second species of anchoring group or polypeptide composite. Assembly of differing species of anchoring groups or polypeptide composites may be controlled by complementary coupling chemistries on each available species of anchoring group or polypeptide composites. For example, a species of anchoring group or polypeptide composite may comprise a first utility surface with one or more coupling groups (e.g., oligonucleotides, streptavidin or biotin, click reactants, etc.) that are configured to selectively hybridize anchoring groups or polypeptide composites of the same species, and a second utility surface with one or more coupling groups (e.g., complementary oligonucleotides, streptavidin or biotin, complementary click reactants, etc.) that are configured to selectively hybridize anchoring groups or polypeptide composites of a second species.

Two differing species of anchoring groups or polypeptide composites in an assembled array may be distinguished by differing types of displayed polypeptides. Differing polypeptides may be sorted on the basis of any polypeptide property, including, but not limited to peptide sequence, size, weight, length, cellular location (e.g., extracellular, membrane, cytoplasmic, organelle, nuclear, etc.), organism or system of origin (e.g., cell-free synthesis), isoelectric point, hydrodynamic radius, post-translational modification, or any other measurable or observable polypeptide characteristic. For example, a first species of anchoring groups or polypeptide composites in a polypeptide array may comprise polypeptides from a polypeptide-containing sample and a second species of anchoring groups or polypeptide composites in a polypeptide array may comprise polypeptides from a standard or control sample (i.e., a quality control marker polypeptide, positive control polypeptide, negative control polypeptide, etc.). In another example, polypeptides from a first organism may be placed on a first species of anchoring groups or polypeptide composites, and polypeptides from a second

organism may be placed on a second species of anchoring groups or polypeptide composites. A polypeptide array may comprise two or more species of anchoring groups, with each species of anchoring group coupled or conjugated to sample polypeptides from a different sample. Preparation and combining of multiple samples by formation of unique species of sample polypeptide composites corresponding to each sample may permit multiplexed polypeptide assays.

A polypeptide array may be formed by simultaneous or sequential deposition of pluralities of polypeptides and fiducial elements. A fiducial element may comprise a detectable moiety that is deposited on a polypeptide array for procedures associated with array detection, such as landmarking of array positions, image registration during detection, and focusing for signal detection. In some cases, a method of forming a polypeptide array may comprise one or more steps of: a) depositing a plurality of fiducial elements on an array, in which each fiducial element of the plurality of fiducial elements is located at an address of a plurality of array addresses; and b) depositing a plurality of polypeptides on the polypeptide array, in which each polypeptide of the plurality of polypeptides is located at an array address of the plurality of array addresses. In other cases, formation of a polypeptide array may comprise one or more steps of: a) combining a plurality of polypeptides with a plurality of fiducial elements to form a combined plurality of array moieties; and b) depositing the combined plurality of array moieties on a polypeptide array, in which each array moiety of the combined plurality of array moieties is deposited at an address of a plurality of array addresses. In some cases, a method of forming a polypeptide array may further comprise a step of detecting a presence or absence of a fiducial element at each address of a plurality of addresses of the polypeptide array.

A fiducial element may be deposited on a polypeptide array. A fiducial element may comprise a detectable label. A fiducial element may be configured to be detected by an optical method (e.g., fluorescence detection, luminescence lifetime detection, etc.). A fiducial element may comprise a fluorescent or luminescent detectable label. A fiducial element may comprise a detectable particle, such as a fluorescent polymer (e.g., Invitrogen FluoSpheres™), fluorescent semiconductor or metal nanoparticles (e.g., quantum dots), or fluorescently-labeled oligonucleotides. In some cases, a fiducial element of a plurality of fiducial elements may be coupled to an anchoring group. For example, a fluorescent polymer particle (e.g., a carboxylated FluoSphere™, catalog number F8803) may be covalently coupled to a SNAP moiety, in which the SNAP is configured to couple the fluorescent polymer particle to a polypeptide array.

**Use of Internal Standards**

One or more internal standards may be provided to a sample preparation process. Standard polypeptides are a particularly useful class of internal standards for use when preparing polypeptide samples. A standard polypeptide can have a uniquely identifiable peptide sequence that functions as a tag. Typically, the tag of the standard polypeptide is uniquely distinguishable from sequences present in other polypeptides found in the sample with which the standard polypeptide is to be deployed. The standard polypeptide can also have a chemical composition (e.g. a peptide sequence), that imparts a known or predicted characteristic. A standard polypeptide that includes both a unique tag and a characteristic chemical composition, can be added to a sample at a particular stage of processing the sample, and then the sample can be analyzed for presence or absence of the tag. Presence or absence of the tag can provide information regarding the fate of sample polypeptides having characteristics similar to the standard polypeptide. Optionally, the quantity of tag that is retained or lost after sample processing can indicate the extent to which sample polypeptides having similar characteristics as the standard polypeptide may have been retained or lost during processing. For example, a lower than expected recovery of the tag from a sample after being subjected to a sample preparation process may indicate that the process caused the loss of certain sample polypeptides having a protease recognition sequence that was also present in the standard polypeptide. Optionally, the sample can be further analyzed for structural or functional characteristics of the standard polypeptide as a way to evaluate changes that may have occurred for sample polypeptides that were processed with the standard polypeptide. For example, the presence or absence of a particular post-translational modification on the standard polypeptide can indicate the extent to which sample polypeptides with similar peptide sequences were also modified during processing. An internal standard may comprise a plurality of internal standard polypeptides, such as a plurality of separation standard polypeptides, a plurality of functionalization standard polypeptides, a plurality of coupling standard polypeptides, a plurality of attachment standard polypeptides or a plurality of other standard polypeptides set forth herein. Several examples illustrating the use of internal standards are set forth in further detail below.

A sample preparation method may comprise the step of providing one or more standard polypeptides, such as separation standard polypeptides. One or more standard polypeptides may be provided by combining the standard polypeptide(s) with a sample. For example, one or more separation standard polypeptides may be provided by combining the separation standard polypeptide(s) with one or more sample polypeptides derived from a particular sample. In some cases, combining the separation standard polypeptide(s) with the sample polypeptide(s) may occur before separating the sample polypeptide(s) from other components of the sample. Alternatively, the standard polypeptide(s) can be combined with one or more fractions after fractionation of the sample. As such, one or more standard polypeptides can be combined with one or more sample polypeptides that have been separated from the sample.

In some cases, a sample preparation method can include a step of functionalizing one or more sample

polypeptides to form sample polypeptides having reactive handles. In one configuration of the method, the functionalization step can be carried out after the sample polypeptide(s) have been separated from one or more other components of the sample. In another configuration of the method, the functionalization step can be carried out before the sample polypeptide(s) have been separated from one or more other components of the sample. In either configuration, combining the separation standard polypeptide(s) with the sample polypeptide(s) may occur before, during or after functionalizing the sample polypeptide(s) from the sample.

In some cases, a sample preparation method can include a step of coupling one or more sample polypeptides to one or more anchoring groups to form one or more sample polypeptide composites that each include a sample polypeptide and an anchoring group. In one configuration of the method, the coupling step can be carried out after the sample polypeptide(s) have been separated from one or more other components of the sample and/or after the sample polypeptide(s) have been functionalized with reactive handles. In another configuration of the method, the coupling step can be carried out before the sample polypeptide(s) have been separated from one or more other components of the sample and/or before the sample polypeptide(s) have been functionalized with reactive handles. In either configuration, combining the separation standard polypeptide(s) with the sample polypeptide(s) may occur before, during or after coupling the sample polypeptide(s) to form polypeptide composites.

In some cases, a sample preparation method can include a step of attaching sample polypeptide(s) to a solid support, whereby each of the sample polypeptides is attached to an address of a polypeptide array on the solid support. In one configuration of the method, the attaching step can be carried out after the sample polypeptide(s) have been separated from one or more other components of the sample, after the sample polypeptide(s) have been functionalized with reactive handles and/or after the functionalized sample polypeptides have been coupled to anchoring group(s). In another configuration of the method, the attaching step can be carried out before the sample polypeptide(s) have been separated from one or more other components of the sample. In either configuration, combining the separation standard polypeptide(s) with the sample polypeptide(s) may occur before, during or after attaching the sample polypeptide(s) to the solid support.

Separation standard polypeptides may comprise a plurality of separation standard polypeptide composites, where each separation standard polypeptide composite of the plurality of separation standard polypeptide composites comprises a separation standard polypeptide coupled to an anchoring group. The separation standard polypeptide composites can be formed before, during or after providing the separation standard polypeptide(s) to a sample or fraction thereof. The separation standard polypeptide composites may be formed before, during or after functionalizing the sample polypeptide(s) with reactive handle(s). The separation standard polypeptide composites may be formed before, during or after coupling the sample polypeptide(s) to an anchoring group(s). The separation standard polypeptide composites may be formed before, during or after attaching the sample polypeptide(s) to address(es) on a solid support. An internal standard, such as a standard polypeptide, may be detected by any suitable method. In some configurations, the internal standard can be detected on a solid support, for example, at an address of an array of polypeptides. An internal standard polypeptide may comprise a detectable label that permits identification of the internal standard polypeptide. In some configurations, an internal standard polypeptide may be coupled to an anchoring group that comprises a detectable label that permits identification of the internal standard polypeptide. An internal standard polypeptide may comprise a detectable label that produces a detectable signal, such as a signal produced by fluorescence, luminescence, phosphorescence, enzymatic activity, radioactivity, affinity reagent binding, or sequencing. A detectable label may be covalently or non-covalently coupled to the internal standard polypeptide or to an anchoring group that is coupled or conjugated to the internal standard polypeptide. A detectable label may comprise a fluorescent group, a luminescent group, a phosphorescent group, an enzyme, a radiolabel, or a nucleic acid barcode. In some configurations, two differing internal standard polypeptides (e.g., a separation standard polypeptide and a coupling standard polypeptide) may comprise unique detectable labels that distinguish one type of polypeptide from the other.

When forming polypeptide arrays from a plurality of sample polypeptide composites, the attachment of each sample polypeptide composite to a solid support may further comprise attaching one or more separation standard polypeptide composites to an address on the solid support. The total quantity of separation standard polypeptides or separation standard polypeptide composites (relative to the plurality of separation standard polypeptides provided to a sample preparation process) that are bound to the polypeptide array may be utilized to determine the completeness of a polypeptide separation method or to determine the extent of removing sample polypeptides from a sample. The quantity of separation standard polypeptide composites bound to a solid support of a polypeptide array may be at least about 0.01%, 0.05%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, 99.5%, 99.9%, 99.99%, 99.999% or more than 99.999% of the quantity of the plurality of separation standard polypeptides provided to a polypeptide sample preparation method. Alternatively or additionally, the quantity of separation standard polypeptide composites bound to a solid support of a polypeptide array may be no more than about 99.999%, 99.99%, 99.9%, 99.5%, 99%, 95%, 90%, 85%, 80%, 75%, 70%, 65%, 60%, 55%, 50%, 45%, 40%, 35%, 30%, 25%, 20%, 15%, 10%, 5%, 1%, 0.5%, 0.1%, 0.05%, 0.01%, or less than 0.01% of the quantity of the plurality of separation standard polypeptides provided to a polypeptide sample preparation method.

A sample preparation method may comprise the step of providing one or more functionalization standard polypeptides. The functionalization standard polypeptide(s) may be provided by combining the separation standard polypeptide(s) with a sample. For example, the functionalization standard polypeptide(s) may be provided by combining the functionalization standard polypeptide(s) with one or more sample polypeptides derived from a sample. Combining the functionalization standard polypeptide(s) with the sample polypeptide(s) may occur before, during or after functionalizing the sample polypeptide(s) to provide reactive handle(s) to the sample polypeptide(s). The functionalization standard polypeptide(s) can be combined with the sample polypeptide(s) before, during or after separating the sample polypeptide(s) from the sample. The functionalization standard polypeptide(s) can be combined with the sample polypeptide(s) before during or after coupling the sample polypeptide(s) to anchoring group(s). The functionalization standard polypeptide(s) can be combined with the sample polypeptides before during or after attaching the sample polypeptide(s) to solid support(s).

Functionalization standard polypeptides may comprise a plurality of functionalization standard polypeptide composites, where each functionalization standard polypeptide composite of the plurality of functionalization standard polypeptide composites comprises a functionalization standard polypeptide coupled to an anchoring group. The functionalization standard polypeptide composites can be formed before, during or after providing separation standard polypeptides to a sample or fraction thereof. The functionalization standard polypeptide composites can be formed before during or after providing functionalization standard polypeptides to a sample or fraction thereof. The functionalization standard polypeptide composites may be formed before during or after coupling the sample polypeptides to anchoring groups. The functionalization standard polypeptide composites may be formed before, during or after attaching the sample polypeptide(s) to address(es) on a solid support.

When forming polypeptide arrays from a plurality of sample polypeptide composites, the attachment of each sample polypeptide composite to a solid support may further comprise attaching one or more functionalization standard polypeptide composites to an address on the solid support. The total quantity of functionalization standard polypeptides or functionalization standard polypeptide composites (relative to the plurality of functionalization standard polypeptides provided to a sample preparation process) that are bound to the polypeptide array may be utilized to determine the completeness of a polypeptide functionalization method or determine the extent of functionalization of sample polypeptides from a sample. The quantity of functionalization standard polypeptide composites bound to a solid support of a polypeptide array may be at least about 0.01%, 0.05%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, 99.5%, 99.9%, 99.99%, 99.999% or more than 99.999% of the quantity of the plurality of functionalization standard polypeptides provided to a polypeptide sample preparation method. Alternatively or additionally, the quantity of functionalization standard polypeptide composites bound to a solid support of a polypeptide array may be no more than about 99.999%, 99.99%, 99.9%, 99.5%, 99%, 95%, 90%, 85%, 80%, 75%, 70%, 65%, 60%, 55%, 50%, 45%, 40%, 35%, 30%, 25%, 20%, 15%, 10%, 5%, 1%, 0.5%, 0.1%, 0.05%, 0.01%, or less than 0.01% of the quantity of the plurality of functionalization standard polypeptides provided to a polypeptide sample preparation method.

A sample preparation method may comprise the step of providing one or more coupling standard polypeptides. The coupling standard polypeptide(s) may be provided by combining the coupling standard polypeptide(s) with a sample. The coupling standard polypeptide(s) may be provided by combining the coupling standard polypeptide(s) with one or more sample polypeptides derived from the sample. Combining the coupling standard polypeptide(s) with the sample polypeptide(s) may occur before, during or after coupling the sample polypeptide(s) to provide reactive handle(s) to the sample polypeptide(s). Combining the coupling standard polypeptide(s) with the sample polypeptide(s) may occur before, during or after separating the sample polypeptide(s) from the sample. The coupling standard polypeptide(s) can be combined with the sample polypeptide(s) before, during or after coupling the sample polypeptide(s) to anchoring group(s). The coupling standard polypeptide(s) can be combined with the sample polypeptide(s) before during or after attaching the sample polypeptide(s) to solid support(s).

Coupling standard polypeptides may comprise a plurality of coupling standard polypeptide composites, where each coupling standard polypeptide composite of the plurality of coupling standard polypeptide composites comprises a coupling standard polypeptide coupled to an anchoring group. The coupling standard polypeptide composites can be formed before, during or after providing separation standard polypeptides to a sample or fraction thereof. The coupling standard polypeptide composites can be formed before, during or after providing functionalization standard polypeptides to a sample or fraction thereof. The coupling standard polypeptide composites can be formed before, during or after providing the coupling standard polypeptides to a sample or fraction thereof. The coupling standard polypeptide composites may be formed before, during or after attaching the sample polypeptide(s) to address(es) on a solid support.

When forming polypeptide arrays from a plurality of sample polypeptide composites, the attachment of each sample polypeptide composite to a solid support may further comprise attaching one or more coupling standard polypeptide composites to an address on the solid support. The total quantity of coupling standard polypeptides or coupling standard polypeptide composites (relative to the plurality of coupling standard polypeptides provided to a sample preparation process) that are bound to the polypeptide array may be utilized to determine the completeness of a

polypeptide coupling method or determine the extent of coupling of sample polypeptides from a sample. The quantity of coupling standard polypeptide composites bound to a solid support of a polypeptide array may be at least about 0.01%, 0.05%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, 99.5%, 99.9%, 99.99%, 99.999% or more than 99.999% of the quantity of the plurality of coupling standard polypeptides provided to a polypeptide sample preparation method. Alternatively or additionally, the quantity of coupling standard polypeptide composites bound to a solid support of a polypeptide array may be no more than about 99.999%, 99.99%, 99.9%, 99.5%, 99%, 95%, 90%, 85%, 80%, 75%, 70%, 65%, 60%, 55%, 50%, 45%, 40%, 35%, 30%, 25%, 20%, 15%, 10%, 5%, 1%, 0.5%, 0.1%, 0.05%, 0.01%, or less than 0.01% of the quantity of the plurality of coupling standard polypeptides provided to a polypeptide sample preparation method.

A sample preparation method may comprise the step of providing one or more attachment standard polypeptides. The attachment standard polypeptide(s) may be provided by combining the attachment standard polypeptide(s) with a sample or fraction thereof. The attachment standard polypeptides may be provided by combining the attachment standard polypeptides with one or more sample polypeptides derived from a sample. Combining the attachment standard polypeptide(s) with the sample polypeptide(s) may occur before, during or after attaching the sample polypeptides to a solid support. Combining the attachment standard polypeptides with the sample polypeptide(s) may occur before separating the sample polypeptide(s) from a component of the sample. The attachment standard polypeptide(s) can be combined with the sample polypeptides before, during or after coupling the sample polypeptide(s) to anchoring group(s).

The plurality of attachment standard polypeptides may comprise a plurality of attachment standard polypeptide composites, where each attachment standard polypeptide composite of the plurality of attachment standard polypeptide composites comprises an attachment standard polypeptide coupled to an anchoring group. The attachment standard polypeptide composites can be formed before, during or after providing the attachment standard polypeptides to a sample or fraction thereof. The attachment standard polypeptide composites can be formed before, during or after providing separation standard polypeptides to a sample or fraction thereof. The attachment standard polypeptide composites can be formed before, during or after providing functionalization standard polypeptides to a sample or fraction thereof. The attachment standard polypeptide composites can be formed before, during or after providing the coupling standard polypeptides to a sample or fraction thereof. When forming polypeptide arrays from a plurality of sample polypeptide composites, the attaching of each sample polypeptide composite to a solid support may further comprise attaching one or more attachment standard polypeptide composites to an address on the solid support The total quantity of attachment standard polypeptides or attachment standard polypeptide composites (relative to the plurality of attachment standard polypeptides provided to a sample preparation process) that are attached to the polypeptide array may be utilized to determine the completeness of a polypeptide attachment method or determine the extent of attaching of sample polypeptides from a sample. The quantity of attachment standard polypeptide composites bound to a solid support of a polypeptide array may be at least about 0.01%, 0.05%, 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 99%, 99.5%, 99.9%, 99.99%, 99.999% or more than 99.999% of the quantity of the plurality of attachment standard polypeptides provided to a polypeptide sample preparation method. Alternatively or additionally, the quantity of attachment standard polypeptide composites bound to a solid support of a polypeptide array may be no more than about 99.999%, 99.99%, 99.9%, 99.5%, 99%, 95%, 90%, 85%, 80%, 75%, 70%, 65%, 60%, 55%, 50%, 45%, 40%, 35%, 30%, 25%, 20%, 15%, 10%, 5%, 1%, 0.5%, 0.1%, 0.05%, 0.01%, or less than 0.01% of the quantity of the plurality of attachment standard polypeptides provided to a polypeptide sample preparation method.

The outcome of a sample polypeptide preparation method or a sample polypeptide preparation process within a sample polypeptide preparation method may be characterized quantitatively or qualitatively utilizing zero, one or more than one internal standard. The outcome of a sample polypeptide preparation method or a sample polypeptide preparation process within a sample polypeptide preparation method may be characterized by measuring a relative weight ratio of sample polypeptides or sample polypeptide composites to internal standard polypeptides or internal standard polypeptide composites. For example, the sample polypeptide : separation standard polypeptide ratio may be measured before and after a sample polypeptide separation process to determine if the sample polypeptide separation process separated sample polypeptides in an expected manner. In some cases, a weight ratio of a quantity of sample polypeptides in the sample to the plurality of internal standard polypeptides is larger than a weight ratio of a quantity of sample polypeptides in the polypeptide array to a quantity of separation standard polypeptides in the polypeptide array. In other cases, a weight ratio of a quantity of sample polypeptides in the sample to the plurality of internal standard polypeptides is smaller than a weight ratio of a quantity of sample polypeptides in the polypeptide array to a quantity of separation standard polypeptides in the polypeptide array. The weight ratio of a quantity of sample polypeptides in the sample to the plurality of internal standard polypeptides may increase or decrease relative to a weight ratio of a quantity of sample polypeptides in the polypeptide array to a quantity of separation standard polypeptides in the polypeptide array by about 1%, 5%, 10%, 15%, 20%, 25%, 30%, 35%, 40%, 45%, 50%, 55%, 60%, 65%, 70%, 75%, 80%, 85%, 90%, 95%, 99%, 99.9%, or about 100%. The weight ratio of a quantity of sample polypeptides in the sample to the plurality of internal standard polypeptides may

increase or decrease relative to a weight ratio of a quantity of sample polypeptides in the polypeptide array to a quantity of separation standard polypeptides in the polypeptide array by at least about 1%, 5%, 10%, 15%, 20%, 25%, 30%, 35%, 40%, 45%, 50%, 55%, 60%, 65%, 70%, 75%, 80%, 85%, 90%, 95%, 99%, 99.9%, or more than 99.9%. Alternatively or additionally, the weight ratio of a quantity of sample polypeptides in the sample to the plurality of internal standard polypeptides may increase or decrease relative to a weight ratio of a quantity of sample polypeptides in the polypeptide array to a quantity of separation standard polypeptides in the polypeptide array by no more than about 100%, 99.9%, 99%, 95%, 90%, 85%, 80%, 75%, 70%, 65%, 60%, 55%, 50%, 45%, 40%, 35%, 30%, 25%, 20%, 15%, 10%, 5%, 1%, or less than 1%.

A mixture of standard polypeptides may be provided to a polypeptide sample preparation method, for example, before, during or after a step set forth herein or otherwise known in the art. A mixture of standard polypeptides may include two or more standard polypeptides provided from the group consisting of sample collection standard polypeptides, sample handling standard polypeptides, separation standard polypeptides, functionalization standard polypeptides, coupling standard polypeptides, and attachment standard polypeptides. In some cases, a mixture of each species of standard polypeptide may be combined with a sample before a sample preparation method.

One or more internal standards may be provided to a polypeptide assay, for example, after one or more of the sample preparation processes set forth above or elsewhere herein. Polypeptide standards that are provided to a polypeptide assay can be used to evaluate characteristics such as the specificity or promiscuity of probes used in a binding assay, the quantity of particular sample polypeptides present in the assay, the intensity of labels used to detect particular sample polypeptides, the level of background signal (i.e. noise) resulting from a particular detection technique, the length of particular sample polypeptides in an assay, the robustness of particular sample polypeptides to repeated exposure to assay reagents, or the robustness of particular sample polypeptides to repeated exposure to energy from detection hardware.

In some configurations, a full set of polypeptide standards can contain all known peptide sequences of a particular length. Alternatively, a partial set of polypeptide standards can contain a subset of all known peptide sequences of a particular length. Whether present in a full set or partial set, each polypeptide standard can include one and only one of the known sequences. Optionally, each of the standard polypeptides in the full or partial set can further include a unique tag that distinguishes it from all other standard polypeptides in the set. Alternatively or additionally, all of the standard polypeptides in a particular set (whether a full set or partial set) can include a tag that is universal to the particular set. The universal tag can be useful for conveniently distinguishing the set from other polypeptides that are present in the same assay. Exemplary tags, whether used as universal tags or unique tags, can be peptide sequences, signal producing labels (e.g. fluorophores), or a combination thereof. The length of the sequences in the set can be at least 2 amino acids, 3 amino acids, 4 amino acids, 5 amino acids, 6 amino acids, 7 amino acids, 8 amino acids, 9 amino acids, 10 amino acids or more. Alternatively or additionally, the length of the sequences in the set can be at most 10 amino acids, 9 amino acids, 8 amino acids, 7 amino acids, 6 amino acids, 5 amino acids, 4 amino acids, 3 amino acids, or 2 amino acids. A full or partial set of polypeptide standards having sequences of a particular length can be useful, for example, to evaluate the specificity or promiscuity of probes that bind to epitopes of a particular length, the level of background signal (i.e. noise) resulting from use of particular probes, the robustness of particular sample polypeptides to repeated exposure to binding reagents, or the degree to which binding reagents are removed from an assay when repeated binding steps are employed.

In some configurations, a full set of polypeptide standards can contain all known peptide sequences that include the same epitope flanked on one or both sides by a different amino acid. Alternatively, a partial set of polypeptide standards can contain a subset of all known peptide sequences that include the same epitope flanked on one or both sides by a different amino acid. In some configurations, a full set of polypeptide standards can contain all known peptide sequences that include the same epitope flanked on one or both sides by different peptide sequences of a particular length. Alternatively, a partial set of polypeptide standards can contain a subset of all known peptide sequences that include the same epitope flanked on one or both sides by different peptide sequences of a particular length. The length of an epitope used in a method or compositon set forth herein can be at least 2 amino acids, 3 amino acids, 4 amino acids, 5 amino acids, 6 amino acids, 10 amino acids or more. Alternatively or additionally, the length of an epitope can be at most 10 amino acids, 6 amino acids, 5 amino acids, 4 amino acids, 3 amino acids, or 2 amino acids. The length of a flanking sequence on either or both sides of an epitope can be at least 2 amino acids, 3 amino acids, 4 amino acids, 5 amino acids, 6 amino acids, 7 amino acids, 8 amino acids, 9 amino acids, 10 amino acids or more. Alternatively or additionally, the length of a flanking sequence on either or both sides of an epitope can be at most 10 amino acids, 9 amino acids, 8 amino acids, 7 amino acids, 6 amino acids, 5 amino acids, 4 amino acids, 3 amino acids, or 2 amino acids. Whether present in a full set or partial set, each polypeptide standard can include one and only one of the flanking amino acids or sequences. Optionally, each of the standard polypeptides in the full or partial set can further include a unique tag that distinguishes it from all other standard polypeptides in the set. Alternatively or additionally, all of the standard polypeptides in a particular set (whether a full set or partial set) can include a tag that is universal to the particular set. The universal tag can be useful for conveniently distinguishing the set from other polypeptides that are present in the same assay. Exemplary tags, whether used as universal tags or unique tags, can be peptide sequences, signal producing labels (e.g. fluorophores) or a combination

thereof. A full or partial set of polypeptide standards having the same epitope flanked on one or both sides by a different amino acid or different peptide sequence can be useful, for example, to evaluate the specificity or promiscuity of probes that bind to epitopes of a particular length, the level of background signal (i.e. noise) resulting from use of particular probes, the robustness of particular sample polypeptides to repeated exposure to binding reagents, or the degree to which binding reagents are removed from an assay when repeated binding steps are employed.

In some configurations, a polypeptide standard may be provided with a full set of target epitopes or sequences containing specific amino acids that are likely to be modified (e.g., due to post-translational modification; due to functionalization reactions). For example, a polypeptide standard may comprise a plurality of polypeptides that collectively contain every possible trimer epitope with an amine- or thiol-containing amino acid (e.g., arginine, lysine, cysteine). Such standards can be useful, for example, if amine- or thiol-targeting functionalization chemistries are utilized for polypeptide sample preparation. In some configurations, a polypeptide internal standard comprising a full set of target epitopes or sequences containing specific amino acids that are likely to be modified may be utilized in an unmodified form and/or included with a sample undergoing a sample preparation process. For example, a polypeptide array may be formed by first applying an unmodified polypeptide internal standard, then depositing a polypeptide sample on the array comprising the same internal standard that has undergone one or more steps of the sample preparation process. Differences in assay behavior between the unmodified and modified polypeptide standards may provide qualitiative and/or quantitative information on the impact of modifications to certain amino acids or epitopes during a sample preparation process.

In some configurations, a set of polypeptide standards can contain polypeptide standards that are uniquely tagged, one from the other, and present in different quantities. As such, the quantity of each tag in the set will be different from the quantity of the other tags in the set. As an additional option, all of the standard polypeptides in the set can include a tag that is universal to all members of the set. The universal tag can be useful for conveniently distinguishing the set from other polypeptides that are present in the same assay. Exemplary tags, whether used as universal tags or unique tags, can be peptide sequences, signal producing labels (e.g. fluorophores) or a combination thereof. The standard polypeptides in the set can cover a variety of ranges including, for example, a range of at least 0.1, 0.5, 1, 2, 3, 4, 5, 6, 7, 8, 9 or 10 orders of magnitude. Alternatively or additionally, the standard polypeptides in the set can cover a range of at most 10, 9, 8, 7, 6, 5, 4, 3, 2, 1, 0.5 or 0.1 orders of magnitude. When used in single molecule, array-based formats, the standard polypeptides can be quantified by counting the number of addresses on the array that are occupied by each tag. In some formats, the standard polypeptides can be quantified by detecting the concentration or total amount of each tag in an assay sample. A set of polypeptide standards having tagged members that are present in different quantities can be useful, for example, to evaluate the quantity of particular sample polypeptides present in the assay, the intensity of labels used to detect particular sample polypeptides, the level of background signal (i.e. noise) resulting from a particular detection technique, the robustness of particular sample polypeptides to repeated exposure to assay reagents, or the robustness of particular sample polypeptides to repeated exposure to energy from detection hardware.

In some configurations, polypeptide standards in a set of polypeptide standards can contain peptide sequences of a different length from other polypeptide standards in the set. Optionally, each of the standard polypeptides in the set can further include a unique tag that distinguishes it from all other standard polypeptides in the set. Alternatively or additionally, all of the standard polypeptides in a particular set (whether a full set or partial set) can include a tag that is universal to the particular set. The universal tag can be useful for conveniently distinguishing the set from other polypeptides that are present in the same assay. Exemplary tags, whether used as universal tags or unique tags, can be peptide sequences, signal producing labels (e.g. fluorophores), or a combination thereof. The length of the sequences in the set can be at least 5 amino acids, 10 amino acids, 25 amino acids, 50 amino acids, 100 amino acids, or more. Alternatively or additionally, the length of the sequences in the set can be at most 100 amino acids, 50 amino acids, 25 amino acids, 10 amino acids, or 5 amino acids. A full or partial set of polypeptide standards having sequences of a particular length can be useful, for example, to evaluate the specificity or promiscuity of probes used in a binding assay, the level of background signal (i.e. noise) resulting from a particular detection technique, or the length of particular sample polypeptides in an assay.

A polypeptide internal standard may be utilized as a reporter or proxy for conditions during a polypeptide assay. For example, an enzyme with an orthogonal reactivity or specificity may be used to assess proper denaturing conditions during a polypeptide assay. In the presence of a substrate, the presence or absence of detectable enzymatic activity would provide a proxy signal for the successful denaturation of polypeptides in the assay. Likewise, similar reporting polypeptides could be provided for other assay conditions, such as pH, ionic strength, or temperature.

The description describes a method of identifying a quality control metric for a polypeptide array, comprising: a) contacting a plurality of polypeptides with a polypeptide array; b) contacting a plurality of internal standard moieties with a polypeptide array; c) detecting a presence or absence of an internal standard moiety at each address of the polypeptide array; and d) based upon the presence or absence of each internal standard moiety of the plurality of internal standard moieties, determining the quality control metric for the polypeptide array. A quality control metric may comprise a metric that provides information on the outcome (e.g., efficiency, completion, state, etc.) of a polypeptide array formation process. A quality control metric may be calculated for any step or set of steps of a polypeptide array formation process, including but not limited to: a) sample collection; b) sample storage; c) sample purification; d) sample pre-processing; e) polypeptide

purification; f) polypeptide functionalization; g) polypeptide conjugate formation; and h) polypeptide conjugate deposition. An internal standard may be provided for any step of a polypeptide array formation process to facilitate determining a quality control metric. Based upon a quality control metric, a polypeptide array formation process may further comprise one or more steps, selected from a group consisting of: a) repeating a step of the polypeptide array formation process; b) repeating an entire polypeptide array formation process; c) discarding the polypeptide array; d) stripping one or more moieties from the polypeptide array; and e) performing an alternative polypeptide array formation method.

An internal standard may be provided during a polypeptide array formation process to facilitate the determination of a quality control metric. An internal standard, as set forth herein, may be specifically designed and/or selected based upon chemical, physical, and/or biological properties that provide information on an outcome of a polypeptide array formation process. For example, an internal standard for a polypeptide conjugate formation process (e.g., coupling of a polypeptide to an anchoring group) may comprise: i) a first polypeptide with a plurality of functionalization sites; and ii) a second polypeptide with a single functionalization, in which detection of a magnitude of a signal (e.g., a step-wise change in fluorescent signal) from a polypeptide conjugate comprising the first polypeptide or the second polypeptide is used to determine a quality control metric regarding the average number of anchoring groups conjugated to a polypeptide.

Table I provides a non-exhaustive listing of quality control metrics and methods for determining them. In some cases, a method of determining a quality control metric may occur before a polypeptide assay of a polypeptide array is initiated. In other cases, a method of determining a quality control metric may occur during or after a polypetide assay of a polypeptide array is initiated. In some cases, a polypeptide assay may comprise a step of determining a quality control metric before completing the polypeptide assay. In other cases, a quality control metric may be determined from data collected during a polypeptide assay (e.g., by cyclic contacting of affinity agents with a polypeptide array, by cyclic degradation steps of a sequencing assay, etc.).

**Table I. Quality Control Metrics for Polypeptide Arrays**

| Quality Control Metric | Exemplary Method of Determination | Exemplary Detection Method |
|---|---|---|
| Average number of anchoring groups per polypeptide | i) provide a ladder of coupling standards (e.g., laddered by polypeptide length, laddered by quantity of functionalized side chains, ladder of all possible epitopes containing a functionalized group, etc.) to a polypeptide sample;<br>ii) conjugate labeled anchoring groups to the polypeptide sample comprising the ladder of coupling standards;<br>iii) couple polypeptide conjugates to a polypeptide array;<br>iv) measure signal intensity from each conjugate containing a coupling standard | Fluorescence intensity from anchoring group; coupling of labeled molecules to anchoring groups; expect step-wise change in intensity for each additional anchoring group |
| Average number of polypeptides per anchoring group | i) provide a ladder of coupling standards (e.g., laddered by polypeptide length, laddered by quantity of functionalized side chains, ladder of all possible epitopes containing a functionalized group, etc.) to a polypeptide sample;<br>ii) conjugate labeled coupling standards to anchoring groups;<br>iii) couple polypeptide conjugates to a polypeptide array;<br>iv) measure signal intensity from each conjugate containing a coupling standard | Fluorescence intensity from labeled standard polypeptides; coupling of labeled molecules to polypeptides; expect step-wise change in intensity for each additional polypeptide |
| Separation efficiency of binding pairs | i) provide a ladder of binding-pair separation standards (e.g., laddered by length, laddered by net electrical charge, laddered by polarity, laddered by hydrophobicity, laddered by sequence diversity, etc.) to a polypeptide sample (e.g., receptor-ligand pairs, antibody-antigen pairs);<br>ii) disrupt binding interactions (e.g., via a chaotrope);<br>iii) couple polypeptides to a polypeptide array; | Affinity agent binding and decoding of binding patterns to determine an identity of each polypeptide |

(continued)

| Quality Control Metric | Exemplary Method of Determination | Exemplary Detection Method |
|---|---|---|
| | iv) detect presence of each binding pair member at differing array addresses | |

**Table I Cont'd. Quality Control Metrics for Polypeptide Arrays**

| Quality Control Metric | Exemplary Method of Determination | Exemplary Detection Method |
|---|---|---|
| Average number of anchoring groups per array address | i) conjugate labeled anchoring groups to a polypeptide sample;<br>ii) couple polypeptide conjugates to a polypeptide array;<br>iii) measure signal intensity from each array address | Fluorescence intensity; expect step-wise change in signal intensity for each additional anchoring groups coupled to an array address |
| Non-polypeptide separation efficiency | i) provide a separation standard comprising labeled non-polypeptide standards to a polypeptide-containing sample;<br>ii) separate polypeptide-containing fraction from sample;<br>iii) couple polypeptide-containing fraction to anchoring groups;<br>iv) coupled polypeptide conjugates to a polypeptide array<br>v) measure presence or absence of non-polypeptide standards on the array | Fluorescence intensity of labeled standards; non-polypeptide specific affinity agent binding to non-polypeptide standards |
| Array reuse efficiency | i) couple a ladder of storage standards comprising varying levels of temperature sensitivity (e.g., laddered by length, laddered by denaturation temperature, laddered by oxidation susceptibility, laddered by reduction susceptibility, laddered by hydrolyzability, etc.) to a polypeptide array;<br>ii) store the polypeptide array at a set thermal condition (e.g., -80 °C, 4 °C, 20 °C);<br>iii) after storage, measure a presence or absence of thermal damage to storage standard polypeptides | Affinity agent binding; thermal damage should alter or disrupt particular epitopes in storage standards; presence or absence of epitopes suggests extent of thermal damage |

**Array Compositions**

In another aspect, provided herein is a composition comprising a) a solid support comprising a plurality of addresses, in which each address of the plurality of addresses is spatially resolvable from each other address of the plurality of addresses, and in which each address of the plurality of addresses is configured to couple a polypeptide, and b) a sample polypeptide mixture, in which the sample polypeptide mixture comprises a plurality of sample polypeptides, a plurality of separation standard polypeptides, and a plurality of coupling standard polypeptides, and in which each polypeptide of the sample polypeptide mixture is coupled to an anchoring group, in which the anchoring group is configured to couple a polypeptide to an address of the plurality of addresses.

Upon completion of a sample polypeptide preparation process, an array comprising a plurality of polypeptides, including a plurality of standard polypeptides may be formed. A polypeptide array may comprise a plurality of addresses, in which each address of the plurality of addresses is spatially resolvable from each other address of the plurality of addresses, and in which a subset of the plurality of addresses comprises a plurality of standard polypeptides, in which each address of the subset of the plurality of addresses comprises a standard polypeptide of the plurality of standard polypeptides. In a particular configuration, a polypeptide array may comprise a plurality of addresses, in which each address of the plurality of addresses is spatially resolvable from each other address of the plurality of addresses, and in which a subset of the plurality of addresses comprises a plurality of standard polypeptides, in which each address of the subset of the plurality of addresses comprises one and only one standard polypeptide of the plurality of standard

polypeptides. A skilled person will readily recognize that a polypeptide array, as set forth herein, may comprise a plurality of standard polypeptides for various purposes (e.g., experimental controls, quality control, data analysis, etc.) that can be assayed simultaneously with sample polypeptides. This differs from many bulk or ensemble polypeptide assay methods (e.g., Western blot, ELISA, mass spectrometry), in which control and/or quality control samples must be analyzed via a separate experiments, then compared to sample results.

In some configurations, a polypeptide array, as set forth herein, may comprise a plurality of standard polypeptides, in which the plurality of standard polypeptides comprises a random spatial distribution on the polypeptide array. A plurality of standard polypeptides may be characterized as comprising a random spatial distribution on a polypeptide array if a subset of addresses comprising a standard polypeptide lack a spatial grouping (e.g., an array does not comprise a contiguous cluster of addresses comprising standard polypeptides) and/or lack a spatial order (e.g., a polypeptide array does not comprise a column of addresses, in which an address comprises a standard polypeptide once every 10 addresses). Alternatively, a plurality of standard polypeptides may be characterized as comprising a random spatial distribution on a polypeptide array if a likelihood of any group of one or more addresses of a plurality of addresses on the polypeptide array containing a standard polypeptide is described by a probabilistic function (e.g., a Poisson distribution). For example, a group of ten randomly chosen addresses may have a 30% chance of containing no standard polypeptides, a 40% chance of containing only one standard polypeptide, and a 30% chance of containing two or more standard polypeptides. In some configurations, a probabilistic function describing a random spatial distribution of standard polypeptides on a polypeptide array may be correlated to and/or a function of a quantity and/or concentration of standard polypeptides in a sample polypeptide mixture from which a polypeptide array is prepared. A random spatial distribution of standard polypeptides on a polypeptide array may occur if sample polypeptides and standard polypeptides are deposited by a same deposition chemistry (e.g., same anchoring groups for polypeptide composites of sample polypeptides and standard polypeptides). **FIG. 17D** depicts an array with standard polypeptides (denoted by black addresses) in a random spatial distribution. A skilled person will readily recognize that a polypeptide array, as set forth herein, may comprise a plurality of standard polypeptides for various purposes (e.g., experimental controls, quality control, data analysis, etc.) that can be assayed simultaneously with sample polypeptides. This differs from many bulk or ensemble polypeptide assay methods (e.g., Western blot, ELISA, mass spectrometry), in which control and/or quality control samples must be analyzed via a separate experiments, then compared to sample results.

In other configurations, a polypeptide array, as set forth herein, may comprise a plurality of standard polypeptides, in which the plurality of standard polypeptides comprises an ordered spatial distribution on the polypeptide array. A plurality of standard polypeptides may be characterized as comprising an ordered spatial distribution on a polypeptide array if a subset of addresses comprising a standard polypeptide comprise a spatial grouping (e.g., an array comprises one or more contiguous clusters of addresses comprising standard polypeptides) and/or possess a spatial order (e.g., a polypeptide array comprises one or more columns of addresses, in which an address of a column comprises a standard polypeptide once every 10 addresses). **FIGs. 17A - 17C** depict examples of arrays with standard polypeptides (denoted by black addresses) in ordered spatial distributions. **FIG. 17A** depicts a polypeptide array with multiple ordered clusters of control polypeptides. **FIG. 17B** depicts a polypeptide array with a recurring diagonal pattern of addresses containing standard polypeptides. **FIG. 17C** depicts a polypeptide array with two different recurring patterns, for example to distinguish a region containing a first type of standard polypeptide from a region containing a second type of standard polypeptide. An ordered spatial distribution of standard polypeptides on a polypeptide array may occur if, for example, sample polypeptides and standard polypeptides are deposited on the polypeptide array by differing deposition chemistries (e.g., differing anchoring groups that correspond to differing surface chemistries on particular array addresses).

A polypeptide array, as set forth herein, may comprise a plurality of standard polypeptides. A standard polypeptide of a plurality of standard polypeptides, when coupled to a polypeptide array, may be utilized for various purposes, including qualititative and/or quantitative evalution of a polypeptide assay. A plurality of standard polypeptides coupled to a polypeptide array may include a positive control polypeptide (e.g., a synthetic polypeptide with an amino acid sequence that is identical to an amino acid sequence of a sample polypeptide) and/or a negative control polypeptide (e.g., a synthetic polypeptide with an amino acid sequence that is not naturally found in a sample). A plurality of standard polypeptides coupled to a polypeptide array may include a polypeptide that can be analyzed to provide a quantitiative measure of an efficiency of a step of a sample preparation process (e.g., a functionalization standard polypeptide, a storage standard polypeptide, a coupling standard polypeptide, a separation standard polypeptide, an attachment standard polypeptide, etc.). A plurality of standard polypeptides coupled to a polypeptide array may include a polypeptide that can be analyzed to provide a quantitiative measure of a binding characteristic of an affinity agent (e.g., a binding affinity, a binding specificity, a binding promiscuity, etc.). A plurality of standard polypeptides coupled to a polypeptide array may include a polypeptide that can be analyzed to provide a quantitiative quality control measure for a polypeptide array and/or a polypeptide assay.

A polypeptide array, as set forth herein, may comprise a plurality of standard polypeptides, in which the plurality of standard polypeptides comprises a ladder of properties. A ladder of properties may comprise a plurality of polypeptides that encompass a range of a polypeptide property or characteristic. For example, a ladder of length standards may

comprise a plurality of peptides with chain lengths from 5 amino acids to 1000 amino acids, including intermediate lengths. In some cases, a ladder of polypeptides may comprise a first bounding standard polypeptide, a second bounding standard polypeptide, and one or more intermediate standard polypeptides, in which the first and second bounding standard polypeptides encompass endpoints of a selected range of a property and the one or more intermediate standard polypeptides have a value of the property between the endpoints. A ladder of standard polypeptides may contain one or more intermediate standard polypeptides, such as at least about 1, 2, 3, 4, 5, 6, 7, 8, 9, 10, 15, 20, 25, 30, 35, 40, 45, 50, 60, 70, 80, 90, 100 or more than 100 intermediate standard polypeptides. Alternatively or additionally, a ladder of standard polypeptides may contain one or more intermediate standard polypeptides, such as no more than about 100, 90, 80, 70, 60, 50, 45, 40, 35, 30, 25, 20, 15, 10, 9, 8, 7, 6, 5, 4, 3, 2, or less than 2 intermediate standard polypeptides.

A ladder of standard polypeptides may be laddered based on one or more polypeptide properties. A ladder of standard polypeptides may comprise a range of polypeptide properties that can affect the outcome of a polypeptide sample preparation process and/or a polypeptide assay. For example, a plurality of standard polypeptides may comprise a ladder of functionalization standard polypeptides, in which the ladder of functionalization standard polypeptides comprises a range of polypeptides characterized by number of available functionalizable amino acid residues (e.g., from 0 lysines to 20 or more lysines). A ladder of standard polypeptides may contain a range of standard polypeptides encompassing a polypeptide property, such as polypeptide length, polypeptide net electrical charge, polypeptide isoelectric point, polypeptide polarity, and/or polypeptide hydrodynamic radius. A ladder of standard polypeptides may contain a range of standard polypeptides based upon a measure of sequence diversity or sequence content. For example, a ladder of polypeptides may comprise a ladder of polypeptides with varying numbers of a particular amino acid (e.g., from 0 lysines to 20 or more lysines, from 0 arginines to 20 or more arginines, etc.). In another example, a ladder of polypeptides may comprise a ladder of polypeptides with a range of sequence epitope diversity (e.g., all possible dimer, trimer, or quadramer epitopes comprising lysine, arginine, typtophan, etc.). In another example, a ladder of polypeptides may comprise a ladder of polypeptides with a range of sequence epitope position (e.g., a trimer epitope located at any position between the C-terminus and N-terminus of a polypeptide). A ladder of standard polypeptides may be designed or assembled based upon a range of a polypeptide property for a reference sequence length. For example, a ladder based upon net electrical charge may be based upon a maximum net electrical charge for any 10 amino acid residue sequence of a given polypeptide. A polypeptide property for a standard polypeptide may be based upon a reference sequence length of at least about 5, 10, 15, 20, 25, 30, 35, 40, 45, 50, 60, 70, 80, 90, 100, 125, 150, 200, or more than 200 amino acid residues. Alternatively or additionally, a polypeptide property for a standard polypeptide may be based upon a reference sequence length of no more than about 200, 150, 125, 100, 90, 80, 70, 60, 50, 45, 40, 35, 30, 25, 20, 15, 10, 5, or less than 5 amino acid residues.

A plurality of standard polypeptides coupled to a polypeptide array, as set forth herein, may comprise a standard polypeptide that is chemically or structurally identical to a sample polypeptide on the polypeptide array. For example, a plurality of sample polypeptide derived from a blood sample that is expected to contain a protein (e.g., VEGF, EGFR, etc.) may be coupled to a polypeptide array that further contains a tagged standard of the same polypeptide (e.g., a his-tagged VEGF, a hig-tagged EGFR, etc.). A plurality of standard polypeptides coupled to a polypeptide array may comprise a standard polypeptide that is not chemically or structurally identical to a sample polypeptide on the polypeptide array. In some cases, a plurality of standard polypeptides coupled to a polypeptide array may comprise a standard polypeptide that does not naturally occur or is not expected to naturally occur in a sample, a sample source, a proteome, and/or a sub-proteome. For example, a standard polypeptide may comprise a synthetic polypeptide or a polypeptide from a cloned microorganism that has a sequence that is not known to exist naturally. In another example, a standard polypeptide may comprise an unmodified version of a polypeptide that is expected to naturally occur in a post-translationally modified form (e.g., a splice variant, a glycosylated polypeptide, a phosphorylated polypeptide, etc.). In another example, a plurality of standard polypeptides may comprise one or more polypeptides with a non-natural amino acid composition (e.g., an overabundance or underabundance of a particular amino acid per sequence length relative to a source organism, a deviation from a natural ratio between two amino acids for a source organism, etc.).

A polypeptide array, as set forth herein, may comprise a plurality of standard polypeptides, in which the plurality of standard polypeptides comprises one or more unexpected or anomalous standard polypeptides. In some cases, a polypeptide array may comprise one or more standard polypeptides that were designed, engineered and/or selected to not be coupled to the polypeptide array. For example, a functionalization standard polypeptide may comprise no functional groups that are targeted by a functionalization chemistry and is thus expected to not be configured to couple to an anchoring group, but becomes coupled to an anchoring group by an unknown or unexpected mechanism. In another example, a separation standard polypeptide may be designed to not be captured by an affinity-based separation method (e.g., affinity chromatography), but becomes coupled to a polypeptide array due to anomalous or inefficient separation during the separation process. A presence or absence of an unexpected or anomalous standard polypeptide may be correlated to a presence or an absence of an unexpected or anomalous sample polypeptide on a polypeptide array. A presence or absence of an unexpected or anomalous standard polypeptide may inform a performance of a polypeptide assay or may inform an interpretation of data or results from a polypeptide assay. For example, based upon the presence or absence of an unexpected or anomalous standard polypeptide, a polypeptide assay may be performed with a set of affinity

agents that are selected based upon the presence or absence of the unexpected or anomalous standard polypeptide. In another example, a polypeptide assay may be re-performed with a second set of affinity agents that are selected based upon the presence or absence of the unexpected or anomalous standard polypeptide. In another example, an assay result may be reinterpreted (e.g., utilizing a different result database, utilizing a different data analysis algorithm, etc.) based upon a presence or absence of the unexpected or anomalous standard polypeptide.

A polypeptide array, as set forth herein, may comprise a plurality of standard polypeptides, in which the standard polypeptides comprise a fragment library. A fragment library may comprise one or more peptides or polypeptides that comprise a truncated amino acid sequence of a known or characterized protein or polypeptide. In some cases, a fragement library may be comprise one or more synthetic peptides or polypeptides. In other cases, a fragment library may comprise one or more natural or biologically-derived peptides or polypeptides (e.g., fragmented polypeptides from a control sample, fragmented peptides of a polypeptide produced by an engineered microorganism, etc.). A fragment library may comprise a complete range of possible peptide fragments of a known protein or polypeptide, or a subset thereof. For example, polypeptides from a blood sample that may contain VEGF may be coupled to a polypeptide array with a standard fragment library comprising each possible truncated amino acid sequence of VEGF. A fragment library may be especially useful for a polypeptide assay that utilizes a fragmentation approach to polypeptide analysis (e.g., a fluorosequencing method, an affinity agent-based sequencing method, etc.).

A polypeptide array, as set forth herein, may comprise a plurality of standard polypeptides, in which the standard polypeptides comprise a proteoform library. A proteoform library may comprise one or more standard polypeptides that are proteoforms of a sample polypeptide. For example, a proteoform library for a protein that is typically glycosylated on serine and/or threonine residues may comprise each possible permutation of glycosylation amongst all serine and threonine residues of the protein, or a subset thereof. In some cases, a proteoform library may comprise a plurality of standard polypeptide proteoforms selected from a group consisting of: i) sequence-based variants; ii) splice isoforms; and iii) post-translationally modified isoforms. In some cases, a proteoform library may comprise damaged, degraded, truncated, and/or modified versions of a sample polypeptide. In particular cases, a proteoform library may comprise a polypeptide arising due to an environmental constituent, such as a contaminant (e.g., carcinogens, teratogens, mutagens, toxins, nanoparticles, oxidants, reductants, etc.) and/or pharmaceutical compounds.

A polypeptide array, as set forth herein, may comprise a plurality of standard polypeptides, in which two or more standard polypeptides of the plurality of standard polypeptides are configured to form a polypeptide complex. In some cases, a polypeptide complex standard pair may be introduced during a polypeptide preparation process to assess the outcome of a similar or identical sample polypeptide complex in the array format. In some configurations, a polypeptide array may comprise a first standard polypeptide of a polypeptide complex and a second standard polypeptide of the polypeptide complex, in which the first standard polypeptide and the second standard polypeptide are coupled to different addresses of the polypeptide array. In other configurations, a polypeptide array may comprise a first standard polypeptide of a polypeptide complex and a second standard polypeptide of the polypeptide complex, in which the first standard polypeptide and the second standard polypeptide are coupled to a same address of the polypeptide array. In particular configurations, a polypeptide array may comprise a first standard polypeptide of a polypeptide complex and a second standard polypeptide of the polypeptide complex, in which the first standard polypeptide and the second standard polypeptide are coupled to a same address of the polypeptide array, and in which the the first standard polypeptide and the second standard polypeptide are cross-linked to each other. In other configurations, a polypeptide array may comprise a first standard polypeptide of a polypeptide complex and a second standard non-polypeptide of the polypeptide complex, in which the first standard polypeptide and the second standard non-polypeptide are coupled to a same address of the polypeptide array. For example, a DNA-binding protein standard may be coupled to an array with a DNA molecule to which the DNA-binding molecule is configured to bind.

A polypeptide array, as set forth herein, may comprise a plurality of non-polypeptide standards. A non-polypeptide standard may comprise a standard for an impurity or a waste component of a sample (e.g, a lipid, saccharide, or nucleic acid from a sample organism). A non-polypeptide standard may comprise a standard for a reagent, component, material, and/or system utilized during a sample polypeptide preparation process, or a step thereof. For example, a plurality of sample polypeptides purified by an affinity chromatography separation process may be coupled to an array comprising a standard for the chromatographic affinity agent and a standard for the chromatographic support material to which the chromatographic affinity agent is bound. In some configurations, a polypeptide array may comprise a non-polypeptide standard, in which the non-polypeptide standard is a standard for a post-translational modification. For example, a polypeptide array may comprise a glycan standard or a phosphorylated amino acid standard (e.g., phosphotyrosine).

## Polypeptide Assays

The present disclosure provides compositions, apparatus and methods that can be useful for characterizing sample components, such as proteins, nucleic acids, cells or other species, by obtaining multiple separate and non-identical measurements of the sample components. In particular configurations, the individual measurements may not, by themselves, be sufficiently accurate or specific to make the characterization, but an aggregation of the multiple non-identical measurements can allow the characterization to be made with a high degree of accuracy, specificity and confidence. For example, the multiple separate measurements can include subjecting the sample to reagents that are promiscuous with regard to recognizing multiple components of the sample. Accordingly, a first measurement carried out using a first promiscuous reagent may perceive a first subset of sample components without distinguishing one component from another. A second measurement carried out using a second promiscuous reagent may perceive a second subset of sample components, again, without distinguishing one component from another. However, a comparison of the first and second measurements can distinguish: (i) a sample component that is uniquely present in the first subset but not the second; (ii) a sample component that is uniquely present in the second subset but not the first; (iii) a sample component that is uniquely present in both the first and second subsets; or (iv) a sample component that is uniquely absent in the first and second subsets. The number of promiscuous reagents used, the number of separate measurements acquired, and degree of reagent promiscuity (*e.g.* the diversity of components recognized by the reagent) can be adjusted to suit the component diversity expected for a particular sample.

The present disclosure provides assays that are useful for detecting one or more analytes. Exemplary assays are set forth herein in the context of detecting proteins. Those skilled in the art will recognize that methods, compositions and apparatus set forth herein can be adapted for use with other analytes such as nucleic acids, polysaccharides, metabolites, vitamins, hormones, enzyme co-factors and others set forth herein or known in the art. Particular configurations of the methods, apparatus and compositions set forth herein can be made and used, for example, as set forth in US Pat. No. 10,473,654 or US Pat. App. Pub. Nos. 2020/0318101 A1 or 2020/0286584 A1.

Exemplary methods, systems and compositions are set forth in further detail below.

A composition, apparatus or method set forth herein can be used to characterize an analyte, or moiety thereof, with respect to any of a variety of characteristics or features including, for example, presence, absence, quantity (*e.g.* amount or concentration), chemical reactivity, molecular structure, structural integrity (*e.g.* full length or fragmented), maturation state (e.g. presence or absence of pre- or pro- sequence in a protein), location (e.g. in an analytical system, subcellular compartment, cell or natural environment), association with another analyte or moiety, binding affinity for another analyte or moiety, biological activity, chemical activity or the like. An analyte can be characterized with regard to a relatively generic characteristic such as the presence or absence of a common structural feature (*e.g.* amino acid sequence length, overall charge or overall pKa for a protein) or common moiety (*e.g.* a short primary sequence motif or post-translational modification for a protein). An analyte can be characterized with regard to a relatively specific characteristic such as a unique amino acid sequence (*e.g.* for the full length of the protein or a motif), an RNA or DNA sequence that encodes a protein (*e.g.* for the full length of the protein or a motif), or an enzymatic or other activity that identifies a protein. A characterization can be sufficiently specific to identify an analyte, for example, at a level that is considered adequate or unambiguous by those skilled in the art.

In particular configurations, a protein can be detected using one or more affinity agents having known or measurable binding affinity for the protein. For example, an affinity agent can bind a protein to form a complex and a signal produced by the complex can be detected. A protein that is detected by binding to a known affinity agent can be identified based on the known or predicted binding characteristics of the affinity agent. For example, an affinity agent that is known to selectively bind a candidate protein suspected of being in a sample, without substantially binding to other proteins in the sample, can be used to identify the candidate protein in the sample merely by observing the binding event. This one-to-one correlation of affinity agent to candidate protein can be used for identification of one or more proteins. However, as the protein complexity (*i.e.* the number and variety of different proteins) in a sample increases, or as the number of different candidate proteins to be identified increases, the time and resources to produce a commensurate variety of affinity agents having one-to-one specificity for the proteins approaches limits of practicality.

Methods set forth herein, can be advantageously employed to overcome these constraints. In particular configurations, the methods can be used to identify a number of different candidate proteins that exceeds the number of affinity agents used. For example, the number of candidate proteins identified can be at least 5x, 10x, 25x, 50x, 100x or more than the number of affinity agents used. This can be achieved, for example, by (1) using promiscuous affinity agents that bind to multiple different candidate proteins suspected of being present in a given sample, and (2) subjecting the protein sample to a set of promiscuous affinity agents that, taken as a whole, are expected to bind each candidate protein in a different combination, such that each candidate protein is expected to be encoded by a unique profile of binding and non-binding events. Promiscuity of an affinity agent is a characteristic that can be understood relative to a given population of proteins. Promiscuity can arise due to the affinity agent recognizing an epitope that is known to be present in a plurality of different candidate proteins suspected of being present in the given population of unknown proteins. For example, epitopes having relatively short amino acid lengths such as dimers, trimers, or tetramers can be expected to occur in a substantial number of different proteins in the human proteome. Alternatively or additionally, a promiscuous affinity agent can recognize different epitopes (*e.g.* epitopes differing from each other with regard to amino acid composition or sequence), the different epitopes being present in a plurality of different candidate proteins. For example, a promiscuous

affinity agent that is designed or selected for its affinity toward a first trimer epitope may bind to a second epitope that has a different sequence of amino acids when compared to the first epitope.

Although performing a single binding reaction between a promiscuous affinity agent and a complex protein sample may yield ambiguous results regarding the identity of the different proteins to which it binds, the ambiguity can be resolved when the results are combined with other identifying information about those proteins. The identifying information can include characteristics of the protein such as length (*i.e.* number of amino acids), hydrophobicity, molecular weight, charge to mass ratio, isoelectric point, chromatographic fractionation behavior, enzymatic activity, presence or absence of post translational modifications or the like. The identifying information can include results of binding with other promiscuous affinity agents. For example, a plurality of different promiscuous affinity agents can be contacted with a complex population of proteins, wherein the plurality is configured to produce a different binding profile for each candidate protein suspected of being present in the population. In this example, each of the affinity agents can be distinguishable from the other affinity agents, for example, due to unique labeling (*e.g.* different affinity agents having different luminophore labels), unique spatial location (*e.g.* different affinity agents being located at different addresses in an array), and/or unique time of use (*e.g.* different affinity agents being delivered in series to a population of proteins). Accordingly, the plurality of promiscuous affinity agents produces a binding profile for each individual protein that can be decoded to identify a unique combination of epitopes present in the individual protein, and this can in turn be used to identify the individual protein as a particular candidate protein having the same or similar unique combination of epitopes. The binding profile can include observed binding events as well as observed non-binding events and this information can be evaluated in view of the expectation that particular candidate proteins produce a similar binding profile, for example, based on presence and absence of particular epitopes in the candidate proteins.

In some configurations, distinct and reproducible binding profiles may be observed for one or more unknown proteins in a sample. However, in many cases one or more binding events produces inconclusive or even aberrant results and this, in turn, can yield ambiguous binding profiles. For example, observation of binding outcome for a single-molecule binding event can be particularly prone to ambiguities due to stochasticity in the behavior of single molecules when a observed using certain detection hardware. The present disclosure provides methods that provide accurate protein identification despite ambiguities and imperfections that can arise in many contexts. In some configurations, methods for identifying, quantitating or otherwise characterizing one or more proteins in a sample utilize a binding model that evaluates the likelihood or probability that one or more candidate proteins that are suspected of being present in the sample will have produced an empirically observed binding profile. The binding model can include information regarding expected binding outcomes (*e.g.* binding or non-binding) for binding of one or more affinity reagent with one or more candidate proteins. The information can include an *a priori* characteristic of a candidate protein, such as presence or absence of a particular epitope in the candidate protein or length of the candidate protein. Alternatively or additionally, the information can include empirically determined characteristics such as propensity or likelihood that the candidate protein will bind to a particular affinity reagent. Accordingly, a binding model can include information regarding the propensity or likelihood of a given candidate protein generating a false positive or false negative binding result in the presence of a particular affinity reagent, and such information can optionally be included for a plurality of affinity reagents.

Methods set forth herein can be used to evaluate the degree of compatibility of one or more empirical binding profiles with results computed for various candidate proteins using a binding model. For example, to identify an unknown protein in a sample of many proteins, an empirical binding profile for the protein can be compared to results computed by the binding model for many or all candidate proteins suspected of being in the sample. In some configurations of the methods set forth herein, identity for the unknown protein is determined based on a likelihood of the unknown protein being a particular candidate protein given the empirical binding pattern or based on the probability of a particular candidate protein generating the empirical binding pattern. Optionally a score can be determined from the measurements that are acquired for the unknown protein with respect to many or all candidate proteins suspected of being in the sample. A digital or binary score that indicates one of two discrete states can be determined. In particular configurations, the score can be non-digital or non-binary. For example, the score can be a value selected from a continuum of values such that an identity is made based on the score being above or below a threshold value. Moreover, a score can be a single value or a collection of values. Particularly useful methods for identifying proteins using promiscuous reagents, serial binding measurements and/or decoding with binding models are set forth, for example, in US Pat. No. 10,473,654 US Pat. App. Pub. No. 2020/0318101 A1 or Egertson et al., BioRxiv (2021), DOI: 10.1101/2021.10.11.463967.

The present disclosure provides compositions, apparatus and methods for detecting one or more proteins. A protein can be detected using one or more affinity agents having binding affinity for the protein. The affinity agent and the protein can bind each other to form a complex and, during or after formation, the complex can be detected. The complex can be detected directly, for example, due to a label that is present on the affinity agent or protein. In some configurations, the complex need not be directly detected, for example, in formats where the complex is formed and then the affinity agent, protein, or a label component that was present in the complex is detected.

Many protein detection methods, such as enzyme linked immunosorbent assay (ELISA), achieve high-confidence characterization of one or more protein in a sample by exploiting high specificity binding of antibodies, aptamers or

other binding agents to the protein(s) and detecting the binding event while ignoring all other proteins in the sample. ELISA is generally carried out at low plex scale (*e.g.* from one to a hundred different proteins detected in parallel or in succession) but can be used at higher plexity. ELISA methods can be carried out by detecting immobilized binding agents and/or proteins in multiwell plates, on arrays, or on particles in microfluidic devices. Exemplary plate-based methods include, for example, the MULTI-ARRAY technology commercialized by MesoScale Diagnostics (Rockville, Maryland) or Simple Plex technology commercialized by Protein Simple (San Jose, CA). Exemplary, array-based methods include, but are not limited to those utilizing Simoa® Planar Array Technology or Simoa® Bead Technology, commercialized by Quanterix (Billerica, MA). Further exemplary array-based methods are set forth in US Pat. Nos. 9,678,068; 9,395,359; 8,415,171; 8,236,574; or 8,222,047.

Exemplary microfluidic detection methods include those commercialized by Luminex (Austin, Texas) under the trade name xMAP® technology or used on platforms identified as MAGPIX®, LUMINEX® 100/200 or FEXMAP 3D®.

Other detection methods that can also be used, for example at low plex scale, include procedures that employ SOMAmer reagents and SOMAscan assays commercialized by Soma Logic (Boulder, CO). In one configuration, a sample is contacted with aptamers that are capable of binding proteins with specificity for the amino acid sequence of the proteins. The resulting aptamer-protein complexes can be separated from other sample components, for example, by attaching the complexes to beads (or other solid support) that are removed from other sample components. The aptamers can then be isolated and, because the aptamers are nucleic acids, the aptamers can be detected using any of a variety of methods known in the art for detecting nucleic acids, including for example, hybridization to nucleic acid arrays, PCR-based detection, or nucleic acid sequencing. Exemplary methods and compositions are set forth in US Patent Nos. 7,855,054; 7,964,356; 8,404,830; 8,945,830; 8,975,026; 8,975,388; 9,163,056; 9,938,314; 9,404,919; 9,926,566; 10,221,421; 10,239,908; 10,316,321 10,221,207 or 10,392,621.

In some detection assays, a protein can be cyclically modified and the modified products from individual cycles can be detected. In some configurations, a protein can be sequenced by a sequential process in which each cycle includes steps of detecting the protein and removing one or more terminal amino acids from the protein. Optionally, one or more of the steps can include adding a label to the protein, for example, at the amino terminal amino acid or at the carboxy terminal amino acid. In particular configurations, a method of detecting a protein can include steps of (i) exposing a terminal amino acid on the protein; (ii) detecting a change in signal from the protein; and (iii) identifying the type of amino acid that was removed based on the change detected in step (ii). The terminal amino acid can be exposed, for example, by removal of one or more amino acids from the amino terminus or carboxyl terminus of the protein. Steps (i) through (iii) can be repeated to produce a series of signal changes that is indicative of the sequence for the protein.

In a first configuration of a cyclical protein detection method, one or more types of amino acids in the protein can be attached to a label that uniquely identifies the type of amino acid. In this configuration, the change in signal that identifies the amino acid can be loss of signal from the respective label. For example, lysines can be attached to a distinguishable label such that loss of the label indicates removal of a lysine. Alternatively or additionally, other amino acid types can be attached to other labels that are mutually distinguishable from lysine and from each other. For example, lysines can be attached to a first label and cysteines can be attached to a second label, the first and second labels being distinguishable from each other. Exemplary compositions and techniques that can be used to remove amino acids from a protein and detect signal changes are those set forth in Swaminathan et al., Nature Biotech. 36:1076-1082 (2018); or US Pat. Nos. 9,625,469 or 10,545,153.

Methods and apparatus under development by Erisyon, Inc. (Austin, TX) may also be useful for detecting proteins.

In a second configuration of a cyclical protein detection method, a terminal amino acid of a protein can be recognized by an affinity agent that is specific for the terminal amino acid or specific for a label moiety that is present on the terminal amino acid. The affinity agent can be detected on the array, for example, due to a label on the affinity agent. Optionally, the label is a nucleic acid barcode sequence that is added to a primer nucleic acid upon formation of a complex. For example, a barcode can be added to the primer via ligation of an oligonucleotide having the barcode sequence or polymerase extension directed by a template that encodes the barcode sequence. The formation of the complex and identity of the terminal amino acid can be determined by decoding the barcode sequence. Multiple cycles can produce a series of barcodes that can be detected, for example, using a nucleic acid sequencing technique. Exemplary affinity agents and detection methods are set forth in US Pat. App. Pub. No. 2019/0145982 A1; 2020/0348308 A1; or 2020/0348307 A1 . Methods and apparatus under development by Encodia, Inc. (San Diego, CA) may also be useful for detecting proteins.

Cyclical removal of terminal amino acids from a protein can be carried out using an Edman-type sequencing reaction in which a phenyl isothiocyanate reacts with a N-terminal amino group under mildly alkaline conditions (e.g. about pH 8) to form a cyclical phenylthiocarbamoyl Edman complex derivative. The phenyl isothiocyanate may be substituted or unsubstituted with one or more functional groups, linker groups, or linker groups containing functional groups. An Edman-type sequencing reaction can include variations to reagents and conditions that yield a detectable removal of amino acids from a protein terminus, thereby facilitating determination of the amino acid sequence for a protein or portion thereof. For example, the phenyl group can be replaced with at least one aromatic, heteroaromatic or aliphatic group which may

participate in an Edman-type sequencing reaction, non-limiting examples including: pyridine, pyrimidine, pyrazine, pyridazoline, fused aromatic groups such as naphthalene and quinoline), methyl or other alkyl groups or alkyl group derivatives (*e.g.*, alkenyl, alkynyl, cyclo-alkyl). Under certain conditions, for example, acidic conditions of about pH 2, derivatized terminal amino acids may be cleaved, for example, as a thiazolinone derivative. The thiazolinone amino acid derivative under acidic conditions may form a more stable phenylthiohydantoin (PTH) or similar amino acid derivative which can be detected. This procedure can be repeated iteratively for residual protein to identify the subsequent N-terminal amino acid. Many variations of Edman-type degradation have been described and may be used including, for example, a one-step removal of an N-terminal amino acid using alkaline conditions (Chang, J.Y., FEBS LETTS., 1978, 91(1), 63-68). In some cases, Edman-type reactions may be thwarted by N-terminal modifications which may be selectively removed, for example, N-terminal acetylation or formylation (e.g., see Gheorghe M.T., Bergman T. (1995) in Methods in Protein Structure Analysis, Chapter 8: Deacetylation and internal cleavage of Proteins for N-terminal Sequence Analysis. Springer, Boston, MA. https://doi.org/ 10.1007/978-1-4899-1031-8_8).

Non-limiting examples of functional groups for substituted phenyl isothiocyanate may include ligands (*e.g.* biotin and biotin analogs) for known receptors, labels such as luminophores, or reactive groups such as click functionalities (*e.g.* compositions having an azide or acetylene moiety). The functional group may be a DNA, RNA, peptide or small molecule barcode or other tag which may be further processed and/or detected.

The removal of an amino terminal amino acid using Edman-type processes can utilize at least two main steps, the first step includes reacting an isothiocyanate or equivalent with protein N-terminal residues to form a relatively stable Edman complex, for example, a phenylthiocarbamoyl complex. The second step can include removing the derivatized N-terminal amino acid, for example, via heating. The protein, now having been shortened by one amino acid, may be detected, for example, by contacting the protein with a labeled affinity agent that is complementary to the amino terminus and examining the protein for binding to the agent, or by detecting loss of a label that was attached to the removed amino acid.

Edman-type processes can be carried out in a multiplex format to detect, characterize or identify a plurality of proteins. A method of detecting a protein can include steps of (i) exposing a terminal amino acid on a protein at an address of an array; (ii) binding an affinity agent to the terminal amino acid, where the affinity agent includes a nucleic acid tag, and where a primer nucleic acid is present at the address; (iii) extending the primer nucleic acid, thereby producing an extended primer having a copy of the tag; and (iv) detecting the tag of the extended primer. The terminal amino acid can be exposed, for example, by removal of one or more amino acids from the amino terminus or carboxyl terminus of the protein. Steps (i) through (iv) can be repeated to produce a series of tags that is indicative of the sequence for the protein. The method can be applied to a plurality of proteins on the array and in parallel. Whatever the plexity, the extending of the primer can be carried out, for example, by polymerase-based extension of the primer, using the nucleic acid tag as a template. Alternatively, the extending of the primer can be carried out, for example, by ligase- or chemical-based ligation of the primer to a nucleic acid that is hybridized to the nucleic acid tag. The nucleic acid tag can be detected via hybridization to nucleic acid probes (*e.g.* in an array), amplification-based detections (*e.g.* PCR-based detection, or rolling circle amplification-based detection) or nuclei acid sequencing (*e.g.* cyclical reversible terminator methods, nanopore methods, or single molecule, real time detection methods). Exemplary methods that can be used for detecting proteins using nucleic acid tags are set forth in US Pat. App. Pub. No. 2019/0145982 A1; 2020/0348308 A1; or 2020/0348307 A1.

A protein can optionally be detected based on its enzymatic or biological activity. For example, a protein can be contacted with a reactant that is converted to a detectable product by an enzymatic activity of the protein. In other assay formats, a first protein having a known enzymatic function can be contacted with a second protein to determine if the second protein changes the enzymatic function of the first protein. As such, the first protein serves as a reporter system for detection of the second protein. Exemplary changes that can be observed include, but are not limited to, activation of the enzymatic function, inhibition of the enzymatic function, attenuation of the enzymatic function, degradation of the first protein or competition for a reactant or cofactor used by the first protein. Proteins can also be detected based on their binding interactions with other molecules such as proteins, nucleic acids, nucleotides, metabolites, hormones, vitamins, small molecules that participate in biological signal transduction pathways, biological receptors or the like. For example, a protein that participates in a signal transduction pathway can be identified as a particular candidate protein by detecting binding to a second protein that is known to be a binding partner for the candidate protein in the pathway.

The presence or absence of post-translational modifications (PTM) can be detected using a composition, apparatus or method set forth herein. A PTM can be detected using an affinity agent that recognizes the PTM or based on a chemical property of the PTM. Exemplary PTMs that can be detected, identified or characterized include, but are not limited to, myristoylation, palmitoylation, isoprenylation, prenylation, farnesylation, geranylgeranylation, lipoylation, flavin moiety attachment, Heme C attachment, phosphopantetheinylation, retinylidene Schiff base formation, dipthamide formation, ethanolamine phosphoglycerol attachment, hypusine, beta-Lysine addition, acylation, acetylation, deacetylation, formylation, alkylation, methylation, C-terminal amidation, arginylation, polyglutamylation, polyglyclyation, butyrylation, gamma-carboxylation, glycosylation, glycation, polysialylation, malonylation, hydroxylation, iodination, nucleotide addition, phosphoate ester formation, phosphoramidate formation, phosphorylation, adenylylation, uridylylation, propionylation, pyrolglutamate formation, S-glutathionylation, S-nitrosylation, S-sulfenylation, S-sulfinylation, S-sulfonylation, succinylation, sulfation, glycation, carbamylation, carbonylation, isopeptide bond formation, biotinylation, carbamylation, oxidation, reduction, pegylation, ISGylation, SUMOylation, ubiquitination, neddylation, pupylation, citrullination, deamidation, elminylation, disulfide bridge formation, proteolytic cleavage, isoaspartate formation, racemization, and protein splicing.

PTMs may occur at particular amino acid residues of a protein. For example, the phosphate moiety of a particular proteoform can be present on a serine, threonine, tyrosine, histidine, cysteine, lysine, aspartate or glutamate residue of the protein. In other examples, an acetyl moiety can be present on the N-terminus or on a lysine; a serine or threonine residue can have an O-linked glycosyl moiety; an asparagine residue can have an N-linked glycosyl moiety; a proline, lysine, asparagine, aspartate or histidine amino acid can be hydroxylated; an arginine or lysine residue can be methylated; or the N-terminal methionine or at a lysine amino acid can be ubiquitinated.

In some configurations of the apparatus and methods set forth herein, one or more proteins can be detected on a solid support. For example, protein(s) can be attached to a support, the support can be contacted with detection agents (*e.g.* affinity agents) in solution, the agents can interact with the protein(s), thereby producing a detectable signal, and then the signal can be detected to determine the presence of the protein(s). In multiplexed versions of this approach, different proteins can be attached to different addresses in an array, and the probing and detection steps can occur in parallel. In another example, affinity agents can be attached to a solid support, the support can be contacted with proteins in solution, the proteins can interact with the affinity agents, thereby producing a detectable signal, and then the signal can be detected to determine presence, quantity or characteristics of the proteins. This approach can also be multiplexed by attaching different affinity agents to different addresses of an array.

Proteins, affinity agents or other objects of interest can be attached to a solid support via covalent or non-covalent bonds. For example, a linker can be used to covalently attach a protein or other object of interest to an array. A particularly useful linker is a structured nucleic acid particle such as a nucleic acid nanoball (*e.g.* a concatemeric amplicon produced by rolling circle replication of a circular nucleic acid template) or a nucleic acid origami. For example, a plurality of proteins can be conjugated to a plurality of structured nucleic acid particles, such that each protein-conjugated particle forms an address in the array. Exemplary linkers for attaching proteins, or other objects of interest, to an array or other solid support are set forth in US Pat. App. Pub. No. 2021/0101930 A1.

A protein can be detected based on proximity of two or more affinity agents. For example, the two affinity agents can include two components each: a receptor component and a nucleic acid component. When the affinity agents bind in proximity to each other, for example, due to ligands for the respective receptors being on a single protein, or due to the ligands being present on two proteins that associate with each other, the nucleic acids can interact to cause a modification that is indicative of the two ligands being in proximity. Optionally, the modification can be polymerase catalyzed extension of one of the nucleic acids using the other nucleic acid as a template. As another option, one of the nucleic acids can form a template that acts as splint to position other nucleic acids for ligation to an oligonucleotide. Exemplary methods are commercialized by Olink Proteomics AB (Uppsala Sweden) or set forth in US Pat. Nos. 7,306,904; 7,351,528; 8,013,134; 8,268,554 or 9,777,315.

A method or apparatus of the present disclosure can optionally be configured for optical detection (e.g. luminescence detection). Analytes or other entities can be detected, and optionally distinguished from each other, based on measurable characteristics such as the wavelength of radiation that excites a luminophore, the wavelength of radiation emitted by a luminophore, the intensity of radiation emitted by a luminophore (*e.g.* at particular detection wavelength(s)), luminescence lifetime (*e.g.* the time that a luminophore remains in an excited state) or luminescence polarity. Other optical characteristics that can be detected, and optionally used to distinguish analytes, include, for example, absorbance of radiation, resonance Raman, radiation scattering, or the like. A luminophore can be an intrinsic moiety of a protein or other analyte to be detected, or the luminophore can be an exogenous moiety that has been synthetically added to a protein or other analyte.

A method or apparatus of the present disclosure can use a light sensing device that is appropriate for detecting a characteristic set forth herein or known in the art. Particularly useful components of a light sensing device can include, but are not limited to, optical subsystems or components used in nucleic acid sequencing systems. Examples of useful sub systems and components thereof are set forth in US Pat. App. Pub. No. 2010/0111768 A1 or U.S. Pat. Nos. 7,329,860; 8,951,781 or 9,193,996.

Other useful light sensing devices and components thereof are described in U.S. Pat. Nos. 5,888,737; 6,175,002; 5,695,934; 6,140,489; or 5,863,722; or US Pat. Pub. Nos. 2007/007991 A1, 2009/0247414 A1, or 2010/0111768; or WO2007/123744 ·

Light sensing devices and components that can be used to detect luminophores based on luminescence lifetime are described, for example, in US Pat. Nos. 9,678,012; 9,921,157; 10,605,730; 10,712,274; 10,775,305; or 10,895,534.

Luminescence lifetime can be detected using an integrated circuit having a photodetection region configured to receive incident photons and produce a plurality of charge carriers in response to the incident photons. The integrated circuit can include at least one charge carrier storage region and a charge carrier segregation structure configured to

selectively direct charge carriers of the plurality of charge carriers directly into the charge carrier storage region based upon times at which the charge carriers are produced. See, for example, US Pat. Nos. 9,606,058, 10,775,305, and 10,845,308.

Optical sources that produce short optical pulses can be used for luminescence lifetime measurements. For example, a light source, such as a semiconductor laser or LED, can be driven with a bipolar waveform to generate optical pulses with FWHM durations as short as approximately 85 ps having suppressed tail emission. See, for example, in US 10,605,730.

For configurations that use optical detection (*e.g*. luminescent detection), one or more analytes (e.g. proteins) may be immobilized on a surface, and this surface may be scanned with a microscope to detect any signal from the immobilized analytes. The microscope itself may include a digital camera or other luminescence detector configured to record, store, and analyze the data collected during the scan. A luminescence detector of the present disclosure can be configured for epiluminescent detection, total internal reflection (TIR) detection, waveguide assisted excitation, or the like.

A light sensing device may be based upon any suitable technology, and may be, for example, a charged coupled device (CCD) sensor that generates pixilated image data based upon photons impacting locations in the device. It will be understood that any of a variety of other light sensing devices may also be used including, but not limited to, a detector array configured for time delay integration (TDI) operation, a complementary metal oxide semiconductor (CMOS) detector, an avalanche photodiode (APD) detector, a Geiger-mode photon counter, a photomultiplier tube (PMT), charge injection device (CID) sensors, JOT image sensor (Quanta), or any other suitable detector. Light sensing devices can optionally be coupled with one or more excitation sources, for example, lasers, light emitting diodes (LEDs), arc lamps or other energy sources known in the art.

An optical detection system can be configured for single molecule detection. For example, waveguides or optical confinements can be used to deliver excitation radiation to locations of a solid support where analytes are located. Zero-mode waveguides can be particularly useful, examples of which are set forth in U.S. Pat. Nos. 7,181,122, 7,302,146, or 7,313,308. Analytes can be confined to surface features, for example, to facilitate single molecule resolution. For example, analytes can be distributed into wells having nanometer dimensions such as those set forth in US Pat. Nos. 7,122,482 or 8,765,359, or US Pat. App. Pub. No 2013/0116153 A1.

The wells can be configured for selective excitation, for example, as set forth in US Pat. No. 8,798,414 or 9,347,829.

Analytes can be distributed to nanometer-scale posts, such as high aspect ratio posts which can optionally be dielectric pillars that extend through a metallic layer to improve detection of an analyte attached to the pillar. See, for example, US Pat. Nos. 8,148,264, 9,410,887 or 9,987,609. Further examples of nanostructures that can be used to detect analytes are those that change state in response to the concentration of analytes such that the analytes can be quantitated as set forth in WO 2020/176793 A1.

An apparatus or method set forth herein need not be configured for optical detection. For example, an electronic detector can be used for detection of protons or charged labels (see, for example, US Pat. App. Pub. Nos. 2009/0026082 A1; 2009/0127589 A1; 2010/0137143 A1; or 2010/0282617 A1

). A field effect transistor (FET) can be used to detect analytes or other entities, for example, based on proximity of a field disrupting moiety to the FET. The field disrupting moiety can be due to an extrinsic label attached to an analyte or affinity agent, or the moiety can be intrinsic to the analyte or affinity agent being used. Surface plasmon resonance can be used to detect binding of analytes or affinity agents at or near a surface. Exemplary sensors and methods for attaching molecules to sensors are set forth in US Pat. App. Pub. Nos. 2017/0240962 Al; 2018/0051316 Al; 2018/0112265 Al; 2018/0155773 A1 or 2018/0305727 Al; or US Pat. Nos. 9,164,053; 9,829,456; 10,036,064.

In some configurations of the compositions, apparatus and methods set forth herein, one or more proteins can be present on a solid support, where the proteins can optionally be detected. For example, a protein can be attached to a solid support, the solid support can be contacted with a detection agent (*e.g*. affinity agent) in solution, the affinity agent can interact with the protein, thereby producing a detectable signal, and then the signal can be detected to determine the presence, absence, quantity, a characteristic or identity of the protein. In multiplexed versions of this approach, different proteins can be attached to different addresses in an array, and the detection steps can occur in parallel, such that proteins at each address are detected, quantified, characterized or identified. In another example, detection agents can be attached to a solid support, the support can be contacted with proteins in solution, the proteins can interact with the detection agents, thereby producing a detectable signal, and then the signal can be detected to determine the presence of the proteins. This approach can also be multiplexed by attaching different probes to different addresses of an array.

In multiplexed configurations, different proteins can be attached to different unique identifiers (*e.g*. addresses in an array), and the proteins can be manipulated and detected in parallel. For example, a fluid containing one or more different affinity agents can be delivered to an array such that the proteins of the array are in simultaneous contact with the affinity agent(s). Moreover, a plurality of addresses can be observed in parallel allowing for rapid detection of binding events. A plurality of different proteins can have a complexity of at least 5, 10, 100, 1 x $10^3$, 1 x $10^4$, 1 x $10^5$ or more different native-length protein primary sequences. Alternatively or additionally, a proteome, proteome subfraction or other protein

sample that is analyzed in a method set forth herein can have a complexity that is at most 1 x $10^5$, 1 x $10^4$, 1 x $10^3$, 100, 10, 5 or fewer different native-length protein primary sequences. The total number of proteins of a sample that is detected, characterized or identified can differ from the number of different primary sequences in the sample, for example, due to the presence of multiple copies of at least some protein species. Moreover, the total number of proteins of a sample that is detected, characterized or identified can differ from the number of candidate proteins suspected of being in the sample, for example, due to the presence of multiple copies of at least some protein species, absence of some proteins in a source for the sample, or loss of some proteins prior to analysis.

A protein can be attached to a unique identifier using any of a variety of means. The attachment can be covalent or non-covalent. Exemplary covalent attachments include chemical linkers such as those achieved using click chemistry or other linkages known in the art or described in US Pat. App. Ser. No. 17/062,405.
Non-covalent attachment can be mediated by receptor-ligand interactions (*e.g.* (strept)avidin-biotin, antibody-antigen, or complementary nucleic acid strands), for example, wherein the receptor is attached to the unique identifier and the ligand is attached to the protein or vice versa. In particular configurations, a protein is attached to a solid support (*e.g.* an address in an array) via a structured nucleic acid particle (SNAP). A protein can be attached to a SNAP and the SNAP can interact with a solid support, for example, by non-covalent interactions of the DNA with the support and/or via covalent linkage of the SNAP to the support. Nucleic acid origami or nucleic acid nanoballs are particularly useful. The use of SNAPs and other moieties to attach proteins to unique identifiers such as tags or addresses in an array are set forth in US Pat. App. Ser. Nos. 17/062,405 and 63/159,500.

The methods, compositions and apparatus of the present disclosure are particularly well suited for use with proteins. Although proteins are exemplified throughout the present disclosure, it will be understood that other analytes can be similarly used. Exemplary analytes include, but are not limited to, biomolecules, polysaccharides, nucleic acids, lipids, metabolites, hormones, vitamins, enzyme cofactors, therapeutic agents, candidate therapeutic agents or combinations thereof. An analyte can be a non-biological atom or molecule, such as a synthetic polymer, metal, metal oxide, ceramic, semiconductor, mineral, or a combination thereof.

One or more proteins that are used in a method, composition or apparatus herein, can be derived from a natural or synthetic source. Exemplary sources include, but are not limited to biological tissues, fluids, cells or subcellular compartments (*e.g*. organelles). For example, a sample can be derived from a tissue biopsy, biological fluid (*e.g.* blood, sweat, tears, plasma, extracellular fluid, urine, mucus, saliva, semen, vaginal fluid, synovial fluid, lymph, cerebrospinal fluid, peritoneal fluid, pleural fluid, amniotic fluid, intracellular fluid, extracellular fluid, etc.), fecal sample, hair sample, cultured cell, culture media, fixed tissue sample (*e.g*. fresh frozen or formalin-fixed paraffin-embedded) or product of a protein synthesis reaction. A protein source may include any sample where a protein is a native or expected constituent. For example, a primary source for a cancer biomarker protein may be a tumor biopsy sample or bodily fluid. Other sources include environmental samples or forensic samples.

Exemplary organisms from which proteins or other analytes can be derived include, for example, a mammal such as a rodent, mouse, rat, rabbit, guinea pig, ungulate, horse, sheep, pig, goat, cow, cat, dog, primate, non-human primate or human; a plant such as *Arabidopsis thaliana,* tobacco, corn, sorghum, oat, wheat, rice, canola, or soybean; an algae such as *Chlamydomonas reinhardtii*; a nematode such as *Caenorhabditis elegans*; an insect such as *Drosophila melanogaster,* mosquito, fruit fly, honey bee or spider; a fish such as zebrafish; a reptile; an amphibian such as a frog or *Xenopus laevis*; *a dictyostelium discoideum*; a fungi such as *Pneumocystis carinii, Takifugu rubripes,* yeast, *Saccharamoyces cerevisiae* or *Schizosaccharomyces pombe;* or a *Plasmodium falciparum.* Proteins can also be derived from a prokaryote such as a bacterium, *Escherichia coli, staphylococci* or *Mycoplasma pneumoniae*; an archae; a virus such as Hepatitis C virus, influenza virus, coronavirus, or human immunodeficiency virus; or a viroid. Proteins can be derived from a homogeneous culture or population of the above organisms or alternatively from a collection of several different organisms, for example, in a community or ecosystem.

In some cases, a protein or other biomolecule can be derived from an organism that is collected from a host organism. For example, a protein may be derived from a parasitic, pathogenic, symbiotic, or latent organism collected from a host organism. A protein can be derived from an organism, tissue, cell or biological fluid that is known or suspected of being linked with a disease state or disorder (*e.g.*, cancer). Alternatively, a protein can be derived from an organism, tissue, cell or biological fluid that is known or suspected of not being linked to a particular disease state or disorder. For example, the proteins isolated from such a source can be used as a control for comparison to results acquired from a source that is known or suspected of being linked to the particular disease state or disorder. A sample may include a microbiome or substantial portion of a microbiome. In some cases, one or more proteins used in a method, composition or apparatus set forth herein may be obtained from a single source and no more than the single source. The single source can be, for example, a single organism (*e.g*. an individual human), single tissue, single cell, single organelle (e.g. endoplasmic reticulum, Golgi apparatus or nucleus), or single protein-containing particle (*e.g.,* a viral particle or vesicle).

A method, composition or apparatus of the present disclosure can use or include a plurality of proteins having any of a variety of compositions such as a plurality of proteins composed of a proteome or fraction thereof. For example, a plurality of proteins can include solution-phase proteins, such as proteins in a biological sample or fraction thereof, or a

plurality of proteins can include proteins that are immobilized, such as proteins attached to a particle or solid support. By way of further example, a plurality of proteins can include proteins that are detected, analyzed or identified in connection with a method, composition or apparatus of the present disclosure. The content of a plurality of proteins can be understood according to any of a variety of characteristics such as those set forth below or elsewhere herein.

A plurality of proteins can be characterized in terms of total protein mass. The total mass of protein in a liter of plasma has been estimated to be 70 g and the total mass of protein in a human cell has been estimated to be between 100 pg and 500 pg depending upon cells type. See Wisniewski et al. Molecular & Cellular Proteomics13:10.1074/mcp.M113.037309, 3497-3506 (2014). A plurality of proteins used or included in a method, composition or apparatus set forth herein can include at least 1 pg, 10 pg, 100 pg, 1 ng, 10 ng, 100 ng, 1 μg, 10 μg, 100 μg, 1 mg, 10 mg, 100 mg or more protein by mass. Alternatively or additionally, a plurality of proteins may contain at most 100 mg, 10 mg, 1 mg, 100 μg, 10 μg, 1 μg, 100 ng, 10 ng, 1 ng, 100 pg, 10 pg, 1 pg or less protein by mass.

A plurality of proteins can be characterized in terms of percent mass relative to a given source such as a biological source (*e.g.* cell, tissue, or biological fluid such as blood). For example, a plurality of proteins may contain at least 60%, 75%, 90%, 95%, 99%, 99.9% or more of the total protein mass present in the source from which the plurality of proteins was derived. Alternatively or additionally, a plurality of proteins may contain at most 99.9%, 99%, 95%, 90%, 75%, 60% or less of the total protein mass present in the source from which the plurality of proteins was derived.

A plurality of proteins can be characterized in terms of total number of protein molecules. The total number of protein molecules in a *Saccharomyces cerevisiae* cell has been estimated to be about 42 million protein molecules. See Ho et al., Cell Systems (2018), DOI: 10.1016/j.cels.2017.12.004. A plurality of proteins used or included in a method, composition or apparatus set forth herein can include at least 1 protein molecule, 10 protein molecules, 100 protein molecules, 1 x $10^4$ protein molecules, 1 x $10^6$ protein molecules, 1 x $10^8$ protein molecules, 1 x $10^{10}$ protein molecules, 1 mole ($6.02214076 \times 10^{23}$ molecules) of protein, 10 moles of protein molecules, 100 moles of protein molecules or more. Alternatively or additionally, a plurality of proteins may contain at most 100 moles of protein molecules, 10 moles of protein molecules, 1 mole of protein molecules, 1 x $10^{10}$ protein molecules, 1 x $10^8$ protein molecules, 1 x $10^6$ protein molecules, 1 x $10^4$ protein molecules, 100 protein molecules, 10 protein molecules, 1 protein molecule or less.

A plurality of proteins can be characterized in terms of the variety of full-length primary protein structures in the plurality. For example, the variety of full-length primary protein structures in a plurality of proteins can be equated with the number of different protein-encoding genes in the source for the plurality of proteins. Whether or not the proteins are derived from a known genome or from any genome at all, the variety of full-length primary protein structures can be counted independent of presence or absence of post translational modifications in the proteins. A human proteome is estimated to have about 20,000 different protein-encoding genes such that a plurality of proteins derived from a human can include up to about 20,000 different primary protein structures. See Aebersold et al., Nat. Chem. Biol. 14:206-214 (2018).

Other genomes and proteomes in nature are known to be larger or smaller. A plurality of proteins used or included in a method, composition or apparatus set forth herein can have a complexity of at least 2, 5, 10, 100, 1 x $10^3$, 1 x $10^4$, 2 x $10^4$, 3 x $10^4$ or more different full-length primary protein structures. Alternatively or additionally, a plurality of proteins can have a complexity that is at most 3 x $10^4$, 2 x $10^4$, 1 x $10^4$, 1 x $10^3$, 100, 10, 5, 2 or fewer different full-length primary protein structures.

In relative terms, a plurality of proteins used or included in a method, composition or apparatus set forth herein may contain at least one representative for at least 60%, 75%, 90%, 95%, 99%, 99.9% or more of the proteins encoded by the genome of a source from which the sample was derived. Alternatively or additionally, a plurality of proteins may contain a representative for at most 99.9%, 99%, 95%, 90%, 75%, 60% or less of the proteins encoded by the genome of a source from which the sample was derived.

A plurality of proteins can be characterized in terms of the variety of primary protein structures in the plurality including transcribed splice variants. The human proteome has been estimated to include about 70,000 different primary protein structures when splice variants ae included. See Aebersold et al., Nat. Chem. Biol. 14:206-214 (2018).

Moreover, the number of the partial-length primary protein structures can increase due to fragmentation that occurs in a sample. A plurality of proteins used or included in a method, composition or apparatus set forth herein can have a complexity of at least 2, 5, 10, 100, 1 x $10^3$, 1 x $10^4$, 7 x $10^4$, 1 x $10^5$, 1 x $10^6$ or more different primary protein structures. Alternatively or additionally, a plurality of proteins can have a complexity that is at most 1 x $10^6$, 1 x $10^5$, 7 x $10^4$, 1 x $10^4$, 1 x $10^3$, 100, 10, 5, 2 or fewer different primary protein structures.

A plurality of proteins can be characterized in terms of the variety of protein structures in the plurality including different primary structures and different proteoforms among the primary structures. Different molecular forms of proteins expressed from a given gene are considered to be different proteoforms. Protoeforms can differ, for example, due to differences in primary structure (*e.g*. shorter or longer amino acid sequences), different arrangement of domains (*e.g.* transcriptional splice variants), or different post translational modifications (*e.g.* presence or absence of phosphoryl, glycosyl, acetyl, or ubiquitin moieties). The human proteome is estimated to include hundreds of thousands of proteins when counting the different primary structures and proteoforms. See Aebersold et al., Nat. Chem. Biol. 14:206-214 (2018). A plurality of proteins used or included in a method, composition or apparatus set forth herein can have a complexity of at

least 2, 5, 10, 100, 1 x $10^3$, 1 x $10^4$, 1 x $10^5$, 1 x $10^6$, 5 x $10^6$, 1 x $10^7$ or more different protein structures. Alternatively or additionally, a plurality of proteins can have a complexity that is at most 1 x $10^7$, 5 x $10^6$, 1 x $10^6$, 1 x $10^5$, 1 x $10^4$, 1 x $10^3$, 100, 10, 5, 2 or fewer different protein structures.

A plurality of proteins can be characterized in terms of the dynamic range for the different protein structures in the sample. The dynamic range can be a measure of the range of abundance for all different protein structures in a plurality of proteins, the range of abundance for all different primary protein structures in a plurality of proteins, the range of abundance for all different full-length primary protein structures in a plurality of proteins, the range of abundance for all different full-length gene products in a plurality of proteins, the range of abundance for all different proteoforms expressed from a given gene, or the range of abundance for any other set of different proteins set forth herein. The dynamic range for all proteins in human plasma is estimated to span more than 10 orders of magnitude from albumin, the most abundant protein, to the rarest proteins that have been measured clinically. See Anderson and Anderson Mol Cell Proteomics 1:845-67 (2002). The dynamic range for plurality of proteins set forth herein can be a factor of at least 10, 100, 1 x $10^3$, 1 x $10^4$, 1 x $10^6$, 1 x $10^8$, 1 x $10^{10}$, or more. Alternatively or additionally, the dynamic range for plurality of proteins set forth herein can be a factor of at most 1 x $10^{10}$, 1 x $10^8$, 1 x $10^6$, 1 x $10^4$, 1 x $10^3$, 100, 10 or less.

A method set forth herein can be carried out in a fluid phase or on a solid phase. For fluid phase configurations, a fluid containing one or more proteins can be mixed with another fluid containing one or more affinity agents. For solid phase configurations one or more proteins or affinity agents can be attached to a solid support. One or more components that will participate in a binding event can be contained in a fluid and the fluid can be delivered to a solid support, the solid support being attached to one or more other component that will participate in the binding event.

A method of the present disclosure can be carried out at single analyte resolution. Alternatively to single-analyte resolution, a method can be carried out at ensemble-resolution or bulk-resolution. Bulk-resolution configurations acquire a composite signal from a plurality of different analytes or affinity agents in a vessel or on a surface. For example, a composite signal can be acquired from a population of different protein-affinity agent complexes in a well or cuvette, or on a solid support surface, such that individual complexes are not resolved from each other. Ensemble-resolution configurations acquire a composite signal from a first collection of proteins or affinity agents in a sample, such that the composite signal is distinguishable from signals generated by a second collection of proteins or affinity agents in the sample. For example, the ensembles can be located at different addresses in an array. Accordingly, the composite signal obtained from each address will be an average of signals from the ensemble, yet signals from different addresses can be distinguished from each other.

A composition, apparatus or method set forth herein can be configured to contact one or more proteins (*e.g.* an array of different proteins) with a plurality of different affinity agents. For example, a plurality of affinity agents (whether configured separately or as a pool) may include at least 2, 5, 10, 25, 50, 100, 250, 500 or more types of affinity agents, each type of affinity agent differing from the other types with respect to the epitope(s) recognized. Alternatively or additionally, a plurality of affinity agents may include at most 500, 250, 100, 50, 25, 10, 5, or 2 types of affinity agents, each type of affinity agent differing from the other types with respect to the epitope(s) recognized. Different types of affinity agents in a pool can be uniquely labeled such that the different types can be distinguished from each other. In some configurations, at least two, and up to all, of the different types of affinity agents in a pool may be indistinguishably labeled with respect to each other. Alternatively or additionally to the use of unique labels, different types of affinity agents can be delivered and detected serially when evaluating one or more proteins (*e.g.* in an array).

A method of the present disclosure can be performed in a multiplex format. In multiplexed configurations, different proteins can be attached to different unique identifiers (*e.g.* the proteins can be attached to different addresses in an array). Multiplexed proteins can be manipulated and detected in parallel. For example, a fluid containing one or more different affinity agents can be delivered to a protein array such that the proteins of the array are in simultaneous contact with the affinity agent(s). Moreover, a plurality of addresses can be observed in parallel allowing for rapid detection of binding events. A plurality of different proteins can have a complexity of at least 5, 10, 100, 1 x $10^3$, 1 x $10^4$, 2 x $10^4$, 3 x $10^4$ or more different native-length protein primary sequences. Alternatively or additionally, a proteome or proteome subfraction that is analyzed in a method set forth herein can have a complexity that is at most 3 x $10^4$, 2 x $10^4$, 1 x $10^4$, 1 x $10^3$, 100, 10, 5 or fewer different native-length protein primary sequences. The plurality of proteins can constitute a proteome or subfraction of a proteome. The total number of proteins that is detected, characterized or identified can differ from the number of different primary sequences in the sample from which the proteins are derived, for example, due to the presence of multiple copies of at least some protein species. Moreover, the total number of proteins that are detected, characterized or identified can differ from the number of candidate proteins suspected of being present, for example, due to the presence of multiple copies of at least some protein species, absence of some proteins in a source for the proteins, or loss of some proteins prior to analysis.

A particularly useful multiplex format uses an array of proteins and/or affinity agents. A polypeptide, anchoring group, polypeptide composite or other analyte can be attached to a unique identifier, such as an address in an array, using any of a variety of means. The attachment can be covalent or non-covalent. Exemplary covalent attachments include chemical linkers such as those achieved using click chemistry or other linkages known in the art or described in US Pat.

App. Pub. No. 2021/0101930 A1.

Non-covalent attachment can be mediated by receptor-ligand interactions (*e.g.* (strept)avidin-biotin, antibody-antigen, or complementary nucleic acid strands), for example, in which the receptor is attached to the unique identifier and the ligand is attached to the protein or *vice versa.* In particular configurations, a protein is attached to a solid support (*e.g.* an address in an array) via a structured nucleic acid particle (SNAP). A protein can be attached to a SNAP and the SNAP can interact with a solid support, for example, by non-covalent interactions of the DNA with the support and/or via covalent linkage of the SNAP to the support. Nucleic acid origami or nucleic acid nanoballs are particularly useful. The use of SNAPs and other moieties to attach proteins to unique identifiers such as tags or addresses in an array are set forth in US Pat. App. Pub. No. 2021/0101930 A1.

A solid support or a surface thereof may be configured to display an analyte or a plurality of analytes. A solid support may contain one or more patterned, formed, or prepared surfaces that contain at least one address for displaying an analyte. In some cases, a solid support may contain one or more patterned, formed, or prepared surfaces that contain a plurality of addresses, with each address configured to display one or more analytes. Accordingly, an array as set forth herein may comprise a plurality of analytes coupled to a solid support or a surface thereof. In some configurations, a solid support or a surface thereof may be patterned or formed to produce an ordered or patterned array of addresses. The deposition of analytes on the ordered or patterned array of addresses may be controlled by interactions between the solid support and the analytes such as, for example, electrostatic interactions, magnetic interactions, hydrophobic interactions, hydrophilic interactions, covalent interactions, or non-covalent interactions. Accordingly, the coupling of an analyte at each address of an array may produce an ordered or patterned array of analytes whose average spacing between analytes is determined based upon the tolerance of the ordering or patterning of the solid support and the size of an analyte-binding region for each address. An ordered or patterned array of analytes may be characterized as having a regular geometry, such as a rectangular, triangular, polygonal, or annular grid. In other configurations, a solid support or a surface thereof may be non-patterned or non-ordered. The deposition of analytes on the non-ordered or non-patterned array of addresses may be controlled by interactions between the solid support and the analytes, or inter-analyte interactions such as, for example, steric repulsion, electrostatic repulsion, electrostatic attraction, magnetic repulsion, magnetic attraction, covalent interactions, or non-covalent interactions.

A solid support or a surface thereof may contain one or more structures or features. A structure or feature may comprise an elevation, profile, shape, geometry, or configuration that deviates from an average elevation, profile, shape, geometry, or configuration of a solid support or surface thereof. A structure or feature may be a raised structure or feature, such as a ridge, post, pillar, or pad, if the structure or feature extends above the average elevation of a surface of a solid support. A structure or feature may be a depressed structure, such as a channel, well, pore, or hole, if the structure or feature extends below the average elevation of a surface of a solid support. A structure or feature may be an intrinsic structure or feature of a substrate (*i.e.,* arising due to the physical or chemical properties of the substrate, or a physical or chemical mechanism of formation), such as surface roughness structures, crystal structures, or porosity. A structure or feature may be formed by a method of processing a solid support. In some configurations, a solid support or a surface may be processed by a lithographic method to form one or more structures or features. A solid support or a surface thereof may be formed by a suitable lithographic method, including, but not limited to photolithography, Dip-Pen nanolithography, nanoimprint lithography, nanosphere lithography, nanoball lithography, nanopillar arrays, nanowire lithography, immersion lithography, neutral particle lithography, plasmonic lithography, scanning probe lithography, thermochemical lithography, thermal scanning probe lithography, local oxidation nanolithography, molecular self-assembly, stencil lithography, laser interference lithography, soft lithography, magnetolithography, stereolithography, deep ultraviolet lithography, x-ray lithography, ion projection lithography, proton-beam lithography, or electron-beam lithography.

A solid support or surface may comprise a plurality of structures or features. A plurality of structures or features may comprise an ordered or patterned array of structures or features. A plurality of structures or features may comprise an non-ordered, non-patterned, or random array of structures or features. A structure or feature may have an average characteristic dimension (*e.g.*, length, width, height, diameter, circumference, etc.) of at least about 1 nanometer (nm), 5 nm, 10 nm, 20 nm, 30 nm, 40 nm, 50 nm, 75 nm, 100 nm, 150 nm, 200 nm, 250 nm, 300 nm, 400 nm, 500 nm, 750 nm, 1000 nm, or more than 1000 nm. Alternatively or additionally, a structure or feature may have an average characteristic dimension of no more than about 1000 nm, 750 nm, 500 nm, 400 nm, 300 nm, 250 nm, 200 nm, 150 nm, 100 nm, 75 nm, 50 nm, 40 nm, 30 nm, 20 nm, 10 nm, 5 nm, 1 nm, or less than 1 nm. An array of structures or features may have an average pitch, in which the pitch is measured as the average separation between respective centerpoints of neighboring structures or features. An array may have an average pitch of at least about 1 nm, 5 nm, 10 nm, 20 nm, 30 nm, 40 nm, 50 nm, 75 nm, 100 nm, 150 nm, 200 nm, 250 nm, 300 nm, 400 nm, 500 nm, 750 nm, 1 micron ($\mu$m), 2 $\mu$m , 5 $\mu$m , 10 $\mu$m , 50 $\mu$m , 100 $\mu$m, or more than 100 $\mu$m. Alternatively or additionally, an array may have an average pitch of no more than about 100 $\mu$m, 50 $\mu$m, 10 $\mu$m, 5 $\mu$m, 1 $\mu$m, 750 nm, 500 nm, 400 nm, 300 nm, 250 nm, 200 nm, 150 nm, 100 nm, 75 nm, 50 nm, 40 nm, 30 nm, 20 nm, 10 nm, 5 nm, 1 nm, or less than 1 nm.

A solid support or a surface thereof may include a base substrate material and, optionally, one or more additional materials that are contacted or adhered with the substrate material. A solid support may comprise one or more additional

materials that are deposited, coated, or inlayed onto the substrate material. Additional materials may be added to the substrate material to alter the properties of the substrate material. For example, materials may be added to alter the surface chemistry (*e.g.*, hydrophobicity, hydrophilicity, non-specific binding, electrostatic properties), alter the optical properties (*e.g.*, reflective properties, refractive properties), alter the electrical or magnetic properties (*e.g.*, dielectric materials, conducting materials, electrically-insulating materials), or alter the heat transfer characteristics of the substrate material. Additional materials contacted or adhered with a substrate material may be ordered or patterned onto the substrate material to, for example, locate the additional material at addresses or locate the additional material at interstitial regions between addresses. Exemplary additional materials may include metals (*e.g.,* gold, silver, copper, etc.), metal oxides (*e.g.,* titanium oxide, silicon dioxide, alumina, iron oxides, etc.), metal nitrides (*e.g.*, silicon nitride, aluminum nitride, boron nitride, gallium nitride, etc.), metal carbides (*e.g.*, tungsten carbide, titanium carbide, iron carbide, etc.), metal sulfides (*e.g.*, iron sulfide, silver sulfide, etc.), and organic moieties (*e.g.*, polyethylene glycol (PEG), dextrans, chemically-reactive functional groups, etc.).

A method of the present disclosure can include the step of coupling one or more analytes to a solid support or a surface thereof prior to performing a detection step set forth herein. The coupling of one or more analytes to a solid support surface may include covalent or non-covalent coupling of the one or more analytes to the solid support. Covalent coupling of an analyte to a solid support can include direct covalent coupling of an analyte to a solid support (*e.g.*, formation of coordination bonds) or indirect covalent coupling between a reactive functional group of the analyte and a reactive functional group that is coupled to the solid support (*e.g.,* a CLICK-type reaction). Non-covalent coupling can include the formation of any non-covalent interaction between an analyte and a solid support, including electrostatic or magnetic interactions, or non-covalent bonding interactions (*e.g.*, ionic bonds, van der Waals interactions, hydrogen bonding, etc.). The skilled person will readily recognize that the particular analyte and the choice of solid support can affect the selection of a coupling chemistry for the compositions and methods set forth herein.

Accordingly, a coupling chemistry may be selected based upon the criterium that it provides a sufficiently stable coupling of an analyte to a solid support for a time scale that meets or exceeds the time scale of a method as set forth herein. For example, a polypeptide identification method can require a coupling of the analyte to the solid support for a sufficient amount of time to permit a series of empirical measurements of the analyte to occur. An analyte may be continuously coupled to a solid support for an observable length of time such as, for example, at least about 1 minute, 1 hour (hr), 3 hrs, 6 hrs, 12 hrs, 1 day, 1.5 days, 2 days, 3 days, 1 week (wk), 2 wks, 3 wks, 1 month, or more. The coupling of an analyte to a solid support can occur with a solution-phase chemistry that promotes the deposition of the analyte on the solid support. Coupling of an analyte to a solid support may occur under solution conditions that are optimized for any conceivable solution property, including solution composition, species concentrations, pH, ionic strength, solution temperature, etc. Solution composition can be varied by chemical species, such as buffer type, salts, acids, bases, and surfactants. In some configurations, species such as salts and surfactants may be selected to facilitate the formation of interactions between an analyte and a solid support. Covalent coupling methods for coupling an analyte to a solid support may include species such as catalyst, initiators, and promoters to facilitate particular reactive chemistries.

Coupling of an analyte to a solid support may be facilitated by a mediating group. A mediating group may modify the properties of the analyte to facilitate the coupling. Useful mediating groups have been set forth herein (e.g., structured nucleic acid particles). In some configurations, a mediating group can be coupled to an analyte prior to coupling the analyte to a solid support. Accordingly, the mediating group may be chosen to increase the strength, control, or specificity of the coupling of the analyte to the solid support. In other configurations, a mediating group can be coupled to a solid support prior to coupling an analyte to the solid support. Accordingly, the mediating group may be chosen to provide a more favorable coupling chemistry than can be provided by the solid support alone.

**Sample Polypeptide Compositions**

Numerous compositions of the above-described sample polypeptide preparation will be readily apparent to the skilled person. The sample polypeptide preparation compositions may include compositions of sample polypeptides and/or internal standard polypeptides at intermediate or final stages of sample polypeptide fraction preparation or sample polypeptide array preparation.

In some configurations, a sample polypeptide composition may comprise: 1) an anchoring group containing a surface electrical charge coupled or conjugated to a sample polypeptide; 2) an aqueous solution comprising a metal salt; and 3) a surfactant.

In some configurations, a sample polypeptide composition may comprise: 1) a charged anchoring group coupled or conjugated to a sample polypeptide; 2) an aqueous solution comprising a metal salt; 3 3 a solid support comprising a plurality of charged binding sites; and 4) a surfactant. The charged anchoring group may be configured to bind to the charged binding site.

In some configurations, a sample polypeptide composition may comprise: 1) an anchoring group comprising a first reactive functional group, 2) a solid support comprising a surface; 3) a metal salt; 4) a sample polypeptide comprising a

second reactive functional group; and 5) a surfactant. The anchoring group may be conjugated to the solid support surface by a conjugated interaction between the first reactive functional group and the second reactive functional group.

One or more fluid media may be utilized during a sample preparation method. A sample preparation process of a sample preparation method may utilize one or more fluid media Exemplary sample preparation processes that may utilize a fluid medium may include sample collection, sample storage, sample polypeptide separation, sample polypeptide functionalization, sample polypeptide coupling, sample polypeptide binding, rinsing processes, detectable label measurement, and internal standard addition. As such, a fluid medium utilized during a sample preparation method may be referred to by the sample preparation process in which it is utilized, for example a sample storage medium, a sample polypeptide separation medium, a polypeptide composite coupling medium, an internal standard medium, etc,

A fluid medium for a sample preparation method or process may comprise any of a variety of components, such as a solvent species, pH buffering species, a cationic species, an anionic species, a surfactant species, a denaturing species, or a combination thereof. A solvent species may include water, acetic acid, methanol, ethanol, n-propanol, isopropyl alcohol, n-butanol, formic acid, ammonia, propylene carbonate, nitromethane, dimethyl sulfoxide, acetonitrile, dimethylformamide, acetone, ethyl acetate, tetrahydrofuran, dichloromethane, chloroform, carbon tetrachloride, dimethyl ether, diethyl ether, 1-4, dioxane, toluene, benzene, cyclohexane, hexane, cyclopentane, pentane, or combinations thereof. A fluid medium may include a buffering species including, but not limited to, MES, Tris, Bis-tris, Bis-tris propane, ADA, ACES, PIPES, MOPSO, MOPS, BES, TES, HEPES, HEPBS, HEPPSO, DIPSO, MOBS, TAPSO, TAPS, TABS, POPSO, TEA, EPPS, Tricine, Gly-Gly, Bicine, AMPD, AMPSO, AMP, CHES, CAPSO, CAPS, and CABS. A fluid medium may include cationic species such as $Na^+$, $K^+$, $Ag^+$, $Cu^+$, $NH_4^+$, $Mg^{2+}$, $Ca^{2+}$, $Cu^{2+}$, $Cd^{2+}$, $Zn^{2+}$, $Fe^{2+}$, $Co^{2+}$, $Ni^{2+}$, $Cr^{2+}$, $Mn^{2+}$, $Ge^{2+}$, $Sn^{2+}$, $Al^{3+}$, $Cr^{3+}$, $Fe^{3+}$, $Co^{3+}$, $Ni^{3+}$, $Ti^{3+}$, $Mn^{3+}$, $Si^{4+}$, $V^{4+}$, $Ti^{4+}$, $Mn^{4+}$, $Ge^{4+}$, $Se^{4+}$, $V^{5+}$, $Mn^{5+}$, $Mn^{6+}$, $Se^{6+}$, and combinations thereof. A fluid medium may include anionic species such as $F^-$, $Cl^-$, $Br^-$, $ClO_3^-$, $H_2PO_4^-$, $HCO_3^-$, $HSO_4^-$, $OH^-$, $I^-$, $NO_3^-$, $NO_2^-$, $MnO_4^-$, $SCN^-$, $CO_3^{2-}$, $CrO_4^{2-}$, $Cr_2O_7^{2-}$, $HPO_4^{2-}$, $SO_4^{2-}$, $SO_3^{2-}$, $PO_4^{3-}$, and combinations thereof. A fluid medium may include a surfactant species, such as a cationic surfactant, an anionic surfactant, a zwitterionic surfactant, or an amphoteric surfactant. A fluid medium may include a surfactant species including, but not limited to, stearic acid, lauric acid, oleic acid, sodium dodecyl sulfate, sodium dodecyl benzene sulfonate, dodecylamine hydrochloride, hexadecyltrimethylammonium bromide, polyethylene oxide, nonylphenyl ethoxylates, Triton X, pentapropylene glycol monododecyl ether, octapropylene glycol monododecyl ether, pentaethylene glycol monododecyl ether, octaethylene glycol monododecyl ether, lauramide monoethylamine, lauramide diethylamine, octyl glucoside, decyl glucoside, lauryl glucoside, Tween 20, Tween 80, n-dodecyl-β-D-maltoside, nonoxynol 9, glycerol monolaurate, polyethoxylated tallow amine, poloxamer, digitonin, zonyl FSO, 2,5-dimethyl-3-hexyne-2,5-diol, Igepal CA630, Aerosol-OT, triethylamine hydrochloride, cetrimonium bromide, benzethonium chloride, octenidine dihydrochloride, cetylpyridinium chloride, adogen, dimethyldioctadecylammonium chloride, CHAPS, CHAPSO, cocamidopropyl betaine, amidosulfobetaine-16, lauryl-N,N-(dimethylammonio)butyrate, lauryl-N,N-(dimethyl)-glycinebetaine, hexadecyl phosphocholine, lauryldimethylamine N-oxide, lauryl-N,N-(dimethyl)-propanesulfonate, 3-(1-pyridinio)-1-propanesulfonate, 3-(4-tert-butyl-1-pyridinio)-1-propanesulfonate, N-laurylsarcosine, and combinations thereof.

A fluid medium may be formulated with any combination of a solvent species, a pH buffering species, a cationic species, an anionic species, or a surfactant species. The components of a fluid medium may be formulated in amounts to optimize the deposition of anchoring groups or polypeptide composites to a solid support. A fluid medium may be formulated to be a homogeneous liquid medium. A fluid medium may be formulated to be a single-phase liquid medium. A fluid medium may be formulated to be a multi-phase liquid medium, such as an oil-in-water emulsion or a water-in-oil emulsion. For a fluid medium formulated as an emulsion, anchoring groups or polypeptide composites may be solvated or suspended within the dissolved phase.

A buffering species may be formulated in a fluid medium in any quantity. A buffering species may be present in a fluid medium at a concentration of at least about 0.0001M, 0.001M, 0.01M, 0.02M, 0.03M, 0.04M, 0.05M, 0.06M, 0.07M, 0.08M, 0.09M, 0.1M, 0.2M, 0.3M, 0.4M, 0.5M, 0.6M, 0.7M, 0.8M, 0.9M, 1M, 1.1M, 1.2M, 1.3M, 1.4M, 1.5M, 1.6M, 1.7M, 1.8M, 1.9M, 2M, 2.1M, 2.2M, 2.3M, 2.4M, 2.5M, 2.6M, 2.7M, 2.8M, 2.9M, 3M, 3.1M, 3.2M, 3.3M, 3.4M, 3.5M, 3.6M, 3.7M, 3.8M, 3.9M, 4M, 4.1M, 4.2M, 4.3M, 4.4M, 4.5M, 4.6M, 4.7M, 4.8M, 4.9M, 5M, 5.1M, 5.2M, 5.3M, 5.4M, 5.5M, 5.6M, 5.7M, 5.8M, 5.9M, 6M, 7M, 8M, 9M or more than 10M. Alternatively or additionally, a buffering species may be present in a fluid medium at a concentration of no more than about 10 M, 9M, 8M, 7M, 6M, 5.9M, 5.8M, 5.7M, 5.6M, 5.5M, 5.4M, 5.3M, 5.2M, 5.1M, 5.0M, 4.9M, 4.8M, 4.7M, 4.6M, 4.5M, 4.4M, 4.3M, 4.2M, 4.1M, 4.0M, 3.9M, 3.8M, 3.7M, 3.6M, 3.5M, 3.4M, 3.3M, 3.2M, 3.1M, 3.0M, 2.9M, 2.8M, 2.7M, 2.6M, 2.5M, 2.4M, 2.3M, 2.2M, 2.1M, 2.0M, 1.9M, 1.8M, 1.7M, 1.6M, 1.5M, 1.4M, 1.3M, 1.2M, 1.1M, 1.0M, 0.9M, 0.8M, 0.7M, 0.6M, 0.5M, 0.4M, 0.3M, 0.2M, 0.1M, 0.09M, 0.08M, 0.07M, 0.06M, 0.05M, 0.04M, 0.03M, 0.02M, 0.01M, 0.001M, 0.001M, or less than about 0.001M.

A buffering species may be present in a fluid medium in a weight percentage of at least about 0.0001 weight percent (wt%), 0.001 wt%, 0.002 wt%, 0.003 wt%, 0.004 wt%, 0.005 wt%, 0.006 wt%, 0.007 wt%, 0.008 wt%, 0.009 wt%, 0.01 wt%, 0.02 wt%, 0.03 wt%, 0.04 wt%, 0.05 wt%, 0.06 wt%, 0.07 wt%, 0.08 wt%, 0.09 wt%, 0.1 wt%, 0.2 wt%, 0.3 wt%, 0.4 wt%, 0.5 wt%, 0.6 wt%, 0.7 wt%, 0.8 wt%, 0.9 wt%, 1.0 wt%, 1.1 wt%, 1.2 wt%, 1.3 wt%, 1.4 wt%, 1.5 wt%, 1.6 wt%, 1.7 wt%, 1.8 wt%, 1.9 wt%, 2 wt%, 2.1 wt%, 2.2 wt%, 2.3 wt%, 2.4 wt%, 2.5 wt%, 2.6 wt%, 2.7 wt%, 2.8 wt%, 2.9 wt%, 3 wt%, 3.1

wt%, 3.2 wt%, 3.3 wt%, 3.4 wt%, 3.5 wt%, 3.6 wt%, 3.7 wt%, 3.8 wt%, 3.9 wt%, 4 wt%, 4.1 wt%, 4.2 wt%, 4.3 wt%, 4.4 wt%, 4.5 wt%, 4.6 wt%, 4.7 wt%, 4.8 wt%, 4.9 wt%, 5 wt%, 6 wt%, 7 wt%, 8 wt%, 9wt%, 10 wt%, or more than 10 wt%. Alternatively or additionally, a buffering species may be present in a fluid medium in a weight percentage of no more than about 10 wt%, 9 wt%, 8 wt%, 7 wt%, 6 wt%, 5 wt%, 4.9 wt%, 4.8 wt%, 4.7 wt%, 4.6 wt%, 4.5 wt%, 4.4 wt%, 4.3 wt%, 4.2 wt%, 4.1 wt%, 4.0 wt%, 3.9 wt%, 3.8 wt%, 3.7 wt%, 3.6 wt%, 3.5 wt%, 3.4 wt%, 3.3 wt%, 3.2 wt%, 3.1 wt%, 3.0 wt%, 2.9 wt%, 2.8 wt%, 2.7 wt%, 2.6 wt%, 2.5 wt%, 2.4 wt%, 2.3 wt%, 2.2 wt%, 2.1 wt%, 2.0 wt%, 1.9 wt%, 1.8 wt%, 1.7 wt%, 1.6 wt%, 1.5 wt%, 1.4 wt%, 1.3 wt%, 1.2 wt%, 1.1 wt%, 1.0 wt%, 0.9 wt%, 0.8 wt%, 0.7 wt%, 0.6 wt%, 0.5 wt%, 0.4 wt%, 0.3 wt%, 0.2 wt%, 0.1 wt%, 0.09 wt%, 0.08 wt%, 0.07 wt%, 0.06 wt%, 0.05 wt%, 0.04 wt%, 0.03 wt%, 0.02 wt%, 0.01 wt%, 0.009 wt%, 0.008 wt%, 0.007 wt%, 0.006 wt%, 0.005 wt%, 0.004 wt%, 0.003 wt%, 0.002 wt%, 0.001 wt%, 0.0001 wt%, or less than 0.0001 wt%.

A cationic species may be formulated in a fluid medium in any quantity. A cationic species may be present in a fluid medium at a concentration of at least about 0.0001M, 0.001M, 0.01M, 0.02M, 0.03M, 0.04M, 0.05M, 0.06M, 0.07M, 0.08M, 0.09M, 0.1M, 0.2M, 0.3M, 0.4M, 0.5M, 0.6M, 0.7M, 0.8M, 0.9M, 1M, 1.1M, 1.2M, 1.3M, 1.4M, 1.5M, 1.6M, 1.7M, 1.8M, 1.9M, 2M, 2.1M, 2.2M, 2.3M, 2.4M, 2.5M, 2.6M, 2.7M, 2.8M, 2.9M, 3M, 3.1M, 3.2M, 3.3M, 3.4M, 3.5M, 3.6M, 3.7M, 3.8M, 3.9M, 4M, 4.1M, 4.2M, 4.3M, 4.4M, 4.5M, 4.6M, 4.7M, 4.8M, 4.9M, 5M, 5.1M, 5.2M, 5.3M, 5.4M, 5.5M, 5.6M, 5.7M, 5.8M, 5.9M, 6M, 7M, 8M, 9M or more than 10M. Alternatively or additionally, a cationic species may be present in a fluid medium at a concentration of no more than about 10 M, 9M, 8M, 7M, 6M, 5.9M, 5.8M, 5.7M, 5.6M, 5.5M, 5.4M, 5.3M, 5.2M, 5.1M, 5.0M, 4.9M, 4.8M, 4.7M, 4.6M, 4.5M, 4.4M, 4.3M, 4.2M, 4.1M, 4.0M, 3.9M, 3.8M, 3.7M, 3.6M, 3.5M, 3.4M, 3.3M, 3.2M, 3.1M, 3.0M, 2.9M, 2.8M, 2.7M, 2.6M, 2.5M, 2.4M, 2.3M, 2.2M, 2.1M, 2.0M, 1.9M, 1.8M, 1.7M, 1.6M, 1.5M, 1.4M, 1.3M, 1.2M, 1.1M, 1.0M, 0.9M, 0.8M, 0.7M, 0.6M, 0.5M, 0.4M, 0.3M, 0.2M, 0.1M, 0.09M, 0.08M, 0.07M, 0.06M, 0.05M, 0.04M, 0.03M, 0.02M, 0.01M, 0.001M, 0.001M, or less than about 0.001M.

A cationic species may be present in a fluid medium in a weight percentage of at least about 0.0001 weight percent (wt%), 0.001 wt%, 0.002 wt%, 0.003 wt%, 0.004 wt%, 0.005 wt%, 0.006 wt%, 0.007 wt%, 0.008 wt%, 0.009 wt%, 0.01 wt%, 0.02 wt%, 0.03 wt%, 0.04 wt%, 0.05 wt%, 0.06 wt%, 0.07 wt%, 0.08 wt%, 0.09 wt%, 0.1 wt%, 0.2 wt%, 0.3 wt%, 0.4 wt%, 0.5 wt%, 0.6 wt%, 0.7 wt%, 0.8 wt%, 0.9 wt%, 1.0 wt%, 1.1 wt%, 1.2 wt%, 1.3 wt%, 1.4 wt%, 1.5 wt%, 1.6 wt%, 1.7 wt%, 1.8 wt%, 1.9 wt%, 2 wt%, 2.1 wt%, 2.2 wt%, 2.3 wt%, 2.4 wt%, 2.5 wt%, 2.6 wt%, 2.7 wt%, 2.8 wt%, 2.9 wt%, 3 wt%, 3.1 wt%, 3.2 wt%, 3.3 wt%, 3.4 wt%, 3.5 wt%, 3.6 wt%, 3.7 wt%, 3.8 wt%, 3.9 wt%, 4 wt%, 4.1 wt%, 4.2 wt%, 4.3 wt%, 4.4 wt%, 4.5 wt%, 4.6 wt%, 4.7 wt%, 4.8 wt%, 4.9 wt%, 5 wt%, 6 wt%, 7 wt%, 8 wt%, 9wt%, 10 wt%, or more than 10 wt%. Alternatively or additionally, a cationic species may be present in a fluid medium in a weight percentage of no more than about 10 wt%, 9 wt%, 8 wt%, 7 wt%, 6 wt%, 5 wt%, 4.9 wt%, 4.8 wt%, 4.7 wt%, 4.6 wt%, 4.5 wt%, 4.4 wt%, 4.3 wt%, 4.2 wt%, 4.1 wt%, 4.0 wt%, 3.9 wt%, 3.8 wt%, 3.7 wt%, 3.6 wt%, 3.5 wt%, 3.4 wt%, 3.3 wt%, 3.2 wt%, 3.1 wt%, 3.0 wt%, 2.9 wt%, 2.8 wt%, 2.7 wt%, 2.6 wt%, 2.5 wt%, 2.4 wt%, 2.3 wt%, 2.2 wt%, 2.1 wt%, 2.0 wt%, 1.9 wt%, 1.8 wt%, 1.7 wt%, 1.6 wt%, 1.5 wt%, 1.4 wt%, 1.3 wt%, 1.2 wt%, 1.1 wt%, 1.0 wt%, 0.9 wt%, 0.8 wt%, 0.7 wt%, 0.6 wt%, 0.5 wt%, 0.4 wt%, 0.3 wt%, 0.2 wt%, 0.1 wt%, 0.09 wt%, 0.08 wt%, 0.07 wt%, 0.06 wt%, 0.05 wt%, 0.04 wt%, 0.03 wt%, 0.02 wt%, 0.01 wt%, 0.009 wt%, 0.008 wt%, 0.007 wt%, 0.006 wt%, 0.005 wt%, 0.004 wt%, 0.003 wt%, 0.002 wt%, 0.001 wt%, 0.0001 wt%, or less than 0.0001 wt%.

An anionic species may be formulated in a fluid medium in any quantity. An anionic species may be present in a deposition solvent composition at a concentration of at least about 0.0001M, 0.001M, 0.01M, 0.02M, 0.03M, 0.04M, 0.05M, 0.06M, 0.07M, 0.08M, 0.09M, 0.1M, 0.2M, 0.3M, 0.4M, 0.5M, 0.6M, 0.7M, 0.8M, 0.9M, 1M, 1.1M, 1.2M, 1.3M, 1.4M, 1.5M, 1.6M, 1.7M, 1.8M, 1.9M, 2M, 2.1M, 2.2M, 2.3M, 2.4M, 2.5M, 2.6M, 2.7M, 2.8M, 2.9M, 3M, 3.1M, 3.2M, 3.3M, 3.4M, 3.5M, 3.6M, 3.7M, 3.8M, 3.9M, 4M, 4.1M, 4.2M, 4.3M, 4.4M, 4.5M, 4.6M, 4.7M, 4.8M, 4.9M, 5M, 5.1M, 5.2M, 5.3M, 5.4M, 5.5M, 5.6M, 5.7M, 5.8M, 5.9M, 6M, 7M, 8M, 9M or more than 10M. Alternatively or additionally, an anionic species may be present in a fluid medium at a concentration of no more than about 10 M, 9M, 8M, 7M, 6M, 5.9M, 5.8M, 5.7M, 5.6M, 5.5M, 5.4M, 5.3M, 5.2M, 5.1M, 5.0M, 4.9M, 4.8M, 4.7M, 4.6M, 4.5M, 4.4M, 4.3M, 4.2M, 4.1M, 4.0M, 3.9M, 3.8M, 3.7M, 3.6M, 3.5M, 3.4M, 3.3M, 3.2M, 3.1M, 3.0M, 2.9M, 2.8M, 2.7M, 2.6M, 2.5M, 2.4M, 2.3M, 2.2M, 2.1M, 2.0M, 1.9M, 1.8M, 1.7M, 1.6M, 1.5M, 1.4M, 1.3M, 1.2M, 1.1M, 1.0M, 0.9M, 0.8M, 0.7M, 0.6M, 0.5M, 0.4M, 0.3M, 0.2M, 0.1M, 0.09M, 0.08M, 0.07M, 0.06M, 0.05M, 0.04M, 0.03M, 0.02M, 0.01M, 0.001M, 0.001M, or less than about 0.001M.

An anionic species may be present in a fluid medium in a weight percentage of at least about 0.0001 weight percent (wt%), 0.001 wt%, 0.002 wt%, 0.003 wt%, 0.004 wt%, 0.005 wt%, 0.006 wt%, 0.007 wt%, 0.008 wt%, 0.009 wt%, 0.01 wt%, 0.02 wt%, 0.03 wt%, 0.04 wt%, 0.05 wt%, 0.06 wt%, 0.07 wt%, 0.08 wt%, 0.09 wt%, 0.1 wt%, 0.2 wt%, 0.3 wt%, 0.4 wt%, 0.5 wt%, 0.6 wt%, 0.7 wt%, 0.8 wt%, 0.9 wt%, 1.0 wt%, 1.1 wt%, 1.2 wt%, 1.3 wt%, 1.4 wt%, 1.5 wt%, 1.6 wt%, 1.7 wt%, 1.8 wt%, 1.9 wt%, 2 wt%, 2.1 wt%, 2.2 wt%, 2.3 wt%, 2.4 wt%, 2.5 wt%, 2.6 wt%, 2.7 wt%, 2.8 wt%, 2.9 wt%, 3 wt%, 3.1 wt%, 3.2 wt%, 3.3 wt%, 3.4 wt%, 3.5 wt%, 3.6 wt%, 3.7 wt%, 3.8 wt%, 3.9 wt%, 4 wt%, 4.1 wt%, 4.2 wt%, 4.3 wt%, 4.4 wt%, 4.5 wt%, 4.6 wt%, 4.7 wt%, 4.8 wt%, 4.9 wt%, 5 wt%, 6 wt%, 7 wt%, 8 wt%, 9wt%, 10 wt%, or more than 10 wt%. Alternatively or additionally, an anionic species may be present in a fluid medium in a weight percentage of no more than about 10 wt%, 9 wt%, 8 wt%, 7 wt%, 6 wt%, 5 wt%, 4.9 wt%, 4.8 wt%, 4.7 wt%, 4.6 wt%, 4.5 wt%, 4.4 wt%, 4.3 wt%, 4.2 wt%, 4.1 wt%, 4.0 wt%, 3.9 wt%, 3.8 wt%, 3.7 wt%, 3.6 wt%, 3.5 wt%, 3.4 wt%, 3.3 wt%, 3.2 wt%, 3.1 wt%, 3.0 wt%, 2.9 wt%, 2.8 wt%, 2.7 wt%, 2.6 wt%, 2.5 wt%, 2.4 wt%, 2.3 wt%, 2.2 wt%, 2.1 wt%, 2.0 wt%, 1.9 wt%, 1.8 wt%, 1.7 wt%, 1.6 wt%, 1.5

wt%, 1.4 wt%, 1.3 wt%, 1.2 wt%, 1.1 wt%, 1.0 wt%, 0.9 wt%, 0.8 wt%, 0.7 wt%, 0.6 wt%, 0.5 wt%, 0.4 wt%, 0.3 wt%, 0.2 wt%, 0.1 wt%, 0.09 wt%, 0.08 wt%, 0.07 wt%, 0.06 wt%, 0.05 wt%, 0.04 wt%, 0.03 wt%, 0.02 wt%, 0.01 wt%, 0.009 wt%, 0.008 wt%, 0.007 wt%, 0.006 wt%, 0.005 wt%, 0.004 wt%, 0.003 wt%, 0.002 wt%, 0.001 wt%, 0.0001 wt%, or less than 0.0001 wt%.

A surfactant species may be formulated in a fluid medium in any quantity. A surfactant species may be present in a fluid medium at a concentration of at least about 0.0001M, 0.001M, 0.01M, 0.02M, 0.03M, 0.04M, 0.05M, 0.06M, 0.07M, 0.08M, 0.09M, 0.1M, 0.2M, 0.3M, 0.4M, 0.5M, 0.6M, 0.7M, 0.8M, 0.9M, 1M, 1.1M, 1.2M, 1.3M, 1.4M, 1.5M, 1.6M, 1.7M, 1.8M, 1.9M, 2M, 2.1M, 2.2M, 2.3M, 2.4M, 2.5M, 2.6M, 2.7M, 2.8M, 2.9M, 3M, 3.1M, 3.2M, 3.3M, 3.4M, 3.5M, 3.6M, 3.7M, 3.8M, 3.9M, 4M, 4.1M, 4.2M, 4.3M, 4.4M, 4.5M, 4.6M, 4.7M, 4.8M, 4.9M, 5M, 5.1M, 5.2M, 5.3M, 5.4M, 5.5M, 5.6M, 5.7M, 5.8M, 5.9M, 6M, 7M, 8M, 9M or more than 10M. Alternatively or additionally, a surfactant species may be present in a fluid medium at a concentration of no more than about 10 M, 9M, 8M, 7M, 6M, 5.9M, 5.8M, 5.7M, 5.6M, 5.5M, 5.4M, 5.3M, 5.2M, 5.1M, 5.0M, 4.9M, 4.8M, 4.7M, 4.6M, 4.5M, 4.4M, 4.3M, 4.2M, 4.1M, 4.0M, 3.9M, 3.8M, 3.7M, 3.6M, 3.5M, 3.4M, 3.3M, 3.2M, 3.1M, 3.0M, 2.9M, 2.8M, 2.7M, 2.6M, 2.5M, 2.4M, 2.3M, 2.2M, 2.1M, 2.0M, 1.9M, 1.8M, 1.7M, 1.6M, 1.5M, 1.4M, 1.3M, 1.2M, 1.1M, 1.0M, 0.9M, 0.8M, 0.7M, 0.6M, 0.5M, 0.4M, 0.3M, 0.2M, 0.1M, 0.09M, 0.08M, 0.07M, 0.06M, 0.05M, 0.04M, 0.03M, 0.02M, 0.01M, 0.001M, 0.001M, or less than about 0.001M.

A surfactant species may be present in a fluid medium in a weight percentage of at least about 0.0001 weight percent (wt%), 0.001 wt%, 0.002 wt%, 0.003 wt%, 0.004 wt%, 0.005 wt%, 0.006 wt%, 0.007 wt%, 0.008 wt%, 0.009 wt%, 0.01 wt%, 0.02 wt%, 0.03 wt%, 0.04 wt%, 0.05 wt%, 0.06 wt%, 0.07 wt%, 0.08 wt%, 0.09 wt%, 0.1 wt%, 0.2 wt%, 0.3 wt%, 0.4 wt%, 0.5 wt%, 0.6 wt%, 0.7 wt%, 0.8 wt%, 0.9 wt%, 1.0 wt%, 1.1 wt%, 1.2 wt%, 1.3 wt%, 1.4 wt%, 1.5 wt%, 1.6 wt%, 1.7 wt%, 1.8 wt%, 1.9 wt%, 2 wt%, 2.1 wt%, 2.2 wt%, 2.3 wt%, 2.4 wt%, 2.5 wt%, 2.6 wt%, 2.7 wt%, 2.8 wt%, 2.9 wt%, 3 wt%, 3.1 wt%, 3.2 wt%, 3.3 wt%, 3.4 wt%, 3.5 wt%, 3.6 wt%, 3.7 wt%, 3.8 wt%, 3.9 wt%, 4 wt%, 4.1 wt%, 4.2 wt%, 4.3 wt%, 4.4 wt%, 4.5 wt%, 4.6 wt%, 4.7 wt%, 4.8 wt%, 4.9 wt%, 5 wt%, 6 wt%, 7 wt%, 8 wt%, 9wt%, 10 wt%, or more than 10 wt%. Alternatively or additionally, a surfactant species may be present in a fluid medium in a weight percentage of no more than about 10 wt%, 9 wt%, 8 wt%, 7 wt%, 6 wt%, 5 wt%, 4.9 wt%, 4.8 wt%, 4.7 wt%, 4.6 wt%, 4.5 wt%, 4.4 wt%, 4.3 wt%, 4.2 wt%, 4.1 wt%, 4.0 wt%, 3.9 wt%, 3.8 wt%, 3.7 wt%, 3.6 wt%, 3.5 wt%, 3.4 wt%, 3.3 wt%, 3.2 wt%, 3.1 wt%, 3.0 wt%, 2.9 wt%, 2.8 wt%, 2.7 wt%, 2.6 wt%, 2.5 wt%, 2.4 wt%, 2.3 wt%, 2.2 wt%, 2.1 wt%, 2.0 wt%, 1.9 wt%, 1.8 wt%, 1.7 wt%, 1.6 wt%, 1.5 wt%, 1.4 wt%, 1.3 wt%, 1.2 wt%, 1.1 wt%, 1.0 wt%, 0.9 wt%, 0.8 wt%, 0.7 wt%, 0.6 wt%, 0.5 wt%, 0.4 wt%, 0.3 wt%, 0.2 wt%, 0.1 wt%, 0.09 wt%, 0.08 wt%, 0.07 wt%, 0.06 wt%, 0.05 wt%, 0.04 wt%, 0.03 wt%, 0.02 wt%, 0.01 wt%, 0.009 wt%, 0.008 wt%, 0.007 wt%, 0.006 wt%, 0.005 wt%, 0.004 wt%, 0.003 wt%, 0.002 wt%, 0.001 wt%, 0.0001 wt%, or less than 0.0001 wt%.

A denaturing species may be formulated in a fluid medium in any quantity. A denaturing species may be present in a fluid medium at a concentration of at least about 0.0001M, 0.001M, 0.01M, 0.02M, 0.03M, 0.04M, 0.05M, 0.06M, 0.07M, 0.08M, 0.09M, 0.1M, 0.2M, 0.3M, 0.4M, 0.5M, 0.6M, 0.7M, 0.8M, 0.9M, 1M, 1.1M, 1.2M, 1.3M, 1.4M, 1.5M, 1.6M, 1.7M, 1.8M, 1.9M, 2M, 2.1M, 2.2M, 2.3M, 2.4M, 2.5M, 2.6M, 2.7M, 2.8M, 2.9M, 3M, 3.1M, 3.2M, 3.3M, 3.4M, 3.5M, 3.6M, 3.7M, 3.8M, 3.9M, 4M, 4.1M, 4.2M, 4.3M, 4.4M, 4.5M, 4.6M, 4.7M, 4.8M, 4.9M, 5M, 5.1M, 5.2M, 5.3M, 5.4M, 5.5M, 5.6M, 5.7M, 5.8M, 5.9M, 6M, 7M, 8M, 9M or more than 10M. Alternatively or additionally, a denaturing species may be present in a fluid medium at a concentration of no more than about 10 M, 9M, 8M, 7M, 6M, 5.9M, 5.8M, 5.7M, 5.6M, 5.5M, 5.4M, 5.3M, 5.2M, 5.1M, 5.0M, 4.9M, 4.8M, 4.7M, 4.6M, 4.5M, 4.4M, 4.3M, 4.2M, 4.1M, 4.0M, 3.9M, 3.8M, 3.7M, 3.6M, 3.5M, 3.4M, 3.3M, 3.2M, 3.1M, 3.0M, 2.9M, 2.8M, 2.7M, 2.6M, 2.5M, 2.4M, 2.3M, 2.2M, 2.1M, 2.0M, 1.9M, 1.8M, 1.7M, 1.6M, 1.5M, 1.4M, 1.3M, 1.2M, 1.1M, 1.0M, 0.9M, 0.8M, 0.7M, 0.6M, 0.5M, 0.4M, 0.3M, 0.2M, 0.1M, 0.09M, 0.08M, 0.07M, 0.06M, 0.05M, 0.04M, 0.03M, 0.02M, 0.01M, 0.001M, 0.001M, or less than about 0.001M.

A denaturing species may be present in a fluid medium in a weight percentage of at least about 0.0001 weight percent (wt%), 0.001 wt%, 0.002 wt%, 0.003 wt%, 0.004 wt%, 0.005 wt%, 0.006 wt%, 0.007 wt%, 0.008 wt%, 0.009 wt%, 0.01 wt%, 0.02 wt%, 0.03 wt%, 0.04 wt%, 0.05 wt%, 0.06 wt%, 0.07 wt%, 0.08 wt%, 0.09 wt%, 0.1 wt%, 0.2 wt%, 0.3 wt%, 0.4 wt%, 0.5 wt%, 0.6 wt%, 0.7 wt%, 0.8 wt%, 0.9 wt%, 1.0 wt%, 1.1 wt%, 1.2 wt%, 1.3 wt%, 1.4 wt%, 1.5 wt%, 1.6 wt%, 1.7 wt%, 1.8 wt%, 1.9 wt%, 2 wt%, 2.1 wt%, 2.2 wt%, 2.3 wt%, 2.4 wt%, 2.5 wt%, 2.6 wt%, 2.7 wt%, 2.8 wt%, 2.9 wt%, 3 wt%, 3.1 wt%, 3.2 wt%, 3.3 wt%, 3.4 wt%, 3.5 wt%, 3.6 wt%, 3.7 wt%, 3.8 wt%, 3.9 wt%, 4 wt%, 4.1 wt%, 4.2 wt%, 4.3 wt%, 4.4 wt%, 4.5 wt%, 4.6 wt%, 4.7 wt%, 4.8 wt%, 4.9 wt%, 5 wt%, 6 wt%, 7 wt%, 8 wt%, 9wt%, 10 wt%, or more than 10 wt%. Alternatively or additionally, a denaturing species may be present in a fluid medium in a weight percentage of no more than about 10 wt%, 9 wt%, 8 wt%, 7 wt%, 6 wt%, 5 wt%, 4.9 wt%, 4.8 wt%, 4.7 wt%, 4.6 wt%, 4.5 wt%, 4.4 wt%, 4.3 wt%, 4.2 wt%, 4.1 wt%, 4.0 wt%, 3.9 wt%, 3.8 wt%, 3.7 wt%, 3.6 wt%, 3.5 wt%, 3.4 wt%, 3.3 wt%, 3.2 wt%, 3.1 wt%, 3.0 wt%, 2.9 wt%, 2.8 wt%, 2.7 wt%, 2.6 wt%, 2.5 wt%, 2.4 wt%, 2.3 wt%, 2.2 wt%, 2.1 wt%, 2.0 wt%, 1.9 wt%, 1.8 wt%, 1.7 wt%, 1.6 wt%, 1.5 wt%, 1.4 wt%, 1.3 wt%, 1.2 wt%, 1.1 wt%, 1.0 wt%, 0.9 wt%, 0.8 wt%, 0.7 wt%, 0.6 wt%, 0.5 wt%, 0.4 wt%, 0.3 wt%, 0.2 wt%, 0.1 wt%, 0.09 wt%, 0.08 wt%, 0.07 wt%, 0.06 wt%, 0.05 wt%, 0.04 wt%, 0.03 wt%, 0.02 wt%, 0.01 wt%, 0.009 wt%, 0.008 wt%, 0.007 wt%, 0.006 wt%, 0.005 wt%, 0.004 wt%, 0.003 wt%, 0.002 wt%, 0.001 wt%, 0.0001 wt%, or less than 0.0001 wt%.

A fluid medium may be formulated to have a pH at a value or within a range of values. A fluid medium may have a pH of about 0, 0.1, 0.2, 0.3, 0.4, 0.5, 0.6, 0.7, 0.8, 0.9, 1.0, 0.1, 0.2, 0.3, 0.4, 0.5, 0.6, 0.7, 0.8, 0.9, 1.0, 1.1, 1.2, 1.3, 1.4, 1.5,

1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, 12.0, 12.1, 12.2, 12.3, 12.4, 12.5, 12.6, 12.7, 12.8, 12.9, 13.0, 13.1, 13.2, 13.3, 13.4, 13.5, 13.6, 13.7, 13.8, 13.9, or about 14.0. A fluid medium may have a pH of at least about 0, 0.1, 0.2, 0.3, 0.4, 0.5, 0.6, 0.7, 0.8, 0.9, 1.0, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, 12.0, 12.1, 12.2, 12.3, 12.4, 12.5, 12.6, 12.7, 12.8, 12.9, 13.0, 13.1, 13.2, 13.3, 13.4, 13.5, 13.6, 13.7, 13.8, 13.9, 14.0 or more than about 14.0. Alternatively or additionally, a fluid medium may have a pH of no more than about 14.0, 13.9, 13.8, 13.7, 13.6, 13.5, 13.4, 13.3, 13.2, 13.1, 13.0, 12.9, 12.8, 12.7, 12.6, 12.5, 12.4, 12.3, 12.2, 12.1, 12.0, 11.9, 11.8, 11.7, 11.6, 11.5, 11.4, 11.3, 11.2, 11.1, 11.0, 10.9, 10.8, 10.7, 10.6, 10.5, 10.4, 10.3, 10.2, 10.1, 10.0, 9.9, 9.8, 9.7, 9.6, 9.5, 9.4, 9.3, 9.2, 9.1, 9.0, 8.9, 8.8, 8.7, 8.6, 8.5, 8.4, 8.3, 8.2, 8.1, 8.0, 7.9, 7.8, 7.7, 7.6, 7.5, 7.4, 7.3, 7.2, 7.1, 7.0, 6.9, 6.8, 6.7, 6.6, 6.5, 6.4, 6.3, 6.2, 6.1, 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.1, 1.0, 0.9, 0.8, 0.7, 0.6, 0.5, 0.4, 0.3, 0.2, 0.1, 0, or less than about 0. A fluid medium may have a pH in a range from about 0 to about 2, about 0 to about 4, about 0 to about 6, about 0 to about 8, about 0 to about 10, about 0 to about 12, about 0 to about 14, about 2 to about 4, about 2 to about 6, about 2 to about 8, about 2 to about 10, about 2 to about 12, about 2 to about 14, about 4 to about 6, about 4 to about 8, about 4 to about 10, about 4 to about 12, about 4 to about 14, about 6 to about 8, about 6 to about 10, about 6 to about 12, about 6 to about 14, about 8 to about 10, about 8 to about 12, about 8 to about 14, about 10 to about 12, about 10 to about 14, or about 12 to about 14.

## EXAMPLES

The presently claimed invention is as defined in the claims. Any of the following Examples that do not fall within the scope of the claims do not form part of the presently claimed invention and are provided for comparative purposes only.

### Example 1: Azide Functionalization of Thiols

A solution containing protein at a concentration of at least 100 μg/ml is provided. 50 μl of the protein solution is removed and set aside for later analysis as a control sample. The remaining protein solution is combined with dithiothreitol (DTT) to reduce any present disulfide linkages. DTT is combined with the solution until at least a final DTT concentration of 2 mM is achieved. The DTT/protein solution is reacted for at least 1 hour to ensure full reduction of all disulfide linkages. DTT may be substituted with any appropriate reducing agent, such as mercaptoethanol or tris (2-carboxyethyl)phosphine (TCEP).

The reduced protein solution is combined with an azide-containing compound to functionalize the protein. 100 mM Bromoacetomido-PEG3-azide (BrPA) is combined with the protein solution until at least a 2-fold molar excess of BrPA is achieved. Iodoacetamide-azide may be used in place of BrPA. The azide-protein solution is incubated at 20°C for at least 1 hour, or at 0°C for at least 2 hours in a dark container.

The azide-functionalized protein solution is purified to remove any unreacted azide-containing compound. 50% trichloroacetic acid (TCA) is mixed with the azide-functionalized protein solution until a TCA concentration of at least 14% is achieved. The solution is incubated at -20°C for 1 hour. The solution is then centrifuged at 13000 rpm for 10 minutes. The supernatant is removed, leaving a precipitated protein fraction. For low protein amounts, there may not be a visible protein pellet or precipitate. The protein pellet or precipitate is washed in cold acetone. The cold acetone solution is centrifuged at 13000 rpm for 10 minutes. The supernatant is removed, leaving a precipitated protein fraction. The protein is resuspended in at least 50 μl of 8M urea.

### Example 2: Azide Functionalization of Amines

A solution containing protein at a concentration of at least 100 μg/ml is provided. 50 μl of the protein solution is removed and set aside for later analysis as a control sample. The protein is provided in a buffer containing no primary amines at a pH within a range between 7 and 9. NHS-$PEG_4$-azide is provided to the protein solution in a molar excess of at least 20x. High concentration protein solutions may be combined with an azide label in a molar excess as low as 10x. NHS-azide may also be used as an azide-containing label. The azide-protein solution is incubated at 20°C for at least 1 hour, or at 0°C for at least 2 hours. The reaction is quenched by the addition of pH 8.0 1M tris buffer until a final concentration of at least 50 mM tris is achieved.

The azide-functionalized protein solution is purified to remove any unreacted azide-containing compound. 50% trichloroacetic acid (TCA) is mixed with the azide-functionalized protein solution until a TCA concentration of at least 14% is achieved. The solution is incubated at -20°C for 1 hour. The solution is then centrifuged at 13000 rpm for 10 minutes. The supernatant is removed, leaving a precipitated protein fraction. For low protein amounts, there may not be a visible protein pellet or precipitate. The protein pellet or precipitate is washed in cold acetone. The cold acetone solution is centrifuged at 13000 rpm for 10 minutes. The supernatant is removed, leaving a precipitated protein fraction. The protein is resuspended in at least 50 μl of 8M urea.

**Example 3: Methyltetrazine Functionalization of Amines**

A solution containing 40 μM maltose binding protein co-tagged with a streptavidin tag and a FLAG tag (MBP-STREP-FLAG) is provided in a pH 8.0 phosphate-buffered solution (PBS). 75 μl of the protein solution is mixed with 39 μl of 1x pH 8.0 PBS. 6 μl of 10 mM methyltetrazine-PEG4-N-hydroxysuccinimidyl ester (mTz-PEG4-NHS) in dimethylformamide (DMF) is mixed to the protein-PBS solution. The mixture is observed and additional DMF is added if full solubility of the mTz-PEG4-NHS solution is not observed. The full reaction mixture is sealed with foil to protect the mixture from light. The mixture is allowed to react for 3 hours at 25°C with continuous mixing. The reaction mixture is quenched with 1M pH 8.0 Tris or 1M L-arginine for at least 2.5 hours at 25°C.

MTz-functionalized MBP-STREP-FLAG protein is purified on a 10DG desalting and buffer exchange column. The reaction mixture is applied to the 10DG sample, followed by a stacking buffer of 1x pH 8.7 PBS solution with 200 mM L-arginine. The column is eluted with 3 fractions of 1 ml each collected. Each elution fraction is measured for protein concentration to determine which fractions contain protein. Fractions containing protein are pooled and concentrated on a Vivaspin filter until the fluid volume is no more than 200 μl. The concentrated protein fraction is collected by reversing the Vivaspin filter. The protein concentration is measured and concentration is continued if the protein concentration is not at least 15 μM. The collected protein fraction may be purified by a second round of desalting or may be purified by FPLC-SEC purification.

**Example 4: Analysis of mTz-functionalized Protein**

A protein sample containing ubiquitin-HIS6 protein was functionalized with mTz according to the protocol described in Example 3, with purification including size exclusion chromatography. Prior to functionalization, a sample or ubiquitin-HIS6 was excluded for comparison to the functionalized protein. Samples were analyzed using a Shimadzu NexeraX2 UHPLC. The HPLC utilized a Phenomenex Aeris Widepore XB-C8 2.1x150 mm column. The HPLC was run using a gradient method, with solvent A as 0.1% trifluoroacetic acid (TFA) and solvent B as 0.1% TFA in acetonitrile. The gradient parameters are shown in Table II. Analysis of eluates was performed by photometric diode array (PDA), with analysis being performed at 280 nm and 530 nm.

**Table II: HPLC gradient method parameters**

| Time (min) | % A | % B | Flow Rate (ml/min) | Max. Pressure (bar) |
|---|---|---|---|---|
| 0 | 100 | 0 | 0.3 | 600 |
| 8 | 63 | 37 | 0.3 | 600 |
| 10 | 63 | 37 | 0.3 | 600 |
| 15 | 50 | 50 | 0.3 | 600 |
| 18 | 100 | 0 | 0.3 | 600 |
| 25 | 100 | 0 | 0.3 | 600 |

Chromatograms for functionalized ubiquitin-HIS6 (upper) and non-functionalized ubiquitin-HIS6 (lower) are shown in **FIG. 13.** The non-functionalized protein was seen to elute primarily as a single peak around 8 min. The functionalized protein eluted in a cluster with multiple peaks between 8.5 mins and 14 mins. The chromatogram for the functionalized protein did not indicate the presence of non-functionalized protein in an amount that exceeds the detection baseline.

**Example 5: Functionalization with Surfactants or Denaturants**

Functionalization of protein molecules was tested in the presence of surfactants or denaturants to determine if

such species would inhibit a functionalization reaction. Hen white egg lysozyme (HEWL) and myoglobin were functionalized with NHS-PEG4-azide according to the protocol of Example 2, with reaction buffer and azide:protein ratio varied. Functionalization of HEWL and myoglobin was tested at azide:protein ratios of 0:1, 3:1 or 30:1.

Each reaction was performed with 4.75 μl of 10 mg/ml protein, 2 μl of NHS-PEG4-azide in DMSO, and 42.25 μl of buffer for a total reaction volume of 50 μl. The four tested buffers were: 1) 42.25 μl of 615 μM PBS buffer; 2) 42.25 μl of 6M guanidinium chloride; 3) 10 μl of 10% sodium dodecyl sulfate (SDS) and 32.25 μl of PBS; and 4) 10 μl of 10% lithium dodecyl sulfate (LDS) and 32.25 μl of PBS. Free azide was not purified after reactions were performed.

Following functionalization and quenching were performed as described in Example 2. After the reaction, all samples were conjugated to DBCO-oligo: protein ratio of 1.5:1. Each conjugation reaction was performed by mixing 3 μl of the functionalized protein mixture with 7 μl of 39.6 μM DBCO-oligos for a total reaction volume of 10 μl. Conjugation reactions were incubated for 14.5 hours at 20°C.

Sample results were checked via SDS-Page gels. Samples were run on a 4-12% BT SDS-Page gel. The 10 μl reactions were mixed with 10 μl of 2x sample buffer and heated to 95 °C for 10 minutes, then spun down. 8 μl of each sample was loaded onto the SDS-Page gel. Gels were run in MES buffer at 220V for 22 minutes. Gels were washed 3 times for 5 minutes in deionized water. After washing, gels were stained with Imperial stain for 3 hours, then rinsed in deionized water. Stained gels were imaged on a lightbox. Table III shows experimental identifiers for the gel images shown in **FIGs. 14A** and **14B.** HEWL is denoted as "H" and myoglobin is denoted as "M" in Table III.

**Table III. Tested Protein Reaction Conditions**

| Identifier | Protein | Buffer | Ratio | Identifier | Protein | Buffer | Ratio |
|---|---|---|---|---|---|---|---|
| H | H | PBS | - | M | M | PBS | - |
| HA | H | PBS | 0:1 | MA | M | PBS | 0:1 |
| HB | H | PBS | 3:1 | MB | M | PBS | 3:1 |
| HC | H | PBS | 30:1 | MC | M | PBS | 30:1 |
| HD | H | GdnCl | 0:1 | MD | M | GdnCl | 0:1 |
| HE | H | GdnCl | 3:1 | ME | M | GdnCl | 3:1 |
| HF | H | GdnCl | 30:1 | MF | M | GdnCl | 30:1 |
| HG | H | SDS | 0:1 | MG | M | SDS | 0:1 |
| HH | H | SDS | 3:1 | MH | M | SDS | 3:1 |
| HI | H | SDS | 30:1 | MI | M | SDS | 30:1 |
| HJ | H | LDS | 0:1 | MJ | M | LDS | 0:1 |
| HK | H | LDS | 3:1 | MK | M | LDS | 3:1 |
| HL | H | LDS | 30:1 | ML | M | LDS | 30:1 |

**FIG. 14A** shows an SDS-Page gel for functionalized HEWL conjugated to DBCO-oligos. Proteins functionalized in the presence of PBS only (lanes HA, HB, and HC) show a trend of increasing molecular weight of the primary protein band as the azide:protein ratio increases, suggesting increased functionalization of the protein. This trend is also observed in guanidinium chloride (HD, HE, HF), SDS (HG, HH, HI), and LDS buffers (HJ, HK, HL), suggesting that the HEWL functionalization is not affected by the presence of denaturants or surfactants. **FIG. 14B** shows an SDS-Page gel for functionalized myoglobin conjugated to DBCO-oligos. Proteins functionalized in the presence of PBS only (lanes MA, MB, and MC) show a trend of increasing molecular weight of the primary protein band as the azide:protein ratio increases, suggesting increased functionalization of the protein. This trend is also observed in guanidinium chloride (MD, ME, MF), SDS (MG, MH, MI), and LDS buffers (MJ, MK, ML), suggesting that the myoglobin functionalization is not affected by the presence of denaturants or surfactants.

**Example 6: Conjugation of Proteins to Anchoring Groups**

MTz- functionalized proteins are conjugated to TCO-functionalized DNA origami anchoring groups comprising one or more TCO functional groups. The TCO-functionalized DNA origami are formed by assembling 5 square-shaped DNA origami tiles into a cross configuration. The central tile of the DNA origami is modified to include 1, 4, or 6 TCO functional groups. **FIG. 16** depicts a top-down schematic of a DNA origami anchoring group comprising a single TCO polypeptide binding site. The TCO-functionalized DNA origami is provided in a buffer comprising 200 mM NaCl, 5 mM Tris-

HCl, 11 mM $MgCl_2$, and 1 mM EDTA at pH 8.0. The amount of protein is calculated based upon the amount of tile to be used in the conjugation reaction. The volume of protein added to the conjugation reaction is calculated according to equation (1):

$$y = (xC_xwz)/C_y \qquad (1)$$

Where y = total volume of mTz-functionalized protein (μl)
x = total volume of DNA origami (μl)
$C_x$ = concentration of DNA origami (μM)
$C_y$ = concentration of mTz-functionalized protein (μM)
w = molar equivalents of protein to TCO
z = number of TCO moieties per DNA origami molecule

Volumes of mTz-functionalized protein and TCO-DNA origami are combined according to the amounts calculated in equation (1). If the volume of mTz-functionalized protein in the reaction mixture exceeds 10% of the total volume (x+y), additional $MgCl_2$ must be added to maintain the magnesium concentration of the reaction mixture. If necessary, 1 μl of $MgCl_2$ should be added to the protein prior to the addition of the DNA origami at a concentration according to equation (2):

$$C_M = 12.4y + 12.4 \qquad (2)$$

Where $C_M$ = concentration of $MgCl_2$ (mM)

The reaction mixture is gently mixed, then placed on a thermomixer or thermocycler at 25°C. The reaction tube is jacketed to prevent exposure to light. Reactions with a 10-fold or higher excess of protein are incubated for 5 hours or more. Reactions with less than a 10-fold excess of protein are incubated for 16 hours or more to ensure complete reaction of mTz with TCO.

Protein conjugates are purified on an Agilent 1100 HPLC with an Agilent Bio-SEC5 4.6 x 300 mm column. The HPLC solvent is filtered 200 mM NaCl, 5 mM Tris-HCl, 11 mM $MgCl_2$, and 1 mM EDTA at pH 8.0. The HPLC is run with isocratic flow at 0.3 ml/min for 25 minutes. Fractions are collected in 30 s intervals between 5 min and 13 mins of the run. Detection of DNA-containing fractions is performed at 260 nm wavelength, with DNA-containing fractions pooled. Pooled DNA-containing fractions are concentrated to a total volume of about 100 μl.

**Example 7. Analysis of Protein Conjugates**

Protein conjugates of Protein A, maltose-binding protein (MBP), and ubiquitin were formed by a mTz-TCO conjugation chemistry, as described in Example 3. Protein conjugates were formed with DNA origami containing a single TCO moiety. Single-TCO DNA origami were conjugated to fluorescently-labeled version of the three aforementioned proteins. Protein A was labeled with an Alexa-Fluor 647 fluorescent dye. MBP was labeled with an Alexa-Fluor 488 fluorescent dye. Ubiquitin was labeled with tetramethylrhodamine (~555 nm wavelength). A control reaction was run using mTz-functionalized protein with DNA origami containing no TCO moiety.

Fluorescently-labeled protein conjugates were run on an Agilent 1100 HPLC with an Agilent Bio-SEC5 4.6 x 300 mm column. The HPLC solvent was filtered 200 mM NaCl, 5 mM Tris-HCl, 11 mM $MgCl_2$, and 1 mM EDTA at pH 8.0. The HPLC was run with isocratic flow at 0.3 ml/min for 25 minutes. The HPLC monitored light absorption across a range of wavelengths between 190 nm and 800 nm. 260 nm wavelength was used to determine the presence of DNA. 488 nm, 553 nm, and 652 nm wavelengths were used to determine the presence of fluorescently-labeled protein as appropriate.

**FIG. 15A** shows HPLC data for Protein A conjugates. The upper chromatogram depicts 260 nm data, showing the elution of DNA origami around 11 mins. The lower chromatogram depicts 652 nm data, showing elution of protein around 11 mins, with excess unconjugated protein following at around 15 mins. Negative control data shown in **FIG. 15B** shows no protein eluting with the DNA origami at 11 mins (lower chromatogram) due to available TCO to complete the conjugation.

**FIG. 15C** shows HPLC data for MBP protein conjugates. The lower chromatogram depicts 260 nm data, showing the elution of DNA origami around 11 mins. The upper chromatogram depicts 488 nm data, showing elution of protein around 11 mins, with excess unconjugated protein following at around 15 mins. **FIG. 15D** shows HPLC data for ubiquitin protein conjugates. The upper chromatogram depicts 260 nm data, showing the elution of DNA origami around 11 mins. The lower chromatogram depicts 553 nm data, showing elution of protein around 11 mins, with excess unconjugated protein following at around 15 mins.

## Claims

1. A method of forming a polypeptide array, comprising:

   (a) providing a sample comprising sample polypeptides;
   (b) separating the sample polypeptides from the sample in the presence of separation standard polypeptides, wherein the separation standard polypeptides comprise polypeptides derived from a differing source than the sample polypeptides, and wherein each separation standard polypeptide of the separation standard polypeptides comprises a universal and/or unique tag, wherein the universal tag distinguishes the separation standard polypeptides from other polypeptides in the method and wherein the unique tag distinguishes a separation standard polypeptide from all other separation standard polypeptides in the method, wherein the tag is a peptide sequence;
   (c) coupling the sample polypeptides to anchoring groups to form sample polypeptide composites, wherein the coupling occurs in the presence of coupling standard polypeptides, wherein the coupling standard polypeptides comprise polypeptides derived from a differing source than the sample polypeptides, and wherein each coupling standard polypeptide of the coupling standard polypeptides comprises a universal and/or unique tag, wherein the universal tag distinguishes the coupling standard polypeptides from other polypeptides in the method and wherein the unique tag distinguishes a coupling standard polypeptide from all other coupling standard polypeptides in the method, wherein the tag is a peptide sequence; and
   (d) attaching at least a fraction of the sample polypeptide composites, the separation standard polypeptides and the coupling standard polypeptides to a solid support, whereby each of the sample polypeptide composites, the separation standard polypeptides and the coupling standard polypeptides that is attached to the solid support comprises an address of the polypeptide array.

2. The method of claim 1, wherein the attaching of step (d) occurs in the presence of attachment standard polypeptides, whereby each of the attachment standards that is attached to the solid support comprises an address of the polypeptide array.

3. The method of claim 1 or claim 2, wherein the attachment standard polypeptides comprise attachment standard polypeptide composites, wherein each of the attachment standard polypeptide composites comprises an attachment standard polypeptide coupled to an anchoring group.

4. The method of any one of the preceding claims, wherein the separation standard polypeptides comprise separation standard polypeptide composites, wherein each of the separation standard polypeptide composites comprises a separation standard polypeptide coupled to an anchoring group.

5. The method of any one of the preceding claims, wherein the coupling standard polypeptides comprise coupling standard polypeptide composites, wherein each of the coupling standard polypeptide composites comprises a coupling standard polypeptide coupled to an anchoring group.

6. The method of any one of the preceding claims, wherein the separation standard polypeptides, coupling standard polypeptides or attachment standard polypeptides comprise a synthetic polypeptide, a non-natural amino acid or a synthetically modified amino acid.

7. The method of any one of the preceding claims, wherein the polypeptide array comprises at least 1 x $10^4$ different full-length primary protein structures.

8. The method of any one of the preceding claims, wherein the anchoring groups comprise particles configured to couple to the solid support, optionally wherein the particles comprise a nucleic acid, a polypeptide, a polymer, an inorganic nanoparticle, an organic nanoparticle, a structured nucleic acid particle (SNAP), a nucleic acid origami or a nucleic acid nanoball.

9. The method of any one of the preceding claims, further comprising:
   (e) detecting the sample polypeptide composites, the separation standard polypeptides and the coupling standard polypeptides at addresses of the polypeptide array.

10. The method of claim 9, further comprising detecting the attachment standard polypeptides at addresses of the polypeptide array.

**11.** The method of claim 9, wherein the sample polypeptide composites, the separation standard polypeptides and the coupling standard polypeptides are detected as single polypeptide resolution.

**12.** The method of any one of claims 9-11, further comprising quantifying the amount of a particular sample polypeptide species in the sample by comparing (i) a number of the addresses attached to a separation standard polypeptide with (ii) the number of the addresses attached to a sample polypeptide composite that comprise the particular sample polypeptide species.

**13.** The method of any one of claims 9-12, further comprising quantifying the amount of a particular sample polypeptide species in the sample by comparing (i) a number of addresses attached to a coupling standard polypeptide with (ii) a number of addresses attached to a sample polypeptide composite that comprise the particular sample polypeptide species.

**14.** The method of any one of claims 9-13, further comprising quantifying the amount of a particular sample polypeptide species in the sample by comparing (i) a number of addresses attached to an attachment standard polypeptide with (ii) a number of addresses attached to a sample polypeptide composite that comprise the particular sample polypeptide species.

## Patentansprüche

**1.** Ein Verfahren zur Bildung eines Polypeptid-Arrays, umfassend:

(a) Bereitstellen einer Probe umfassend Probenpolypeptide;
(b) Trennen der Probenpolypeptide von der Probe in der Gegenwart von Separations-Standardpolypeptiden, wobei die Separations-Standardpolypeptide Polypeptide umfassen, die aus einer anderen Quelle als die Probenpolypeptide stammen, und wobei jedes Separations-Standardpolypeptid der Separations-Standardpolypeptide eine universelle und/oder einzigartige Markierung umfasst, wobei die universelle Markierung die Separations-Standardpolypeptide von anderen Polypeptiden im Verfahren unterscheidet und wobei die einzigartige Markierung ein Separations-Standardpolypeptid von allen anderen Separations-Standardpolypeptiden im Verfahren unterscheidet, wobei die Markierung eine Peptidsequenz ist;
(c) Koppeln der Probenpolypeptide an Ankergruppen, um Probenpolypeptid-Verbünde zu bilden, wobei die Kopplung in der Gegenwart von Kopplungs-Standardpolypeptiden erfolgt, wobei die Kopplungs-Standardpolypeptide Polypeptide umfassen, die aus einer anderen Quelle als die Probenpolypeptide stammen, und wobei jedes Kopplungs-Standardpolypeptid der Kopplungs-Standardpolypeptide eine universelle und/oder einzigartige Markierung umfasst, wobei die universelle Markierung die Kopplungs-Standardpolypeptide von anderen Polypeptiden im Verfahren unterscheidet und wobei die einzigartige Markierung ein Kopplungs-Standardpolypeptid von allen anderen Kopplungs-Standardpolypeptiden im Verfahren unterscheidet, wobei die Markierung eine Peptidsequenz ist; und
(d) Befestigen mindestens eines Teils der Probenpolypeptid-Verbünde, der Separations-Standardpolypeptide und der Kopplungs-Standardpolypeptide an einem festen Träger, wobei jedes der Probenpolypeptid-Verbünde, der Separations-Standardpolypeptide und der Kopplungs-Standardpolypeptide, das am festen Träger befestigt wird, eine Adresse des Polypeptid-Arrays umfasst.

**2.** Das Verfahren aus Anspruch 1, wobei der Befestigungsschritt (d) in der Gegenwart von Befestigungs-Standardpolypeptiden, wobei jedes Befestigungs-Standardpolypeptid, das am festen Träger befestigt ist, eine Adresse des Polypeptid-Arrays umfasst.

**3.** Das Verfahren aus Anspruch 1 oder Anspruch 2, wobei die Befestigungs-Standardpolypeptide Befestigungs-Standardpolypeptid-Verbünde umfassen, wobei jedes der Befestigungs-Standardpolypeptid-Verbünde ein an eine Ankergruppe gekoppeltes Befestigungs-Standardpolypeptid umfasst.

**4.** Das Verfahren aus irgendeinem der vorherigen Ansprüche, wobei die Separations-Standardpolypeptide Separations-Standardpolypeptid-Verbünde umfassen, wobei jedes der Separations-Standardpolypeptid-Verbünde ein an eine Ankergruppe gekoppeltes Separations-Standardpolypeptid umfasst.

**5.** Das Verfahren aus irgendeinem der vorherigen Ansprüche, wobei die Kopplungs-Standardpolypeptide Kopplungs-Standardpolypeptid-Verbünde umfassen, wobei jedes der Kopplungs-Standardpolypeptid-Verbünde ein an eine

Ankergruppe gekoppeltes Kopplungs-Standardpolypeptid umfasst.

**6.** Das Verfahren aus irgendeinem der vorherigen Ansprüche, wobei die Separations-Standardpolypeptide, Kopplungs-Standardpolypeptide oder Befestigungs-Standardpolypeptide ein synthetisches Polypeptid, eine nicht-natürliche Aminosäure oder eine synthetisch modifizierte Aminosäure umfassen.

**7.** Das Verfahren aus irgendeinem der vorherigen Ansprüche, wobei der Polypeptid-Array mindestens 1 x $10^4$ verschiedene primäre Proteinstrukturen voller Länge umfasst.

**8.** Das Verfahren aus irgendeinem der vorherigen Ansprüche, wobei die Ankergruppen Partikel umfassen, die konfiguriert sind, an den festen Träger zu koppeln, optional wobei die Partikel eine Nukleinsäure, ein Polypeptid, ein Polymer, ein anorganisches Nanopartikel, ein organisches Nanopartikel, ein strukturiertes Nukleinsäurepartikel (SNAP), ein Nukleinsäure-Origami oder einen Nukleinsäure-Nanoball umfassen.

**9.** Das Verfahren aus irgendeinem der vorherigen Ansprüche, weiter umfassend:
(e) Detektieren der Probenpolypeptid-Verbünde, der Separations-Standardpolypeptide und der Kopplungs-Standardpolypeptide an Adressen des Polypeptid-Arrays.

**10.** Das Verfahren aus Anspruch 9, weiter umfassend Detektieren der Befestigungs-Standardpolypeptide an Adressen des Polypeptid-Arrays.

**11.** Das Verfahren aus Anspruch 9, wobei die Probenpolypeptid-Verbünde, die Separations-Standardpolypeptide und die Kopplungs-Standardpolypeptide als Einzelpolypeptidauflösung detektiert werden.

**12.** Das Verfahren aus irgendeinem der Ansprüche 9-11, weiter umfassend Quantifizieren der Menge einer bestimmten Probenpolypeptid-Spezies in der Probe durch Vergleichen (i) einer Anzahl der an einem Separations-Standardpolypeptid befestigten Adressen mit (ii) der Anzahl der an einem Probenpolypeptid-Verbund, der die bestimmte Probenpolypeptid-Spezies umfasst, befestigten Adressen.

**13.** Das Verfahren aus irgendeinem der Ansprüche 9-12, weiter umfassend Quantifizieren der Menge einer bestimmten Probenpolypeptid-Spezies in der Probe durch Vergleichen (i) einer Anzahl von an einem Kopplungs-Standardpolypeptid befestigten Adressen mit (ii) einer Anzahl von an einem Probenpolypeptid-Verbund, der die bestimmte Probenpolypeptid-Spezies umfasst, befestigten Adressen.

**14.** Das Verfahren aus irgendeinem der Ansprüche 9-13, weiter umfassend Quantifizieren der Menge einer bestimmten Probenpolypeptid-Spezies in der Probe durch Vergleichen (i) einer Anzahl von an einem Befestigungs-Standardpolypeptid befestigten Adressen mit (ii) einer Anzahl von an einem Probenpolypeptid-Verbund, der die bestimmte Probenpolypeptid-Spezies umfasst, befestigten Adressen.

## Revendications

**1.** - Procédé de formation d'un réseau de polypeptides, comprenant :

(a) fournir un échantillon comprenant des polypeptides d'échantillon ;
(b) séparer les polypeptides d'échantillon à partir de l'échantillon en présence de polypeptides standards de séparation, les polypeptides standards de séparation comprenant des polypeptides dérivés d'une source différente de celle des polypeptides d'échantillon, et chaque polypeptide standard de séparation des polypeptides standards de séparation comprenant une étiquette universelle et/ou unique, l'étiquette universelle distinguant les polypeptides standards de séparation des autres polypeptides dans le procédé, et l'étiquette unique distinguant un polypeptide standard de séparation de tous les autres polypeptides standards de séparation dans le procédé, l'étiquette étant une séquence peptidique ;
(c) coupler les polypeptides d'échantillon à des groupes d'ancrage pour former des composites polypeptidiques d'échantillon, le couplage s'effectuant en présence de polypeptides standards de couplage, les polypeptides standards de couplage comprenant des polypeptides dérivés d'une source différente de celle des polypeptides d'échantillon, et chaque polypeptide standard de couplage des polypeptides standards de couplage comprenant une étiquette universelle et/ou unique, l'étiquette universelle distinguant les polypeptides standards de couplage des autres polypeptides dans le procédé, et l'étiquette unique distinguant un polypeptide standard de couplage

de tous les autres polypeptides standards de couplage dans le procédé, l'étiquette étant une séquence peptidique ; et

(d) fixer au moins une fraction des composites polypeptidiques d'échantillon, des polypeptides standards de séparation et des polypeptides standards de couplage à un support solide, ce par quoi chacun des composites polypeptidiques d'échantillon, des polypeptides standards de séparation et des polypeptides standards de couplage qui est fixé au support solide comprend une adresse du réseau de polypeptides.

**2.** - Procédé selon la revendication 1, dans lequel la fixation de l'étape (d) se produit en présence de polypeptides standards de fixation, ce par quoi chacun des standards de fixation qui est fixé au support solide comprend une adresse du réseau de polypeptides.

**3.** - Procédé selon la revendication 1 ou la revendication 2, dans lequel les polypeptides standards de fixation comprennent des composites polypeptidiques standards de fixation, chacun des composites polypeptidiques standards de fixation comprenant un polypeptide standard de fixation couplé à un groupe d'ancrage.

**4.** - Procédé selon l'une quelconque des revendications précédentes, dans lequel les polypeptides standards de séparation comprennent des composites polypeptidiques standards de séparation, chacun des composites polypeptidiques standards de séparation comprenant un polypeptide standard de séparation couplé à un groupe d'ancrage.

**5.** - Procédé selon l'une quelconque des revendications précédentes, dans lequel les polypeptides standards de couplage comprennent des composites polypeptidiques standards de couplage, chacun des composites polypeptidiques standards de couplage comprenant un polypeptide standard de couplage couplé à un groupe d'ancrage.

**6.** - Procédé selon l'une quelconque des revendications précédentes, dans lequel les polypeptides standards de séparation, les polypeptides standards de couplage ou les polypeptides standards de fixation comprennent un polypeptide synthétique, un acide aminé non naturel ou un acide aminé modifié par voie de synthèse.

**7.** - Procédé selon l'une quelconque des revendications précédentes, dans lequel le réseau de polypeptides comprend au moins 1 x $10^4$ structures protéiques primaires de pleine longueur différentes.

**8.** - Procédé selon l'une quelconque des revendications précédentes, dans lequel les groupes d'ancrage comprennent des particules configurées pour se coupler au support solide, facultativement dans lequel les particules comprennent un acide nucléique, un polypeptide, un polymère, une nanoparticule inorganique, une nanoparticule organique, une particule d'acide nucléique structurée (SNAP), un origami d'acide nucléique ou une nanobille d'acides nucléiques.

**9.** - Procédé selon l'une quelconque des revendications précédentes, comprenant en outre :
(e) détecter les composites polypeptidiques d'échantillon, les polypeptides standards de séparation et les polypeptides standards de couplage aux adresses du réseau de polypeptides.

**10.** - Procédé selon la revendication 9, comprenant en outre la détection des polypeptides standards de fixation aux adresses du réseau de polypeptides.

**11.** - Procédé selon la revendication 9, dans lequel les composites polypeptidiques d'échantillon, les polypeptides standards de séparation et les polypeptides standards de couplage sont détectés en tant que résolution de polypeptides uniques.

**12.** - Procédé selon l'une quelconque des revendications 9 à 11, comprenant en outre la quantification de la quantité d'une espèce polypeptidique particulière d'échantillon dans l'échantillon en comparant (i) un nombre d'adresses attachées à un polypeptide standard de séparation avec (ii) le nombre d'adresses attachées à un composite polypeptidique d'échantillon qui comprennent l'espèce polypeptidique particulière d'échantillon.

**13.** - Procédé selon l'une quelconque des revendications 9 à 12, comprenant en outre la quantification de la quantité d'une espèce polypeptidique particulière d'échantillon dans l'échantillon en comparant (i) un nombre d'adresses attachées à un polypeptide standard de couplage avec (ii) un nombre d'adresses attachées à un composite polypeptidique d'échantillon qui comprennent l'espèce polypeptidique particulière d'échantillon.

**14.** - Procédé selon l'une quelconque des revendications 9 à 13, comprenant en outre la quantification de la quantité d'une

espèce polypeptidique particulière d'échantillon dans l'échantillon en comparant (i) un nombre d'adresses attachées à un polypeptide standard de fixation avec (ii) un nombre d'adresses attachées à un composite polypeptidique d'échantillon qui comprennent l'espèce polypeptidique particulière d'échantillon.

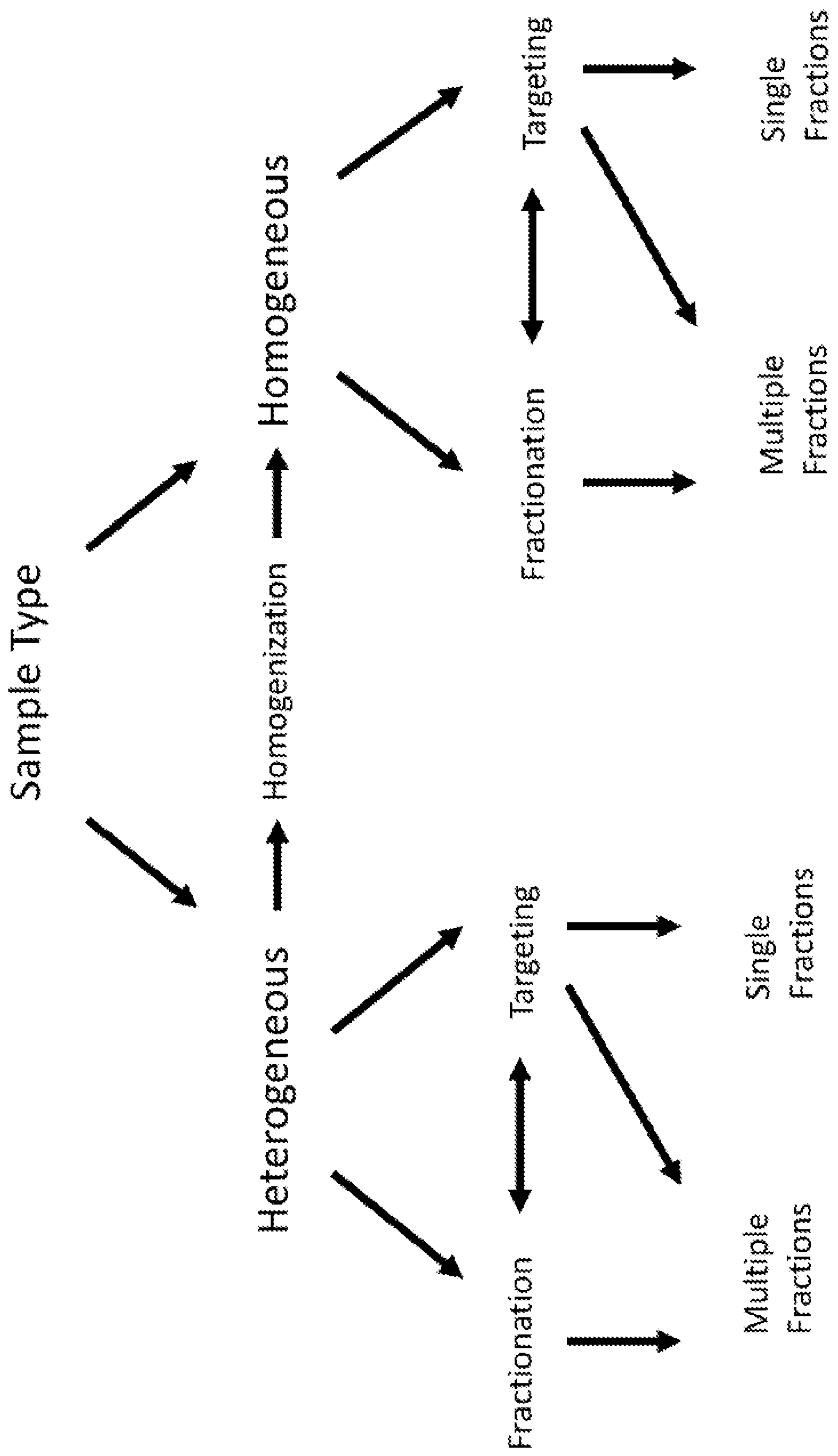
Sample Type
Heterogeneous
Homogenization
Homogeneous
Fractionation
Targeting
Multiple Fractions
Single Fractions
Fractionation
Targeting
Multiple Fractions
Single Fractions

FIG. 1A

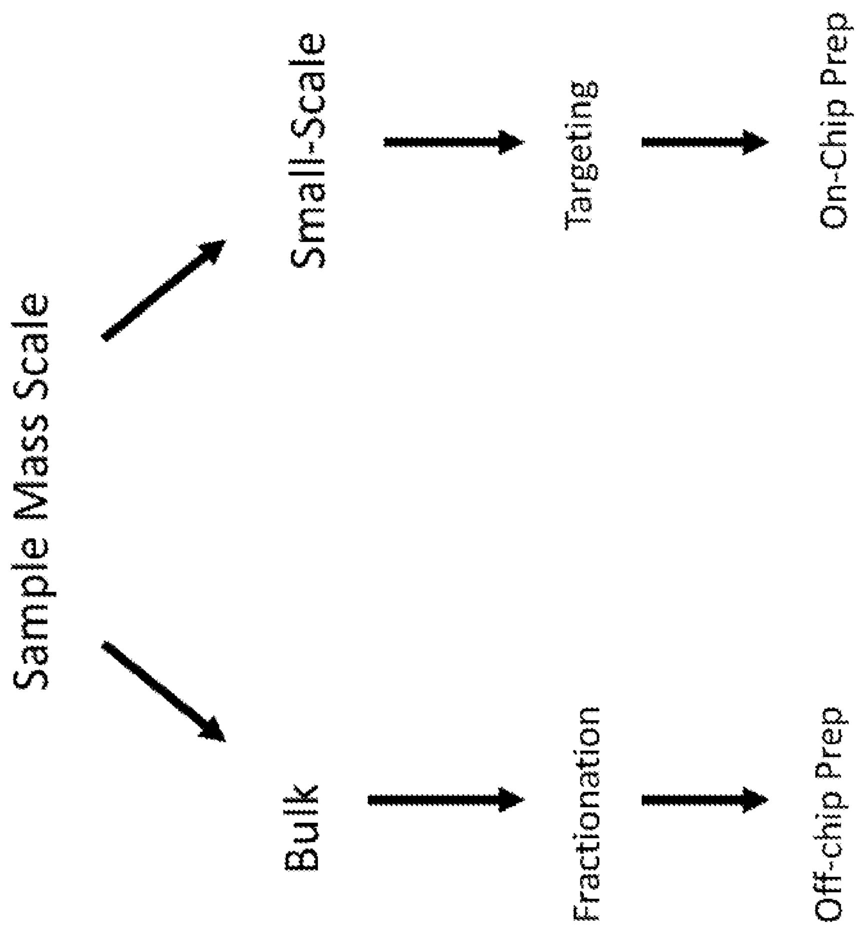
Sample Mass Scale
Bulk
Fractionation
Off-chip Prep
Small-Scale
Targeting
On-Chip Prep

FIG. 1B

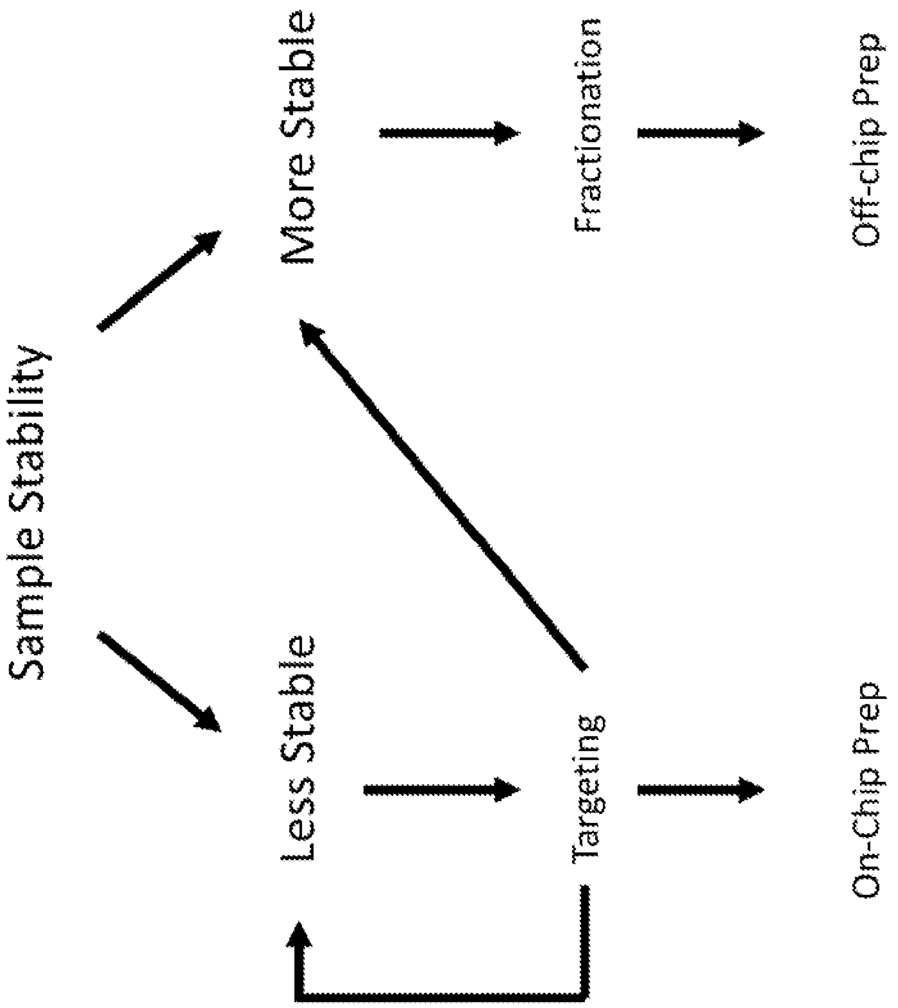
Sample Stability
More Stable
Less Stable
Fractionation
Targeting
Off-chip Prep
On-Chip Prep

FIG. 1C

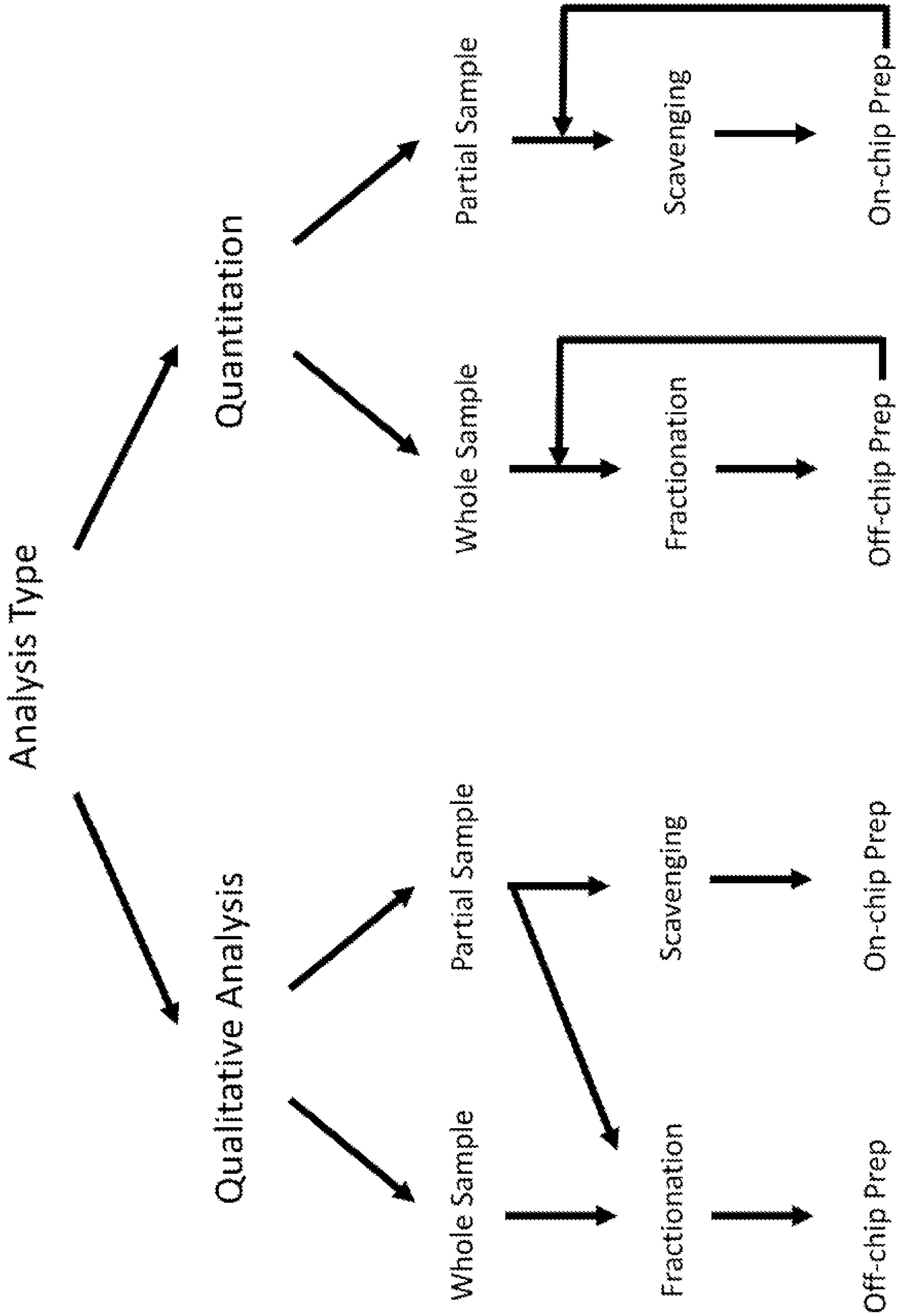
Analysis Type
Quantitation
Qualitative Analysis
Whole Sample
Partial Sample
Whole Sample
Partial Sample
Fractionation
Scavenging
Fractionation
Scavenging
Off-chip Prep
On-chip Prep
Off-chip Prep
On-chip Prep

FIG. 1D

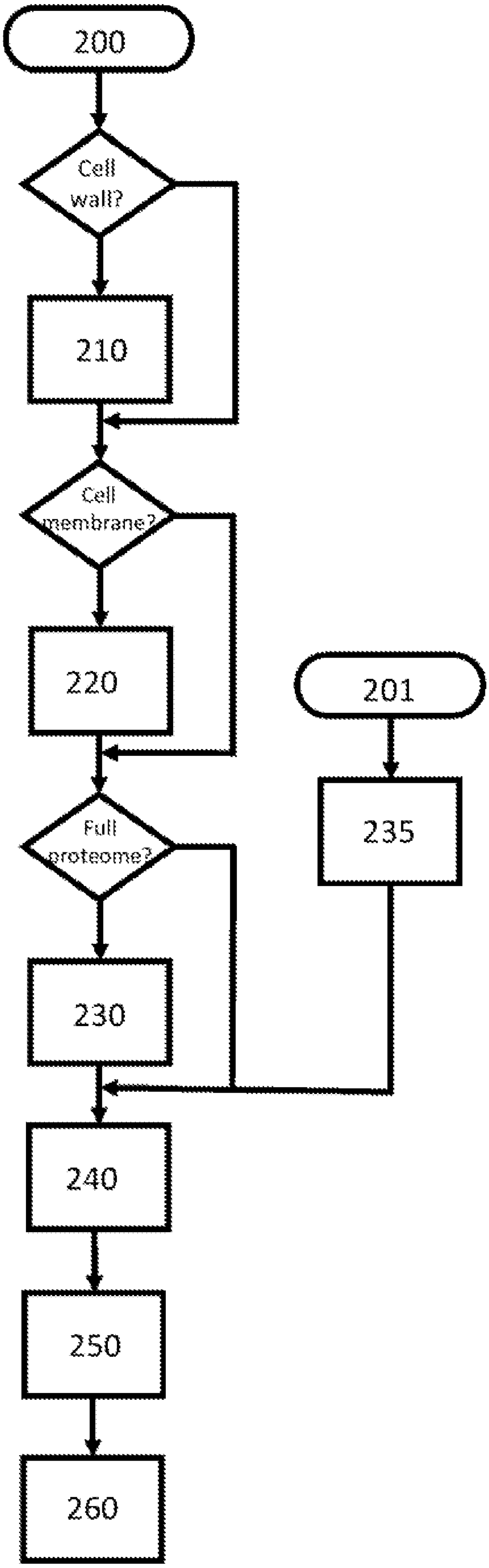
200
Cell wall?
210
Cell membrane?
220
201
235
Full proteome?
230
240
250
260

FIG. 2

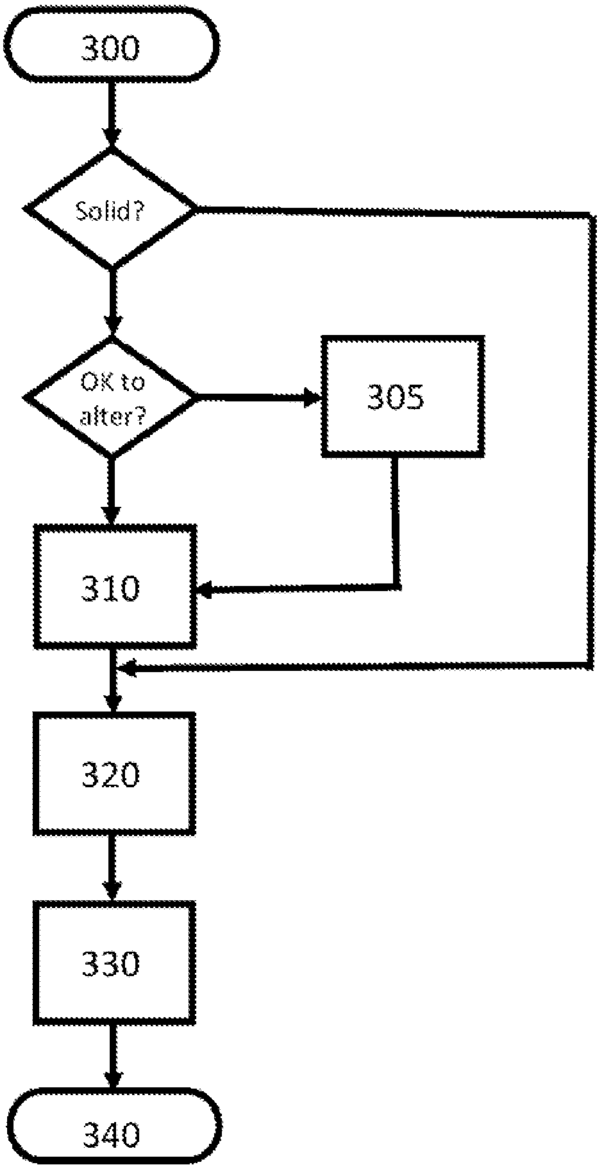


FIG. 3

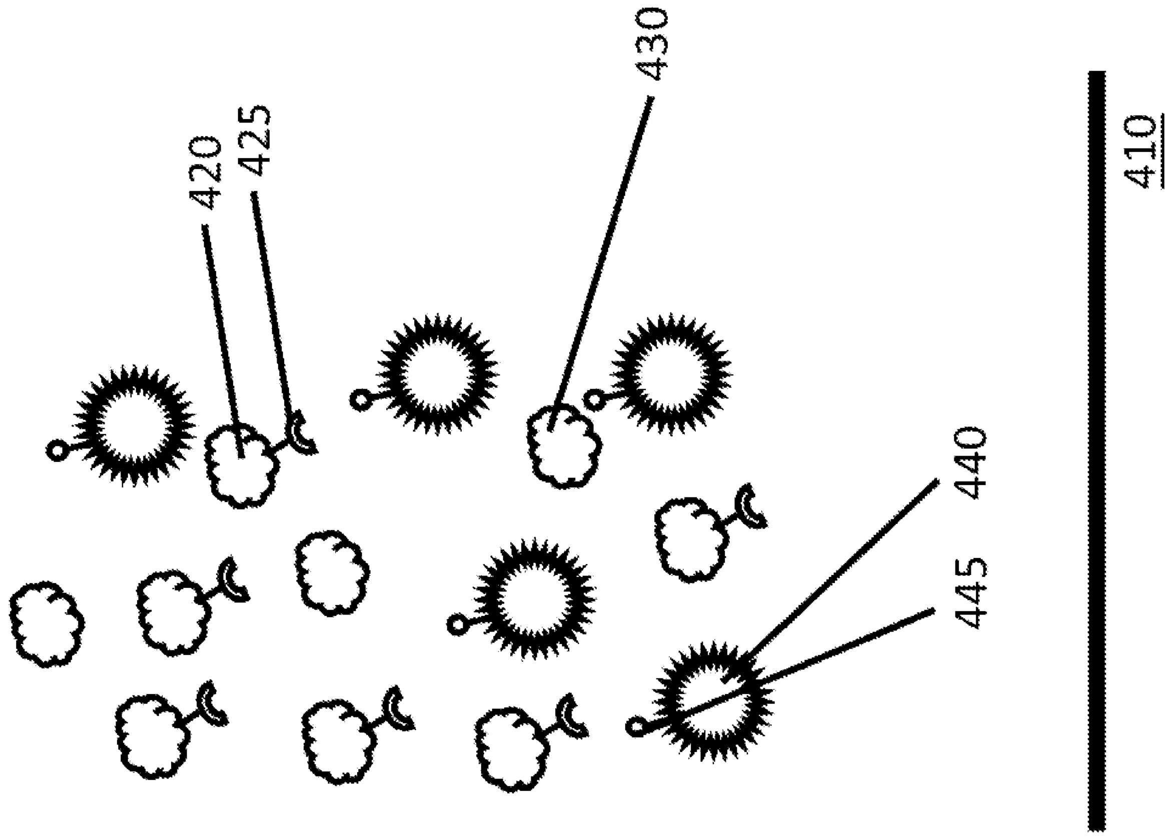


FIG. 4A

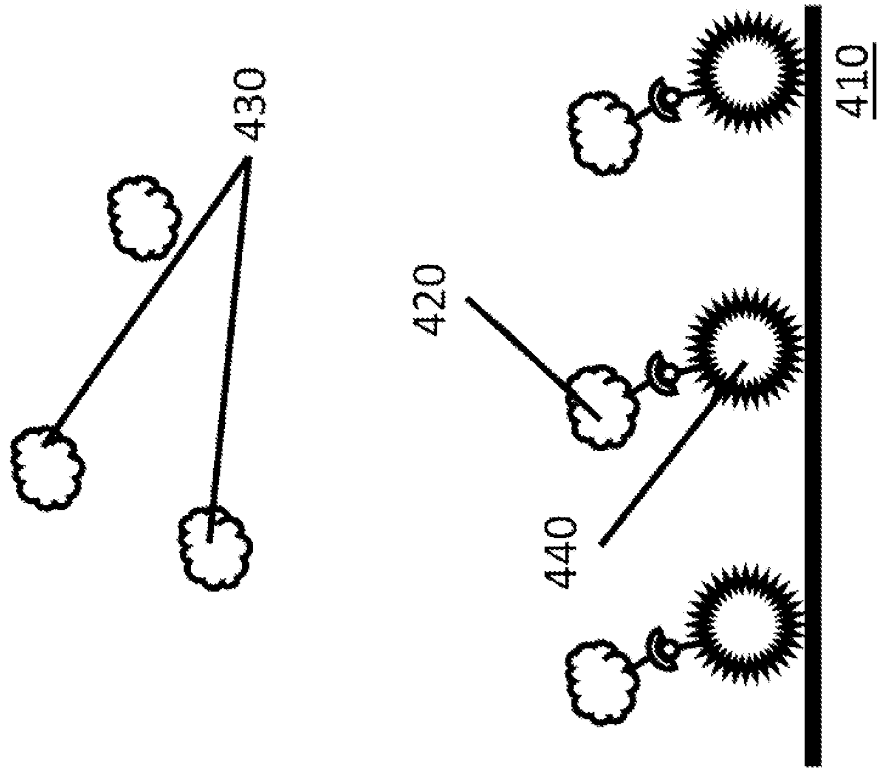


FIG. 4B

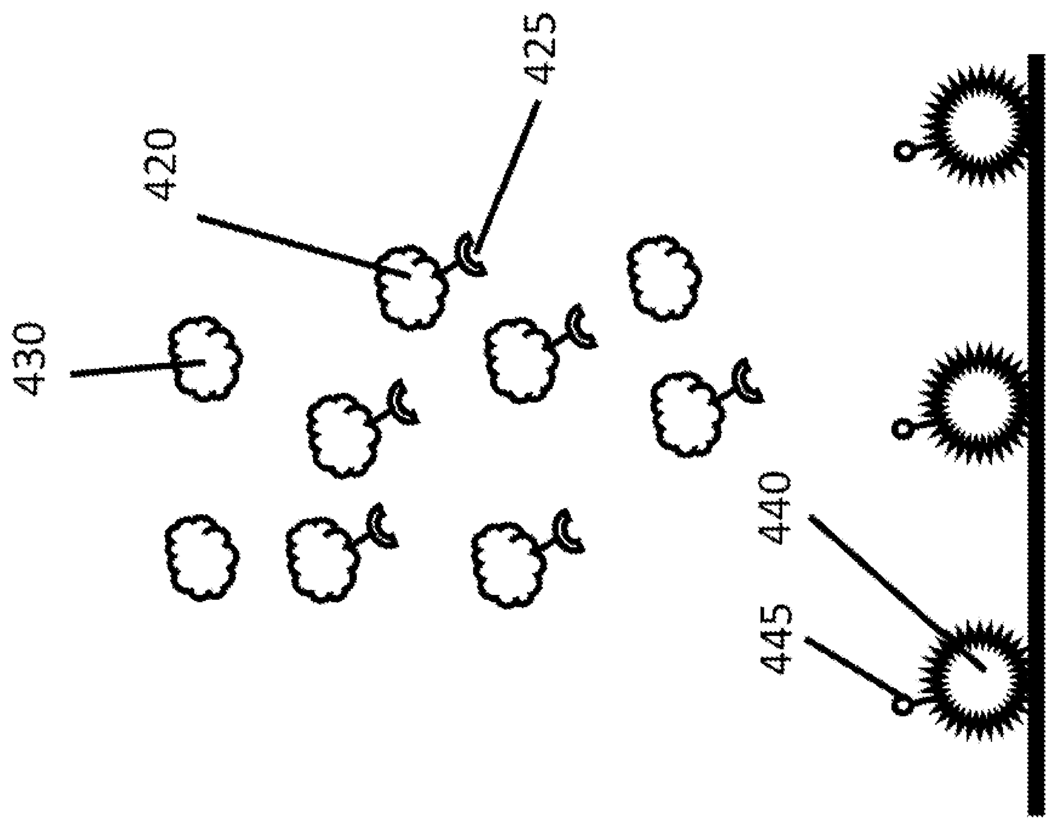


FIG. 4C

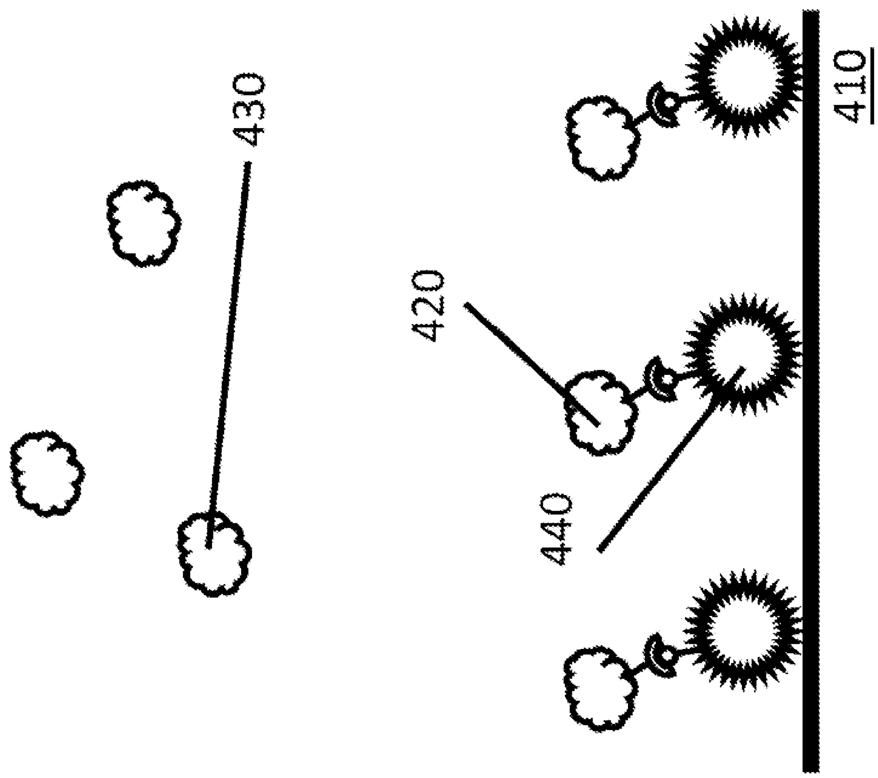


FIG. 4D

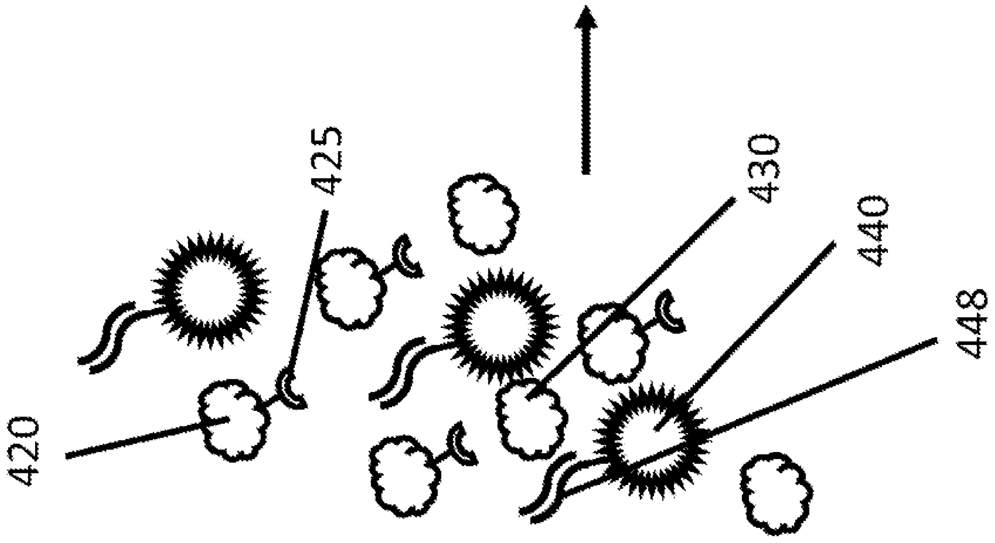


FIG. 4E

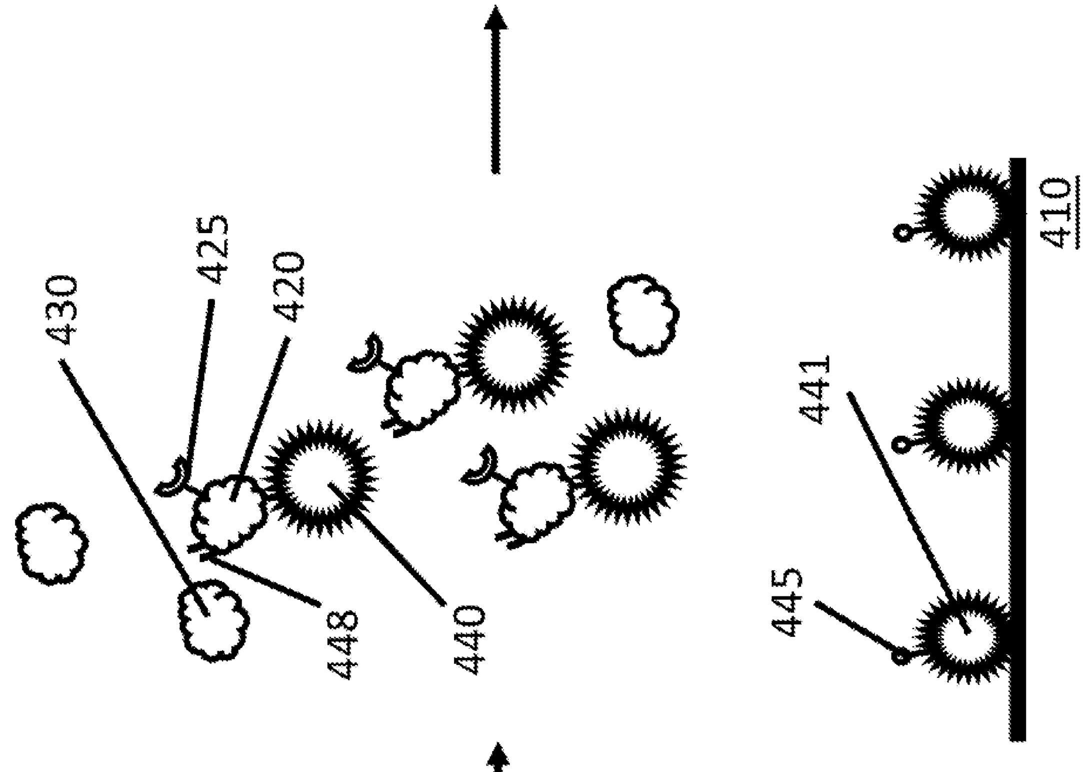


FIG. 4F

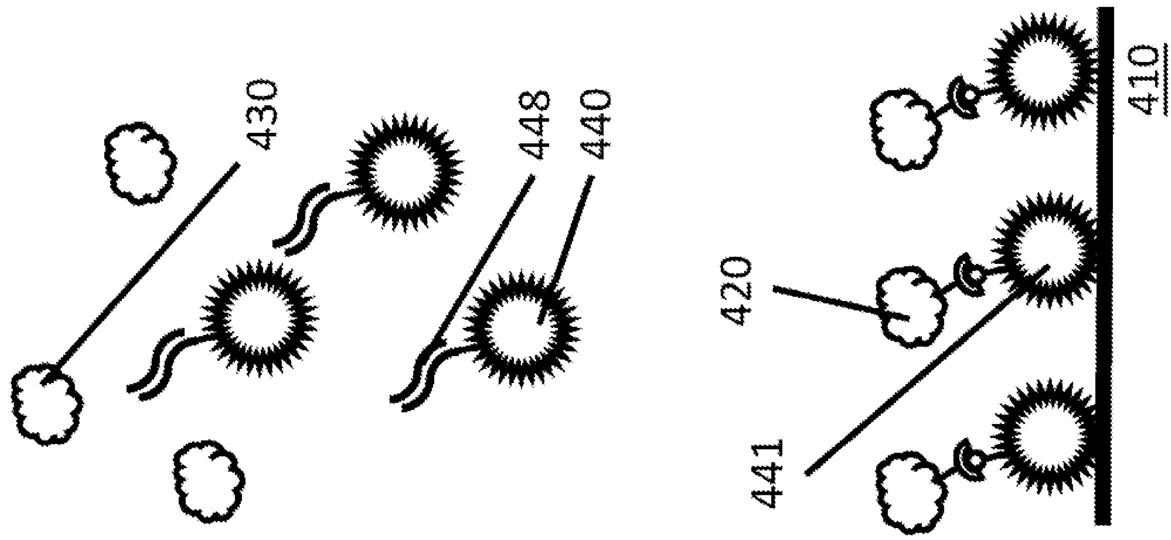


FIG. 4G

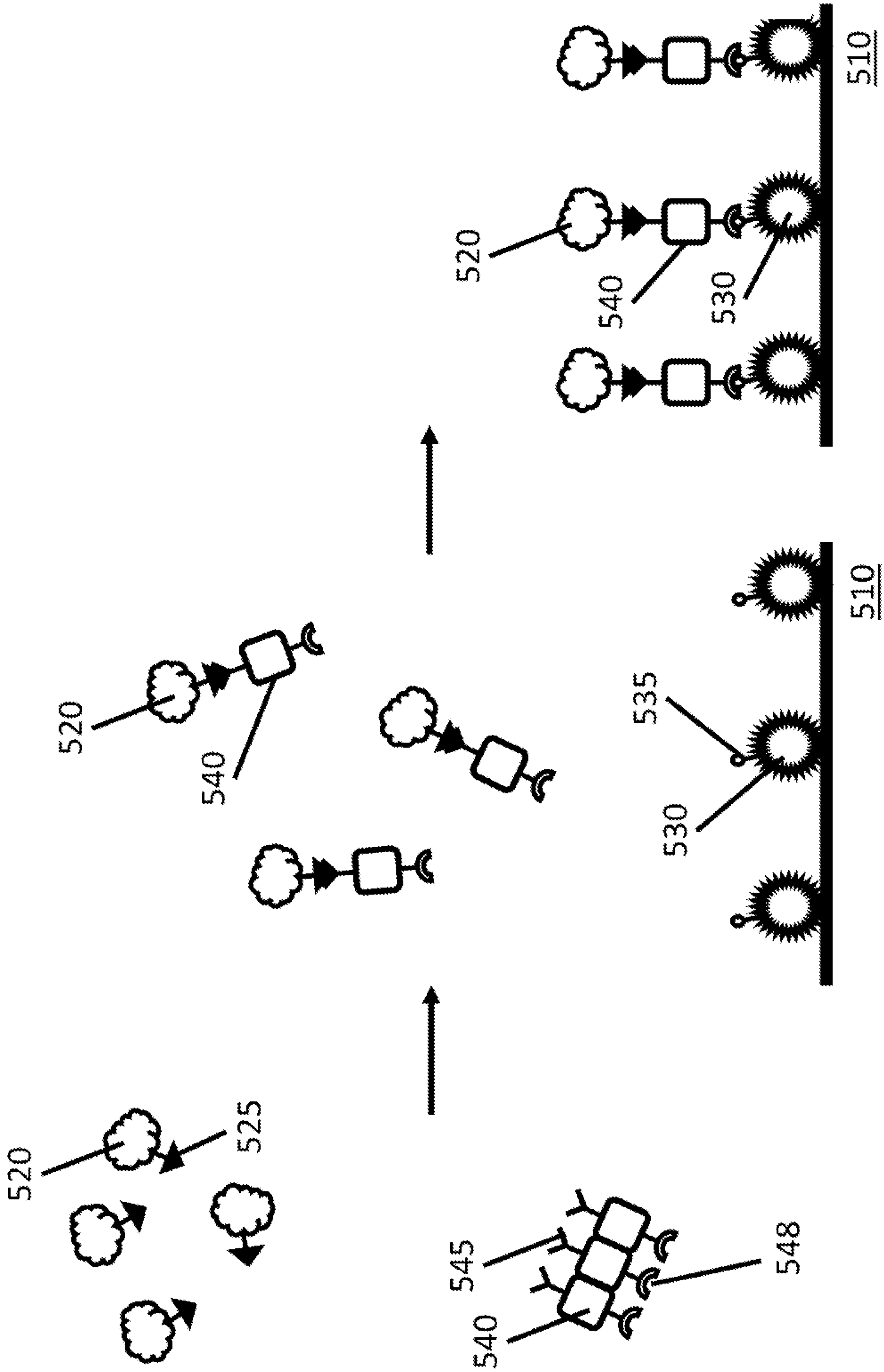


FIG. 5A

FIG. 5B

FIG. 5C

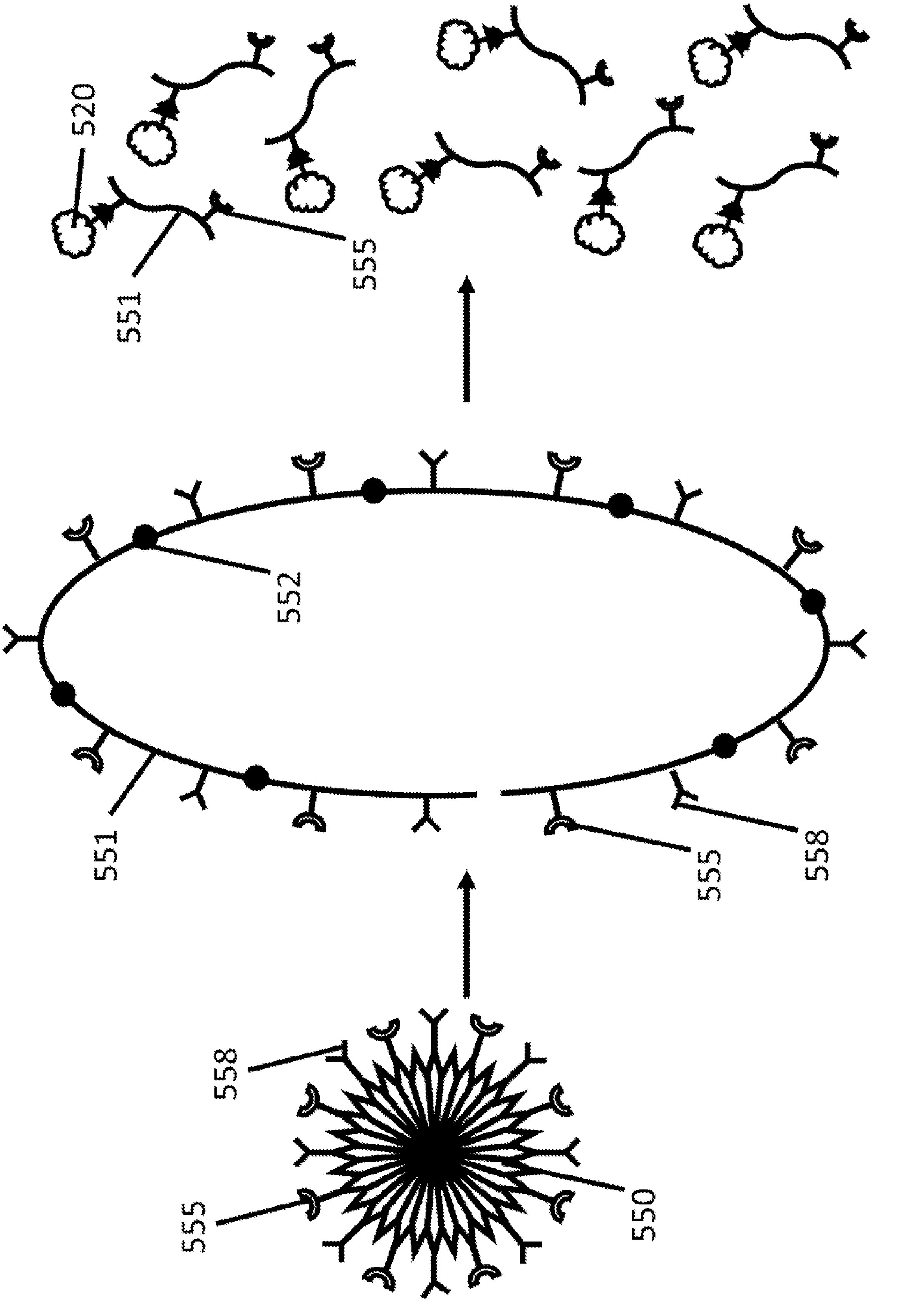


FIG. 5D

FIG. 5E

FIG. 5F

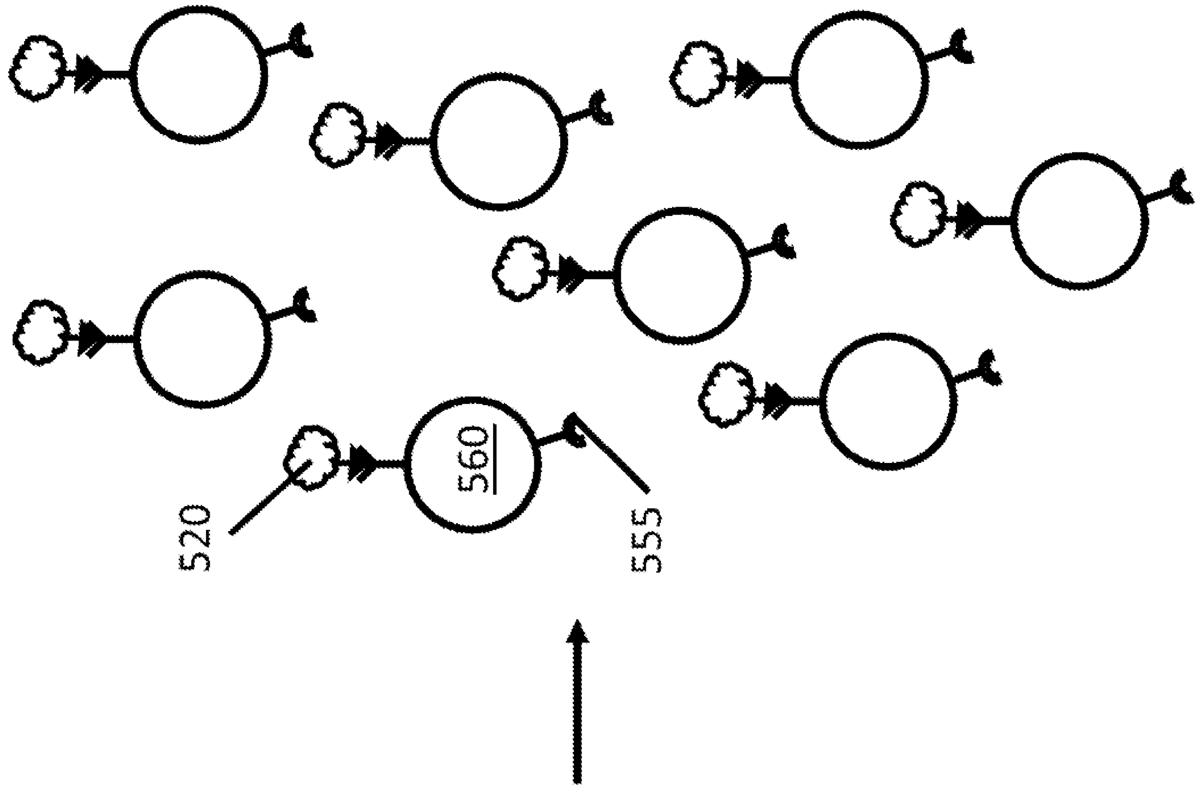


FIG. 5H

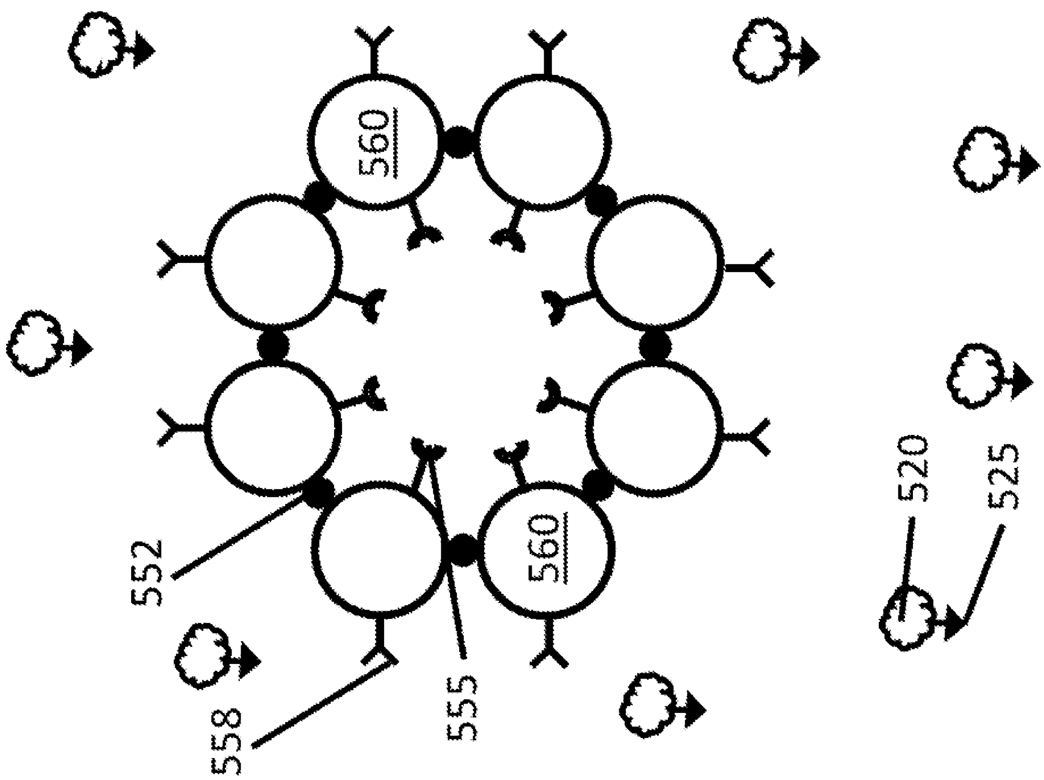


FIG. 5G

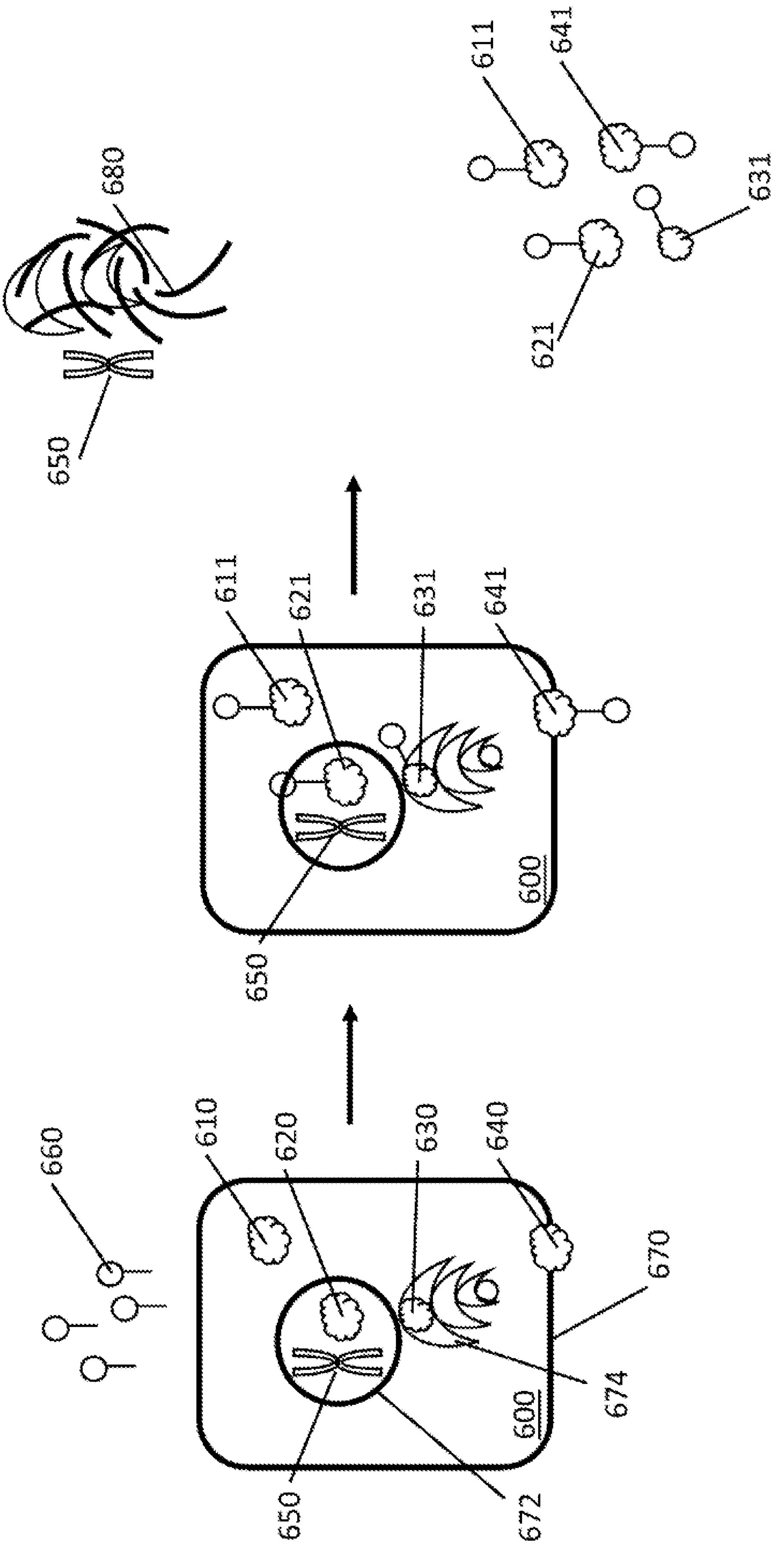
660
610
620
630
640
670
674
672
650
600
611
621
631
641
650
600
650
680
611
641
621
631

FIG. 6A

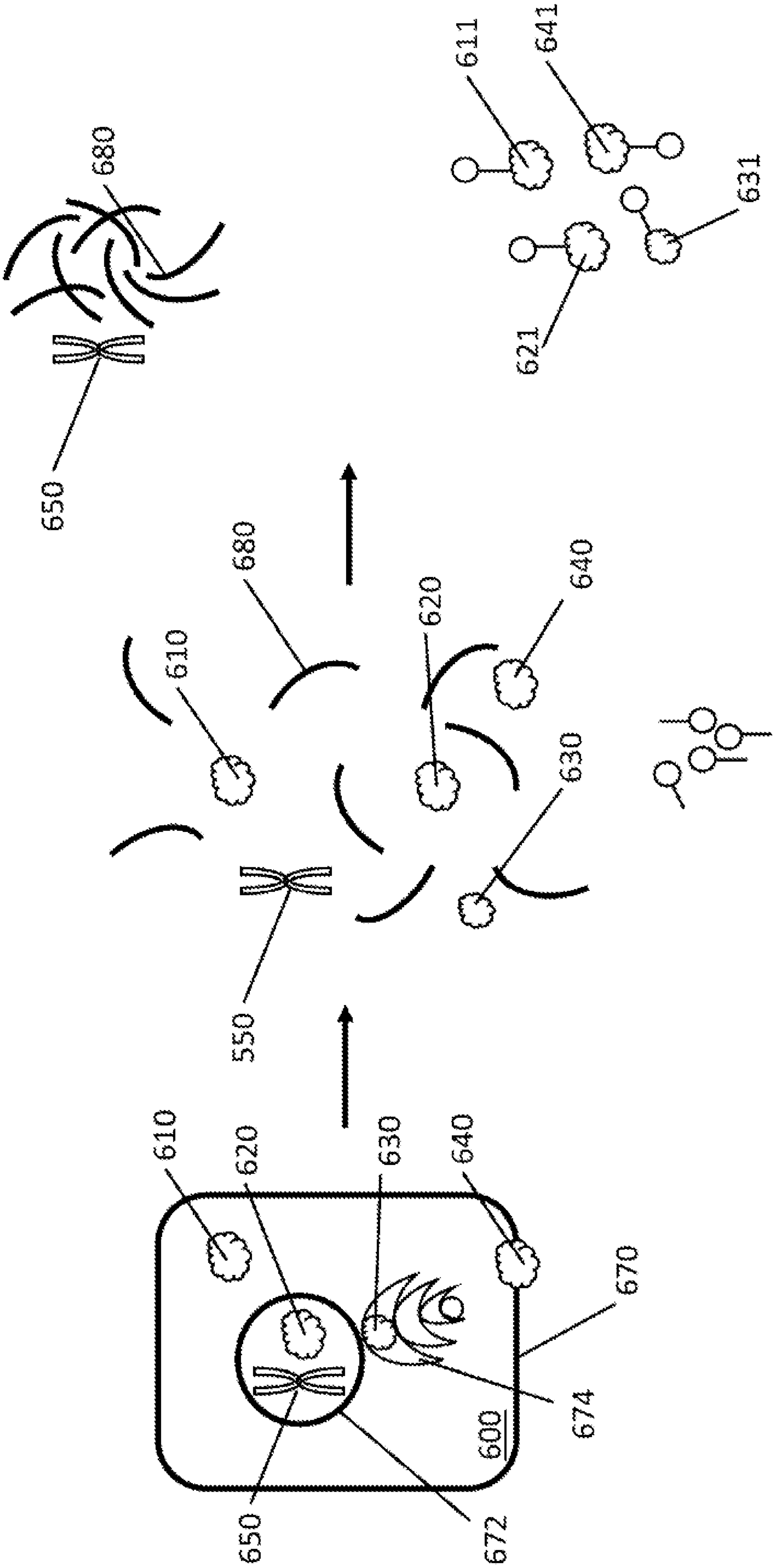


FIG. 6B

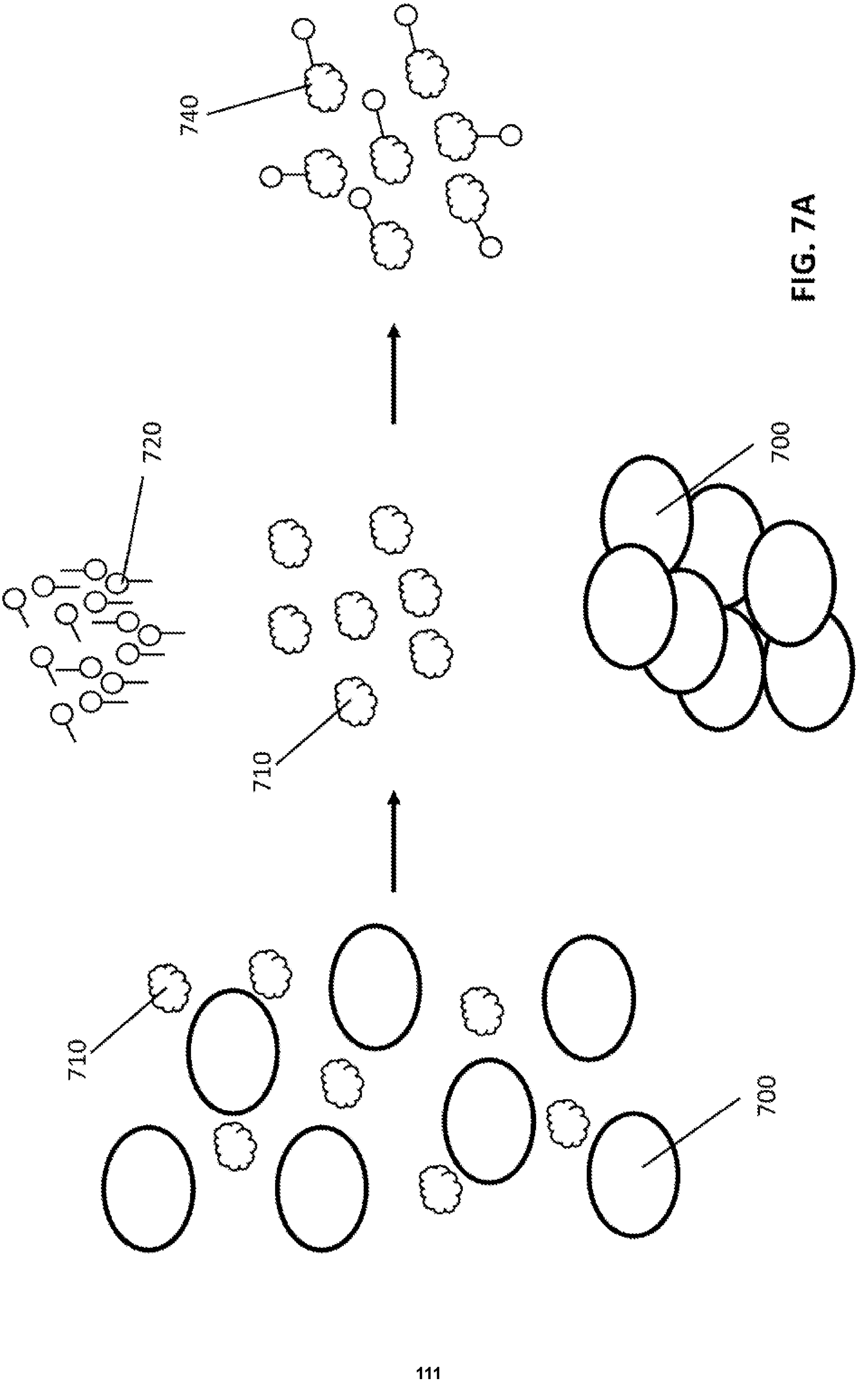


FIG. 7A

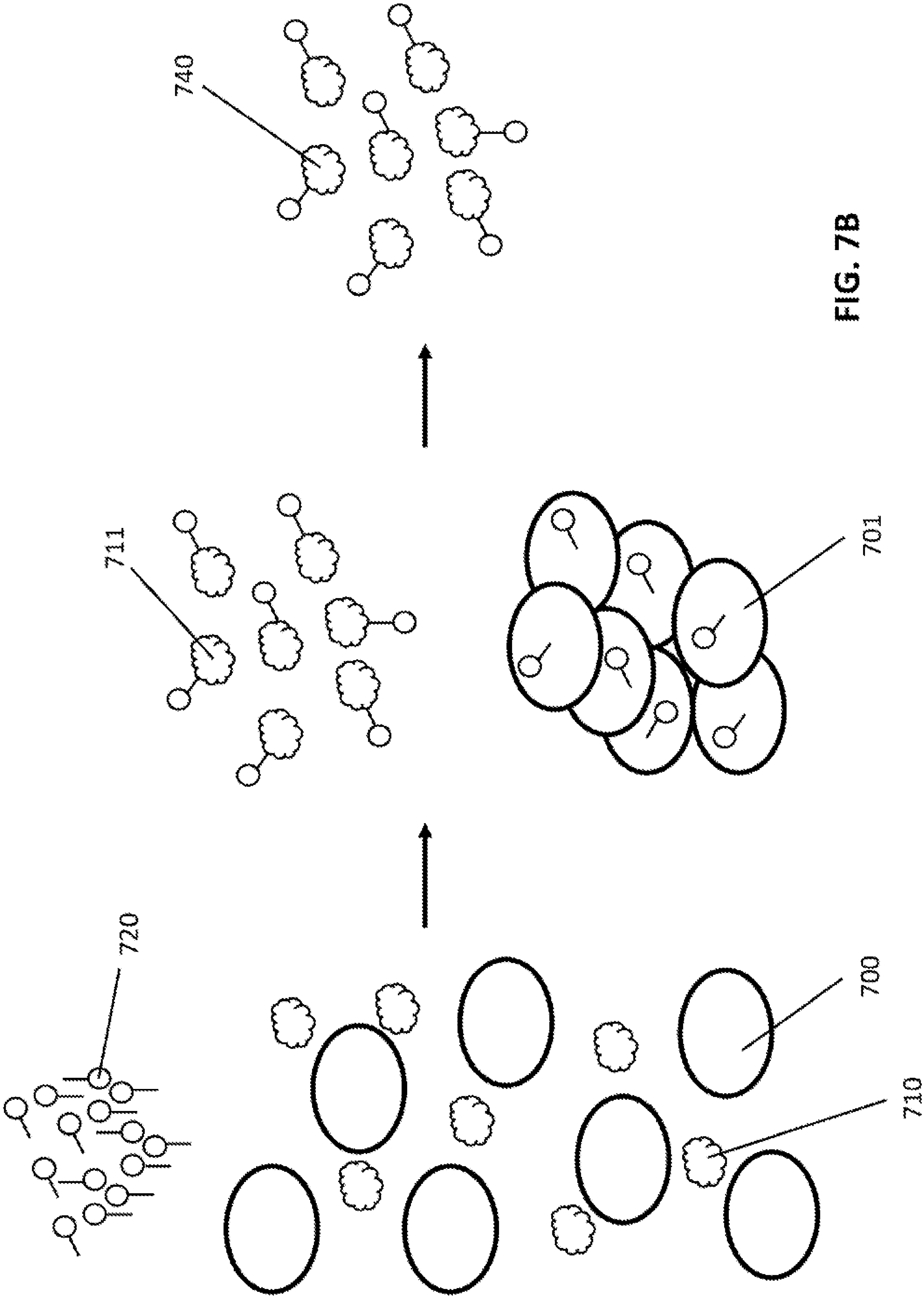


FIG. 7B

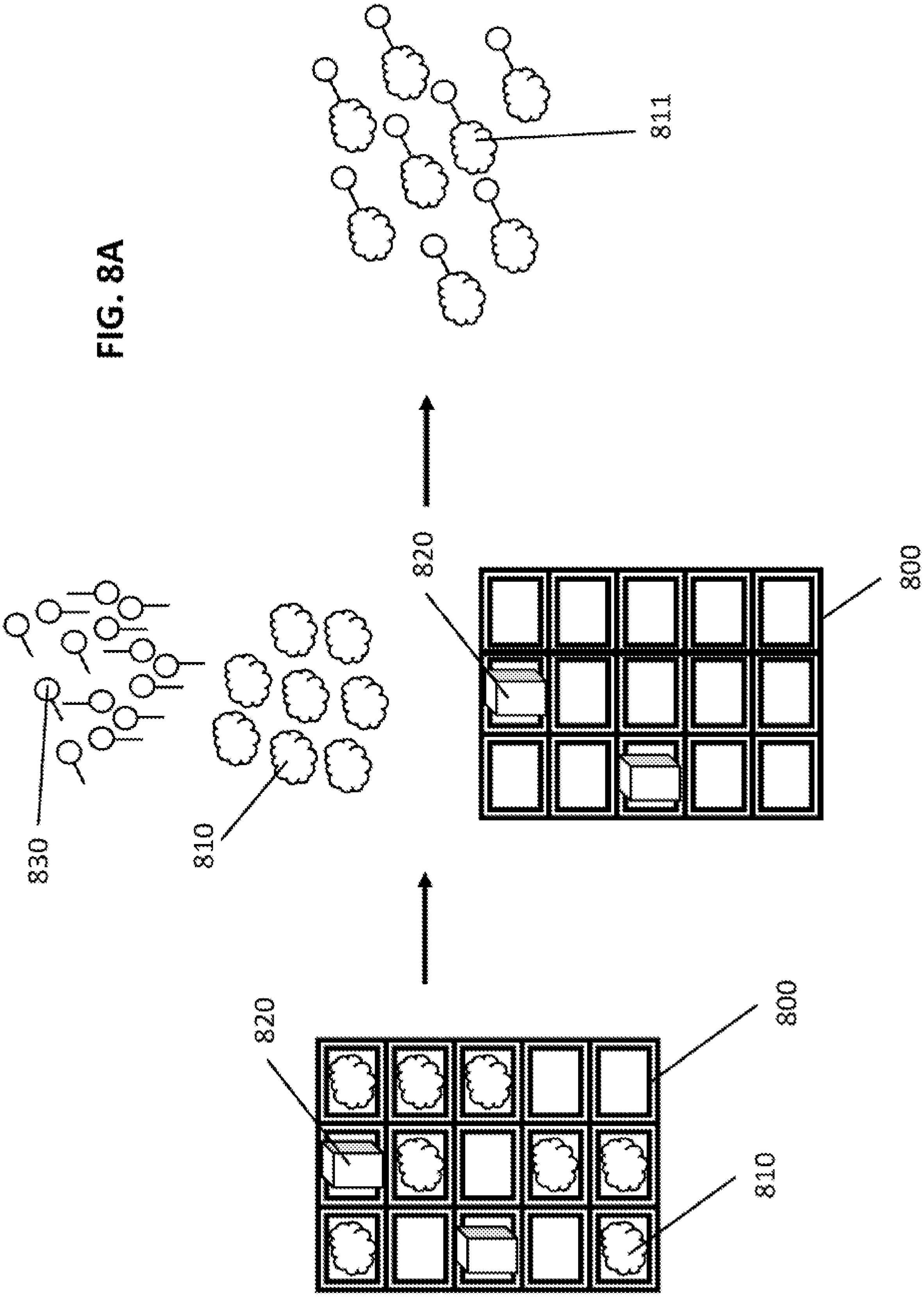


FIG. 8A

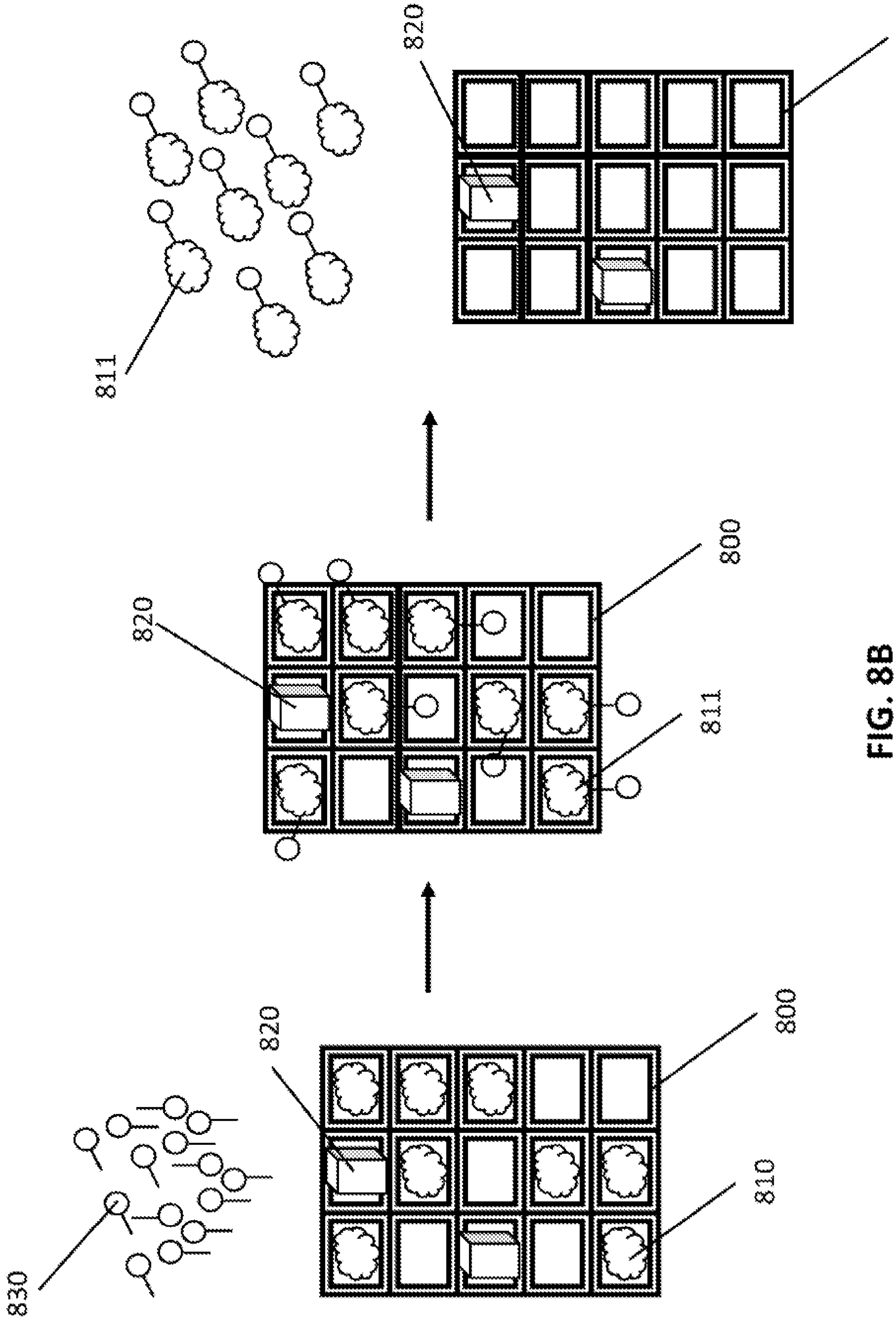


FIG. 8B

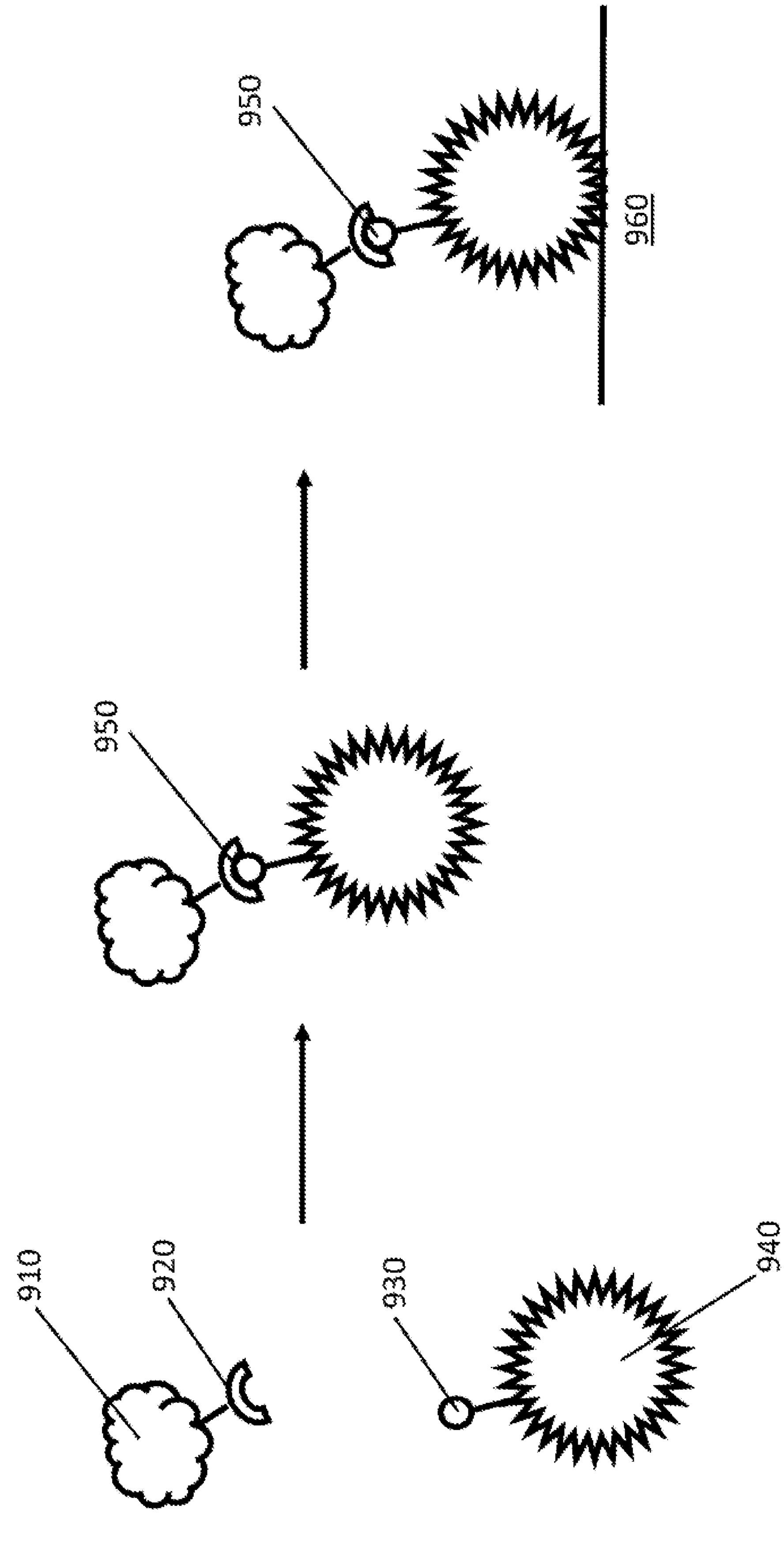


FIG. 9A

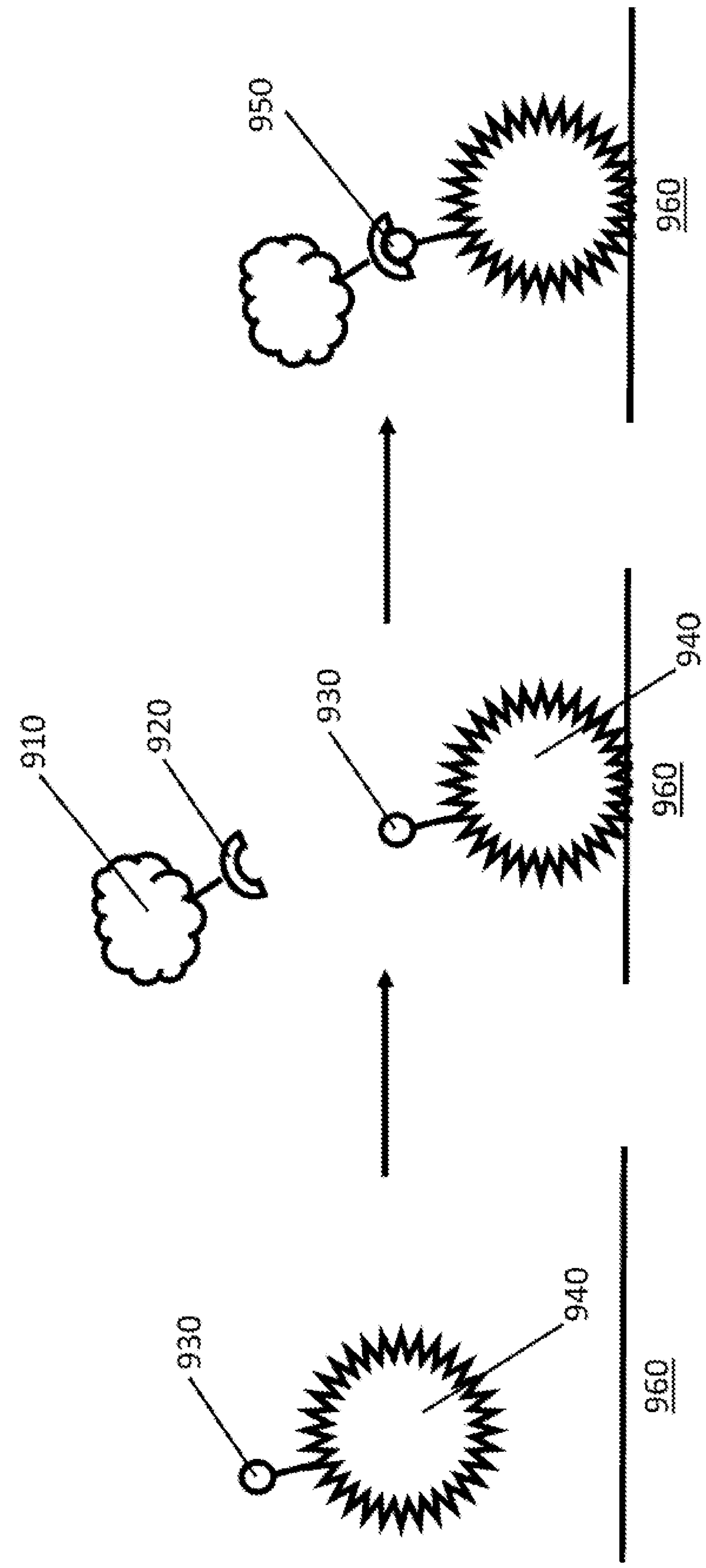


FIG. 9B

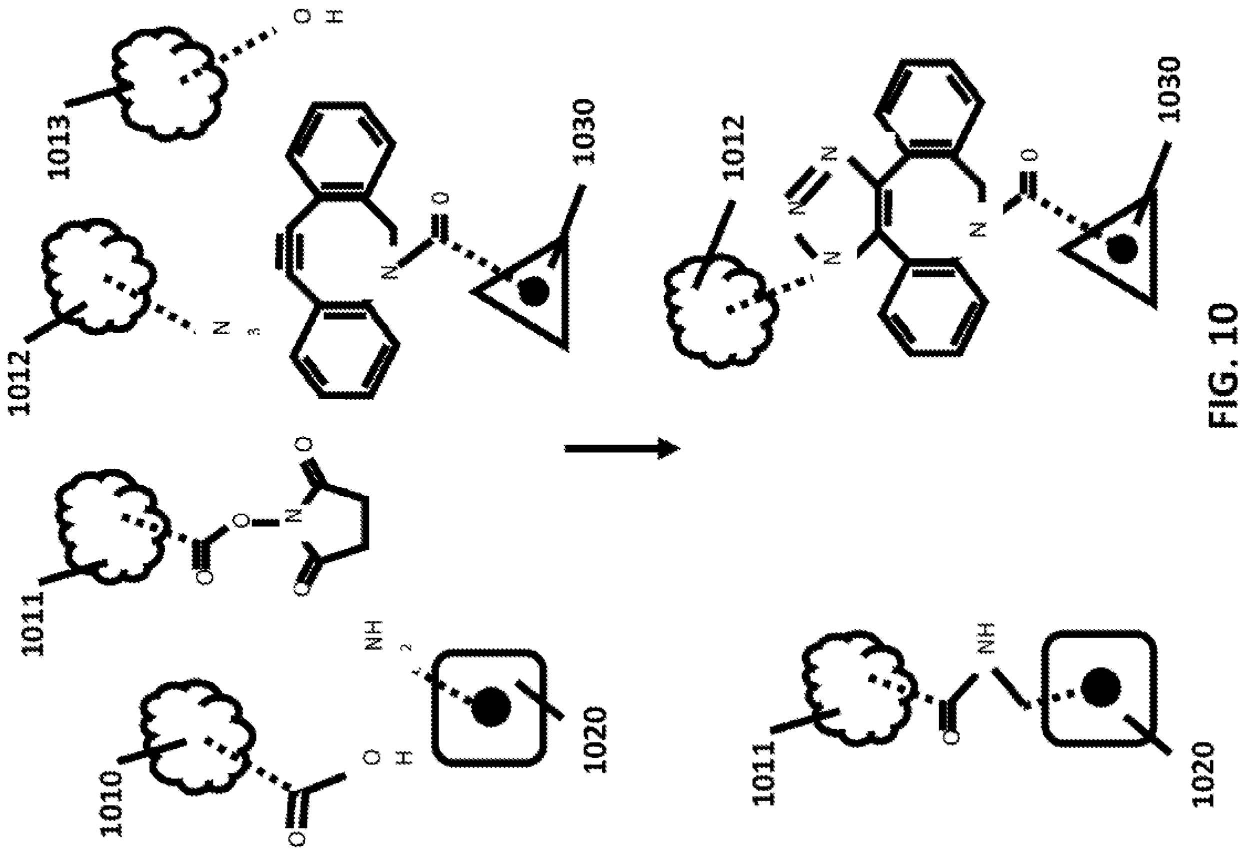

FIG. 10

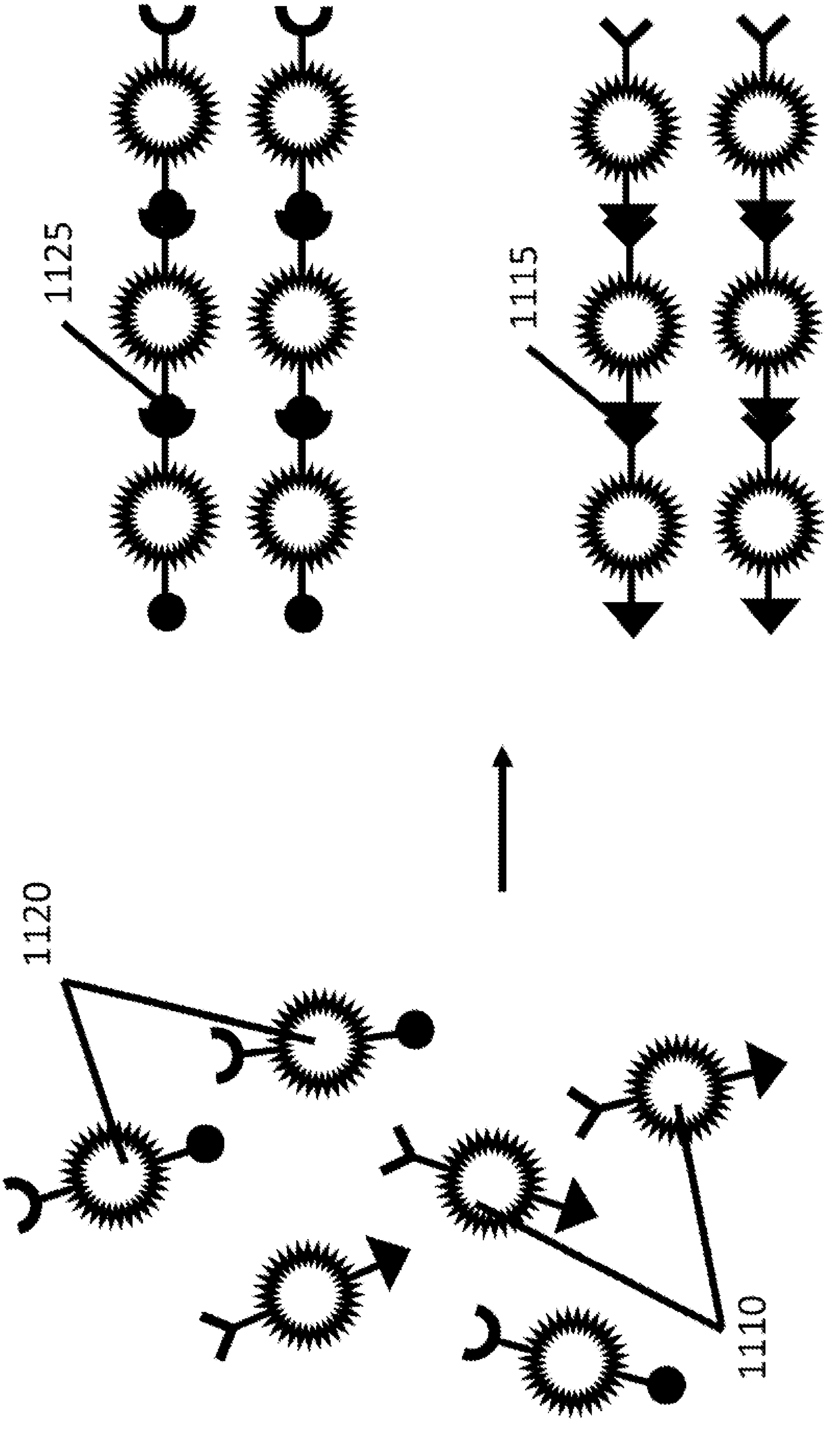


FIG. 11A

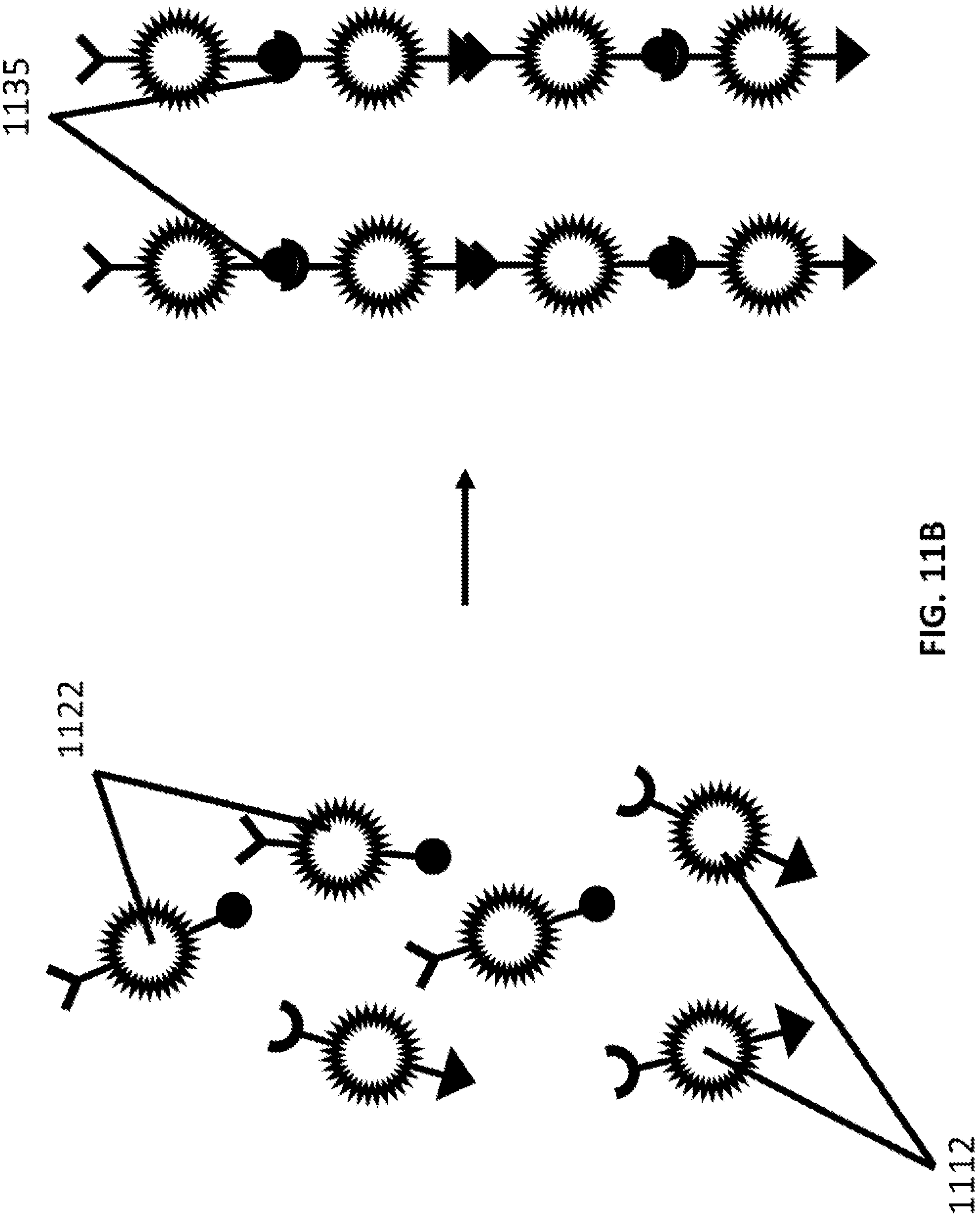


FIG. 11B

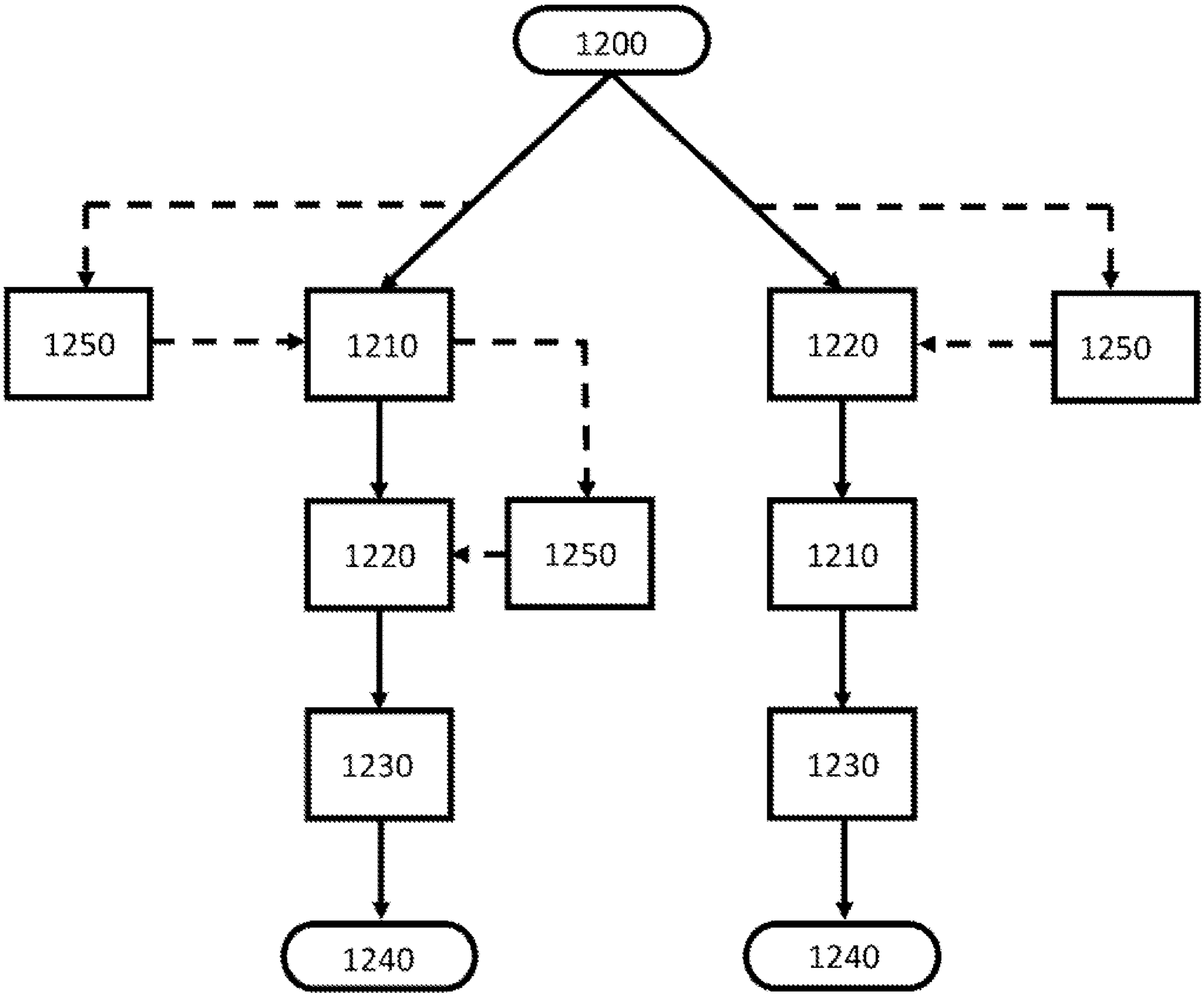


FIG. 12

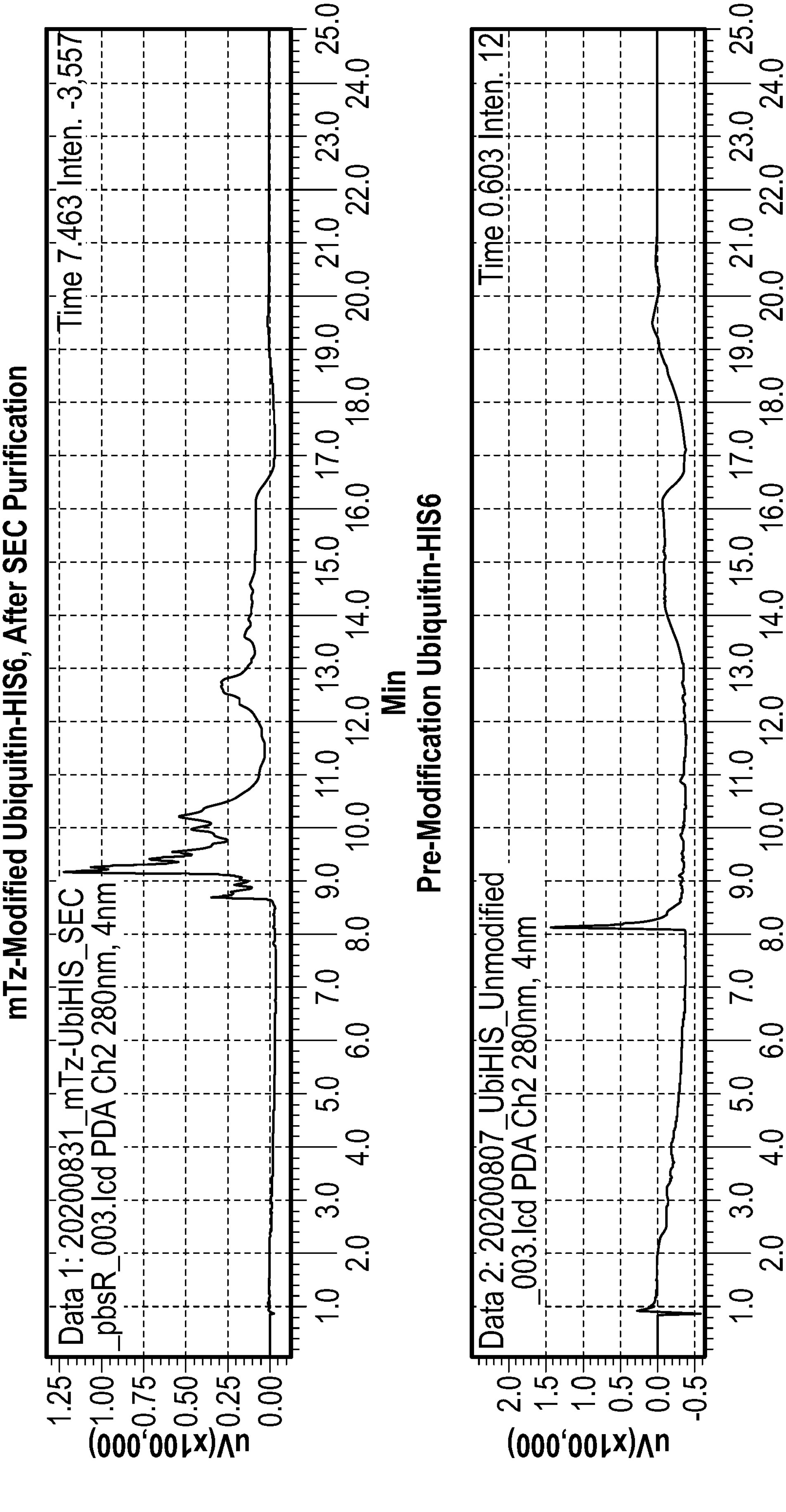
mTz-Modified Ubiquitin-HIS6, After SEC Purification
Data 1: 20200831_mTz-UbiHIS_SEC
_pbsR_003.lcd PDA Ch2 280nm, 4nm
Time 7.463 Inten. -3,557
uV(x100,000)
1.25
1.00
0.75
0.50
0.25
0.00
1.0
2.0
3.0
4.0
5.0
6.0
7.0
8.0
9.0
10.0
11.0
12.0
13.0
14.0
15.0
16.0
17.0
18.0
19.0
20.0
21.0
22.0
23.0
24.0
25.0
Min
Pre-Modification Ubiquitin-HIS6
Data 2: 20200807_UbiHIS_Unmodified
_003.lcd PDA Ch2 280nm, 4nm
Time 0.603 Inten. 12
uV(x100,000)
2.0
1.5
1.0
0.5
0.0
-0.5
1.0
2.0
3.0
4.0
5.0
6.0
7.0
8.0
9.0
10.0
11.0
12.0
13.0
14.0
15.0
16.0
17.0
18.0
19.0
20.0
21.0
22.0
23.0
24.0
25.0
Min

FIG. 13

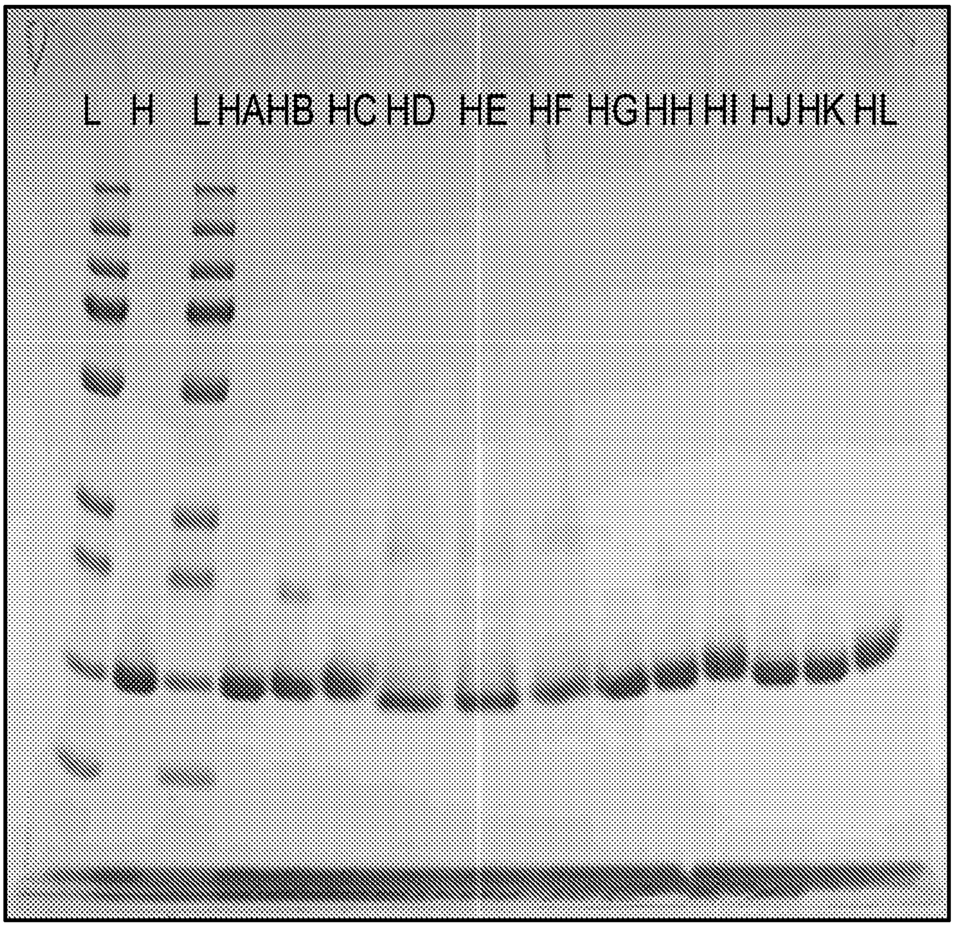


FIG. 14A

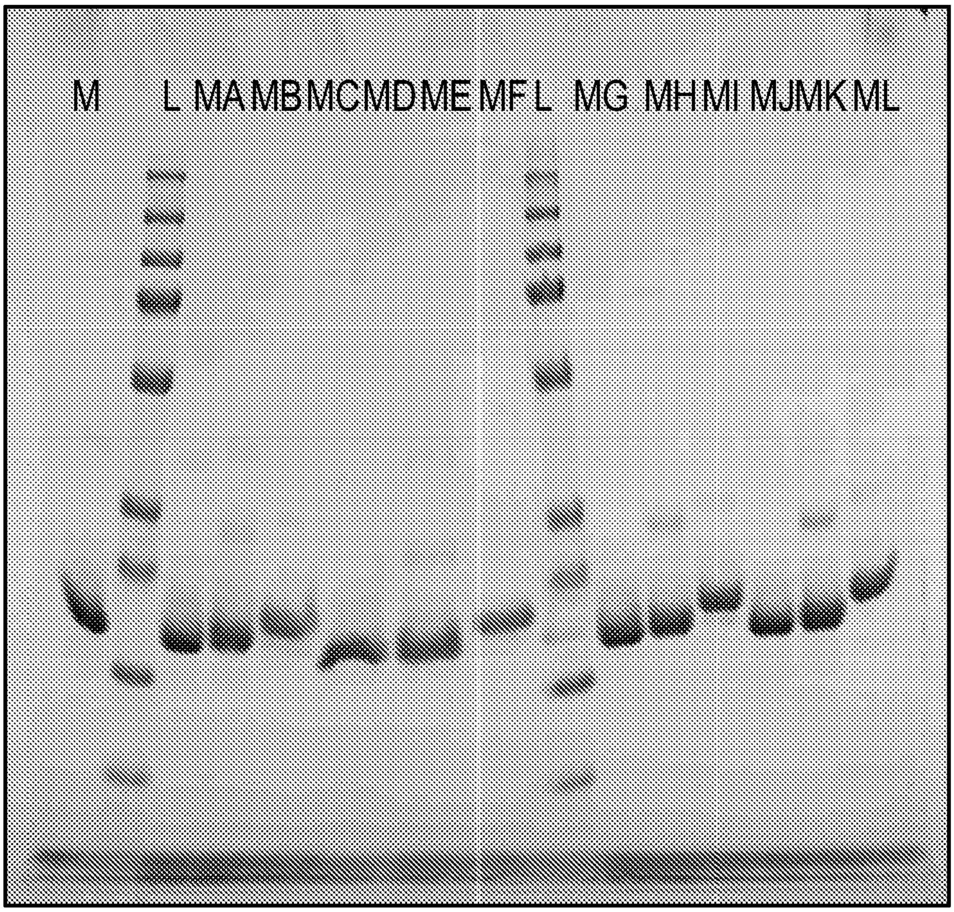


FIG. 14B

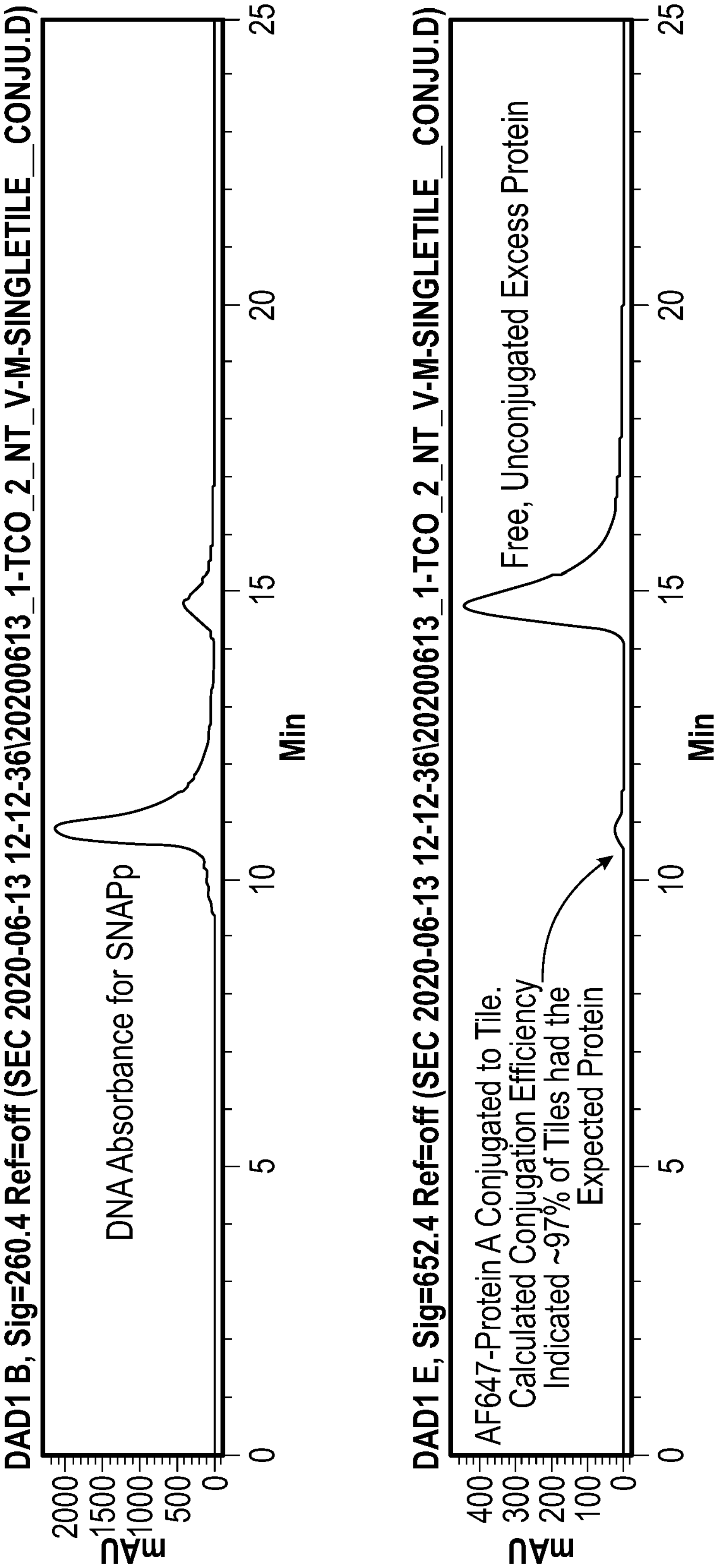
DAD1 B, Sig=260.4 Ref=off (SEC 2020-06-13 12-12-36\20200613_1-TCO_2_NT_V-M-SINGLETILE__CONJU.D)
mAU
2000
1500
1000
500
0
DNA Absorbance for SNAPp
0
5
10
15
20
25
Min
DAD1 E, Sig=652.4 Ref=off (SEC 2020-06-13 12-12-36\20200613_1-TCO_2_NT_V-M-SINGLETILE__CONJU.D)
mAU
400
300
200
100
0
AF647-Protein A Conjugated to Tile. Calculated Conjugation Efficiency Indicated ~97% of Tiles had the Expected Protein
Free, Unconjugated Excess Protein
0
5
10
15
20
25
Min

FIG. 15A

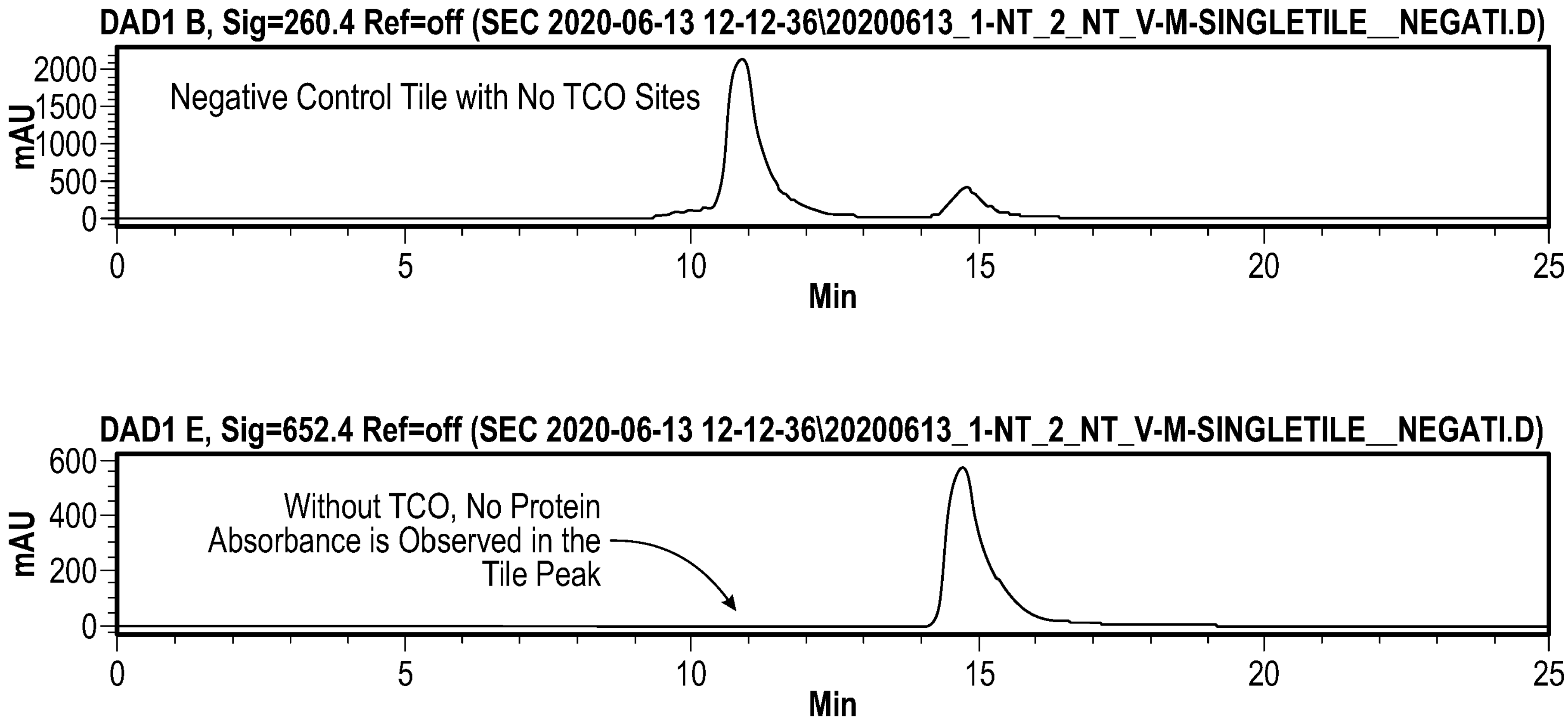
DAD1 B, Sig=260.4 Ref=off (SEC 2020-06-13 12-12-36\20200613_1-NT_2_NT_V-M-SINGLETILE__NEGATI.D)
mAU
2000
1500
1000
500
0
Negative Control Tile with No TCO Sites
0
5
10
15
20
25
Min
DAD1 E, Sig=652.4 Ref=off (SEC 2020-06-13 12-12-36\20200613_1-NT_2_NT_V-M-SINGLETILE__NEGATI.D)
mAU
600
400
200
0
Without TCO, No Protein
Absorbance is Observed in the
Tile Peak
0
5
10
15
20
25
Min

FIG. 15B

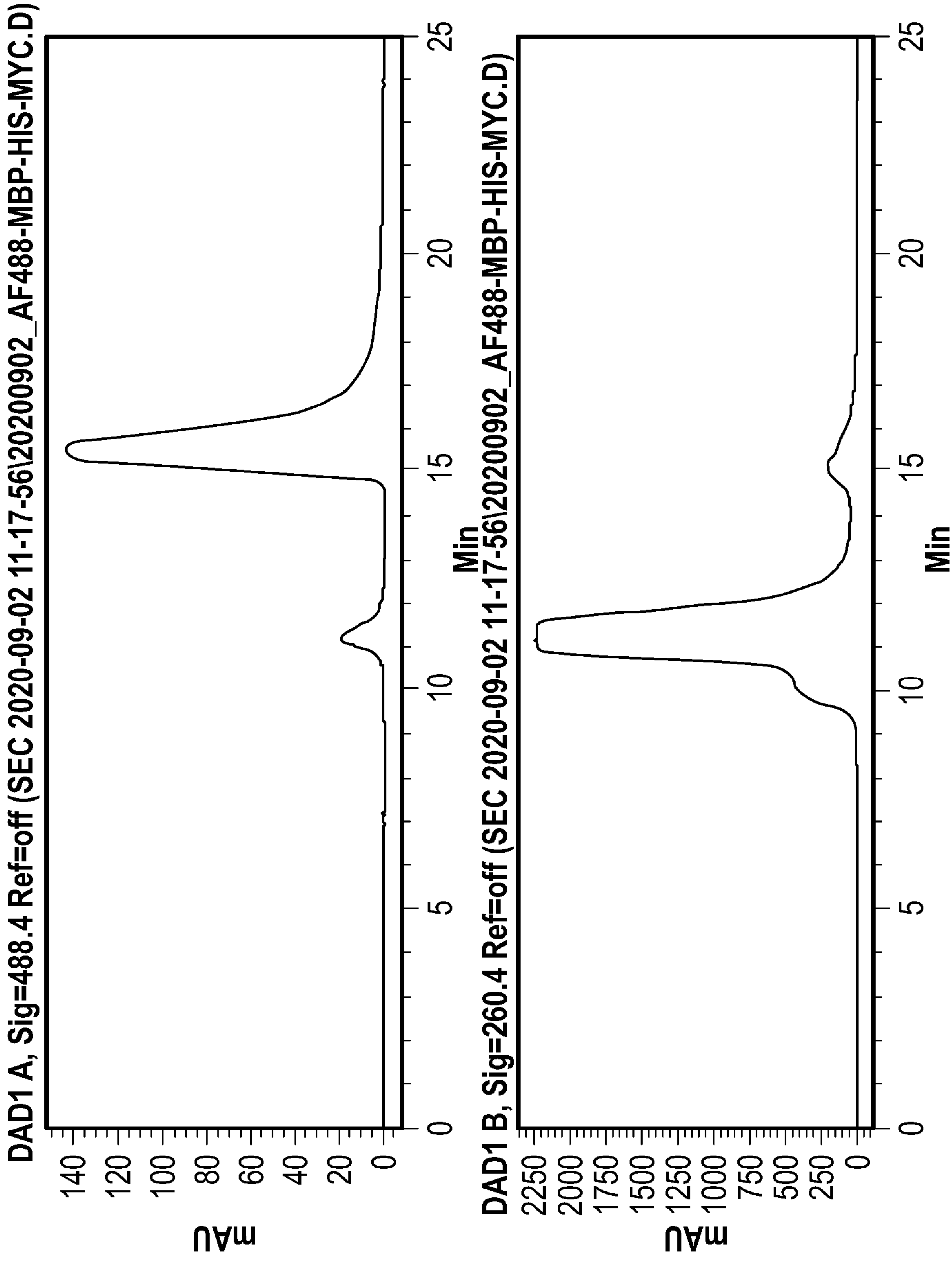
DAD1 A, Sig=488.4 Ref=off (SEC 2020-09-02 11-17-56\20200902_AF488-MBP-HIS-MYC.D)
mAU
140
120
100
80
60
40
20
0
0
5
10
15
20
25
Min
DAD1 B, Sig=260.4 Ref=off (SEC 2020-09-02 11-17-56\20200902_AF488-MBP-HIS-MYC.D)
mAU
2250
2000
1750
1500
1250
1000
750
500
250
0
0
5
10
15
20
25
Min

FIG. 15C

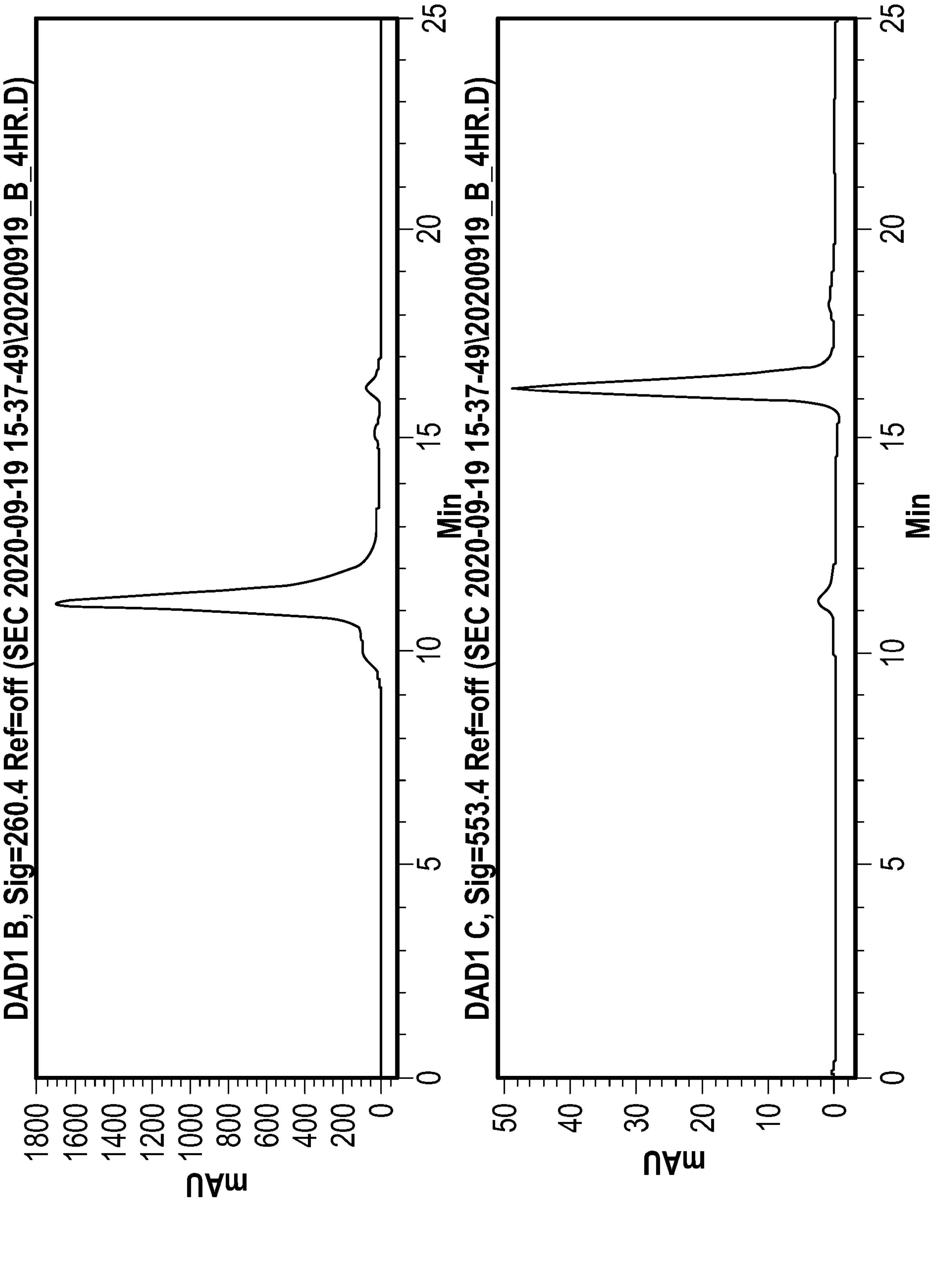
DAD1 B, Sig=260.4 Ref=off (SEC 2020-09-19 15-37-49\20200919_B_4HR.D)
mAU
1800
1600
1400
1200
1000
800
600
400
200
0
0
5
10
15
20
25
Min
DAD1 C, Sig=553.4 Ref=off (SEC 2020-09-19 15-37-49\20200919_B_4HR.D)
mAU
50
40
30
20
10
0
0
5
10
15
20
25
Min

FIG. 15D

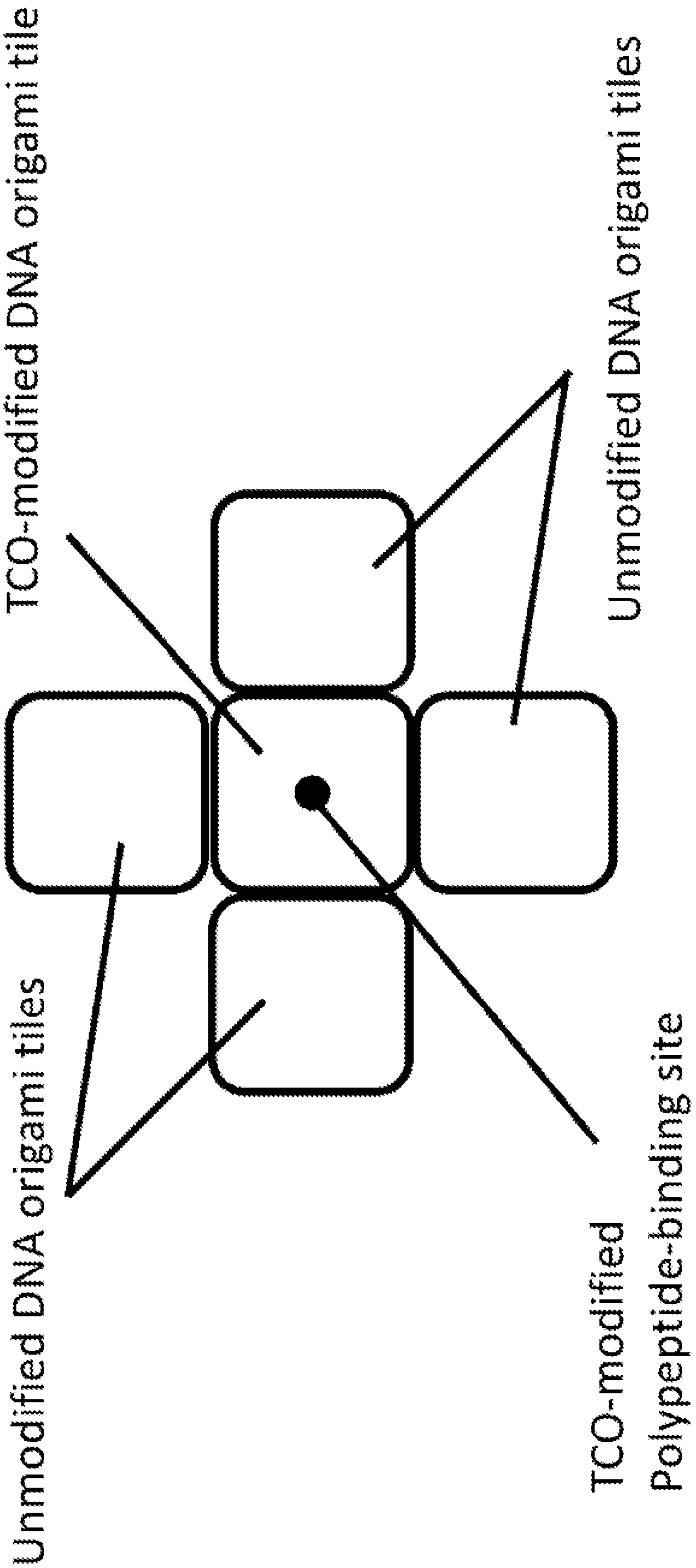
TCO-modified DNA origami tile
Unmodified DNA origami tiles
Unmodified DNA origami tiles
TCO-modified
Polypeptide-binding site

FIG. 16

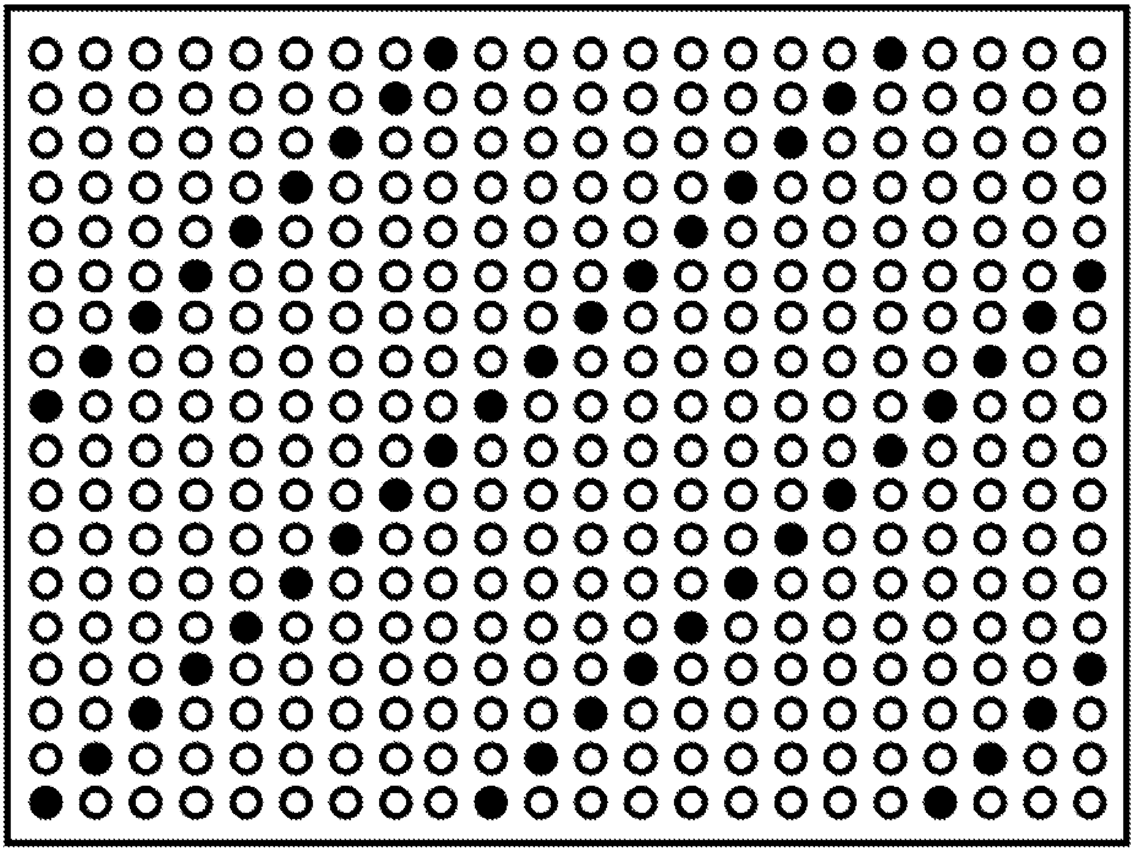

FIG. 17B

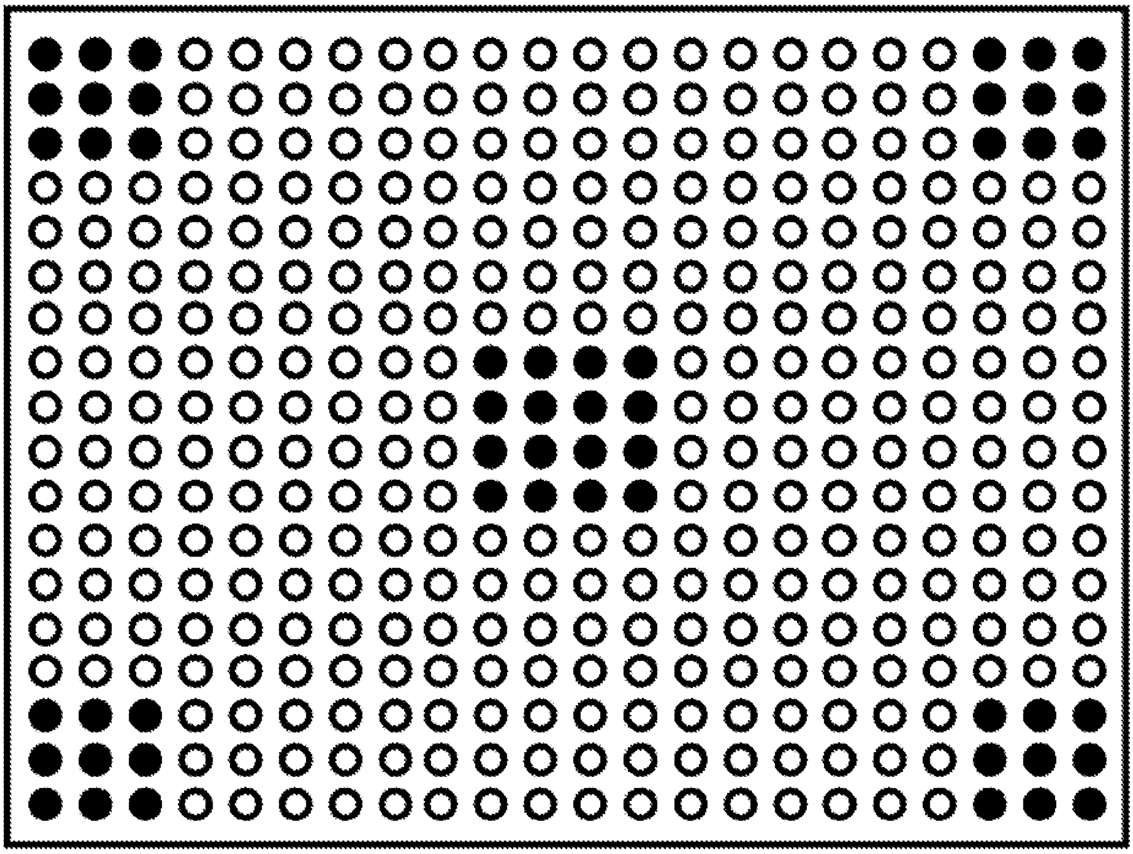

FIG. 17A

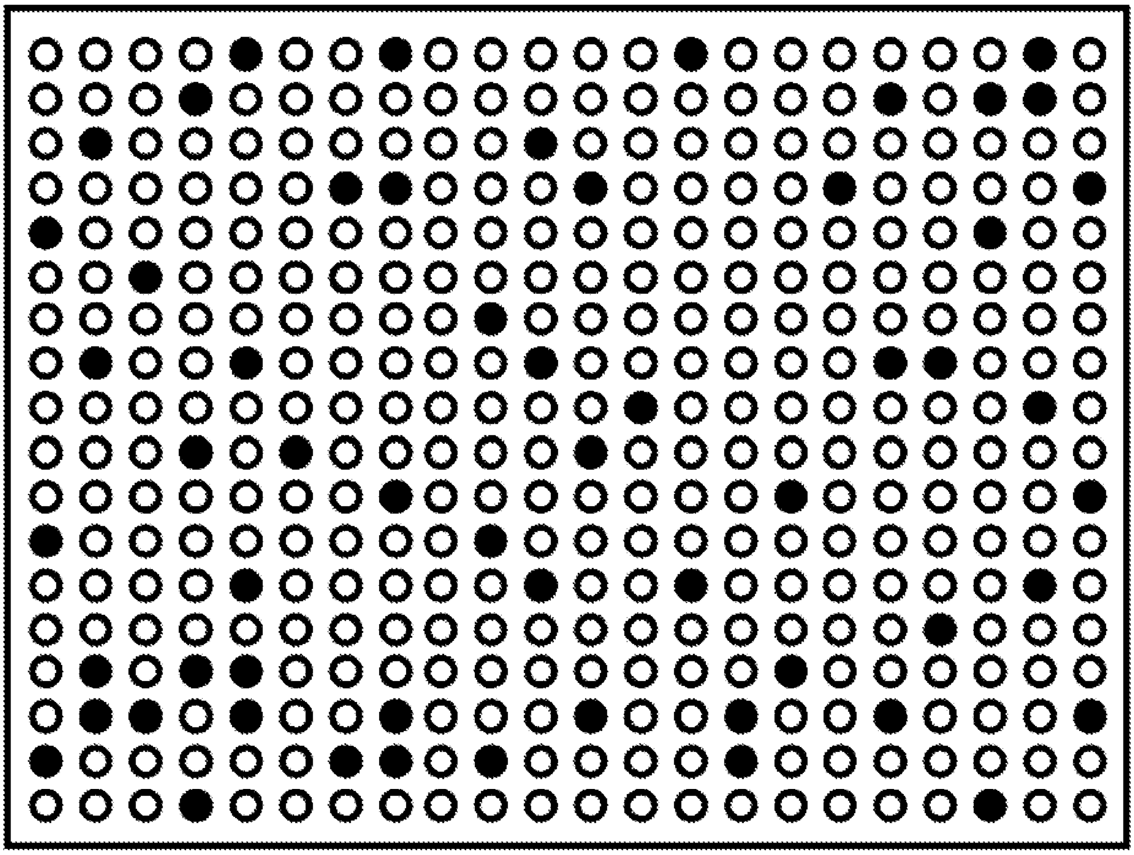

FIG. 17D

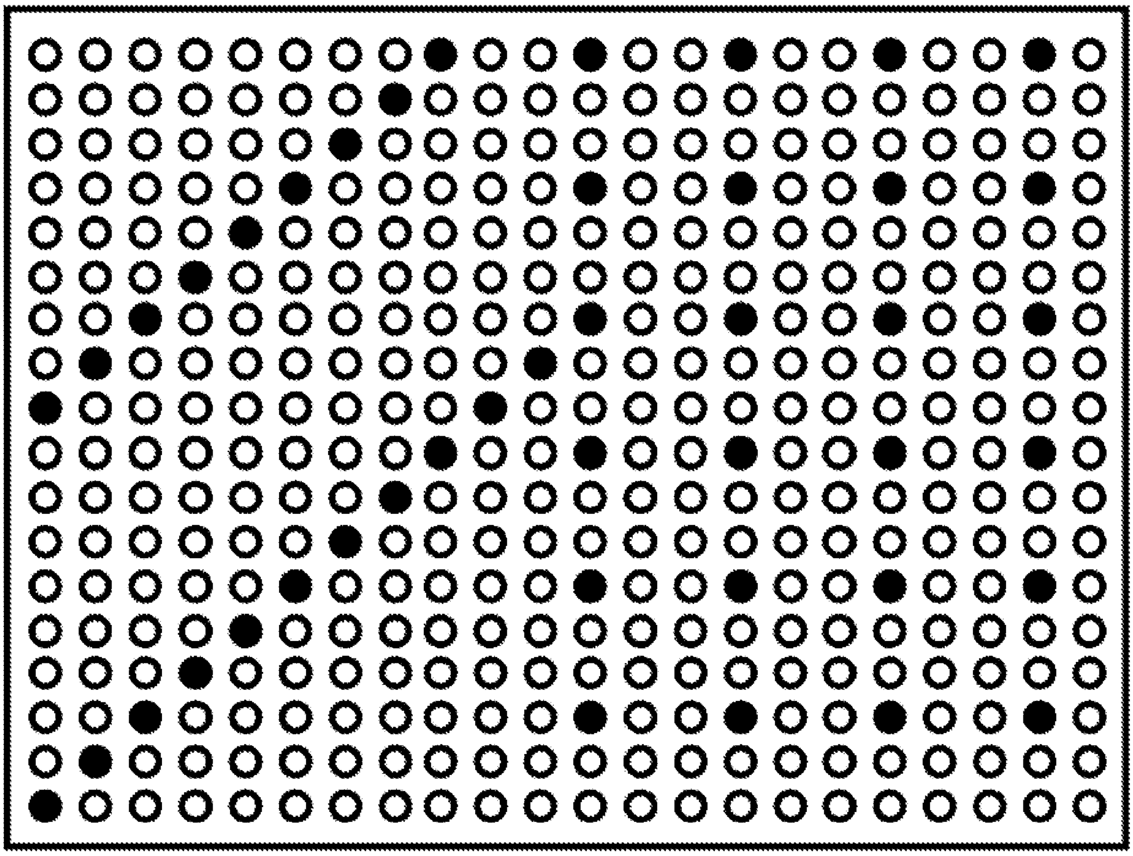

FIG. 17C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63139818
- WO 2017125442 A1
- WO 2019195633 A1
- US 10866242 B
- US 10272050 B
- US 10022334 B
- US 20190350870 A1
- US 20200138728 A1
- US 20200206145 A1
- US 6737236 B
- US 7427678 B
- US 7375234 B
- US 7763736 B
- US 7259258 B
- WO 2005065814 A
- US 10473654 B
- US 20200318101 A1
- US 20200286584 A1
- US 9678068 B
- US 9395359 B
- US 8415171 B
- US 8236574 B
- US 8222047 B
- US 7855054 B
- US 7964356 B
- US 8404830 B
- US 8945830 B
- US 8975026 B
- US 8975388 B
- US 9163056 B
- US 9938314 B
- US 9404919 B
- US 9926566 B
- US 10221421 B
- US 10239908 B
- US 10316321 B
- US 10221207 B
- US 10392621 B
- US 9625469 B
- US 10545153 B
- US 20190145982 A1
- US 20200348308 A1
- US 20200348307 A1
- US 20210101930 A1
- US 7306904 B
- US 7351528 B
- US 8013134 B
- US 8268554 B
- US 9777315 B
- US 20100111768 A1
- US 7329860 B
- US 8951781 B
- US 9193996 B
- US 5888737 A
- US 6175002 B
- US 5695934 A
- US 6140489 A
- US 5863722 A
- US 2007007991 A1
- US 20090247414 A1
- WO 2007123744 A
- US 9678012 B
- US 9921157 B
- US 10605730 B
- US 10712274 B
- US 10775305 B
- US 10895534 B
- US 9606058 B
- US 10845308 B
- US 7181122 B
- US 7302146 B
- US 7313308 B
- US 7122482 B
- US 8765359 B
- US 20130116153 A1
- US 8798414 B
- US 9347829 B
- US 8148264 B
- US 9410887 B
- US 9987609 B
- WO 2020176793 A1
- US 20090026082 A1
- US 20090127589 A1
- US 20100137143 A1
- US 20100282617 A1
- US 20170240962 A1
- US 20180051316 A1
- US 20180112265 A1
- US 20180155773 A1
- US 20180305727 A1
- US 9164053 B
- US 9829456 B
- US 10036064 B
- US 062405
- US 63159500

**Non-patent literature cited in the description**


- **MEANS** ; **FEENEY**. *Bioconjugate Chem.*, 1990, vol. 1, 2-12
- **MATTHEWS et al.** *Meth. Enz.*, 1991, vol. 208, 468-496
- **GLAZER**. *The Proteins*, 1976, vol. 2
- **WONG**. Chemistry of Protein Conjugation and Cross-Linking. CRC Pres, 1991
- Protein Purification: Principles and Practice. Springer Advanced Texts in Chemistry, 1993
- **SAMBROOK et al.** Molecular Cloning: A Laboratory Manual. Cold Spring Harbor Laboratory, 2001
- **AUSUBEL et al.** Current Protocols in Molecular Biology. John Wiley and Sons, 1998
- **BLUME et al.** *Nat. Commun*, 2020, vol. 11, 3662
- **HOWARD et al.** *ACS Chem. Biol.*, 2020, vol. 15, 1401-1407
- **ABBASOV et al.** *Nature Chemistry*, 2021, vol. 13, 1081-1092
- **LUO et al.** *Nat. Comm*, 2019, vol. 10, 142
- **SPICER**. *Chemical Reviews*, 2018, vol. 118, 7702-7743
- **HERMANSON**. Bioconjugate Techniques. Academic Press, 2013
- **EGERTSON et al.** *BioRxiv*, 2021
- **SWAMINATHAN et al.** *Nature Biotech.*, 2018, vol. 36, 1076-1082
- **CHANG, J.Y.** *FEBS LETTS.*, 1978, vol. 91 (1), 63-68
- Deacetylation and internal cleavage of Proteins for N-terminal Sequence Analysis. **GHEORGHE M.T** ; **BERGMAN T.** Methods in Protein Structure Analysis. Springer, 1995
- **WISNIEWSKI et al.** *Molecular & Cellular Proteomics*, 2014, 3497-3506
- **HO et al.** *Cell Systems*, 2018
- **AEBERSOLD et al.** *Nat. Chem. Biol.*, 2018, vol. 14, 206-214
- **ANDERSON** ; **ANDERSON**. *Mol Cell Proteomics*, 2002, vol. 1, 845-67